# EUROPEAN PATENT APPLICATION

(11) **EP 4 137 491 A1**
(43) Date of publication of application: **22.02.2023**
(21) Application number: 21788513.6
(22) Date of filing: 16.04.2021
(51) Int. Cl.: C07D 473/02, A61K 31/522, A61P 43/00, A61P 35/00

(54) **IMIDAZOLINONE DERIVATIVE AND USE THEREOF IN MEDICINE**

(30) Priority: 17.04.2020 CN 202010302603; 15.07.2020 CN 202010679647; 02.04.2021 CN 202110360821
(71) Applicant: Chengdu Baiyu Pharmaceutical Co., Ltd., Chengdu, Sichuan 611130 (CN)
(72) Inventor: WEI, Yonggang, Chengdu, Sichuan 611130 (CN); XU, Xuezhen, Chengdu, Sichuan 611130 (CN); CHU, Hongzhu, Chengdu, Sichuan 611130 (CN); HE, Lvxue, Chengdu, Sichuan 611130 (CN); LEI, Feiquan, Chengdu, Sichuan 611130 (CN); YAN, Jie, Chengdu, Sichuan 611130 (CN); HE, Yang, Chengdu, Sichuan 611130 (CN); WANG, Meiwei, Chengdu, Sichuan 611130 (CN); SU, Guizhuan, Chengdu, Sichuan 611130 (CN); LIU, Bing, Chengdu, Sichuan 611130 (CN); SUN, Yi, Chengdu, Sichuan 611130 (CN)
(74) Representative: Patentanwälte Olbricht Buchhold Keulertz
(86) International application number: PCT/CN2021/087912
(87) International publication number: WO 2021/209055

(57) **Abstract**

The present application relates to imidazolidinone derivatives and medical use thereof, and in particular relates to pyrimidine derivatives represented by general formula (I), or stereoisomers, solvates, metabolites, prodrugs, pharmaceutically acceptable salts or co-crystals thereof, a pharmaceutical composition comprising the same, and use of the compound or pharmaceutical composition according to the present application in the manufacture of a medicament for treatment of cancer.

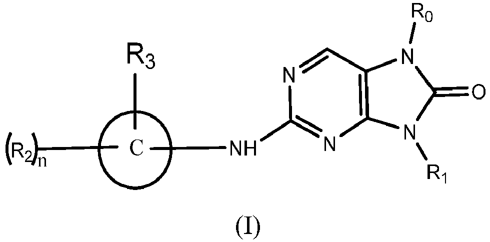

## Description

### Technical Field

The present invention relates to imidazolidinone derivatives represented by general formula (I), or stereoisomers, solvates, prodrugs, metabolites, pharmaceutically acceptable salts or co-crystals thereof, a pharmaceutical composition comprising the same, and use thereof as a DNA-PK inhibitor.

### Background Art

The DNA-dependent protein kinase (DNAPK) is a DNA-PK enzyme complex composed of a Ku70/Ku80 heterodimer and a DNA-dependent protein kinase catalytic subunit (DNA-PKcs). This enzyme complex requires the presence of DNA to be activated and perform the corresponding functions (George et al., 2019). As a serine/threonine protein kinase, DNA-PK is a member of the PIKK (phosphatidylinositol 3-kinase-related kinase) family, which not only plays an important role in repairing intracellular DNA double-strand breaks (DSBs) and cellular DNA recombination or antibody DNA rearrangement (V(D)J recombination), but also participates in chromosomal modification, transcription regulation, telomere maintenance, and other physiological processes.

In normal physiological processes, a variety of factors may cause DSBs in DNA. For example, DSBs often appear as intermediate products in the process of DNA recombination in somatic cells, which is a physiological process very important for the establishment of a functional immune system in all vertebrates. Furthermore, single- or double-strand breaks may be caused by damaged bases found at replication forks during DNA replication. DNA may also generate DSBs due to the attack by reactive oxygen species (ROS) during normal metabolism (Cannan & Pederson, 2016). In addition, various exogenous factors may also lead to DSBs, such as ionizing radiation (IR) and chemotherapeutic agents (such as topoisomerase II inhibitors) (George et al., 2019). If DSBs are not repaired or are incorrectly repaired, mutations and/or chromosomal aberrations will occur, eventually leading to cell death. In order to cope with the harm inflicted by DSBs, eukaryotic cells have evolved multiple mechanisms to repair damaged DNA to maintain cell viability and genome stability. In eukaryotic cells, the most important manner of DNA repair is non-homologous end-joining (NHEJ), which directly rejoins broken DNAs, does not require involvement of homologous DNA fragments, and can occur at any stage of the cell cycle. NHEJ is a dynamic process mediated by DNA-PK that requires participation of multiple proteins and signaling pathways, which basically comprises the following procedures: (1) the Ku70/Ku80 heterodimer recognizes and binds to the broken ends of double-stranded DNAs; (2) proteins such as DNA-PKcs and XRCC4-DNA ligase IV complex are recruited to both sides of the broken DNA double strands; (3) DNA-PKcs is autophosphorylated to activate its own kinase activity; (4) DNA-PKcs acts as an adhesive to join both broken ends of the DNA to prevent DNA degradation by exonuclease; (5) the DNA is processed to remove non-ligatible ends or other forms of damage at the breaks; and (6) the XRCC4-DNA ligase IV complex repairs the DNA ends (in some cases, DNA polymerase may be required to synthesize new ends prior to ligation). When DNA-PKcs is phosphorylated, it can induce conformational changes of proteins and regulate the activities of various proteins (such as Artemis, Ku70, Ku80, the DNA ligase) during NHEJ, which is crucial to the DNA repair process. Therefore, phosphorylated DNA-PKcs (pDNA-PKcs) is often used as a marker of cellular DSBs.

Studies have shown that DNA-PK activity is associated with occurrence and development of various tumors. For example, in melanoma, DNA-PKcs can promote angiogenesis and tumor metastasis; in multiple myeloma, DNA-PKcs expression is significantly up-regulated; and in radiotherapy-resistant thyroid tumors, the content of Ku protein is significantly increased (Ihara, Ashizawa, Shichijo, & Kudo, 2019). Therefore, the combination of a DNA-PK inhibitor with an anti-tumor therapy that causes DNA damage (such as IR, chemotherapeutic agents, etc.) may be contemplated to improve the efficacy. The use of DNA-PK inhibitors may interfere with the DNA repair function of normal cells to a certain extent. However, there are multiple complementary DNA repair pathways in normal cells, while tumor cells face strong DNA replication pressure and lack effective DNA repair means. Inhibition of the activity of DNA-PK in tumor cells can enhance the killing effect of other anti-tumor drugs against the tumor cells. For example, NU7441 can significantly enhance the efficacy of chemotherapy drugs that cause DNA damage against breast cancer and non-small cell lung cancer, and can also improve the sensitivity of colorectal cancer to IR and chemotherapy.

Through years of research, a number of DNA-PK inhibitors have been discovered. The first compound discovered with DNA-PK kinase inhibitory activity was a fungal metabolite, Wortmannin, with an IC50 (DNA-PK) of about 15 nM, which also plays an important role in the acetylation and phosphorylation of the p53 protein (Sarkaria et al., 1998). A quercetin derivative LY294002 reported later also has DNA-PK inhibitory activity (Maira, Stauffer, Schnell, & Garcia-Echeverria, 2009). Afterwards a new generation of DNA-PK inhibitors such as NU7026 and NU7441 were developed based on the structure of LY294002. Although it has been confirmed that these compounds show a good killing effect on tumor cells, they have problems such as high toxicity and poor selectivity, and thus cannot enter clinical trials (Maira et al., 2009). Other DNA-PK inhibitors have also been reported, such as small molecule compounds including OK1035, SU11752, PP121, and KU-0060648, but these compounds also have disadvantages such as low specificity for DNA-PK (George et al., 2019). Therefore, there is still a need to develop DNA-PK inhibitors with high activity, high specificity, and low toxicity that better meet the clinical demands.

### Summary of Invention

One or more embodiments of the present disclosure provide novel imidazolidinone derivatives, or stereoisomers, solvates, metabolites, pharmaceutically acceptable salts, co-crystals, or prodrugs thereof, a pharmaceutical composition comprising the same, and their use as a DNA-PK inhibitor.

The compounds according to one or more embodiments of the present application have high inhibitory activity against and high selectivity for DNA-PK, and can be used as a sensitizer for chemotherapy and radiotherapy to prevent and/or treat cancer, improve the therapeutic efficacy, and reduce toxic and side effects.

One or more embodiments according to the present disclosure provide a compound of general formula (I), or a stereoisomer, solvate, prodrug, metabolite, pharmaceutically acceptable salt, or co-crystal thereof, 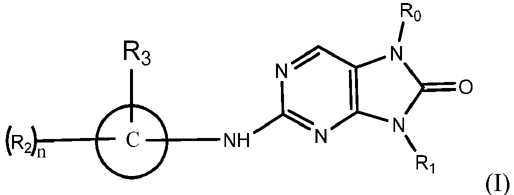 wherein 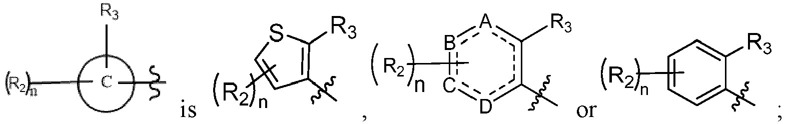
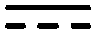 is a single bond or a double bond;
A, B, C, and D in the 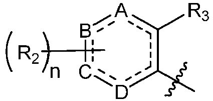 are each independently C or N, and at least one of A, B, C, and D is N;
R₀ is H, C₁₋₆ alkyl, or cyclopropyl, wherein the C₁₋₆ alkyl is optionally further substituted with one or more substituents selected from halogen and deuterium;
R₁ is 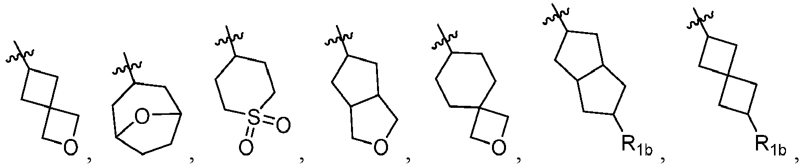 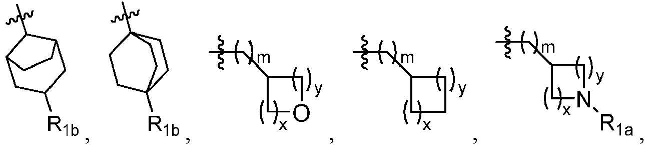 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
R₂ is H, cyano, =O, carboxyl, -C(=O)NR₂ₐR_{2b}, C₁₋₆ alkoxy, C₁₋₆ alkyl, halogen, - S(=O)₂R₂ₐ or -C(=O)OC₁₋₆ alkyl; wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atom to which they are attached form 5- to 6-membered heterocyclyl group containing 1, 2 or 3 heteroatoms selected from N, O and S, wherein the 5- to 6-membered heterocyclyl group is optionally further substituted with one or more substituents selected from C₁₋₆ alkyl, OH, and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2;
x and y are each independently 1, 2, or 3;
provided that
R₁ is not 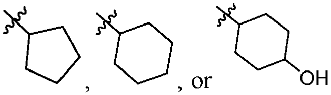 when 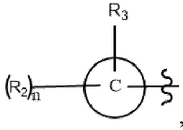 R₀, R₂, and R₃ all satisfy the following condition: 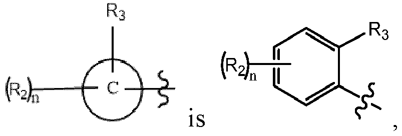
n is 1, R₀ is H or methyl, R₂ is methoxy or - S(=O)₂Me, and R₃ is methyl.

In one or more embodiments according to the present disclosure, R₁ is 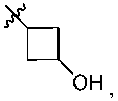 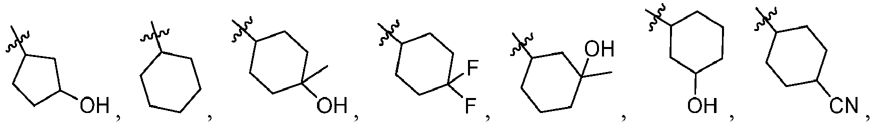 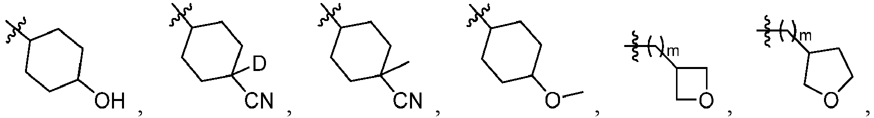 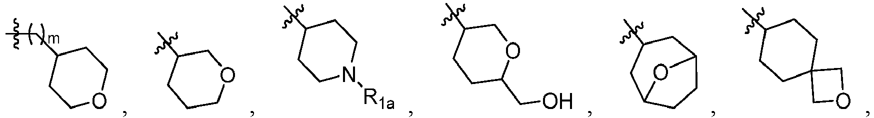 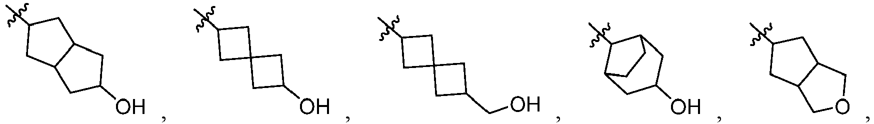 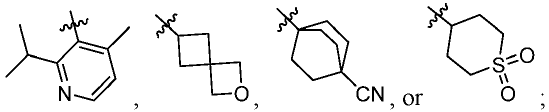
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl; wherein the -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, and halogen;
alternatively, R₂ₐ and R_{2b} together with the atom to which they are attached form 5- to 6-membered heterocyclyl group containing 1 to 3 heteroatoms selected from N, O and S, wherein the heterocyclyl group is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2;
provided that
R₁ is not 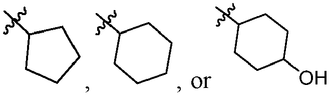 when 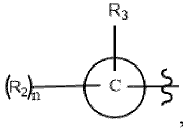 R₀, R₂, and R₃ all satisfy the following condition: 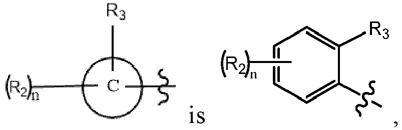 n is 1, R₀ is H or methyl, R₂ is methoxy or - S(=O)₂Me, and R₃ is methyl.

In one or more embodiments according to the present disclosure, 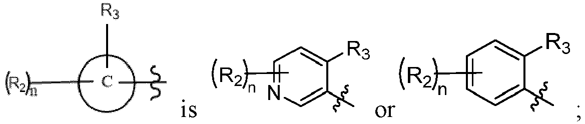
R₀ is H, C₁₋₄ alkyl, or cyclopropyl, wherein the C₁₋₄ alkyl is optionally further substituted with one or more substituents selected from halogen and deuterium;
R₁ is 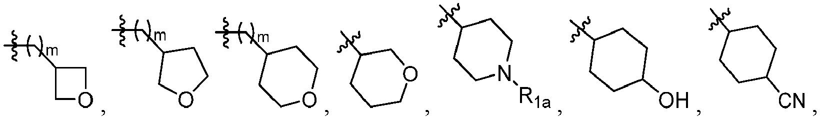 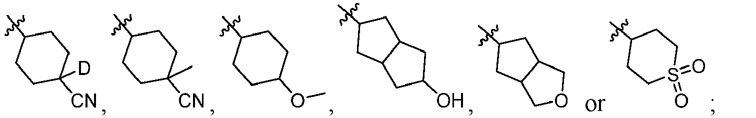
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl; wherein the -C(=O)O alkyl or C₁₋₆ alkoxy is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, and halogen;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2;
provided that
R₁ is not 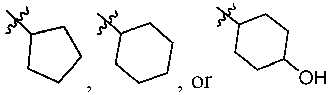 when 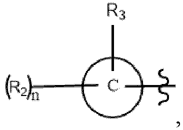 R₀, R₂, and R₃ all satisfy the following condition: 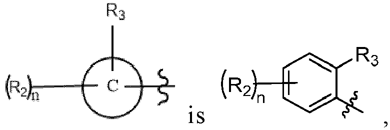 n is 1, R₀ is H or methyl, R₂ is methoxy or - S(=O)₂Me, and R₃ is methyl.

In one or more embodiments according to the present disclosure, the compound according to the present disclosure is a compound of general formula (II): 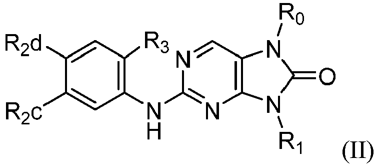 wherein
R₀ is H, C₁₋₆ alkyl, or cyclopropyl, wherein the C₁₋₆ alkyl is optionally further substituted with one or more substituents selected from halogen and deuterium;
R₁ is 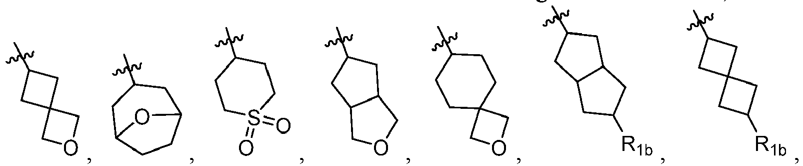 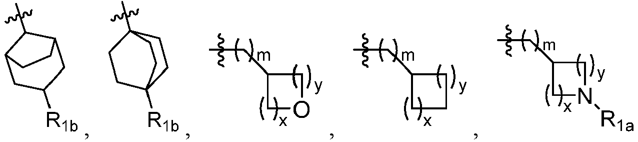 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
R_{2c} is H, cyano, halogen, or C₁₋₆ alkoxy;
R_{2d} is H, cyano, carboxyl, -C(=O)NR₂ₐR_{2b}, C₁₋₆ alkyl, halogen, -S(=O)₂R₂ₐ or - C(=O)OC₁₋₆ alkyl; wherein the C₁₋₆ alkyl or -C(=O)OC₁₋₆ alkyl is optionally substituted with one or more substituents selected from halogen and deuterium;
R₂ₐ and R_{2b} are H, C₁₋₆ alkyl or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from C₁₋₆ alkyl, OH, and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2;
x and y are each independently 1, 2, or 3;
provided that
R₁ is not 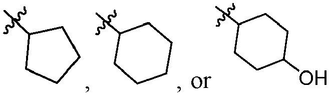 when 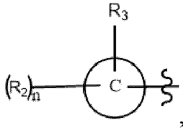 R₀, R₂, and R₃ all satisfy the following condition: 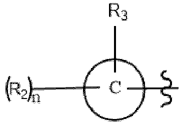 is selected from 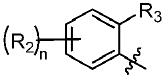 , n is 1, R₀ is H or methyl, R₂ is methoxy or -S(=O)₂Me, and R₃ is methyl.

In one or more embodiments according to the present disclosure, the compound according to the present disclosure is a compound of general formula (III): 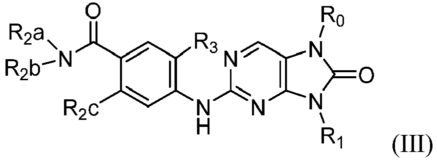 wherein
R₀ is H, C₁₋₆ alkyl, or cyclopropyl, wherein the C₁₋₆ alkyl is optionally further substituted with one or more substituents selected from halogen and deuterium;
R₁ is 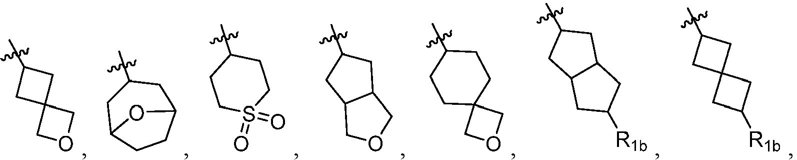 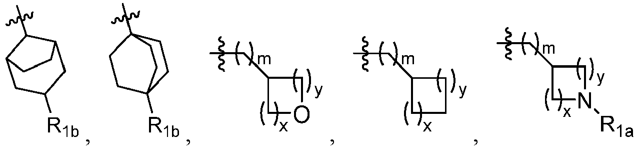 or pyridyl, and R₁ is optionally further substituted with one or two substituents selected from D, halogen, cyano, hydroxy, C₁₋₆ alkyl, and C₁₋₆ alkoxy;
R_{1b} is H, OH, cyano, or hydroxyl-substituted C₁₋₆ alkyl;
R₂ₐ and R_{2b} are each independently H, C₁₋₆ alkyl or 3- to 5-membered cycloalkyl, wherein the C₁₋₆ alkyl is optionally further substituted with one or more substituents selected from D and halogen;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from C₁₋₆ alkyl, OH, and halogen;
R_{2c} is H, cyano, halogen, or C₁₋₆ alkoxy, wherein the C₁₋₆ alkoxy is optionally substituted with one or more D;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2;
x and y are each independently 1, 2, or 3.

One or more embodiments according to the present disclosure provide a compound of general formula (IV), or a stereoisomer, solvate, prodrug, metabolite, pharmaceutically acceptable salt, or co-crystal thereof, 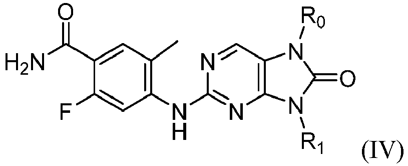 wherein
R₀ is H, C₁₋₆ alkyl, or cyclopropyl, wherein the C₁₋₆ alkyl is optionally further substituted with one or more substituents selected from halogen and deuterium; and
R₁ is a -(CH)ₘ- 4 to 7-membered carbocyclyl, -(CH)ₘ- 4 to 7-membered heterocyclyl, -(CH)ₘ- 8 to 12-membered bridged ring, or -(CH)ₘ- 7 to 12 membered spiro, wherein the -(CH)ₘ- 4 to 7-membered carbocyclyl, -(CH)ₘ- 4 to 7-membered heterocyclyl, - (CH)ₘ- 8 to 12-membered bridged ring, or -(CH)ₘ- 7 to 12 membered spiro is optionally further substituted with one or more substituents selected from hydroxy, cyano, halogen, =O, C₁₋₆ alkyl, C₁₋₆ alkoxy, and hydroxyl-substituted C₁₋₆ alkyl.

In one or more embodiments according to the present disclosure, R₀ is H, C₁₋₆ alkyl, or cyclopropyl, wherein the C₁₋₆ alkyl is optionally further substituted with one or more substituents selected from halogen and deuterium;
R₁ is 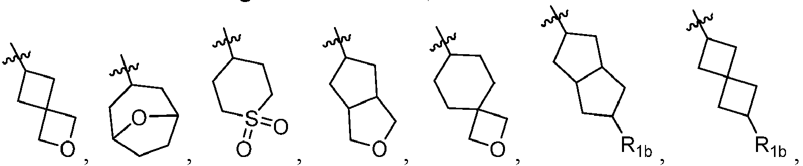 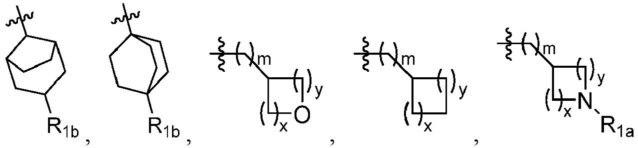 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
m is 0 or 1; and
x and y are each independently 1, 2, or 3.

In one or more embodiments according to the present disclosure, R₀ is C₁₋₄ alkyl, wherein the C₁₋₄ alkyl is optionally further substituted with one or more substituents selected from halogen and deuterium; and
R₁ is

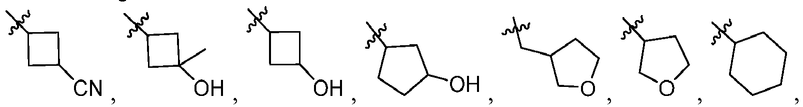

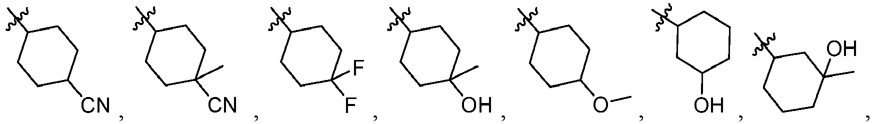

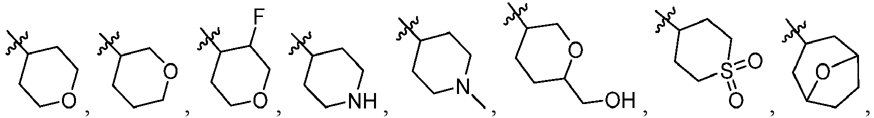

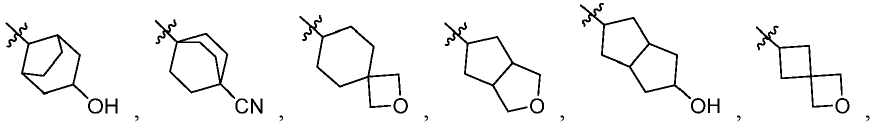

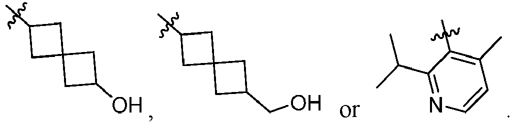

In one or more embodiments according to the present disclosure, the compound according to the present disclosure is

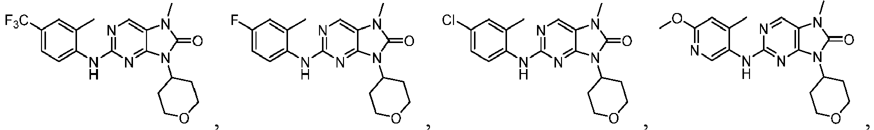

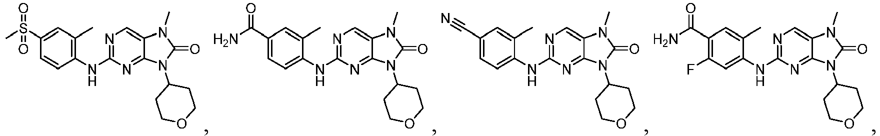

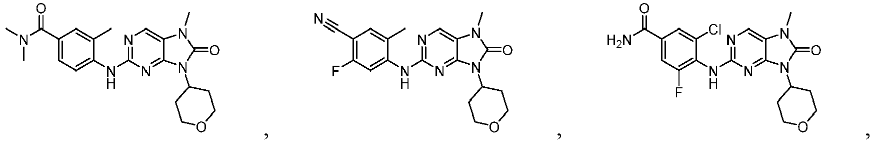

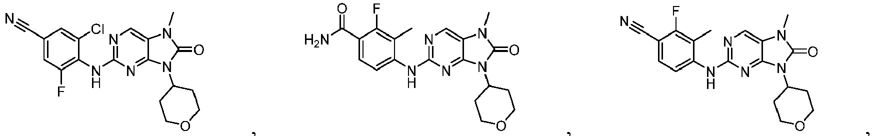

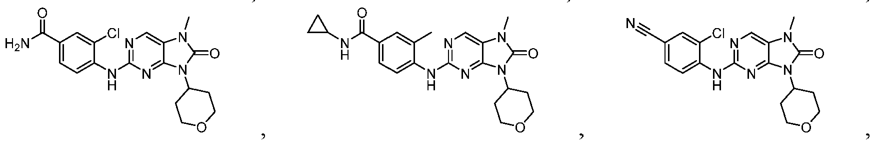

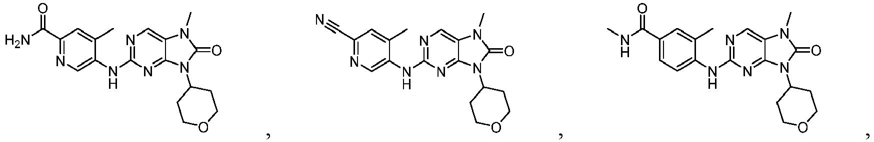

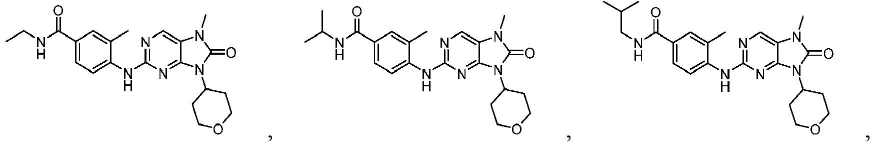

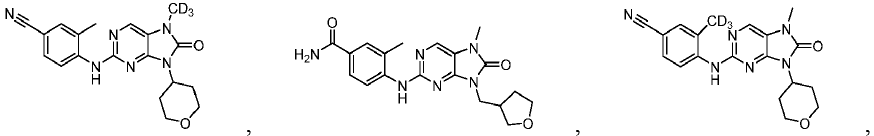

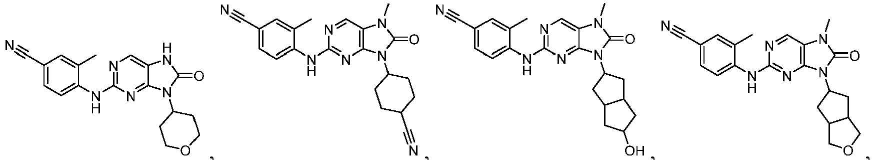

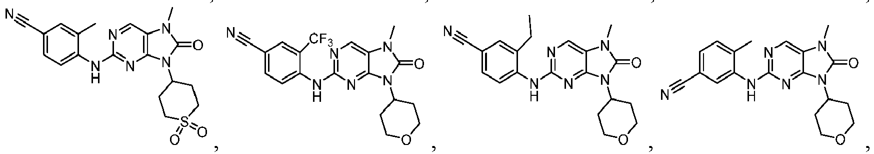

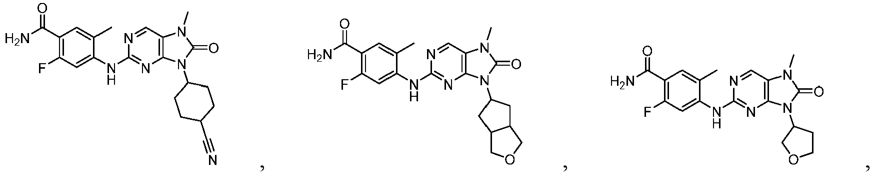

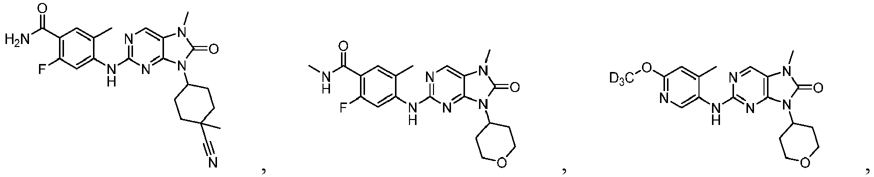

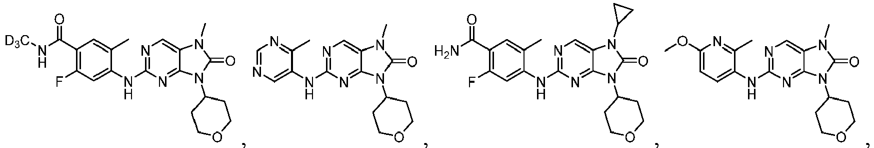

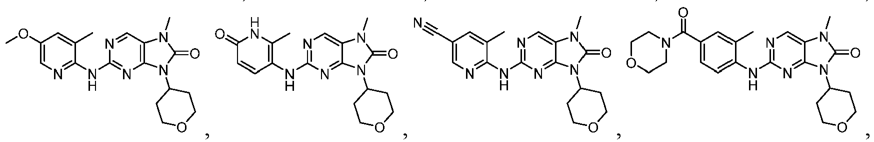

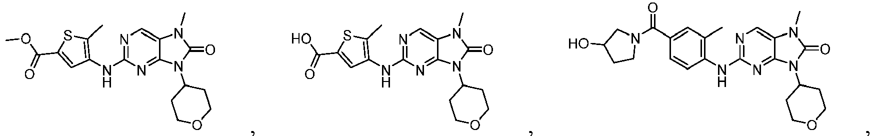

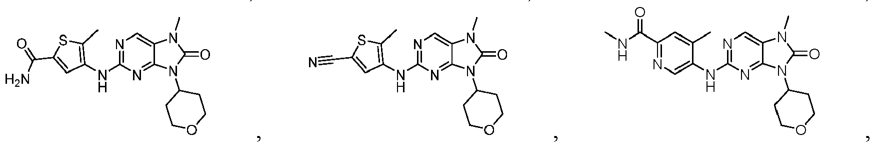

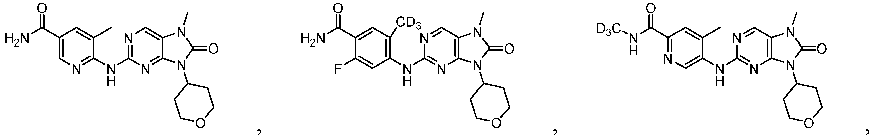

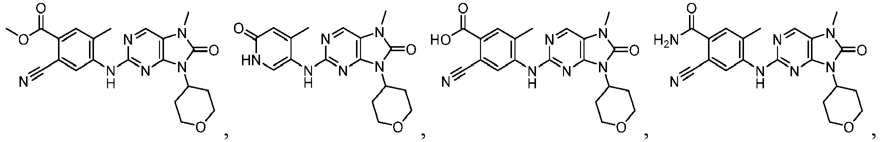

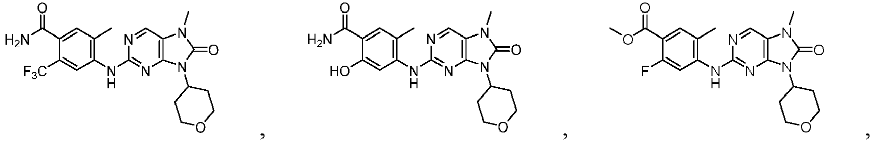

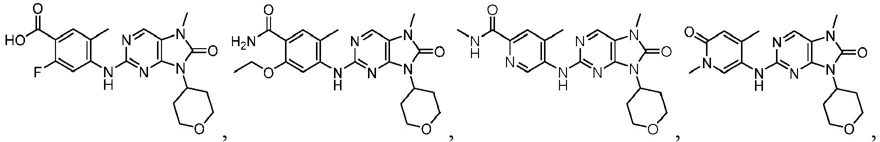

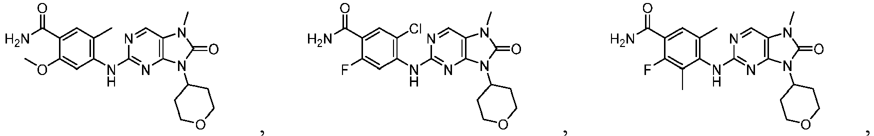

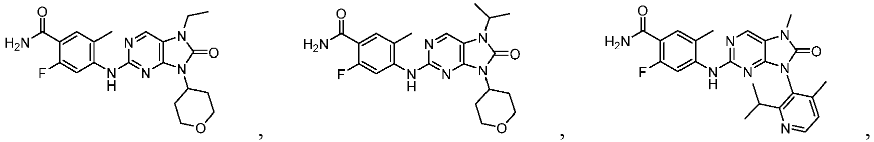

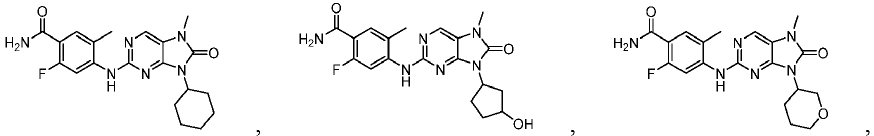

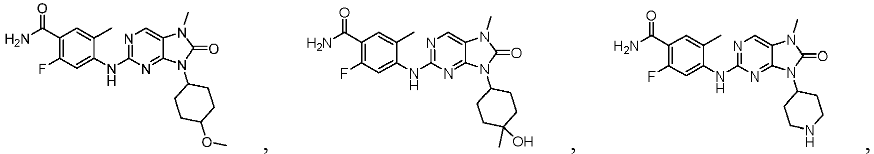

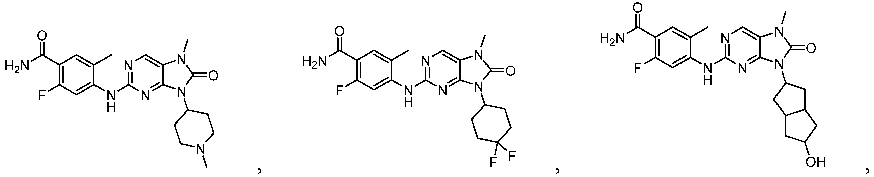

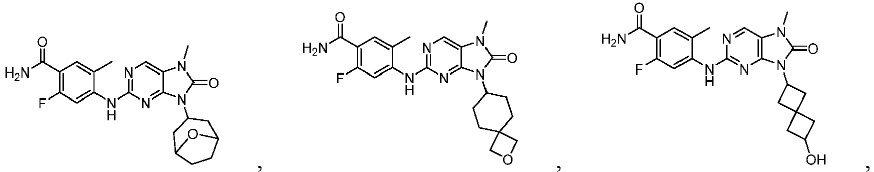

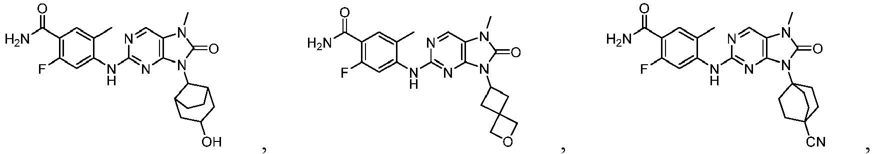

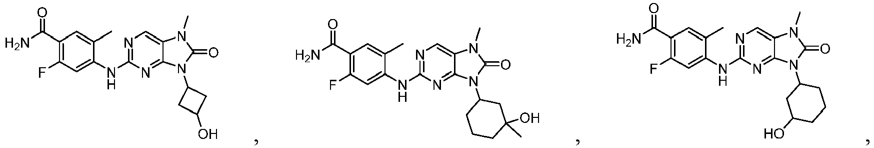

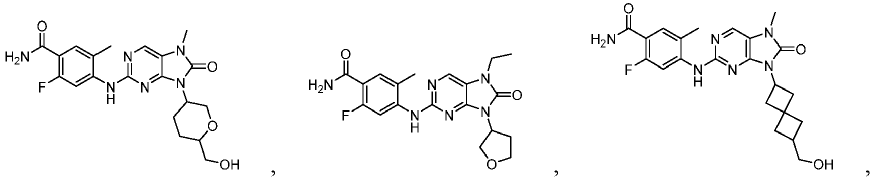

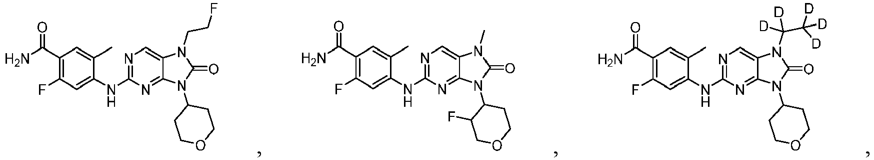

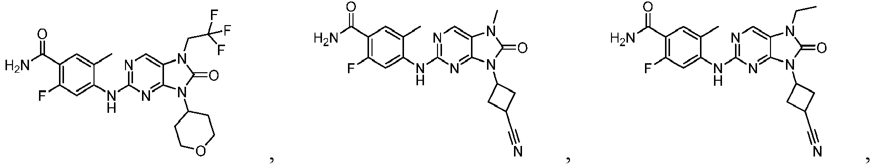

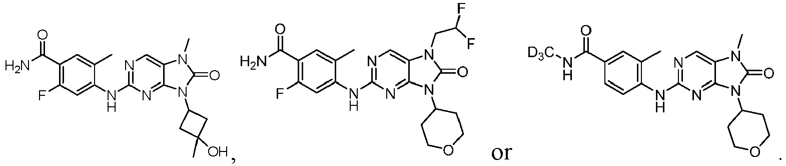

One or more embodiments according to the present disclosure provide a pharmaceutical composition comprising:
(1) a compound according to the present disclosure, or a stereoisomer, solvate, metabolite, pharmaceutically acceptable salt, co-crystal, or prodrug thereof,
(2) optionally one or more additional active ingredients; and
(3) a pharmaceutically acceptable carrier and/or excipient.

One or more embodiments according to the present disclosure provide use of the compound according to the present disclosure, or a stereoisomer, solvate, metabolite, pharmaceutically acceptable salt, co-crystal, or prodrug thereof, or the pharmaceutical composition according to the present disclosure, in the manufacture of a medicament for treatment of cancer.

In one or more embodiments according to the present disclosure, the medicament for treatment of cancer is a DNA-PK inhibitor.

One or more embodiments according to the present disclosure provide a compound of general formula (I'), or a stereoisomer, solvate, prodrug, metabolite, pharmaceutically acceptable salt, or co-crystal thereof, 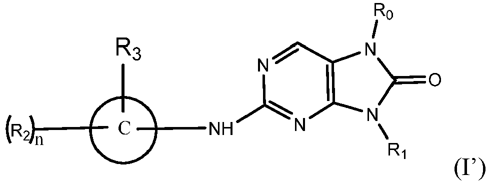 wherein
C is selected from phenyl and 6-membered monocyclic heterocyclic group, wherein the heterocyclic group may contain 1 to 3 heteroatoms selected from N;
R₀ is selected from H, C₁₋₆ alkyl, and cyclopropyl, wherein the alkyl is optionally further substituted with one or more substituents selected from halogen and deuterium;
R₁ is selected from 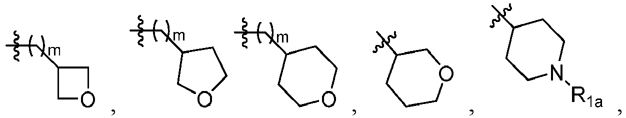 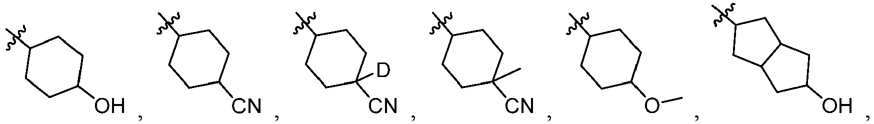 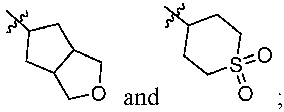
R₁ₐ is selected from H, C₁₋₆ alkyl, and -C(=O)C₁₋₆ alkyl;
R₂ is selected from cyano, carboxyl, -NR₂ₐR_{2b}, -C(=O)NR₂ₐR_{2b}, C₁₋₆ alkoxy, C₁₋₆ alkyl, halogen, -S(=O)₂R₂ₐ and -C(=O)OC₁₋₆ alkyl; wherein the alkyl or alkoxy is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH, D, halogen, C₁₋₆ alkyl and C₁₋₆ alkoxy;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from C₁₋₆ alkyl, OH, and halogen;
R₃ is selected from halogen and C₁₋₆ alkyl, wherein the alkyl is optionally further substituted with 1 to 3 substituents selected from D and halogen;
is selected from 0 and 1; and
n is selected from 0, 1, and 2;
provided that
R₁ is not 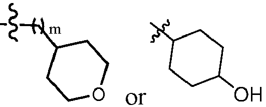 when C is selected from phenyl and a 6-membered monocyclic heterocyclic group and R₀ is H.

In one or more embodiments according to the present disclosure, 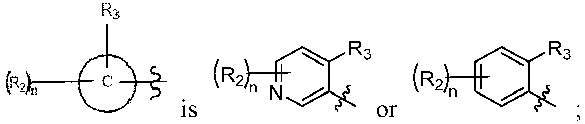
R₀ is selected from H, C₁₋₄ alkyl, and cyclopropyl, wherein the alkyl is optionally further substituted with one or more substituents selected from halogen and deuterium;
R₁ is selected from 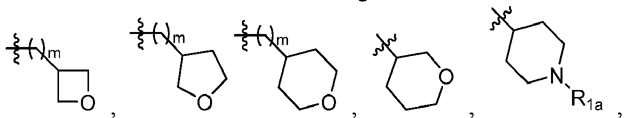 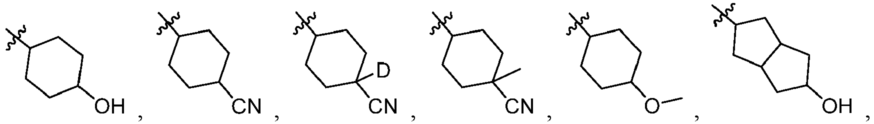 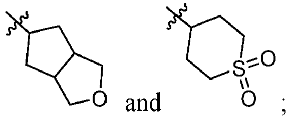
R₁ₐ is selected from H, C₁₋₆ alkyl, and -C(=O)C₁₋₆ alkyl;
R₂ is selected from cyano, -C(=O)NR₂ₐR_{2b}, C₁₋₆ alkoxy, halogen, -S(=O)₂R₂ₐ and - C(=O)OC₁₋₆ alkyl; wherein the alkyl or alkoxy is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH, D, and halogen;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from OH and halogen;
R₃ is halogen or C₁₋₆ alkyl, wherein the alkyl is optionally further substituted with 1 to 3 substituents selected from D and halogen;
m is selected from 0 and 1; and
n is selected from 0, 1, and 2;
provided that
R₁ is not 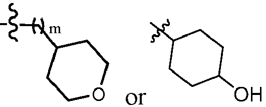 when C is selected from phenyl and a 6-membered monocyclic heterocyclic group and R₀ is H.

One or more embodiments according to the present disclosure provide a compound of general formula (I"), or a stereoisomer, solvate, prodrug, metabolite, pharmaceutically acceptable salt, or co-crystal thereof, 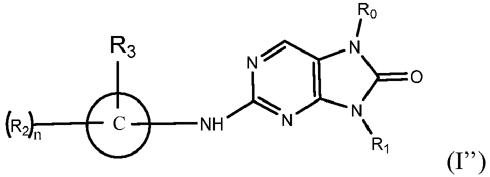 wherein
C is selected from phenyl and 6-membered monocyclic heterocyclic group, wherein the heterocyclic group may contain 1 to 3 heteroatoms selected from N;
R₀ is selected from H and C₁₋₆ alkyl, wherein the alkyl is optionally further substituted with one or more substituents selected from halogen and deuterium;
R₁ is selected from 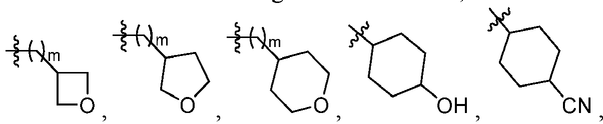 and 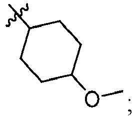
R₂ is selected from cyano, carboxyl, -NR₂ₐR_{2b}, -C(=O)NR₂ₐR_{2b}, C₁₋₆ alkoxy, C₁₋₆ alkyl, halogen, -S(=O)₂R₂ₐ and -C(=O)OC₁₋₆ alkyl; wherein the alkyl is optionally substituted with one or more substituents selected from halogen and deuterium;
R₂ₐ and R_{2b} are selected from H, C₁₋₆ alkyl or 3- to 5-membered cycloalkyl, wherein the C₁₋₆ alkyl is optionally further substituted with one or more substituents selected from OH, halogen, C₁₋₆ alkyl and C₁₋₆ alkoxy;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from C₁₋₆ alkyl, and halogen;
R₃ is selected from halogen and C₁₋₆ alkyl, wherein the alkyl is optionally further substituted with 1 to 3 substituents selected from D and halogen;
m is selected from 0 and 1; and
n is selected from 1 and 2.

In one or more embodiments according to the present disclosure, 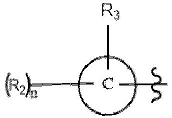 is selected from 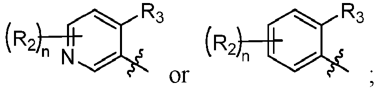
R₀ is selected from H and C₁₋₄ alkyl, wherein the alkyl is optionally further substituted with one or more substituents selected from halogen and deuterium;
R₁ is selected from R₁ is selected from 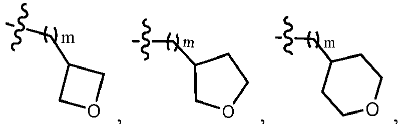 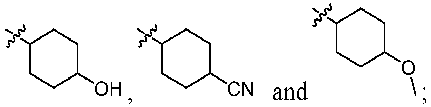
R₂ is selected from cyano, -C(=O)NR₂ₐR_{2b}, C₁₋₆ alkoxy, halogen, -S(=O)₂R₂ₐ and - C(=O)OC₁₋₆ alkyl; wherein the alkyl is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH and halogen;
R₂ₐ and R_{2b} together with the atom to which they are attached may form 6-membered heterocyclyl group;
R₃ is selected from halogen and C₁₋₆ alkyl, wherein the alkyl is optionally further substituted with 1 to 3 substituents selected from D and halogen;
m is selected from 0 and 1; and
n is selected from 1 and 2.

In one or more embodiments according to the present disclosure, the compound according to the present disclosure is 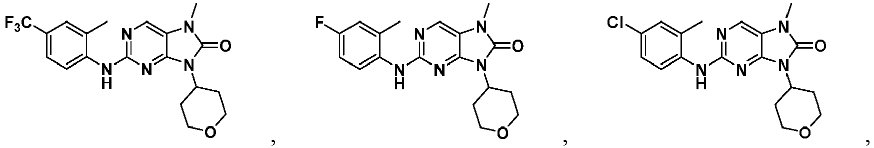 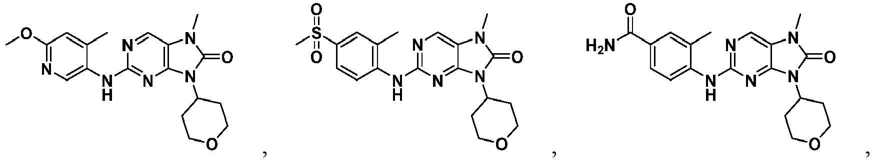 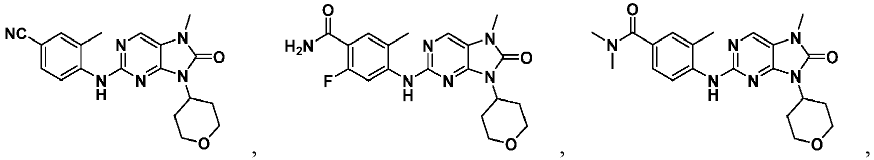 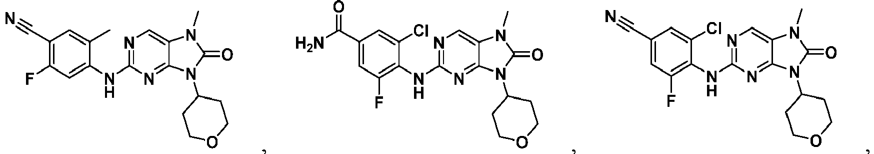 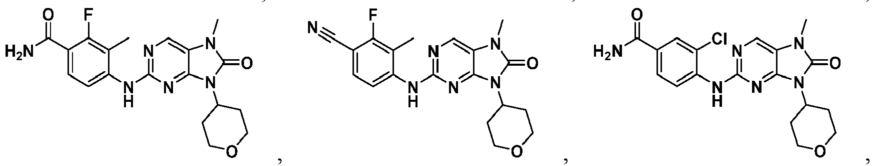 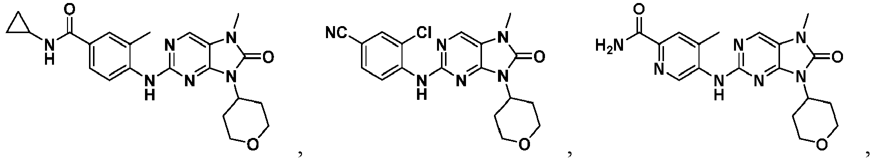 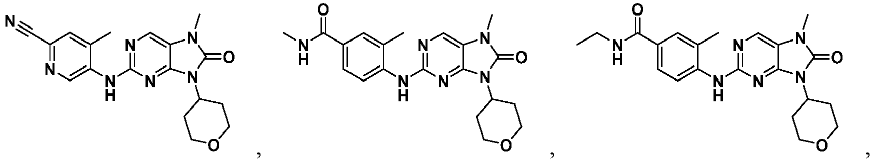 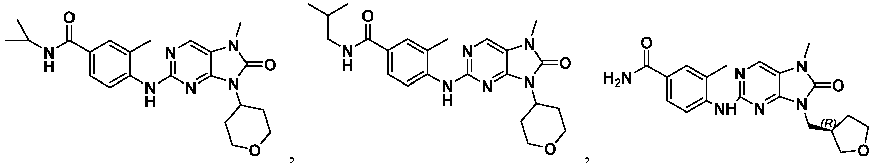 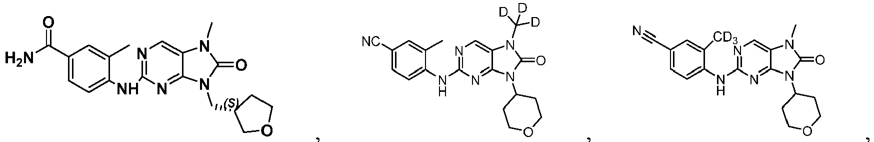 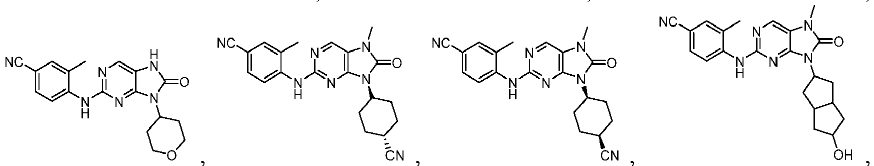 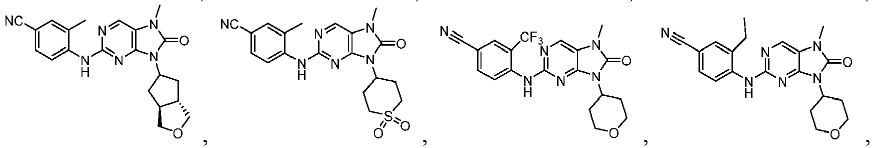 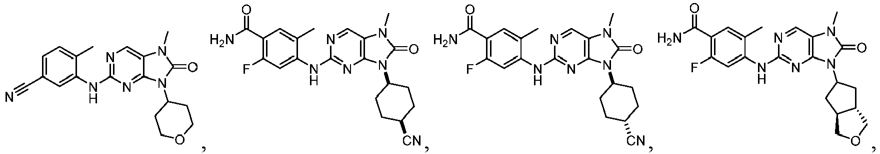 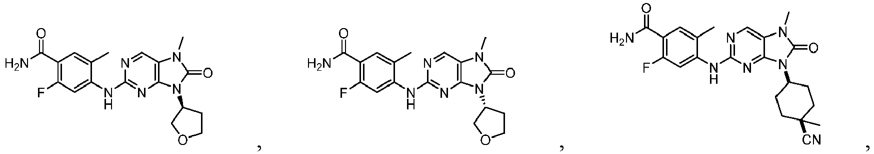 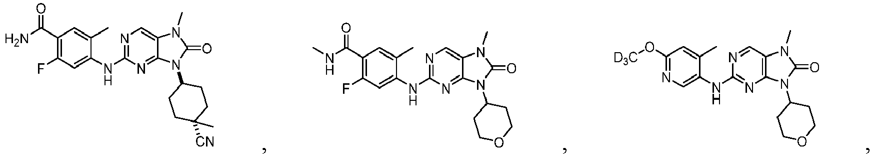 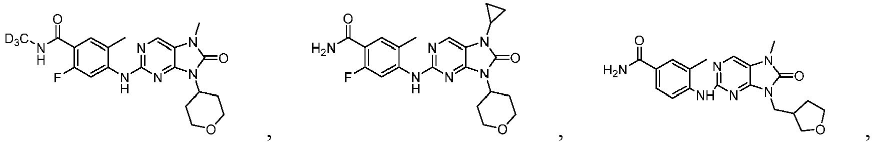 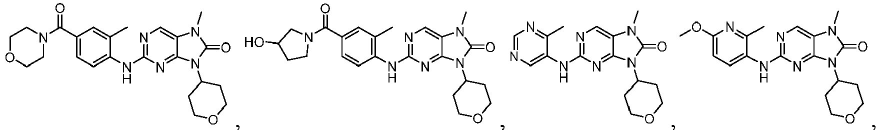 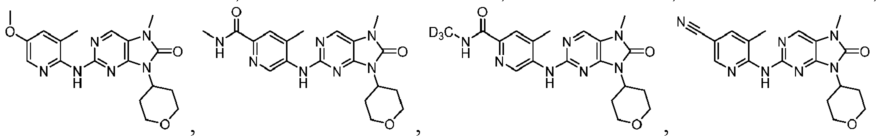 or

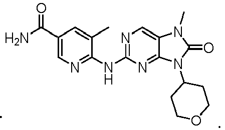

One or more embodiments according to the present disclosure provide a pharmaceutical composition comprising:
(1) a compound according to the present disclosure, or a stereoisomer, solvate, metabolite, pharmaceutically acceptable salt, co-crystal, or prodrug thereof,
(2) optionally one or more additional active ingredients; and
(3) a pharmaceutically acceptable carrier and/or excipient.

One or more embodiments according to the present disclosure provide use of the compound according to the present disclosure, or a stereoisomer, solvate, metabolite, pharmaceutically acceptable salt, co-crystal, or prodrug thereof, or the pharmaceutical composition according to the present disclosure, in the manufacture of a DNA-PK inhibitor.

One or more embodiments according to the present disclosure provide the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, for use as a medicament.

One or more embodiments according to the present disclosure provide the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, for use as a medicament for treatment of cancer or for use in a method for preventing and/or treating cancer.

One or more embodiments according to the present disclosure provide the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, for use as a DNA-PK inhibitor or for use in a method for inhibiting DNA-PK.

One or more embodiments according to the present disclosure provide the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, for use in treatment of DNA-PK-associated diseases.

One or more embodiments according to the present disclosure provide a method for inhibiting DNA-PK, comprising contacting the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, with a subject in need thereof.

One or more embodiments according to the present disclosure provide a method for treating DNA-PK-associated diseases, comprising administering the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, to a subject in need thereof.

One or more embodiments according to the present disclosure provide a method for treating cancer, comprising administering the compound of general formula (I), (II), (III), (IV), (I'), or (I") or the above specific structures according to the present disclosure, or a stereoisomer, solvate, metabolite, pharmaceutically acceptable salt, co-crystal, or prodrug thereof, or the composition according to the present disclosure, to a subject in need thereof.

Unless stated to the contrary, the terms used in the specification and claims have the following meanings.

Carbon, hydrogen, oxygen, sulfur, nitrogen, F, Cl, Br and I involved in the groups and compounds described herein are each inclusive of isotopes thereof, and carbon, hydrogen, oxygen, sulfur or nitrogen involved in the groups and compounds described herein is optionally further replaced by one or more isotopes thereof corresponding thereto, wherein isotopes of carbon include ¹²C, ¹³C and ¹⁴C, isotopes of hydrogen include protium (H), deuterium (D, also called heavy hydrogen) and tritium (T, also called superheavy hydrogen), isotopes of oxygen include ¹⁶O, ¹⁷O and ¹⁸O, isotopes of sulfur include ³²S, ³³S, ³⁴S and ³⁶S, isotopes of nitrogen include ¹⁴N and ¹⁵N, isotopes of fluorine include ¹⁷F and ¹⁹F, isotopes of chlorine include ³⁵Cl and ¹⁷Cl, and isotopes of bromine include ⁷⁹Br and ⁸¹Br.

"Alkyl" refers to a linear or branched saturated aliphatic hydrocarbon group consisting of 1 to 20 carbon atoms, preferably an alkyl group consisting of 1 to 8 (e.g., 1, 2, 3, 4, 5, 6, 7 or 8) carbon atoms, more preferably an alkyl group consisting of 1 to 6 carbon atoms, and further preferably an alkyl group consisting of 1 to 4 carbon atoms. Non-limiting examples include methyl, ethyl, *n*-propyl, isopropyl, *n*-butyl, *sec*-butyl, neobutyl, *tert*-butyl, *n*-pentyl, isopentyl, neopentyl, *n*-hexyl and various branched chain isomers thereof; when the alkyl is substituted, it may be optionally further substituted with 1 or more substituents.

"Alkoxy" refers to a group formed by substitution of at least 1 carbon atom of an alkyl group with an oxygen atom. Non-limiting examples include methoxy, ethoxy, *n-*propoxy, isopropoxy, *n*-butoxy, *sec*-butoxy, *tert*-butoxy, *n*-pentoxy, *n*-hexoxy, cyclopropoxy and cyclobutoxy. The alkyl is defined in the same way as for the "alkyl" described above.

"Alkenyl" refers to a linear or branched unsaturated aliphatic hydrocarbon group containing 1 to 10 (e.g., 1, 2, 3, 4, 5, 6, 7, 8, 9 or 10) carbon-carbon double bonds and consisting of 2 to 20 carbon atoms, preferably an alkenyl group consisting of 2 to 12 (e.g., 2, 3, 4, 5, 6, 7, 8, 9, 10, 11 or 12) carbon atoms, more preferably an alkenyl group consisting of 2 to 8 carbon atoms, and further preferably an alkenyl group consisting of 2 to 6 carbon atoms. Non-limiting examples include vinyl, propen-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9 or 10) carbon-carbon triple bonds and consisting of 2 to 20 carbon atoms, preferably an alkynyl group consisting of 2 to 12 (e.g., 2, 3, 4, 5, 6, 7, 8, 9, 10, 11 or 12) carbon atoms, more preferably an alkynyl group consisting of 2 to 8 carbon atoms, and further preferably an alkynyl group consisting of 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with one or more substituents.

"Aryl" refers to a substituted or unsubstituted aromatic ring. It may be a 5-8 membered (e.g., 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and may be a bridged ring or a spiro ring. Non-limiting examples include phenyl and naphthyl. The aryl may be optionally further substituted with 1 or more substituents.

"Heteroaryl" refers to a substituted or unsubstituted aromatic ring. It may be a 3-8 membered (e.g., 3, 4, 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 6 (e.g., 1, 2, 3, 4, 5 or 6) heteroatoms selected from N, O and S, and is preferably 5-8 membered heteroaryl. 1 to 4 (e.g., 1, 2, 3 or 4) N and S optionally substituted in the ring of the heteroaryl can be oxidized to various oxidation states. Heteroaryl may be attached to a heteroatom or carbon atom and it may be a bridged ring or a spiro ring. Non-limiting examples include cyclic pyridinyl, furanyl, thienyl, pyranyl, pyrrolyl, pyrimidinyl, pyrazinyl, pyridazinyl, imidazolyl, piperidinyl, benzimidazolyl, benzopyridinyl and pyrrolopyridinyl. Heteroaryl is optionally further substituted with 1 or more substituents.

"Carbocyclyl" or "carbocycle" refers to a saturated or unsaturated, aromatic or non-aromatic ring. When being an aromatic ring, it is defined in the same way as for the "aryl" described above; when being an non-aromatic ring, it may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12-membered) bicyclic ring system or a 10-15 membered (e.g., 10, 11, 12, 13, 14 or 15 membered) tricyclic ring system, and it may be a bridged ring or a spiro ring. Non-limiting examples include cyclopropyl, cyclobutyl, cyclopentyl, 1-cyclopentyl-1-alkenyl, 1-cyclopentyl-2-alkenyl, 1-cyclopentyl-3-alkenyl, cyclohexyl, 1-cyclohexyl-2-alkenyl, 1-cyclohexyl-3-alkenyl, cyclohexenyl, cyclohexadienyl, cycloheptyl, cyclooctyl, cyclononyl, cyclodecyl, cycloundecyl, cyclododecyl, 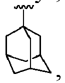 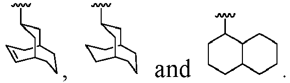 The "carbocyclyl" or "carbocycle" is optionally further substituted with 1 or more substituents.

"Heterocyclyl" or "heterocycle" refers to a saturated or unsaturated, aromatic or non-aromatic heterocycle. When being an aromatic heterocycle, it is defined in the same way as for the "heteroaryl" described above; when being a non-aromatic heterocycle, it may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 4 (e.g., 1, 2, 3 or 4) heteroatoms selected from N, O and S, and is preferably 3-8 membered heterocyclyl. 1 to 4 (e.g., 1, 2, 3 or 4) N and S optionally substituted in the ring of the "heterocyclyl" or "heterocycle" can be oxidized to various oxidation states; "heterocyclyl" or "heterocycle" may be attached to a heteroatom or a carbon atom, and may be a bridged ring or a spiro ring. Non-limiting examples of "heterocyclyl" or "heterocycle" include epoxyethyl, epoxypropyl, aziridinyl, oxetanyl, azetidinyl, thietanyl, 1,3-dioxolanyl, 1,4-dioxolanyl, 1,3-dioxanyl, azepanyl, oxepanyl, thiepanyl, oxoazepinyl, diazepinyl, thiazepinyl, pyridinyl, piperidinyl, homopiperidinyl, furanyl, thienyl, pyranyl, *N*-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, 1,3-dithianyl, dihydrofuranyl, dithiacyclopentyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridinyl, pyrrolopyridinyl, benzodihydrofuranyl, 2-pyrrolinyl, 3-pyrrolinyl, indolinyl, 2*H*-pyranyl, 4*H*-pyranyl, dioxacyclohexyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3*H*-indolylquinolizinyl, *N*-pyridylurea, 1,1-dioxothiomorpholinyl, azabicyclo[3.2.1]octyl, azabicyclo[5.2.0]nonyl, oxatricyclo[5.3.1.1]dodecyl, aza-adamantyl and oxaspiro[3.3]heptyl. The "heterocyclyl" or "heterocycle" may be optionally further substituted with 1 or more substituents.

"Cycloalkyl" refers to a saturated cyclic hydrocarbon group, the ring of which may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-20 membered (e.g., 10, 11, 12, 13, 14, 15, 16, 17, 18, 19 or 20 membered) polycyclic ring system. The ring carbon atoms are preferably 3 to 10 carbon atoms, further preferably 3 to 8 carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, and the like. When the cycloalkyl is substituted, it may be optionally further substituted with 1 or more substituents.

"Heterocycloalkyl" refers to a substituted or unsubstituted saturated non-aromatic cyclic group. It may be a 3-8 membered (e.g., 3, 4, 5, 6, 7 or 8 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1, 2 or 3 heteroatoms selected from N, O and S, and is preferably 3-8 membered heterocyclyl. 1, 2 or 3 N and S optionally substituted in the ring of "heterocycloalkyl" can be oxidized to various oxidation states; "heterocycloalkyl" may be attached to a heteroatom or a carbon atom and may be a bridged ring or a spiro ring. Non-limiting examples of "heterocycloalkyl" include epoxyethyl, aziridinyl, oxetanyl, azetidinyl, 1,3-dioxolanyl, 1,4-dioxolanyl, 1,3-dioxanyl, azepanyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, aza-adamantyl and oxaspiro[3.3]heptyl.

When the "alkyl", "alkoxy", "alkenyl", "alkynyl", "aryl", "heteroaryl", "carbocyclyl", "carbocycle", "heterocyclyl", "heterocycle", "cycloalkyl", "heterocycloalkyl" or "heterocyclyl" described above is substituted, it may be optionally further substituted with 0, 1, 2, 3, 4, 5, 6, 7, 8, 9 or 10 substituents selected from F, Cl, Br, I, hydroxy, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR^{q4}R^{q5}, =NR^{q6}, -C(=O)OC₁₋₆ alkyl, -OC(=O)C₁₋₆ alkyl, -C(=O)NR^{q4}R^{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, - OC(=O)C₆₋₁₀ aryl, -OC(=O)C₅₋₁₀ heteroaryl, -C(=O)OC₅₋₁₀ heteroaryl, -OC(=O)C₃₋₈ heterocycloalkyl, -C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=O)C₆₋₁₀ aryl, -NHC(=O)C₅₋₁₀ heteroaryl, -NHC(=O)C₃₋₈ cycloalkyl, -NHC(=O)C₃₋₈ heterocycloalkyl, -NHC(=O)C₂₋₆ alkenyl and -NHC(=O)C₂₋₆ alkynyl, wherein the substituent C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl or -NHC(=O)C₃₋₈ cycloalkyl is optionally further substituted with 1 to 3 substituents selected from OH, F, Cl, Br, I, C₁₋₆ alkyl, C₁₋₆ alkoxy, -NR^{q4}R^{q5} and =O, R^{q1} is selected from C₁₋₆ alkyl, C₁₋₆ alkoxy and C₆₋₁₀ aryl, and R^{q2} and R^{q3} are selected from H and C₁₋₆ alkyl, wherein R^{q4} and R^{q5} are selected from H, C₁₋₆ alkyl, - NH(C=NR^{q1})NR^{q2}R^{q3}, -S(=O)₂NR^{q2}R^{q3}, -C(=O)R^{q1} and -C(=O)NR^{q2}R^{q3}, wherein the C₁₋₆ alkyl is optionally further substituted with 1 or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl and C₃₋₈ heterocycloalkyl; or R^{q4} and R^{q5}, together with an N atom, form a 3-8 membered heterocycle, which may contain 1 or more heteroatoms selected from N, O and S.

"Pharmaceutically acceptable salt" or "pharmaceutically acceptable salt thereof' refers to a salt obtained by reaction of a compound disclosed herein in a free acid form with a nontoxic inorganic or organic base or by reaction of a compound disclosed herein in a free base form with a nontoxic inorganic or organic acid, and in this salt, the bioavailability and characteristics of the compound disclosed herein in the free acid or free base form is retained.

"Pharmaceutical composition" refers to a mixture of one or more compounds described herein or pharmaceutically acceptable salts or prodrugs thereof and other chemical components, wherein the "other chemical components" refer to pharmaceutically acceptable carriers, excipients and/or one or more other therapeutic agents.

"Carrier" refers to a material that does not cause significant irritation to an organism and does not eliminate the biological activity and characteristics of the administered compound.

"Excipient" refers to an inert substance added to a pharmaceutical composition to facilitate administration of a compound. Non-limiting examples include calcium carbonate, calcium phosphate, sugars, starches, cellulose derivatives (including microcrystalline cellulose), gelatin, vegetable oils, polyethylene glycols, diluents, granulating agents, lubricants, binders and disintegrants.

"Prodrug" refers to a compound disclosed herein that can be metabolized *in vivo* to become biologically active. A prodrug disclosed herein is prepared by modifying amino or carboxyl in a compound disclosed herein, and the modification can be removed by conventional operation or be removed *in vivo* to obtain the parent compound. When a prodrug disclosed herein is administered to a mammalian subject, the prodrug is cleaved to form free amino or carboxyl.

"Cocrystal" refers to a crystal formed by binding of an active pharmaceutical ingredient (API) and a cocrystal former (CCF) via a hydrogen bond or other non-covalent bonds, wherein the API and CCF are both solid in their pure state at room temperature, and the components are present in a fixed stoichiometric ratio. A cocrystal is a multi-component crystal, including both a binary cocrystal formed by two neutral solids and a multiple cocrystal formed by a neutral solid and a salt or solvate.

"Stereoisomer" refers to isomers resulting from different spatial arrangements of atoms in a molecule, including *cis-trans* isomers, enantiomers and conformers.

"Optional", "optionally", "selective" or "selectively" means that the subsequently described event or circumstance may, but does not necessarily, occur, and the description includes cases where the event or circumstance occurs and cases where it does not. For example, "heterocyclyl optionally substituted with alkyl" means that the alkyl may, but does not necessarily, be present, and the description includes the case where the heterocyclyl is substituted with alkyl and the case where the heterocyclyl is not substituted with alkyl.

### DETAILED DESCRIPTION

The following examples illustrate the technical schemes of the present invention in detail, but the protection scope of the present invention includes but is not limited thereto.
DMSO: dimethyl sulfoxide;
DTT: dithiothreitol;
ATP: adenosine triphosphate;
DNA: deoxyribonucleic acid;
IC50: refers to the concentration of a compound at which the activity of DNA-PK kinase is 50% inhibited;
MPLC: medium-pressure silica gel column chromatography.
The reagents used in the present application are all commercially available.

### Examples

### Intermediate 1

### 2-Chloro-7-methyl-9-(tetrahydro-2H-pyran-4-yl)-7,9-dihydro-8H-purin-8-one (Intermediate 1)

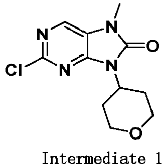

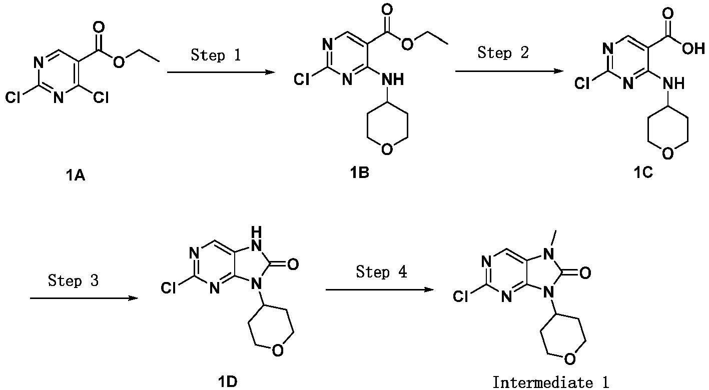

### Step 1:

### Ethyl 2-chloro-4-((tetrahydro-2H-pyran-4-yl)amino)pyrimidine-5-carboxylate (1B)

Ethyl 2,4-dichloropyrimidine-5-carboxylate **1A** (30.00 g, 136.4 mmol) and tetrahydro-2*H*-pyran-4-amine hydrochloride (18.66 g, 136.4 mmol) were dissolved in acetonitrile (600 mL), and after the solution was stirred several times, potassium carbonate (46.92 g, 340.9 mmol) was added. The reaction mixture was stirred at room temperature for 4 h and monitored by TLC. After the reaction is complete, The mixture was filtered, and the filter residue was washed with ethyl acetate (300 mL). The filtrate was concentrated to give a crude product, which was then purified by column separation (n-hexane:ethyl acetate (v/v) = 1:1) to obtain the title compound ethyl 2-chloro-4-((tetrahydro-2*H-*pyran-4-yl)amino)pyrimidine-5-carboxylate **1B** (white solid, 30.0 g, 77.4% yield).

¹H NMR (400 MHz DMSO) δ 8.62 (s,1H), 8.32 (d, 1H), 4.30 (q, 2H), 4.21-4.16 (m, 1H), 3.86-3.83 (m, 2H), 3.48-3.42 (m, 2H), 1.88-1.85 (m, 2H), 1.62-1.53 (m, 2H), 1.31 (t, 3H).

LC-MS m/z(ESI) = **286.10** [M+1]

### Step 2:

### 2-Chloro-4-((tetrahydro-2H-pyran-4-yl)amino)pyrimidine-5-carboxylic acid (1C)

Ethyl 2-chloro-4-((tetrahydro-2*H*-pyran-4-yl)amino)pyrimidine-5-carboxylate **1B** (30 g, 104.99 mmol) was dissolved in tetrahydrofuran/water (200 mL/200 mL), and lithium hydroxide (5.03 g, 209.99 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 6 N hydrochloric acid, and a white solid was precipitated. The reaction mixture was filtered, and the filter cake was washed twice with petroleum ether and collected to obtain the title compound 2-chloro-4-((tetrahydro-2*H*-pyran-4-yl)amino)pyrimidine-5-carboxylic acid **1C** (white solid, 15.0 g, 55.44% yield).

¹H NMR (400 MHz DMSO) δ 8.60 (s, 1H), 8.54 (d, 1H), 4.20-4.15 (m, 1H), 3.86-3.83 (m, 2H), 3.48-3.42 (m, 2H), 1.89-1.86 (m, 2H), 1.60-1.50 (m, 2H).

LC-MS m/z(ESI) = **258.10** [M+1]

### Step 3:

### 2-Chloro-9-(tetrahydro-2H-pyran-4-yl)-7,9-dihydro-8H-purin-8-one (1D)

2-Chloro-4-((tetrahydro-2*H*-pyran-4-yl)amino)pyrimidine-5-carboxylic acid **1C** (15 g, 58.21 mmol) was dissolved in dimethylacetamide (150 mL), and triethylamine (7.38 mL, 58.21 mmol) and diphenylphosphoryl azide (12.06 mL, 58.21 mmol) were added. The reaction mixture was then gradually heated to 120 °C and stirred for 1.5 h. The reaction was monitored by TLC. After completion of the reaction, the reaction mixture was poured into ice water. The solid was collected by filtration and washed 3 times with water, and dried and concentrated in vacuo to give the title compound 2-chloro-9-(tetrahydro-2*H*-pyran-4-yl)-7,9-dihydro-8*H*-purin-8-one **1D** (white solid, 13.0 g, 87.69% yield).

¹H NMR (400 MHz DMSO) δ 11.63 (s, 1H), 8.11 (s, 1H), 4.43-4.37 (m, 1H), 3.98-3.94 (m, 2H), 2.59-2.38 (m, 2H), 1.73-1.65 (m, 2H).

LC-MS m/z(ESI) = **255.10** [M+1]

### Step 4:

### 2-Chloro-7-methyl-9-(tetrahydro-2H-pyran-4-yl)-7,9-dihydro-8H-purin-8-one (intermediate 1)

2-Chloro-9-(tetrahydro-2*H*-pyran-4-yl)-7,9-dihydro-8*H*-purin-8-one **1D** (5 g, 19.63 mmol) was dissolved in dimethylformamide (50 mL), dimethyl sulfate (2.48 g, 19.63 mmol) and cesium carbonate (9.5 g, 29.445 mmol) were added at 0 °C, and the reaction mixture was stirred at 0 °C for 1 h. The reaction was monitored by TLC. After completion of the reaction, the reaction mixture was poured into ice water, and a solid was precipitated. The reaction mixture was filtered and collected to obtain the title compound 2-chloro-7-methyl-9-(tetrahydro-2*H*-pyran-4-yl)-7,9-dihydro-8*H*-purin-8-one **intermediate 1** (white solid, 3.0 g, 56.87% yield).

¹H NMR (400 MHz DMSO) δ 8.36 (s, 1H), 4.50-4.41 (m, 1H), 3.99-3.95 (m, 2H), 3.48-3.42 (m, 2H), 3.34 (s, 3H), 2.47-2.38 (m, 2H), 1.70-1.66 (m, 2H).

LC-MS m/z(ESI) = **268.10** [M+1]

### Intermediate 2

### 4-Amino-2-fluoro-5-methylbenzamide (intermediate 2)

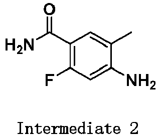

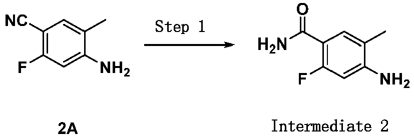

4-Amino-2-fluoro-5-methylbenzonitrile **2A** (300 mg, 2 mmol) and potassium carbonate (41.4 mg, 0.3 mmol) were dissolved in 1 mL of dimethyl sulfoxide, and 300 µL of hydrogen peroxide was added in an ice bath. The reaction mixture was gradually heated to 60 °C and stirred for 2 h. The reaction was monitored by TLC. After completion of the reaction, 5 mL of water was added to the reaction mixture, and a white solid was precipitated. The reaction mixture was filtered and concentrated under reduced pressure to yield the title compound 4-amino-2-fluoro-5-methylbenzamide **intermediate 2** (white solid, 160 mg, 47% yield).

¹H NMR (400 MHz DMSO) δ 7.37 (d, 1H), 7.15 (s, 1H), 6.96 (s, 1H), 6.32 (d, 1H), 5.70 (s, 1H), 2.01 (s, 3H).

LC-MS m/z(ESI) = **169.10** [M+1]

### Example 1

### 7-Methyl-2-((2-methyl-4-(methylsulfonyl)phenyl)amino)-9-(tetrahydro-2H-pyran-4-yl)-7,9-dihydro-8H-purin-8-one (compound 1)

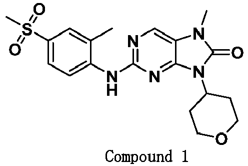

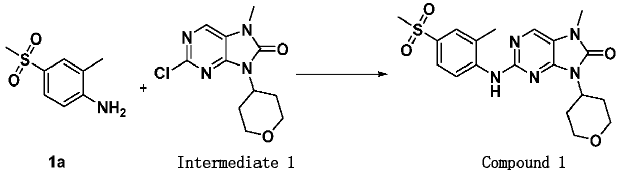

Compound **1a** (290 mg, 1.08 mmol), compound **intermediate** 1 (200 mg, 1.08 mmol), cesium carbonate (703 mg, 2.16 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (98 mg, 0.11 mmol) were dissolved in dioxane (10 mL), followed by nitrogen purging. The resulting mixture was stirred under nitrogen at 100 °C for 4 h and monitored by TLC. After completion of the reaction, the mixture was poured into ice water, and the solid was collected and purified by silica gel column chromatography (dichloromethane/methanol (v/v) = 100/1) to give the title compound 7-methyl-2-((2-methyl-4-(methylsulfonyl)phenyl)amino)-9-(tetrahydro-2*H*-pyran-4-yl)-7,9-dihydro-8*H*-purin-8-one **compound 1** (white solid, 50 mg, 11.09 % yield).

¹H NMR (400 MHz DMSO) δ 8.67 (s, 1H), 8.15 (s, 1H), 8.11 (d, 1H), 7.20 (d, 1H), 7.67 (dd, 1H), 4.47-4.39 (m, 1H), 3.98 (dd, 2H), 3.42 (t, 2H), 3.32 (s, 3H), 3.16 (s, 3H), 2.57-2.49 (m, 2H), 2.37 (s, 3H), 1.67 (dd, 2H).

LC-MS m/z(ESI) = **418.10** [M+1]

### Example 2

### 3-Methyl-4-((7-methyl-8-oxo-9-(tetrahydro-2H-pyran-4-yl)-8,9-dihydro-7H-purin-2-yl)amino)benzamide (compound 2)

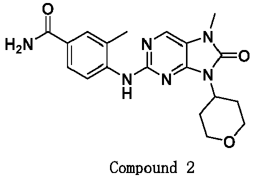

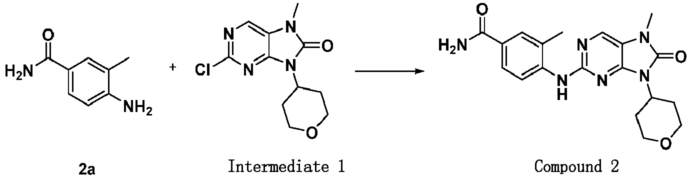

The title compound 3-methyl-4-((7-methyl-8-oxo-9-(tetrahydro-2*H*-pyran-4-yl)-8,9-dihydro-7*H*-purin-2-yl)amino)benzamide **compound 2** (white solid, 100 mg, 19.64% yield) was prepared according to the synthesis method of **compound 1.**

¹H NMR (400 MHz DMSO) δ 8.48 (s,1H), 8.10 (s,1H), 7.82 (d, 2H), 7.71 (s, 1H), 7.66 (dd, 1H), 7.16 (s, 1H), 4.44-4.37 (m, 1H), 3.97 (dd, 2H), 3.41 (t, 2H), 3.33 (s, 3H), 2.57-2.46 (m, 2H), 2.29 (s, 3H), 1.65 (dd, 2H).

LC-MS m/z(ESI) = **383.20** [M+1]

### Example 3

### 7-Methyl-2-((2-methyl-4-(trifluoromethyl)phenyl)amino)-9-(tetrahydro-2H-pyran-4-yl)-7,9-dihydro-8H-purin-8-one (compound 3)

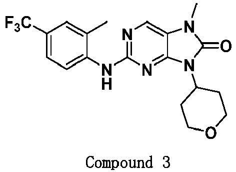

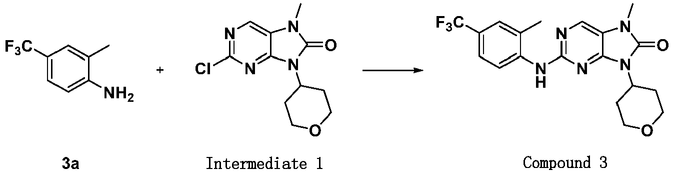

The title compound 7-methyl-2-((2-methyl-4-(trifluoromethyl)phenyl)amino)-9-(tetrahydro-2*H*-pyran-4-yl)-7,9-dihydro-8*H*-purin-8-one **compound 3** (white solid, 50 mg, 21.5% yield) was prepared according to the synthesis method of **compound** 1. ¹H NMR (400 MHz DMSO) δ 8.62 (s, 1H), 8.12 (s, 1H), 8.00 (d, 1H), 7.53 (s, 1H), 7.47 (d, 1H), 4.47-4.38 (m, 1H), 3.97 (dd, 2H), 3.42 (t, 2H), 3.31 (s, 3H), 2.57-2.40 (m, 2H), 2.35 (s, 3H), 1.69-1.65 (m, 2H).

LC-MS m/z(ESI) = **408.20** [M+1]

### Example 4

### 2-((4-Chloro-2-methylphenyl)amino)-7-methyl-9-(tetrahydro-2H-pyran-4-yl)-7,9-dihydro-8H-purin-8-one (compound 4)

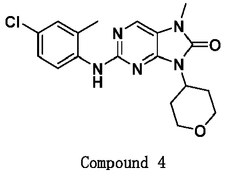

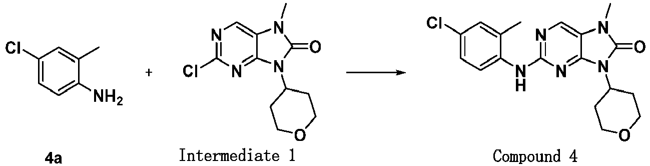

The title compound 2-((4-chloro-2-methylphenyl)amino)-7-methyl-9-(tetrahydro-2*H-*pyran-4-yl)-7,9-dihydro-8*H*-purin-8-one **compound 4** (white solid, 50 mg, 18.94 % yield) was prepared according to the synthesis method of **compound 1.**

¹H NMR (400 MHz DMSO) δ 8.49 (s, 1H), 8.04 (s, 1H), 7.60 (d, 1H), 7.26 (d, 1H), 7.17 (dd, 1H), 4.43-4.35 (m, 1H), 3.96 (dd, 2H), 3.40 (t, 2H), 3.29 (s, 3H), 2.50-2.44 (m, 2H), 2.23 (s, 3H), 1.64 (dd, 2H).

LC-MS m/z(ESI) = **374.10** [M+1]

### Example 5

### 3-Methyl-4-((7-methyl-8-oxo-9-(tetrahydro-2H-pyran-4-yl)-8,9-dihydro-7H-purin-2-yl)amino)benzonitrile (compound 5)

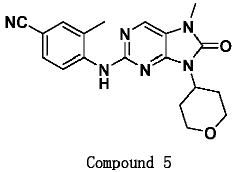

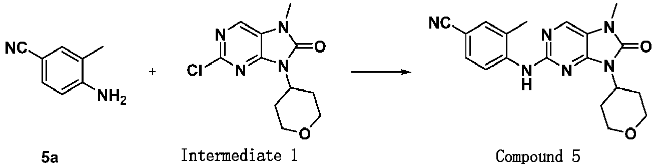

The title compound 3-methyl-4-((7-methyl-8-oxo-9-(tetrahydro-2H-pyran-4-yl)-8,9-dihydro-7H-purin-2-yl)amino)benzonitrile compound 5 (white solid, 50 mg, 18.13 % yield) was prepared according to the synthesis method of compound 1.

¹H NMR (400 MHz DMSO) δ 8.68 (s, 1H), 8.15 (s, 1H), 8.06 (d, 1H), 7.63 (s, 1H), 7.57 (dd, 1H), 4.46-4.38 (m, 1H), 3.97 (dd, 2H), 3.42 (t, 2H), 3.32 (s, 3H), 2.55-2.45 (m, 2H), 2.32 (s, 3H), 1.66 (dd, 2H).

LC-MS m/z(ESI) = **365.20** [M+1]

### Example 6

### 3-Methyl-4-((7-(methyl-d3)-8-oxo-9-(tetrahydro-2H-pyran-4-yl)-8,9-dihydro-7H-purin-2-yl)amino)benzonitrile (compound 6)

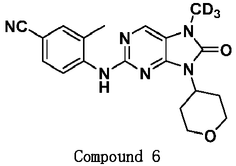

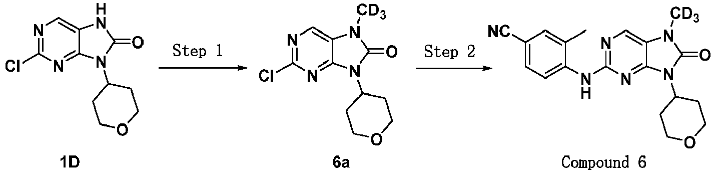

### Step 1:

### 2-Chloro-7-(methyl-d3)-9-(tetrahydro-2H-pyran-4-yl)-7,9-dihydro-8H-purin-8-one (6a)

2-Chloro-9-(tetrahydro-2*H*-pyran-4-yl)-7,9-dihydro-8*H*-purin-8-one **1D** (1.0 g, 3.93 mmol) was dissolved in dimethyl sulfoxide (20 mL), and cesium carbonate (2.03 g, 7.86 mmol) was added at room temperature, followed by addition of deuterated iodomethane (0.45 g, 3.93 mmol) at 0 °C. The reaction mixture was stirred at room temperature for 2 h and monitored by TLC. After the reaction was completed, 5 mL of water was added and the resulting mixture was extracted 3 times with ethyl acetate. The organic phase was dried over anhydrous sodium sulfate and concentrated in vacuo, and a solid was precipitated. The mixture was filtered and purified by medium pressure preparative liquid chromatography, the filtrate was concentrated under reduced pressure to obtain the title compound 2-chloro-7-(methyl-d3)-9-(tetrahydro-2*H*-pyran-4-yl)-7,9-dihydro-8*H*-purin-8-one **6a** (white solid, 0.36 g, 34.66% yield). ¹HNMR (400 MHz DMSO) δ 8.36 (s, 1H), 4.50-4.41 (m, 1H), 3.99-3.95 (m, 2H), 3.48-3.42 (m, 2H), 2.47-2.38 (m, 2H), 1.70-1.66 (m, 2H).

LC-MS m/z(ESI) = **272.10** [M+1]

### Step 2:

### 3-Methyl-4-((7-(methyl-d3)-8-oxo-9-(tetrahydro-2H-pyran-4-yl)-8,9-dihydro-7H-purin-2-yl)amino)benzonitrile (compound 6)

4-Amino-3-methylbenzonitrile **5a** (200 mg, 1.51 mmol), 2-chloro-7-(methyl-d3)-9-(tetrahydro-2*H-*pyran-4-yl)-7,9-dihydro-8*H*-purin-8-one **6a** (330 mg, 1.21 mmol), cesium carbonate (980 mg, 3.02 mmol), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (140 mg, 0.15 mmol) were dissolved in dioxane (10 mL), followed by nitrogen purging. The reaction mixture was stirred under nitrogen at 100 °C for 4 h and monitored by TLC. After the reaction was completed the mixture was poured into ice water, and the solid was collected and purified by silica gel column chromatography (dichloromethane/methanol (v/v) = 100/1) to obtain compound 3-methyl-4-((7-(methyl-d3)-8-oxo-9-(tetrahydro-2*H*-pyran-4-yl)-8,9-dihydro-7*H*-purin-2-yl)amino)benzonitrile **compound 6** (light gray solid, 99 mg, 17.56 % yield).

¹H NMR (400 MHz, Chloroform-d) δ 8.55 (d, 1H), 7.92 (s, 1H), 7.55 (dd, 1H), 7.46 (d, 1H), 7.09 (s, 1H), 4.60-4.50 (m, 1H), 4.14 (dd, 2H), 3.60-3.48 (m, 2H), 2.83-2.67 (m, 2H), 2.39 (s, 3H), 1.80-1.70 (m, 2H).

LC-MS m/z(ESI) = **368.20** [M+1]

### Example 7

### 2-((4-Fluoro-2-methylphenyl)amino)-7-methyl-9-(tetrahydro-2H-pyran-4-yl)-7,9-dihydro-8H-purin-8-one (compound 7)

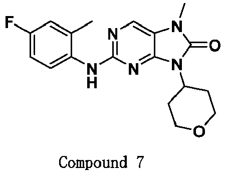

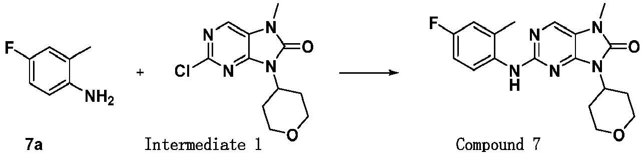

The title compound 2-((4-fluoro-2-methylphenyl)amino)-7-methyl-9-(tetrahydro-2*H-*pyran-4-yl)-7,9-dihydro-8*H*-purin-8-one **compound 7** (white solid, 50 mg, 17.51 % yield) was prepared according to the synthesis method of **compound** 1.

¹H NMR (400 MHz DMSO) δ 8.45 (s, 1H), 7.99 (s, 1H), 7.46-7.43 (m, 1H), 7.05 (dd, 1H), 6.98-6.93 (m, 1H), 4.41-4.33 (m, 1H), 3.97-3.93 (dd, 2H), 3.4 (t, 2H), 3.27 (s, 3H), 2.54-2.44 (m,2H), 2.2 (s, 3H), 1.63 (dd, 2H).

LC-MS m/z(ESI) = **358.20** [M+1]

### Example 8

### 2-Fluoro-5-methyl-4-((7-methyl-8-oxo-9-(tetrahydro-2H-pyran-4-yl)-8,9-dihydro-7H-purin-2-yl)amino)benzonitrile (compound 8)

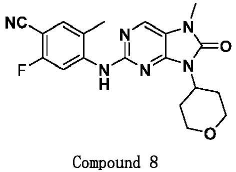

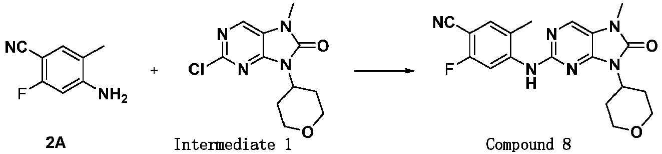

The title compound 2-fluoro-5-methyl-4-((7-methyl-8-oxo-9-(tetrahydro-2*H*-pyran-4-yl)-8,9-dihydro-7*H*-purin-2-yl)amino)benzonitrile **compound 8** (white solid, 18.1 mg, 10.3 % yield) was prepared according to the synthesis method of **compound 1.**

¹H NMR (400 MHz DMSO) δ 8.79 (s, 1H), 8.25 (s, 1H), 8.22 (d, 1H), 7.66 (d, 1H), 4.48-4.41 (m, 1H), 3.97 (dd, 2H), 3.43 (t, 2H), 3.32 (s, 3H), 2.57-2.50 (m, 2H), 2.32(s, 3H), 1.68(dd, 2H).

LC-MS m/z(ESI) = **383.10** [M+1]

### Example 9

### 2-((6-Methoxy-4-methylpyridin-3-yl)amino)-7-methyl-9-(tetrahydro-2H-pyran-4-yl)-7,9-dihydro-8H-purin-8-one (compound 9)

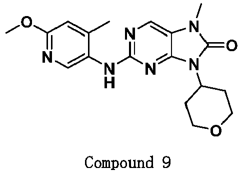

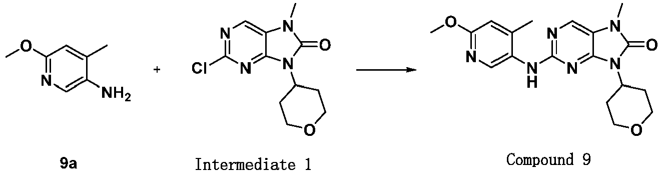

The title compound 2-((6-methoxy-4-methylpyridin-3-yl)amino)-7-methyl-9-(tetrahydro-2*H*-pyran-4-yl)-7,9-dihydro-8*H*-purin-8-one **compound 9** (white solid, 60 mg, 15.2 % yield) was prepared according to the synthesis method of **compound 1.** ¹H NMR (400 MHz DMSO) δ 8.49 (s, 1H), 8.04 (s, 1H), 7.95 (s, 1H), 6.70 (s, 1H), 4.41-4.33 (m, 1H), 3.95 (dd, 2H), 3.81 (s, 3H), 3.40 (t, 2H), 3.27 (s, 3H), 2.54-2.44 (m, 2H), 2.15 (s, 3H), 1.63(dd, 2H).

LC-MS m/z(ESI) = **371.20** [M+1]

### Example 10

### 3-Chloro-4-((7-methyl-8-oxo-9-(tetrahydro-2H-pyran-4-yl)-8,9-dihydro-7H-purin-2-yl)amino)benzonitrile (compound 10)

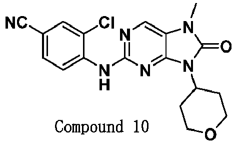

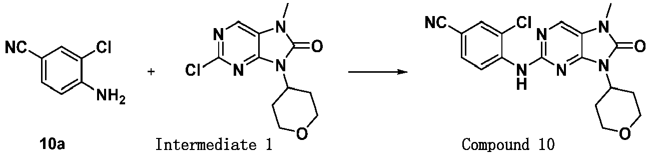

The title compound 3-chloro-4-((7-methyl-8-oxo-9-(tetrahydro-2*H*-pyran-4-yl)-8,9-dihydro-7*H*-purin-2-yl)amino)benzonitrile **compound 10** (white solid, 6.4 mg, 7.8 % yield) was prepared according to the synthesis method of **compound 1.**

¹H NMR (400 MHz DMSO) δ 8.61 (s, 1H), 8.44 (d, 1H), 8.23 (s, 1H), 8.05 (d, 1H), 7.77 (d, 1H), 4.47-4.41 (m, 1H), 3.97 (dd, 2H), 3.44 (t, 2H), 1.67 (d, 2H).

LC-MS m/z(ESI) = **385.10** [M+1]

### Example 11

### 3-Methyl-4-[(9-(oxa-4-yl)-8-oxo-8,9-dihydro-7H-purin-2-yl)amino]benzonitrile (compound 11)

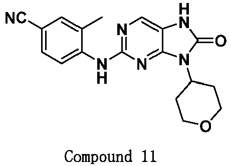

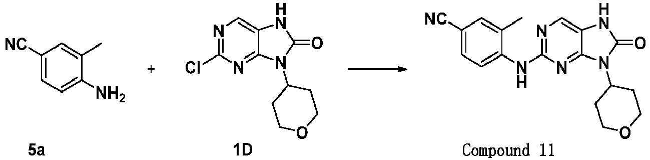

4-Amino-3-methylbenzonitrile **5a** (200 mg, 1.51 mmol), 2-chloro-9-(oxa-4-yl)-8,9-dihydro-7*H*-purin-8-one **1D** (380 mg, 1.51 mmol), cesium carbonate (980 mg, 3.02 mmol), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (140 mg, 0.15 mmol) were dissolved in dioxane (10 mL), followed by nitrogen purging. The mixture was stirred under nitrogen at 100 °C for 4 h and monitored by TLC. After the reaction was completed. The mixture was poured into ice water, and the solid was collected and purified by silica gel column chromatography (dichloromethane/methanol (v/v) = 100/1) to give obtain the title compound 3-methyl-4-[(9-(oxa-4-yl)-8-oxo-8,9-dihydro-7*H*-purin-2-yl)amino]benzonitrile **compound 11** (white solid, 120 mg, 22.68 % yield).

¹H NMR (400 MHz, DMSO-d6) δ 11.09 (s, 1H), 8.60 (s, 1H), 8.08 (d, 1H), 7.96 (s, 1H), 7.62 (d, 1H), 7.57 (dd, 1H), 4.42-4.35 (m, 1H), 3.98 (dd, 2H), 3.41 (t, 2H), 2.32 (s, 3H), 1.64 (dd, 2H).

LC-MS m/z(ESI) = **351.10** [M+1]

### Example 12

### 2-Fluoro-3-methyl-4-((7-methyl-8-oxo-9-(tetrahydro-2H-pyran-4-yl)-8,9-dihydro-7H-purin-2-yl)amino)benzonitrile (compound 12)

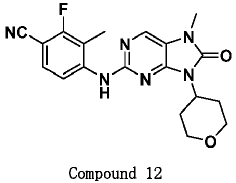

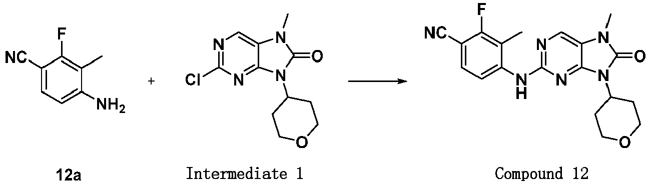

The title compound 2-fluoro-3-methyl-4-((7-methyl-8-oxo-9-(tetrahydro-2*H*-pyran-4-yl)-8,9-dihydro-7*H*-purin-2-yl)amino)benzonitrile **compound 12** (pale yellow solid, 25 mg, 7% yield) was prepared according to the synthesis method of **compound 1.**

¹H NMR (400 MHz DMSO) δ 9.00 (s, 1H), 8.20-8.17 (m, 1H), 7.91 (d, 1H), 7.63 (t, 1H), 4.47-4.39 (m, 1H), 3.97 (dd, 2H), 3.45-3.38 (m, 2H), 2.55-2.45 (m, 2H), 2.22 (d, 3H), 1.67 (dd, 2H).

LC-MS m/z(ESI) = **383.20** [M+1]

### Example 13

### 4-((7-Methyl-8-oxo-9-(tetrahydro-2H-pyran-4-yl)-8,9-dihydro-7H-purin-2-yl)amino)-3-(trifluoromethyl)benzonitrile (compound 13)

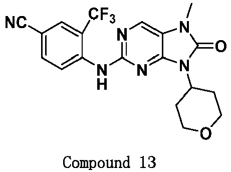

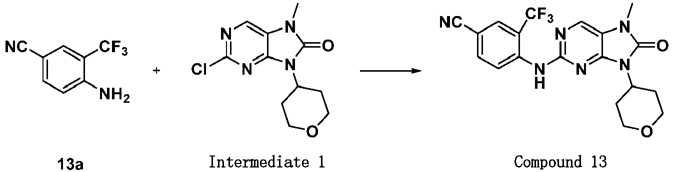

The title compound 4-((7-methyl-8-oxo-9-(tetrahydro-2*H*-pyran-4-yl)-8,9-dihydro-7*H*-purin-2-yl)amino)-3-(trifluoromethyl)benzonitrile **compound 13** (pale yellow solid, 35 mg, 11% yield) was prepared according to the synthesis method of **compound 1.**

¹H NMR (400 MHz CDCl₃) δ 8.77 (d, 1H), 8.94-8.80 (m, 4H), 4.59-4.53 (m, 1H), 4.14 (dd, 2H), 3.54 (t, 2H), 2.74-2.63 (m, 2H), 1.74 (d, 2H).

LC-MS m/z(ESI) = **419.20** [M+1]

### Example 14

### 3-ethyl-4-((7-Methyl-8-oxo-9-(tetrahydro-2H-pyran-4-yl)-8,9-dihydro-7H-purin-2-yl)amino)benzonitrile (compound 14)

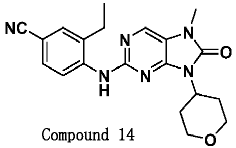

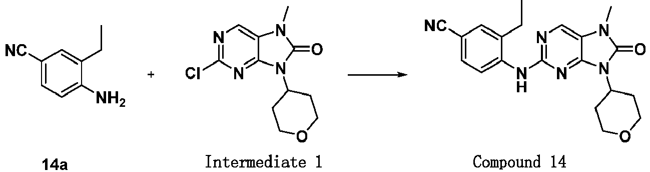

The title compound 3-ethyl-4-((7-methyl-8-oxo-9-(tetrahydro-2H-pyran-4-yl)-8,9-dihydro-7H-purin-2-yl)amino)benzonitrile **compound 14** (pale yellow solid, 27 mg, 10% yield) was prepared according to the synthesis method of **compound 1.**

¹H NMR (400 MHz DMSO) δ 8.71 (s, 1H), 8.13 (d, 1H), 8.00 (d, 1H), 7.61-7.57 (m, 2H), 4.46-4.38 (m, 1H), 3.97 (dd, 2H), 3.45-3.38 (m, 2H), 2.74 (q, 2H), 1.68-1.64 (d, 2H), 1.15 (t, 3H).

LC-MS m/z(ESI) = **379.20** [M+1]

### Example 15

### 4-Methyl-3-((7-methyl-8-oxo-9-(tetrahydro-2H-pyran-4-yl)-8,9-dihydro-7H-purin-2-yl)amino)benzonitrile (compound 15)

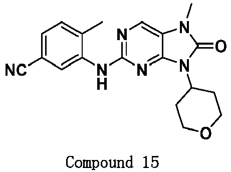

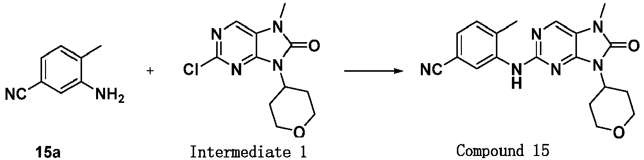

The title compound 4-methyl-3-((7-methyl-8-oxo-9-(tetrahydro-2*H*-pyran-4-yl)-8,9-dihydro-7*H*-purin-2-yl)amino)benzonitrile **compound 15** (pale yellow solid, 27 mg, 10% yield) was prepared according to the synthesis method of **compound 1.**

¹H NMR (400 MHz DMSO) δ 8.67 (s, 1H), 8.14 (d, 2H), 7.39 (d, 2H), 4.41-4.37 (m, 1H), 3.45-3.38 (m, 2H), 3.31 (s, 3H), 2.51-2.47 (m, 2H), 2.35 (s, 3H), 1.70-1.66 (m, 2H).

LC-MS m/z(ESI) = **365.20** [M+1]

### Example 16

### 3-Chloro-5-fluoro-4-((7-methyl-8-oxo-9-(tetrahydro-2H-pyran-4-yl)-8,9-dihydro-7H-purin-2-yl)amino)benzamide (compound 16)

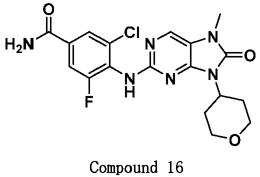

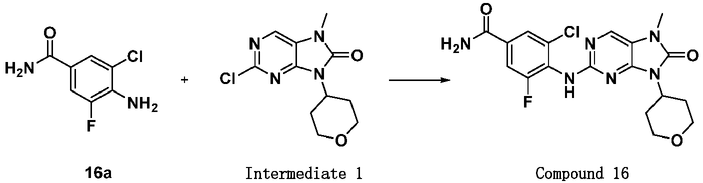

The title compound 3-chloro-5-fluoro-4-((7-methyl-8-oxo-9-(tetrahydro-2*H*-pyran-4-yl)-8,9-dihydro-7*H*-purin-2-yl)amino)benzamide **compound 16** (pale yellow solid, 20 mg, 6% yield) was prepared according to the synthesis method of **compound 1.**

¹H NMR (400 MHz DMSO) δ 10.62 (s, 1H), 8.33 (t, 1H), 7.82 (t, 1H), 7.69 (dd, 1H), 4.47-4.39 (m, 1H), 3.96 (dd, 2H), 3.45-3.39 (m, 1H), 3.36 (s, 3H), 2.60-2.53 (m, 2H), 1.64 (dd, 2H).

LC-MS m/z(ESI) = **421.20** [M+1]

### Example 17

### 4-Methyl-5-((7-methyl-8-oxo-9-(tetrahydro-2H-pyran-4-yl)-8,9-dihydro-7H-purin-2-yl)amino)picolinonitrile (compound 17)

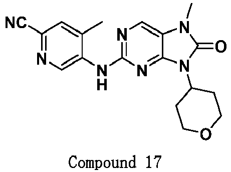

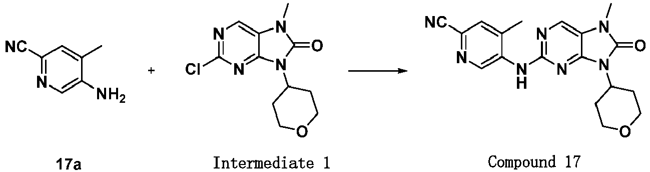

The title compound 4-methyl-5-((7-methyl-8-oxo-9-(tetrahydro-2*H*-pyran-4-yl)-8,9-dihydro-7*H*-purin-2-yl)amino)picolinonitrile **compound 17** (white solid, 44 mg, 29.3% yield) was prepared according to the synthesis method of **compound 1.**

¹H NMR (400 MHz DMSO) δ 9.12 (s, 1H), 9.05 (s, 1H), 8.17 (s, 1H), 7.87 (s, 1H), 4.46-4.39(m, 1H), 3.97 (dd, 2H), 3.41 (t, 2H), 3.32 (s, 3H), 2.34 (s, 3H), 1.67 (d, 2 H). LC-MS m/z(ESI) = **366.10** [M+1]

### Example 18

### 4-Methyl-5-((7-methyl-8-oxo-9-(tetrahydro-2H-pyran-4-yl)-8,9-dihydro-7H-purin-2-yl)amino)picolinamide (compound 18)

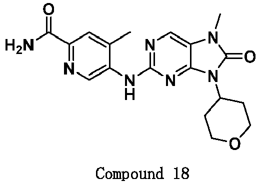

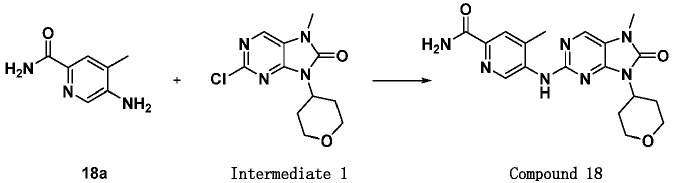

The title compound 4-methyl-5-((7-methyl-8-oxo-9-(tetrahydro-2*H*-pyran-4-yl)-8,9-dihydro-7*H*-purin-2-yl)amino)picolinamide **compound 18** (white solid, 40 mg, 28.1% yield) was prepared according to the synthesis method of **compound** 1.

¹H NMR (400 MHz DMSO) δ 9.04 (s, 1H), 8.89 (s, 1H), 8.12 (s, 1H), 8.05 (s, 1H), 7.95 (s, 1H), 7.57 (s, 1H), 4.44-4.38 (m, 1H), 3.97 (dd, 2H), 3.44 (t, 2H), 3.31 (s, 3H), 2.36 (s, 3H), 1.67 (d, 2H).

LC-MS m/z(ESI) = **384.20** [M+1]

### Example 19

### 2-((5-Methoxy-3-methylpyridin-2-yl)amino)-7-methyl-9-(tetrahydro-2H-pyran-4-yl)-7,9-dihydro-8H-purin-8-one (compound 19)

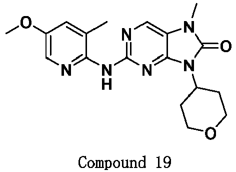

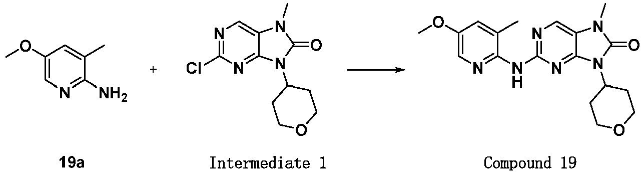

The title compound 2-((5-Methoxy-3-methylpyridin-2yl)-amino)-7-methyl-9-(tetrahydro-2H-pyran-4-yl)-7,9-dihydro-8H-purin-8-one **compound 19** (white solid, 12 mg, 9.6% yield) was prepared according to the synthesis method of **compound 1.** ¹H NMR (400 MHz DMSO) δ 8.73 (s, 1H), 7.96 (s, 1H), 7.90 (d, 1H), 7.28 (d, 1H), 4.38-4.31 (m, 1H), 3.94 (dd, 2H), 3.81 (s, 3H), 3.39 (t, 2H), 3.27 (s, 3H), 2.50-2.42 (m, 2H), 2.15 (s, 3H), 1.61 (dd, 2H).

LC-MS m/z(ESI) = **371.20** [M+1]

### Example 20

### 5-Methyl-6-((7-methyl-8-oxo-9-(tetrahydro-2H-pyran-4-yl)-8,9-dihydro-7H-purin-2-yl)amino)nicotinonitrile (compound 20)

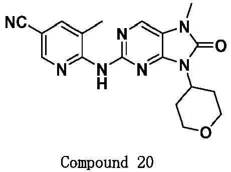

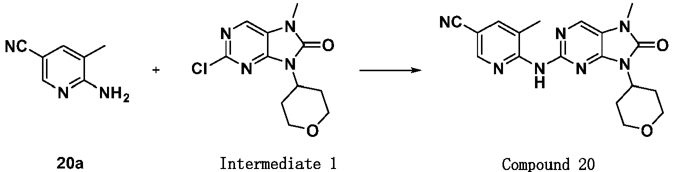

The title compound 5-methyl-6-((7-methyl-8-oxo-9-(tetrahydro-2*H*-pyran-4-yl)-8,9-dihydro-7*H*-purin-2-yl)amino)nicotinonitrile **compound 20** (white solid, 85 mg, 20.4% yield) was prepared according to the synthesis method of **compound** 1.

¹H NMR (400 MHz DMSO) δ 9.50 (s, 1H), 8.49 (d, 1H), 8.21 (s, 1H), 7.98 (d, 1H), 4.44-4.36 (m, 1H), 3.96 (dd, 2H), 3.41 (t, 2H), 3.34 (s, 3H), 2.56-2.45 (m, 2H), 2.24 (s, 3H), 1.65 (dd, 2H).

LC-MS m/z(ESI) = **366.20** [M+1]

### Example 21

### 2-((6-Methoxy-2-methylpyridin-3-yl)amino)-7-methyl-9-(tetrahydro-2H-pyran-4-yl)-7,9-dihydro-8H-purin-8-one (compound 21)

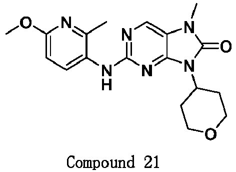

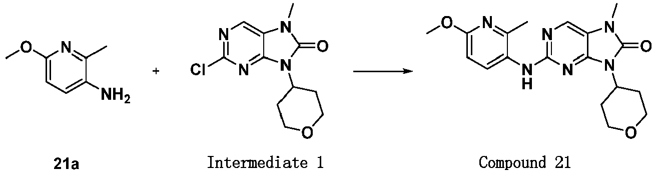

The title compound 2-((6-Methoxy-2-methylpyridin-3-yl)amino)-7-methyl-9-(tetrahydro-2*H*-pyran-4-yl)-7,9-dihydro-8*H*-purin-8-one **compound 21** (white solid, 100 mg, 62.17% yield) was prepared according to the synthesis method of **compound 1.**

¹H NMR (400 MHz DMSO) δ 8.50 (s, 1H), 7.97 (s, 1H), 7.65 (d, 1H), 6.61 (d, 1H), 4.41-4.32(m, 1H), 3.95 (dd, 2H), 3.81 (s, 3H), 3.39 (t, 2H), 3.27 (s, 3H), 2.51-2.45 (m, 2H), 2.31 (s, 3H), 1.63 (dd, 2H).

LC-MS m/z(ESI) = **371.20** [M+1]

### Example 22

### 5-Methyl-4-[(7-methyl-9-(oxa-4-yl)-8-oxo-8,9-dihydro-7H-purin-2-yl)amino]thiophene-2-carboxylate (compound 22)

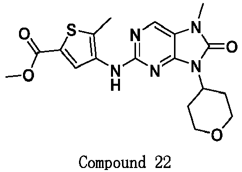

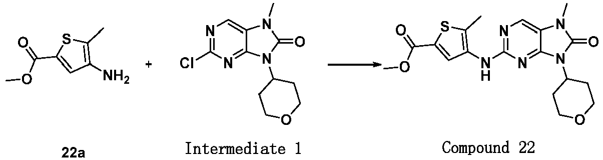

The title compound 5-methyl-4-[(7-methyl-9-(oxa-4-yl)-8-oxo-8,9-dihydro-7*H*-purin-2-yl)amino]thiophene-2-carboxylate **compound 22** (pale yellow solid, 50 mg, 11.09% yield) was prepared according to the synthesis method of **compound** 1.

¹H NMR (400 MHz, DMSO-d6) δ 8.90 (s, 1H), 8.08 (s, 1H), 8.05 (s, 1H), 4.40-4.35 (m, 1H), 3.96 (dd, 2H), 3.79 (s, 3H), 3.41 (t, 2H), 3.29 (s, 3H), 2.37 (s, 3H), 1.67-1.64 (m, 2H).

LC-MS m/z(ESI) = **404.10** [M+1]

### Example 23

### 7-Methyl-2-((4-methylpyrimidin-5-yl)amino)-9-(tetrahydro-2H-pyran-4-yl)-7,9-dihydro-8H-purin-8-one (compound 23)

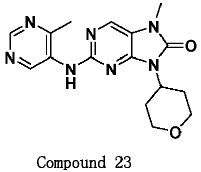

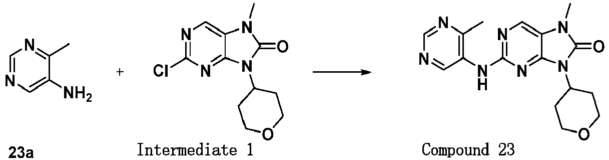

The title compound 7-methyl-2-((4-methylpyrimidin-5-yl)amino)-9-(tetrahydro-2*H-*pyran-4-yl)-7,9-dihydro-8*H*-purin-8-one **compound 23** (white solid, 50 mg, 21.5% yield) was prepared according to the synthesis method of **compound 1.**

¹H NMR (400 MHz DMSO) δ 8.92 (s, 1H), 8.90 (s, 1H), 8.73 (s, 1H), 8.0 (s, 1H), 4.44-4.36 (m, 1H), 3.96 (dd, 2H), 3.40 (t, 2H), 3.30 (s, 3H), 2.53-2.46 (m, 2H), 2.45 (s, 3H), 1.65 (dd, 2H).

LC-MS m/z(ESI) = **342.20** [M+1]

### Example 24

### 2-Fluoro-5-methyl-4-((7-methyl-8-oxo-9-(tetrahydro-2H-pyran-4-yl)-8,9-dihydro-7H-purin-2-yl)amino)benzamide (compound 24)

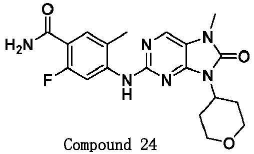

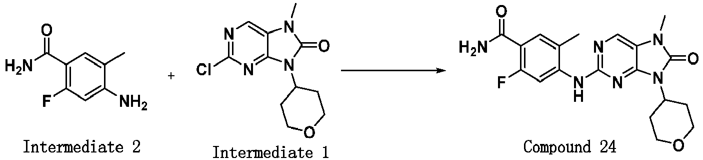

The title compound 2-fluoro-5-methyl-4-((7-methyl-8-oxo-9-(tetrahydro-2*H*-pyran-4-yl)-8,9-dihydro-7*H-*purin-2-yl)amino)benzamide **compound 24** (white solid, 9.5 mg, 8.6% yield) was prepared according to the synthesis method of **compound 1.**

¹H NMR (400 MHz DMSO) δ 8.54 (s, 1H), 8.18 (s, 1H), 7.89 (d, 1H), 7.54 (d, 1H), 7.46 (s, 1H), 7.39 (s, 1H), 4.48-4.40 (m, 1H), 3.98 (dd, 2H), 3.42 (t, 2H), 2.58-2.53 (m, 2H), 2.29 (s, 3H),1.67 (dd, 2H).

LC-MS m/z(ESI) = **401.20** [M+1]

### Example 25

### 3-(Methyl-d3)-4-((7-methyl-8-oxo-9-(tetrahydro-2H-pyran-4-yl)-8,9-dihydro-7H-purin-2-yl)amino)benzonitrile (compound 25)

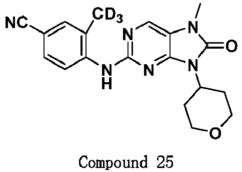

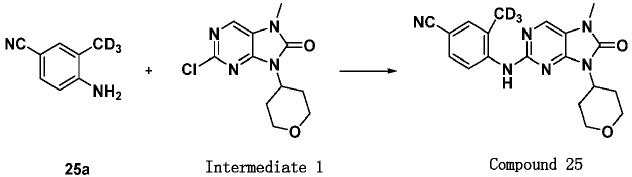

The title compound 3-(methyl-d3)-4-((7-methyl-8-oxo-9-(tetrahydro-2H-pyran-4-yl)-8,9-dihydro-7H-purin-2-yl)amino)benzonitrile **compound 25** (pale yellow solid, 40 mg, 29.4% yield) was prepared according to the synthesis method of **compound** 1.

¹H NMR (400 MHz, DMSO-d₆) δ 8.68 (s, 1H), 8.16 (s, 1H), 8.07 (d, 1H), 7.63 (d, 1H), 7.59-7.57 (m, 1H), 4.46-4.39 (m, 1H), 3.99-3.97 (m, 2H), 3.42 (t, 2H), 3.32 (s, 3H), 2.57-2.50 (m, 2H), 1.69-1.65 (m, 2H).

LC-MS m/z(ESI) = **368.20** [M+1]

### Example 26

### 3-Chloro-4-((7-methyl-8-oxo-9-(tetrahydro-2H-pyran-4-yl)-8,9-dihydro-7H-purin-2-yl)amino)benzamide (compound 26)

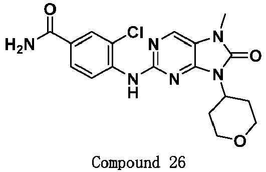

### Step 1:

### 4-Amino-3-chlorobenzamide (26b)

4-Amino-3-chlorobenzonitrile **26a** (2 g, 13 mmol) and potassium carbonate (271 mg, 1.9 mmol) were dissolved in 6 mL of dimethyl sulfoxide, and 1.78 mL of hydrogen peroxide was added in an ice bath. The reaction mixture was then gradually heated to 60 °C and stirred for 2 h. The reaction was completed as monitored by TLC. 10 mL of water was added to the reaction mixture, then a white solid was precipitated. The mixture was filtered, and the filter cake was collected and dried to obtain the title compound 4-amino-3-chlorobenzamide **26b** (white solid, 1.0 g, 48% yield).

¹H NMR (400 MHz DMSO) δ 7.75 (s, 1H), 7.57 (d, 1H), 7.03 (s, 1H), 7.60 (d, 1H), 5.87 (s, 2H).

LC-MS m/z(ESI) = **171.10** [M+1]

### Step 2:

### 3-Chloro-4-((7-methyl-8-oxo-9-(tetrahydro-2H-pyran-4-yl)-8,9-dihydro-7H-purin-2-yl)amino)benzamide (compound 26)

The title compound 3-chloro-4-((7-methyl-8-oxo-9-(tetrahydro-2H-pyran-4-yl)-8,9-dihydro-7H-purin-2-yl)amino)benzamide **compound 26** (white solid, 9.4 mg, 8.3% yield) was prepared according to the synthesis method of **compound 1.**

¹H NMR (400 MHz DMSO) δ 8.39 (s, 1H), 8.27 (d, 1H), 8.19 (s, 1H), 7.99 (d, 1H), 7.97 (s, 1H), 7.83 (dd, 1H), 7.36 (s, 1H), 4.44-4.34 (m, 1H), 3.98 (dd, 2H), 3.44 (t, 2H), 2.55-2.45 (m, 5H), 1.68(dd, 2H).

LC-MS m/z(ESI) = **403.10** [M+1]

### Example 27

### N,3-Dimethyl-4-((7-methyl-8-oxo-9-(tetrahydro-2H-pyran-4-yl)-8,9-dihydro-7H-purin-2-yl)amino)benzamide (compound 27)

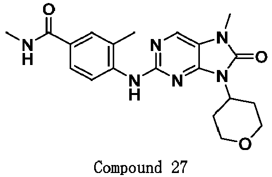

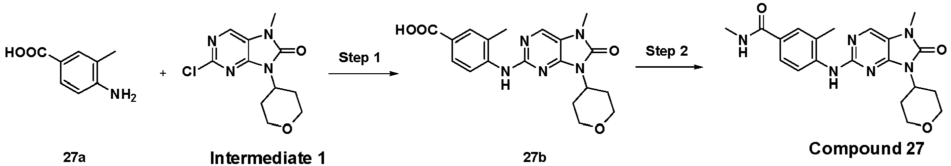

### Step 1:

### 3-Methyl-4-((7-methyl-8-oxo-9-(tetrahydro-2H-pyran-4-yl)-8,9-dihydro-7H-purin-2-yl)amino)benzoic acid (27b)

3-Methyl-4-aminobenzoic acid **27a** (2.0 g, 13.4 mmol), 2-chloro-7-methyl-9-(tetrahydro-2*H*-pyran-4-yl)-7,9-dihydro-8H-purin-8-one **intermediate 1** (3.0 g, 11.2 mmol), cesium carbonate (7.3 g, 22.4 mmol) and catalyst Brettphos-G3-Pd (CAS: 1470372-59-8) methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (1.0 g, 1.12 mmol) were added to a 50 mL flask, followed by addition of 30 mL of 1,4-dioxane. After being purged with nitrogen three times, the reaction system was heated to reflux for 3 h. The reaction was completed as monitored by TLC. The reaction mixture was diluted with dichloromethane and filtered through celite, and water was added to the filtrate. The resulting mixture was concentrated under reduced pressure to remove a small amount of organic solvent, and a large amounts of yellow solid was precipitated. The solid was collected by filtration and slurried with ethanol, and the slurry was filtered and dried by rotary evaporation to obtain the title compound 3-methyl-4-((7-methyl-8-oxo-9-(tetrahydro-2*H*-pyran-4-yl)-8,9-dihydro-7*H*-purin-2-yl)amino)benzoic acid **27b** (pale yellow solid, 2.15 g, 50% yield).

LC-MS m/z(ESI) = **384.20** [M+1]

### Step 2:

### N,3-Dimethyl-4-((7-methyl-8-oxo-9-(tetrahydro-2H-pyran-4-yl)-8,9-dihydro-7H-purin-2-yl)amino)benzamide (compound 27)

3-Methyl-4-((7-methyl-8-oxo-9-(tetrahydro-2*H*-pyran-4-yl)-8,9-dihydro-7*H-*purin-2-yl)amino)benzoic acid **27b** (250 mg, 0.65 mmol), 1-(3-dimethylaminopropyl)-3-ethylcarbodiimide hydrochloride (187.2 mg, 0.98 mmol), 1-hydroxybenzotriazole (132.4 mg, 0.98 mmol), and triethylamine (333 mg, 3.3 mmol) were dissolved in 2 mL of dichloromethane. The reaction mixture was heated to 40 °C and stirred for 1 h, and then methylamine hydrochloride (220 mg, 3.3 mmol) was added. The reaction system was stirred at 40 °C for 3 h. The reaction was completed as monitored by TLC. Water was added to the mixture and extracted three times with an appropriate amount of dichloromethane, and the organic phases were combined and dried over anhydrous sodium sulfate. The reaction mixture was filtered and concentrated to give a crude product, which was then slurried with methanol. The slurry was filtered, and the solid was dried under reduced pressure to obtain the title compound *N*,3-dimethyl-4-((7-methyl-8-oxo-9-(tetrahydro-2*H*-pyran-4-yl)-8,9-dihydro-7*H*-purin-2-yl)amino)benzamide **compound 27** (white solid, 21 mg, 8.1% yield).

¹H NMR (400 MHz DMSO) δ 8.48 (s, 1H), 8.27 (q, 1H), 8.10 (s, 1H), 7.82 (d, 1H), 7.67 (d, 1H), 7.62 (dd, 1H), 4.45-4.37 (m, 1H), 3.97 (dd, 2H), 3.42 (t, 2H), 3.31 (s, 3H), 2.76 (d, 3H), 2.57-2.46 (m, 2H), 2.30 (s, 3H), 1.66 (dd, 2H).

LC-MS m/z(ESI) = **397.20** [M+1]

### Example 28

### N,N,3-Trimethyl-4-((7-methyl-8-oxo-9-(tetrahydro-2H-pyran-4-yl)-8,9-dihydro-7H-purin-2-yl)amino)benzamide (compound 28)

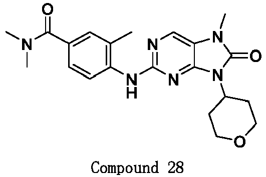

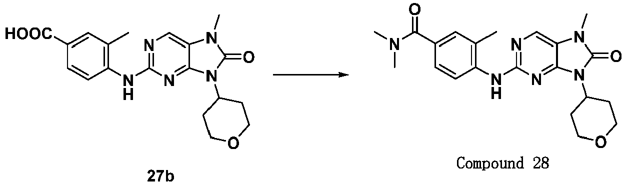

The title compound *N*, *N*,3-trimethyl-4-((7-methyl-8-oxo-9-(tetrahydro-2*H*-pyran-4-yl)-8,9-dihydro-7*H*-purin-2-yl)amino)benzamide **compound 28** (white solid, 65 mg, 24% yield) was prepared according to the synthesis method of **compound 27.**

¹H NMR (400 MHz DMSO) δ 8.49 (s, 1H), 8.07 (s, 1H), 7.72 (d, 1H), 7.24 (d, 1H), 7.19 (dd, 2H), 4.40-4.36 (m, 1H), 3.95 (dd, 2H), 3.43-3.37 (m, 2H), 3.30 (s, 3H), 2.96 (s, 6H), 2.55-2.45 (m, 2H), 2.27 (s, 3H), 1.65 (dd, 2H).

LC-MS m/z(ESI) = **411.20** [M+1]

### Example 29

### N-3-methyl-4-((7-methyl-8-oxo-9-(tetrahydro-2H-pyran-4-yl)-8,9-dihydro-7H-purin-2-yl)amino)benzamide (compound 29)

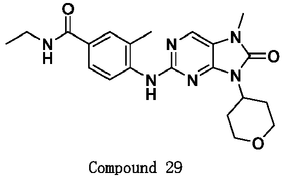

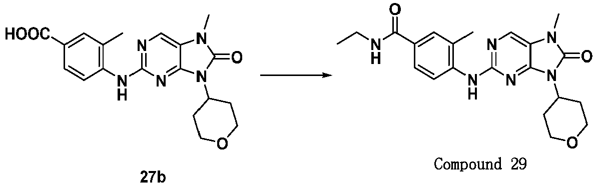

The title compound *N-*ethyl-3-methyl-4-((7-methyl-8-oxo-9-(tetrahydro-2*H*-pyran-4-yl)-8,9-dihydro-7*H*-purin-2-yl)amino)benzamide **compound 29** (white solid, 67 mg, 24% yield) was prepared according to the synthesis method of **compound 27.**

¹H NMR (400 MHz, DMSO-d6) δ 8.47(s, 1H), 8.29 (t, 1H), 8.10 (s, 1H), 7.83 (d, 1H), 7.68 (d, 1H), 7.63 (dd, 1H), 4.45-4.37 (m, 1H), 3.97 (dd, 2H), 3.45-3.38 (m, 2H), 3.33-3.23 (m, 5H), 2.57-2.46 (m, 2H), 2.30 (s, 3H), 1.68-1.64 (m, 2H), 1.11 (t, 3H). LC-MS m/z(ESI) = **411.20** [M+1]

### Example 30

### N-Isopropyl-3-methyl-4-((7-methyl-8-oxo-9-(tetrahydro-2H-pyran-4-yl)-8,9-dihydro-7H-purin-2-yl)amino)benzamide (compound 30)

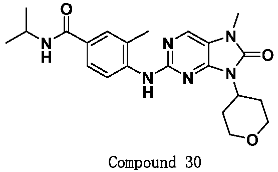

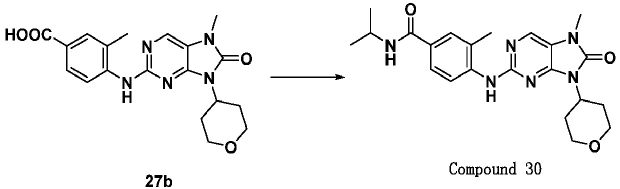

The title compound *N*-isopropyl-3-methyl-4-((7-methyl-8-oxo-9-(tetrahydro-2*H-*pyran-4-yl)-8,9-dihydro-7*H*-purin-2-yl)amino)benzamide **compound 30** (white solid, 67 mg, 23% yield) was prepared according to the synthesis method of **compound 27.** ¹H NMR (400 MHz, DMSO-d6) δ 8.47 (s, 1H), 8.10 (s, 1H), 8.03 (d, 1H), 7.82 (d, 1H), 7.69 (d, 1H), 7.63 (dd, 1H), 4.44-4.38 (m, 1H), 4.11-4.06 (m 1H), 3.97 (dd, 2H), 3.42 (t, 2H), 3.30 (s, 3H), 2.54-2.46 (m, 2H), 2.30 (s, 3H), 1.66 (dd, 2H), 1.16 (d, 6H). LC-MS m/z(ESI) = **425.20** [M+1]

### Example 31

### N-Cyclopropyl-3-methyl-4-((7-methyl-8-oxo-9-(tetrahydro-2H-pyran-4-yl)-8,9-dihydro-7H-purin-2-yl)amino)benzamide (compound 31)

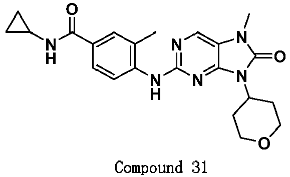

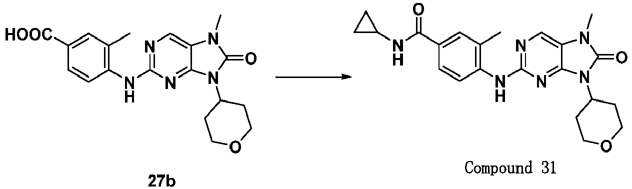

The title compound *N*-cyclopropyl-3-methyl-4-((7-methyl-8-oxo-9-(tetrahydro-2*H-*pyran-4-yl)-8,9-dihydro-7*H*-purin-2-yl)amino)benzamide **compound 31** (white solid, 135 mg, 46% yield) was prepared according to the synthesis method of **compound 27.** ¹H NMR (400 MHz, DMSO-d6) δ 8.46 (s, 1H), 8.30 (d, 1H), 8.10 (s, 1H), 7.81 (d, 1H), 7.68 (d, 1H), 7.62 (dd, 1H), 4.45-4.37 (m, 1H), 3.97 (dd, 2H), 3.42 (t, 2H), 3.31 (s, 3H), 2.86-2.80 (m, 1H), 2.57-2.46 (m, 2H), 2.30 (s, 3H), 1.67 (dd, 2H), 0.69-0.65 (m, 2H), 0.59-0.55 (m, 2H).

LC-MS m/z(ESI) = **423.20** [M+1]

### Example 32

### N-Isobutyl-3-methyl-4-((7-methyl-8-oxo-9-(tetrahydro-2H-pyran-4-yl)-8,9-dihydro-7H-purin-2-yl)amino)benzamide (compound 32)

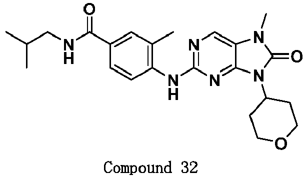

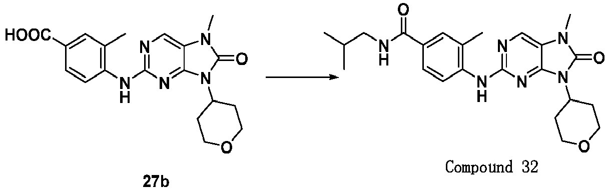

The title compound *N-*isobutyl-3-methyl-4-((7-methyl-8-oxo-9-(tetrahydro-2*H*-pyran-4-yl)-8,9-dihydro-7*H*-purin-2-yl)amino)benzamide **compound 32** (white solid, 21 mg, 8% yield) was prepared according to the synthesis method of **compound 27.**

¹H NMR (400 MHz, DMSO-d6) δ8.48 (s, 1H), 8.28 (t, 1H), 8.10 (s, 1H), 7.82 (d, 1H), 7.70 (d, 1H), 7.64 (dd, 1H), 4.45-4.37 (m, 1H), 4.97 (dd, 2H), 3.42 (q, 2H), 3.30 (s, 3H), 3.70 (dd, 2H), 2.57-2.47 (m, 2H), 2.30 (s, 3H), 1.87-1.80 (m, 1H), 1.67 (dd, 2H), 0.92-0.86 (m, 6H).

LC-MS m/z(ESI) = **439.30** [M+1]

### Example 33

### 7-Methyl-2-((2-methyl-4-(morpholine-4-carbonyl)phenyl)amino)-9-(tetrahydro-2H-pyran-4-yl)-7,9-dihydro-8H-purin-8-one (compound 33)

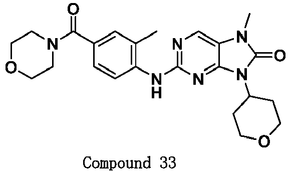

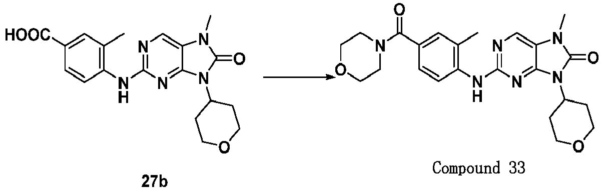

The title compound 7-methyl-2-((2-methyl-4-(morpholine-4-carbonyl)phenyl)amino)-9-(tetrahydro-2*H*-pyran-4-yl)-7,9-dihydro-8*H*-purin-8-one **compound 33** (white solid, 21 mg, 7.1% yield) was prepared according to the synthesis method of **compound 27.** ¹H NMR (400 MHz DMSO) δ 8.52 (s, 1H), 8.07 (s, 1H), 7.73 (d, 1H), 7.25 (d, 1H), 7.19 (dd, 1H), 4.42-4.36 (m, 1H), 3.94 (dd, 2H), 3.59-3.40 (m, 9H), 3.29 (s, 3H), 2.27 (s, 3H), 1.67-1.62 (m, 2H).

LC-MS m/z(ESI) = **453.20** [M+1]

### Example 34

### 2-((4-(3-Hydroxypyrrolidine-1-carbonyl)-2-methylphenyl)amino)-7-methyl-9-(tetrahydro-2H-pyran-4-yl)-7,9-dihydro-8H-purin-8-one (compound 34)

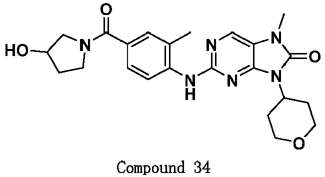

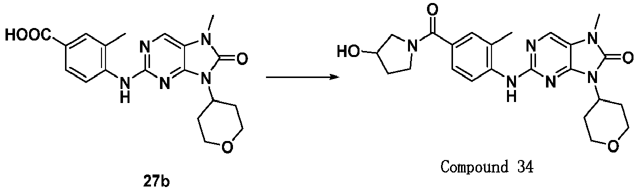

The title compound 2-((4-(3-hydroxypyrrolidine-1-carbonyl)-2-methylphenyl)amino)-7-methyl-9-(tetrahydro-2*H*-pyran-4-yl)-7,9-dihydro-8*H*-purin-8-one **compound 34** (white solid, 65 mg, 25% yield) was prepared according to the synthesis method of **compound 27.**

¹H NMR (400 MHz DMSO) δ 8.49 (s, 1H), 8.08 (s, 1H), 7.75 (d, 1H), 7.29-7.46 (m, 2H), 5.01-4.91 (m, 1H), 4.43-4.22 (m, 2H), 4.03-3.94 (m, 2H), 3.64-3.37 (m, 5H), 3.30 (s,3H), 2.55-2.49 (m, 2H), 2.28 (s, 3H), 2.05-1.79 (m, 2H), 1.65 (d, 2H).

LC-MS m/z(ESI) = **453.20** [M+1]

### Example 35

### 3-Methyl-N-(methyl-d3)-4-((7-methyl-8-oxo-9-(tetrahydro-2H-pyran-4-yl)-8,9-dihydro-7H-purin-2-yl)amino)benzamide (compound 35)

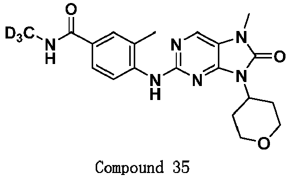

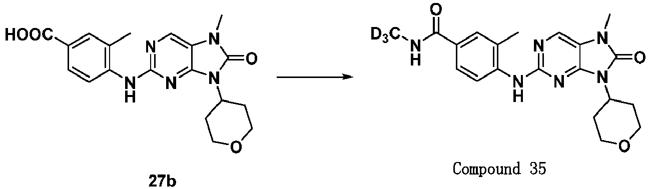

The title compound 3-methyl-N-(methyl-d3)-4-((7-methyl-8-oxo-9-(tetrahydro-2*H-*pyran-4-yl)-8,9-dihydro-7*H*-purin-2-yl)amino)benzamide **compound 35** (white solid, 65 mg, 22% yield) was prepared according to the synthesis method of **compound 27.** ¹H NMR (400 MHz DMSO) δ 8.48 (s, 1H), 8.24 (s, 1H), 8.01 (s, 1H), 7.82 (d, 1H), 7.67 (d, 1H), 7.62 (dd, 1H), 4.44-4.38 (m, 1H), 3.97 (dd, 2H), 3.44-3.38 (m, 2H), 3.30 (s,3H), 2.30 (s, 3H), 1.68-1.64 (m, 2H).

LC-MS m/z(ESI) = **400.20** [M+1]

### Example 36

### 2-Fluoro-N,5-dimethyl-4-((7-methyl-8-oxo-9-(tetrahydro-2H-pyran-4-yl)-8,9-dihydro-7H-purin-2-yl)amino)benzamide (compound 36)

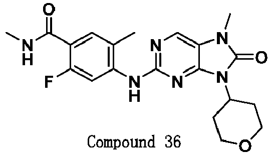

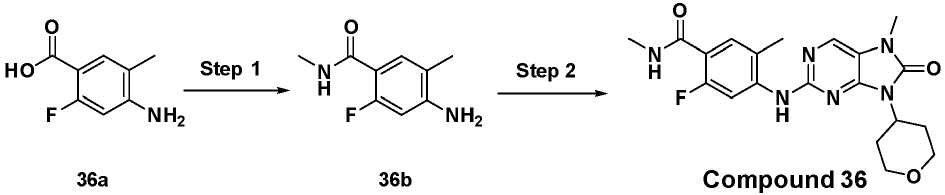

### Step 1:

### 4-Amino-2-fluoro-N,5-dimethylbenzamide (36b)

4-Amino-2-fluoro-5-methylbenzoic acid **36a** (200 mg, 1.18 mmol) was dissolved in thionyl chloride (10 mL). The mixture was heated to reflux for 1 h and concentrated under reduced pressure to evaporate the thionyl chloride, and 10 mL of toluene was added to remove excess thionyl chloride. The residue was dissolved in 10 mL of dichloromethane, and the resulting solution was added dropwise to a solution of potassium carbonate (820 mg, 5.9 mmol) and methylamine hydrochloride (399 mg, 5.90 mmol) in dichloromethane (10 mL). The reaction mixture was stirred at room temperature for 0.5 h. The reaction was monitored by TLC. After completion of the reaction, the reaction mixture was concentrated under reduced pressure to remove dichloromethane, and the residue was purified by column chromatography (dichloromethane/methanol (v/v) = 20/1) to obtain the title compound 4-amino-2-fluoro-N,5-dimethylbenzamide **36b** (pale yellow solid, 0.2 g, 92.84% yield).

¹H NMR (400 MHz, DMSO-d₆) δ 7.52 (t, 1H), 7.32 (d, 1H), 6.32(d, 1H), 5.65 (s, 2H), 2.72 (d, 3H), 2.01 (s, 3H).

LC-MS m/z(ESI) = **183.10** [M+1]

### Step 2:

### 2-Fluoro-N,5-dimethyl-4-((7-methyl-8-oxo-9-(tetrahydro-2H-pyran-4-yl)-8,9-dihydro-7H-purin-2-yl)amino)benzamide (compound 36)

The title compound 2-fluoro-*N*,5-dimethyl-4-((7-methyl-8-oxo-9-(tetrahydro-2*H-*pyran-4-yl)-8,9-dihydro-7*H*-purin-2-yl)amino)benzamide **compound 36** (white solid, 17 mg, 14.95% yield) was prepared according to the synthesis method of **compound 1.** ¹H NMR (401 MHz, DMSO-d₆) δ 8.51 (s, 1H), 8.17 (s, 1H), 7.95 (s, 1H), 7.89 (d, 1H), 7.49 (d, 1H), 4.43 (t, 1H), 3.98 (d, 2H), 3.32 (s, 3H), 2.76 (d, 3H), 2.29 (s, 3H), 1.68 (d, 2H).

LC-MS m/z(ESI) = **415.20** [M+1]

### Example 37

### 2-Fluoro-5-methyl-N-(methyl-d3)-4-((7-methyl-8-oxo-9-(tetrahydro-2H-pyran-4-yl)-8,9-dihydro-7H-purin-2-yl)amino)benzamide (compound 37)

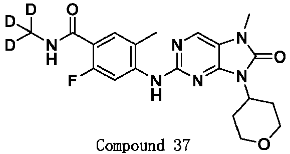

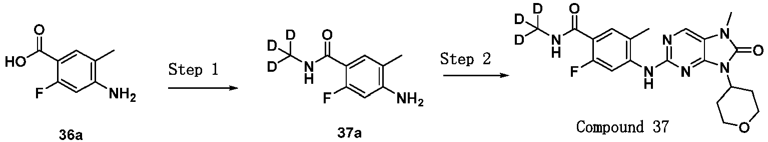

### Step 1:

### 4-Amino-2-fluoro-5-methyl-N-(methyl-d3)benzamide (37a)

4-Amino-2-fluoro-5-methylbenzoic acid **36a** (200 mg, 1.18 mmol) was dissolved in thionyl chloride (10 mL). The mixture was heated at reflux for 1 h and concentrated to evaporate the thionyl chloride, and 10 mL of toluene was added to remove excess thionyl chloride. The residue was dissolved in 10 mL of dichloromethane, and the resulting solution was added dropwise to a solution of potassium carbonate (820 mg, 5.9 mmol) and deuterated methylamine (200 mg, 5.90 mmol) in dichloromethane (10 mL). The reaction mixture was stirred at room temperature for 0.5 h. The reaction was monitored by TLC. After completion of the reaction, the reaction mixture was concentrated, and the residue was purified by column chromatography (dichloromethane/methanol (v/v) = 20/1) to obtain the title compound 4-amino-2-fluoro-5-methyl-*N*-(methyl-d3)benzamide **37a** (pale yellow solid, 0.2 g, 91.51% yield).

¹H NMR (400 MHz, DMSO-d₆) δ 7.54-7.46 (m, 1H), 7.32 (d, 1H), 6.32 (d, 1H), 5.65 (s, 2H), 2.01 (s, 3H).

LC-MS m/z(ESI) = **186.10** [M+1]

### Step 2:

### 2-Fluoro-5-methyl-N-(methyl-d3)-4-((7-methyl-8-oxo-9-(tetrahydro-2H-pyran-4-yl)-8,9-dihydro-7H-purin-2-yl)amino)benzamide (compound 37)

The title compound 2-fluoro-5-methyl-*N*-(methyl-d3)-4-((7-methyl-8-oxo-9-(tetrahydro-2*H*-pyran-4-yl)-8,9-dihydro-7*H*-purin-2-yl)amino)benzamide **compound** 37 (white solid, 15 mg, 13.31% yield) was prepared according to the synthesis method of **compound 1.**

¹H NMR (401 MHz, DMSO-d₆) δ 8.51 (s, 1H), 8.17 (s, 1H), 7.92-7.87 (m, 2H), 7.49 (d, 1H), 4.43 (t, 1H), 3.97 (d, 2H), 3.32 (s, 3H), 2.28 (s, 3H), 1.68 (d, 2H).

LC-MS m/z(ESI) = **418.10** [M+1]

### Example 38

### 2-((6-(Methoxy-d3)-4-methylpyridin-3-yl)amino)-7-methyl-9-(tetrahydro-2H-pyran-4-yl)-7,9-dihydro-8H-purin-8-one (compound 38)

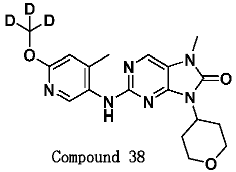

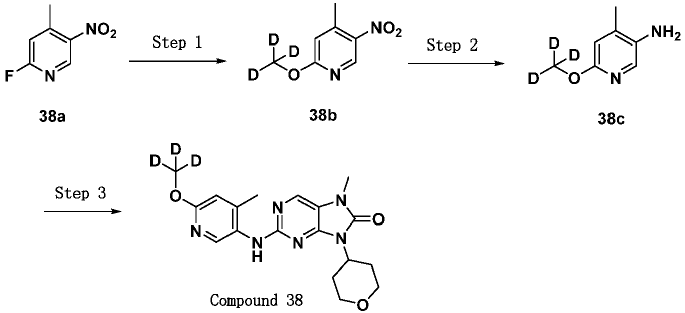

### Step 1:

### 2-(Methoxy-d3)-4-methyl-5-nitropyridine (38b)

2-Fluoro-4-methyl-5-nitropyridine **38a** (0.5 g, 3.20 mmol) was dissolved in acetonitrile (10 mL), and potassium carbonate (1.33 g, 9.60 mmol) and deuterated methanol (0.21 g, 6.4 mmol) were added. The reaction mixture was stirred at room temperature for 2 h. The reaction was monitored by TLC. After completion of the reaction, the reaction mixture was filtered, and the solid was washed with 10 mL of acetonitrile. The filtrates were combined and concentrated to get a crude product, which was then purified by column chromatography (petroleum ether/ethyl acetate (v/v) = 10/1) to give the title compound 2-(methoxy-d3)-4-methyl-5-nitropyridine **38b** (white solid, 0.4 g, 73.03% yield).

¹H NMR (400 MHz, DMSO-d₆) δ 8.91 (s, 1H), 6.96 (s, 1H), 2.55 (s, 3H).

LC-MS m/z(ESI) = **172.10** [M+1]

### Step 2:

### 6-(Methoxy-d3)-4-methylpyridin-3-amine (38c)

2-(Methoxy-d3)-4-methyl-5-nitropyridine **38b** (100 mg, 0.58 mmol) was dissolved in ethanol (20 mL), and iron powder (163.13 mg, 2.92 mmol) and acetic acid (20 µL) were added. The reaction mixture was heated to 90 °C and reacted for 1 h. The reaction was monitored by TLC. After completion of the reaction, the reaction mixture was filtered, and the solid was washed with 20 mL of ethanol and purified by column chromatography (petroleum ether/ethyl acetate (v/v) = 100/1) to get the title compound 6-(methoxy-d3)-4-methylpyridin-3-amine **38c** (yellow solid, 50 mg, 61% yield).

¹H NMR (400 MHz, DMSO-d₆) δ 7.47 (s, 1H), 6.45 (s, 1H), 4.51 (s, 2H), 2.05 (d, 3H). LC-MS m/z(ESI) = **142.10** [M+1]

### Step 3:

### 2-((6-(Methoxy-d3)-4-methylpyridin-3-yl)amino)-7-methyl-9-(tetrahydro-2H-pyran-4-yl)-7,9-dihydro-8H-purin-8-one (compound 38)

The title compound 2-((6-(methoxy-d3)-4-methylpyridin-3-yl)amino)-7-methyl-9-(tetrahydro-2*H*-pyran-4-yl)-7,9-dihydro-8*H*-purin-8-one **compound 38** (white solid, 30 mg, 22.73% yield) was prepared according to the synthesis method of **compound 1.** ¹HNMR (400 MHz, Chloroform-d) δ 8.37 (s, 1H), 7.77 (s, 1H), 6.65 (s, 1H), 4.55-4.45 (m, 1H), 4.10 (dd, 2H), 3.56-3.47 (m, 2H), 3.37 (s, 3H), 2.75-2.68 (m, 2H), 2.27 (s, 3H), 1.68 (dd, 2H).

LC-MS m/z(ESI) = **374.20** [M+1]

### Example 39

### 4-Methyl-N-(methyl-d3)-5-((7-methyl-8-oxo-9-(tetrahydro-2H-pyran-4-yl)-8,9-dihydro-7H-purin-2-yl)amino)picolinamide (compound 39)

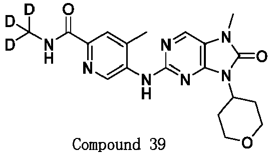

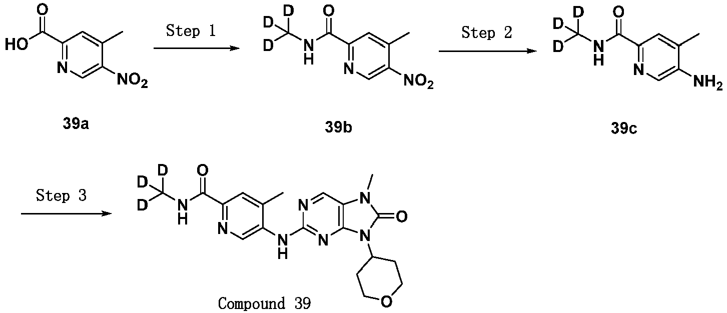

### Step 1:

### 4-Methyl-N-(methyl-d3)-5-nitropicolinamide (39b)

4-Methyl-5-nitropyridine carboxylic acid **39a** (430 mg, 2.3 mmol) and O-(7-azabenzotriazol-1-yl)-*N*,*N*,*N',N'-*tetramethylurea hexafluorophosphate (1.04 g, 2.7 mmol) were dissolved in dichloromethane (10 mL). The resulting solution was stirred at 0 °C for 20 min, and *N*,*N*-diisopropylethylamine (11 g, 8.2 mmol) and methane-d3-amine (500 mg, 7.08 mmol) were added. The reaction mixture was stirred at room temperature for 4 h. The reaction was monitored by TLC. After completion of the reaction, 10 mL of water was added to the reaction mixture, and the organic phase was separated, dried over anhydrous sodium sulfate and purified by preparative medium-pressure normal-phase chromatography to obtain the title compound 4-methyl-N-(methyl-d3)-5-nitropicolinamide **39b** (brown solid, 142 mg, 41% yield).

¹H NMR (400 MHz DMSO) δ 9.07 (s, 1H), 8.21 (s, 1H), 7.96 (s, 1H), 2.72 (s, 3H). LC-MS m/z(ESI) = **199.20** [M+1]

### Step 2:

### 5-Amino-4-methyl-N-(methyl-d3)picolinamide (39c)

4-Methyl-*N*-(methyl-d3)-5-nitronaphthamide **39b** (142 mg, 0.71 mmol) was dissolved in 5 mL of acetic acid, and zinc powder was added with stirring at 0 °C. The reaction mixture was gradually warmed to room temperature and stirred for 1 h. The reaction was monitored by TLC. After completion of the reaction, the reaction mixture was filtered and concentrated to obtain the title compound 5-amino-4-methyl-*N*-(methyl-d3)picolinamide **39c** (yellow solid, 80 mg, 60% yield).

LC-MS m/z(ESI) = **169.20** [M+1]

### Step 3:

### 4-Methyl-N-(methyl-d3)-5-((7-methyl-8-oxo-9-(tetrahydro-2H-pyran-4-yl)-8,9-dihydro-7H-purin-2-yl)amino)picolinamide (compound 39)

The title compound 4-methyl-*N*-(methyl-d3)-5-((7-methyl-8-oxo-9-(tetrahydro-2*H-*pyran-4-yl)-8,9-dihydro-7*H*-purin-2-yl)amino)picolinamide **compound 39** (white solid, 16 mg, 21.1% yield) was prepared according to the synthesis method of **compound 1.**

¹H NMR (400 MHz DMSO) δ 8.83 (s, 2H), 8.56 (s, 1H), 8.09 (s, 1H),7.87 (s, 1H), 4.44-4.38 (m, 1H), 3.97 (d, 2H), 3.44-3.34 (m, 2H), 3.31(s,3H), 2.33 (s, 3H), 1.67 (d, 2H). LC-MS m/z(ESI) = **401.20** [M+1]

### Example 40

### 7-Methyl-2-((2-methyl-4-(trifluoromethyl)phenyl)amino)-9-(tetrahydro-2H-pyran-4-yl)-7,9-dihydro-8H-purin-8-one (compound 40)

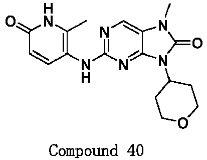

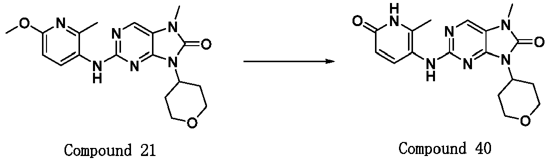

2-((6-Methoxy-2-methylpyridin-3-yl)amino)-7-methyl-9-(tetrahydro-2*H*-pyran-4-yl)-7,9-dihydro-8*H*-purin-8-one **compound 21** (400 mg, 1.08 mmol), lithium chloride (228.88 mg, 5.40 mmol) andp-toluenesulfonic acid (1.03 g, 5.40 mmol) were dissolved in DMF (10 mL), and the mixture was stirred at 120 °C for 1 h. The reaction was monitored by TLC. After completion of the reaction, the reaction mixture was poured into ice water, and the solid was collected and purified by silica gel column chromatography (dichloromethane/methanol (v/v) = 100/1) to obtain the title compound 7-methyl-2-((2-methyl-4-(trifluoromethyl)phenyl)amino)-9-(tetrahydro-2*H*-pyran-4-yl)-7,9-dihydro-8*H*-purin-8-one **compound 40** (white solid, 200 mg, 51.97 % yield).

¹H NMR (400 MHz DMSO) δ 11.53(s,1H), 8.25(s,1H), 7.95 (s,1H), 7.31 (d,1H), 6.12 (d, 1H), 4.38-4.31 (m, 1H), 3.97-3.93 (m, 2H), 3.39 (t, 2H), 3.25 (s, 3H), 2.57-2.42 (m, 2H), 2.07 (s, 3H), 1.64-1.60 (m, 2H).

LC-MS m/z(ESI) = **357.20** [M+1]

### Example 41

### 5-Methyl-4-[(7-methyl-9-(oxepan-4-yl)-8-oxo-8,9-dihydro-7H-purin-2-yl)amino]thiophene-2-carboxylic acid (compound 41)

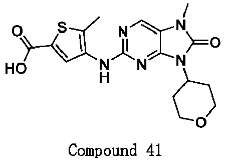

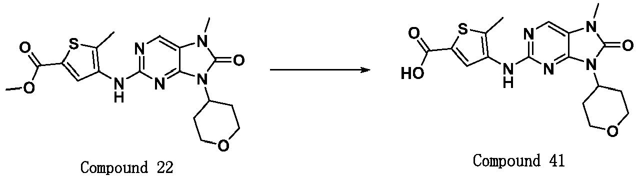

5-Methyl-4-[(7-methyl-9-(oxetan-4-yl)-8-oxo-8,9-dihydro-7*H*-purin-2-yl)amino]thiophene-2-carboxylate **compound 22** (0.78 g, 1.93 mmol) was dissolved in tetrahydrofuran (10 mL) and water (10 mL), and lithium hydroxide (0.09 g, 3.86 mmol) was added. The reaction mixture was stirred at room temperature for 1 h. The reaction was monitored by TLC. After completion of the reaction, the reaction mixture was concentrated to remove tetrahydrofuran and the pH was adjusted to about 5 with 6 N hydrochloric acid, and a white solid was precipitated. The reaction mixture was filtered, and the filter cake was washed twice with petroleum ether and collected to provide the title compound 5-methyl-4-[(7-methyl-9-(oxepan-4-yl)-8-oxo-8,9-dihydro-7H-purin-2-yl)amino]thiophene-2-carboxylic acid **compound 41** (brown solid, 0.4 g, 53.22% yield).

¹H NMR (400 MHz, DMSO-d₆) δ 12.80 (s, 1H), 8.85 (s, 1H), 8.06 (s, 1H), 7.93 (s, 1H), 4.42-4.34 (m, 1H), 3.96 (dd, 2H), 3.39 (t, 2H), 3.29 (s, 3H), 2.35 (s, 3H), 1.67-1.63 (m, 2H).

LC-MS m/z(ESI) = 390.10 [M+1]

### Example 42

### 5-Methyl-4-((7-methyl-8-oxo-9-(tetrahydro-2H-pyran-4-yl)-8,9-dihydro-7H-purin-2-yl)amino)thiophene-2-carboxamide (compound 42)

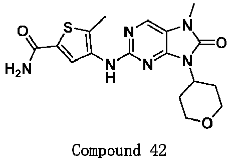

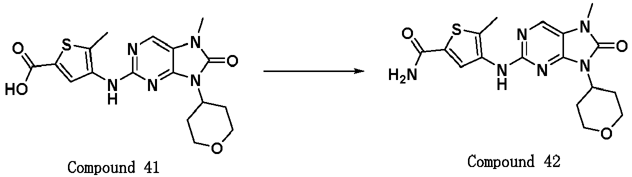

5-Methyl-4-[(7-methyl-9-(oxepan-4-yl)-8-oxo-8,9-dihydro-7*H*-purin-2-yl)amino]thiophene-2-carboxylic acid **compound 41** (0.3 g, 0.77 mmol) was dissolved in anhydrous dichloromethane (20 mL), and a catalytic amount of *N,N-*dimethylformamide was added. The reaction mixture was cooled to 0 °C and stirred for 5 min, and oxalyl dichloride (0.1 mL, 1.16 mmol) was added dropwise to the mixture. The reaction mixture was warmed to room temperature and stirred for 4 h. The reaction was monitored by TLC. After completion of the reaction, the reaction mixture was concentrated under reduced pressure, and the solid was collected and added to 11 mL of dichloromethane/ammonia (10:1) in an ice bath. The mixture was stirred at room temperature for 1 h and concentrated under reduced pressure to give a crude product, which was then purified by preparative medium pressure liquid chromatography to obtain the title compound 5-methyl-4-((7-methyl-8-oxo-9-(tetrahydro-2*H*-pyran-4-yl)-8,9-dihydro-7*H*-purin-2-yl)amino)thiophene-2-carboxamide **compound 42** (white solid, 140 mg, 46% yield).

¹H NMR (400 MHz, DMSO-d6) δ 8.73 (s, 1H), 8.03 (s, 1H), 7.83 (s, 1H), 7.73 (s, 1H), 7.26 (s, 1H), 4.42-4.34 (m, 1H), 3.95 (dd, 2H), 3.40 (t, 2H), 3.29 (s, 3H), 2.29 (s, 3H), 1.65 (dd, 2H).

LC-MS m/z(ESI) = **389.20** [M+1]

### Example 43

### 5-Methyl-4-((7-methyl-8-oxo-9-(tetrahydro-2H-pyran-4-yl)-8,9-dihydro-7H-purin-2-yl)amino)thiophene-2-carbonitrile (compound 43)

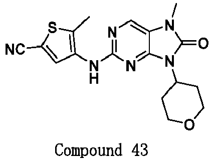

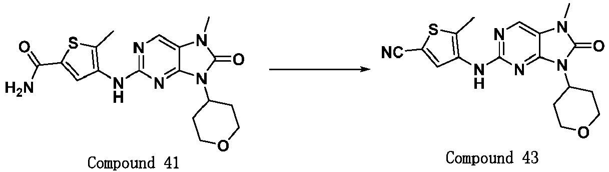

5-Methyl-4-[(7-methyl-9-(oxetan-4-yl)-8-oxo-8,9-dihydro-7*H*-purin-2-yl)amino]thiophene-2-carboxamide **compound 41** (80 mg, 0.21 mmol) was dissolved in dichloromethane (10 mL), and pyridine (70 mg, 0.84 mmol) and trifluoroacetic anhydride (130 mg, 0.63 mmol) were added with stirring at 0 °C. The reaction mixture was stirred for 0.5 h and monitored by TLC. After completion of the reaction, the reaction mixture was quenched with methanol (10 mL) and concentrated to give a crude product, which was then purified by preparative medium pressure liquid chromatography (30% water/acetonitrile) to obtain the title compound 5-methyl-4-[(7-methyl-9-(-4-yl)-8-oxo-8,9-dihydro-7*H*-purin-2-yl)amino]thiophene-2-carbonitrile **compound 43** (pale yellow solid, 20 mg, 25.71% yield).

¹H NMR (400 MHz, DMSO-d₆) δ 9.02 (s, 1H), 8.08 (s, 1H), 8.07 (s, 1H), 4.43-4.36 (m, 1H), 3.98 (dd, 2H), 3.44-3.38 (m, 2H), 3.29 (s, 3H), 2.38 (s, 3H), 1.67-1.64 (m, 2H). LC-MS m/z(ESI) = **371.10** [M+1]

### Example 44

### 2-Fluoro-5-(methyl-d3)-4-((7-methyl-8-oxo-9-(tetrahydro-2H-pyran-4-yl)-8,9-dihydro-7H-purin-2-yl)amino)benzamide (compound 44)

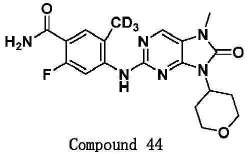

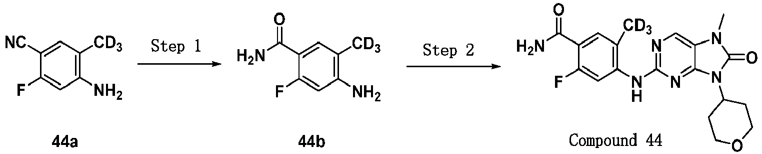

### Step 1:

### 4-Amino-2-fluoro-5-(methyl-d3)benzamide (44b)

4-Amino-2-fluoro-5-(methyl-d3)benzonitrile **44a** (678 mg, 4.72 mmol) was dissolved in DMSO (2 mL), and hydrogen peroxide (3 mL) was slowly added dropwise with stirring at room temperature. The reaction mixture was stirred for 2 h and monitored by TLC. After completion of the reaction,the reaction mixture was poured into purified water (15 mL), and a large amounts of solid was precipitated. The solid was collected by filtration and dried under reduced pressure to remove the solvent and thus to get the title compound 4-amino-2-fluoro-5-(methyl-d3)benzamide **44b** (yellow solid, 609 mg, 75% yield).

¹H NMR (400 MHz, DMSO-*d*₆) δ 7.38 (d, 1H), 7.14 (s, 1H), 6.95 (s, 1H), 6.33 (d, 1H), 5.68 (s, 2H).

LC-MS m/z(ESI) = **172.10** [M+1]

### Step 2:

### 2-Fluoro-5-(methyl-d3)-4-((7-methyl-8-oxo-9-(tetrahydro-2H-pyran-4-yl)-8,9-dihydro-7H-purin-2-yl)amino)benzamide (compound 44)

The title compound 2-fluoro-5-(methyl-d3)-4-((7-methyl-8-oxo-9-(tetrahydro-2*H-*pyran-4-yl)-8,9-dihydro-7*H*-purin-2-yl)amino)benzamide **compound 44** (pale yellow solid, 120 mg, 27% yield) was prepared according to the synthesis method of **compound 1.**

¹H NMR (400 MHz, DMSO-d₆) δ 8.52 (s, 1H), 8.19 (d, 1H), 7.90 (d, 1H), 7.55 (d, 1H), 7.42 (d, 2H), 4.47-4.39 (m, 1H), 4.00-3.96 (m, 2H), 3.44-3.35 (m 2H), 3.33 (s, 3H), 2.57-2.55(m, 2H), 1.71-1.67 (m, 2H).

LC-MS m/z(ESI) = **404.20** [M+1]

### Example 45

### 2-Cyano-5-methyl-4-((7-methyl-8-oxo-9-(tetrahydro-2H-pyran-4-yl)-8,9-dihydro-7H-purin-2-yl)amino)benzoate (compound 45)

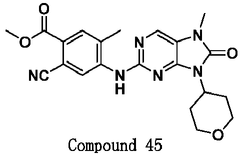

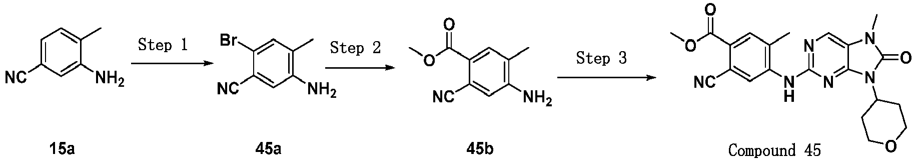

### Step 1:

### 5-Amino-2-bromo-4-methylbenzonitrile (45a)

5-Amino-4-methylbenzonitrile 15a (2.5 g, 18.9 mmol) was dissolved in DMF (15 mL), and NBS (3.37 g, 18.9 mmol) was added. The reaction mixture was stirred at room temperature for 0.5 h and monitored by TLC. After completion of the reaction, the reaction mixture was poured into purified water (100 mL), and a large amounts of solid was precipitated. The solid was collected by filtration and dried under reduced pressure to obtain the title compound 5-amino-2-bromo-4-methylbenzonitrile **45a** (yellow solid, 3.5 g, 87.5% yield).

LC-MS m/z(ESI) = **210.90** [M+1]

### Step 2:

### Methyl 4-amino-2-cyano-5-methylbenzoate (45b)

5-Amino-2-bromo-4-methylbenzonitrile **45a** (3.5 g, 16.6 mmol) was dissolved in toluene (15 mL) and methanol (15 mL), and 4-dimethylaminopyridine (2.0 g, 16.6 mmol), palladium(II) acetate (186 mg, 0.83 mmol) and (9,9-Dimethyl-9H-xanthene-4,5-diyl)bis(diphenylphosphane) (961 mg, 1.66 mmol) were added. The system was purged 4 times with nitrogen. Dicobalt octacarbonate (2.84 g, 8.3 mmol) was rapidly poured into the reaction mixture, and the resulting mixture was refluxed at 100 °C for 4 h. The reaction was monitored by TLC. After completion of the reaction, the reaction mixture was purified by column chromatography to obtain the title compound methyl 4-amino-2-cyano-5-methylbenzoate **45b** (yellow solid, 500 mg, 15.9% yield).

LC-MS m/z(ESI) = **191.10** [M+1]

### Step 3:

### 2-Cyano-5-methyl-4-((7-methyl-8-oxo-9-(tetrahydro-2H-pyran-4-yl)-8,9-dihydro-7H-purin-2-yl)amino)benzoate (compound 45)

The title compound 2-cyano-5-methyl-4-((7-methyl-8-oxo-9-(tetrahydro-2*H*-pyran-4-yl)-8,9-dihydro-7*H*-purin-2-yl)amino)benzoate **compound 45** (pale yellow solid, 502 mg, 45% yield) was prepared according to the synthesis method of **compound 1.**

¹H NMR (400 MHz DMSO) δ 8.92 (s, 1H), 8.55 (s, 1H), 8.25 (s, 1H), 7.94 (s, 1H), 4.45-4.38 (m, 1H), 4.00-3.95 (m, 2H), 3.88 (s, 3H), 3.47-3.42 (m, 2H), 3.33 (s, 3H), 2.67-2.50 (m, 2H), 2.43 (s, 3H), 1.72-1.67 (m, 2H).

LC-MS m/z(ESI) = **423.20** [M+1]

### Example 46

### 2-Cyano-5-methyl-4-((7-methyl-8-oxo-9-(tetrahydro-2H-pyran-4-yl)-8,9-dihydro-7H-purin-2-yl)amino)benzoic acid (compound 46)

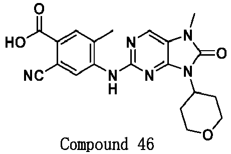

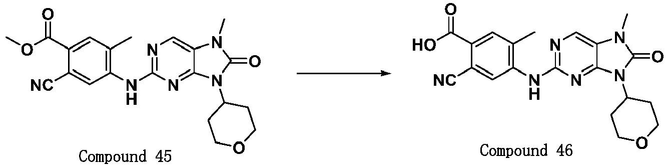

2-Cyano-5-methyl-4-((7-methyl-8-oxo-9-(tetrahydro-2*H*-pyran-4-yl)-8,9-dihydro-7*H-*purin-2-yl)amino)benzoate **compound 45** (200 mg, 0.47 mmol) was added to a mixture of tetrahydrofuran (2 mL) and water (2 mL), and lithium hydroxide monohydrate (94 mg, 2.35 mmol) was added. The resulting mixture was stirred at room temperature overnight. The reaction was monitored by TLC. After completion of the reaction, the pH of the reaction mixture was adjusted to about 5 with concentrated hydrochloric acid in an ice bath and concentrated under reduced pressure to remove the solvent, and the residue was purified by column chromatography to obtain the title compound 2-cyano-5-methyl-4-((7-methyl-8-oxo-9-(tetrahydro-2*H*-pyran-4-yl)-8,9-dihydro-7*H*-purin-2-yl)amino)benzoic acid **compound 46** (pale yellow solid, 90 mg, 47% yield).

¹H NMR (400 MHz, DMSO-d6) δ 13.39 (s, 1H), 8.88 (s, 1H), 8.50 (s, 1H), 8.24 (d, 1H), 7.93 (s, 1H), 4.48-4.40 (m, , 1H), 4.01-3.97 (m, 2H), 3.48-3.35 (m, 2H), 3.34 (s, 3H), 2.57-2.55 (m, 2H), 2.42 (s, 3H), 1.73-1.68 (m, 2H).

LC-MS m/z(ESI) = **409.20** [M+1]

### Example 47

### 2-Cyano-5-methyl-4-((7-methyl-8-oxo-9-(tetrahydro-2H-pyran-4-yl)-8,9-dihydro-7H-purin-2-yl)amino)benzamide (compound 47)

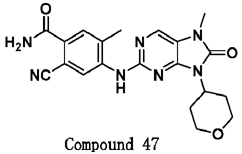

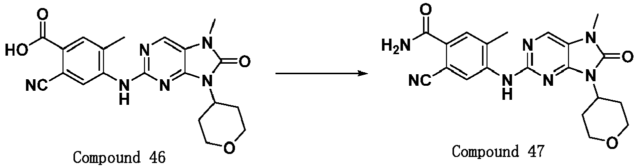

2-Cyano-5-methyl-4-((7-methyl-8-oxo-9-(tetrahydro-2*H*-pyran-4-yl)-8,9-dihydro-7*H-*purin-2-yl)amino)benzoic acid **compound 46** (75 mg, 0.183 mmol) was added to 2 mL of tetrahydrofuran, then HATU (104 mg, 0.275 mmol) and triethylamine (28 mg, 0.275 mmol) were added. The reaction mixture was stirred at room temperature for 0.5 h, and then 2 mL of ammonia in 1,4-dioxane solution (0.5 M in 1,4-dioxane) was slowly added dropwise. The reaction was monitored by TLC. After completion of the reaction, the reaction mixture was concentrated, and the residue was purified by column chromatography to obtain the title compound 2-cyano-5-methyl-4-((7-methyl-8-oxo-9-(tetrahydro-2*H*-pyran-4-yl)-8,9-dihydro-7*H*-purin-2-yl)amino)benzamide **compound 47** (pale yellow solid, 42 mg, 56% yield).

¹H NMR (400 MHz, DMSO-d6) δ 8.80 (s, 1H), 8.34 (s, 1H), 8.20 (s, 1H), 8.00 (s, 1H), 7.72 (s, 1H), 7.53 (s, 1H), 4.47-4.39 (m, 1H), 4.01-3.96 (m, 2H), 3.49-3.38 (m, 2H), 3.32 (s, 3H), 2.52-2.50(m, 2H), 2.39 (s, 3H), 1.72-1.67 (m, 2H).

LC-MS m/z(ESI) = **408.20** [M+1]

### Example 48

### 5-Methyl-4-((7-methyl-8-oxo-9-(tetrahydro-2H-pyran-4-yl)-8,9-dihydro-7H-purin-2-yl)amino)-2-(trifluoromethyl)benzamide (compound 48)

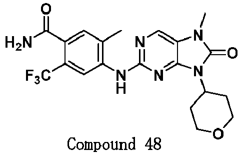

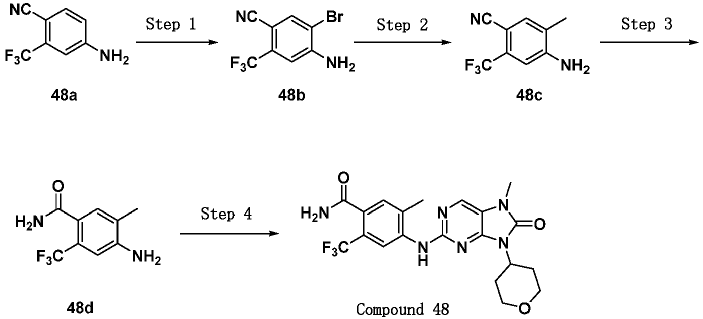

### Step 1:

### 4-Amino-5-bromo-2-(trifluoromethyl)benzonitrile (48b)

4-Amino-2-trifluoromethylbenzonitrile **48a** (1.1 g, 5.9 mmol) was dissolved in DMF (10 mL), and NBS (1.05 g, 5.9 mmol) was added. The reaction mixture was stirred at room temperature for 0.5 h and monitored by TLC. The reaction mixture was poured into purified water (100 mL), and a large amounts of solid was precipitated. The solid was collected by filtration and dried under reduced pressure to obtain the title compound 4-amino-5-bromo-2-(trifluoromethyl)benzonitrile **48b** (yellow solid, 1.5 g, 96.1% yield).

¹H NMR (400 MHz, DMSO-d6) δ8.14 (s, 1H), 7.20(s, 1H), 6.90(s, 2H).

LC-MS m/z(ESI) = **264.90** [M+1]

### Step 2:

### 4-Amino-5-methyl-2-(trifluoromethyl)benzonitrile (48c)

4-Amino-5-bromo-2-(trifluoromethyl)benzonitrile **48b** (1.5 g, 5.66 mmol) was added to a mixture of 1,4-dioxane (20 mL) and water (4 mL), and methylboronic acid (3.4 g, 56.6 mmol), potassium carbonate (2.3 g, 17 mmol) and [1,1'-bis(diphenylphosphino)ferrocene]dichloropalladium (417 mg, 0.57 mmol) were added. The system was purged 4 times with nitrogen, and the reaction mixture was refluxed for 5 h. The reaction was monitored by TLC. After completion of the reaction, the reaction mixture was purified by column chromatography to obtain the title compound 4-amino-5-methyl-2-(trifluoromethyl)benzonitrile **48c** (yellow solid, 850 mg, 75.2% yield).

LC-MS m/z(ESI) = **201.20** [M+1]

### Step 3:

### 4-Amino-5-methyl-2-(trifluoromethyl)benzamide (48d)

4-Amino-2-trifluoromethyl-5-methyl-benzonitrile **48c** (850 mg, 4.25 mmol) was dissolved in DMSO (2 mL), and potassium carbonate (117 mg, 0.85 mmol) was added, followed by the slow dropwise addition of hydrogen peroxide (3 mL) at room temperature. The reaction mixture was stirred for 2 h. The reaction was monitored by TLC. After completion of the reaction,the reaction mixture was poured into purified water (15 mL), and a large amounts of solid was precipitated. The solid was collected by filtration and dried under reduced pressure to yield the title compound 4-amino-5-methyl-2-(trifluoromethyl)benzamide **48d** (yellow solid, 312 mg, 33.6% yield).

LC-MS m/z(ESI) = **219.20** [M+1]

### Step 4:

### 5-Methyl-4-((7-methyl-8-oxo-9-(tetrahydro-2H-pyran-4-yl)-8,9-dihydro-7H-purin-2-yl)amino)-2-(trifluoromethyl)benzamide (compound 48)

The title compound 5-methyl-4-((7-methyl-8-oxo-9-(tetrahydro-2*H*-pyran-4-yl)-8,9-dihydro-7*H*-purin-2-yl)amino)-2-(trifluoromethyl)benzamide **compound 48** (pale yellow solid, 25 mg, 8.0% yield) was prepared according to the synthesis method of **compound 1.**

¹H NMR (400 MHz, DMSO-d6) δ 8.71 (s, 1H), 8.18 (s, 1H), 8.16 (s, 1H),7.83 (s, 1H), 7.44 (s, 1H), 7.38 (s, 1H), 4.44-4.36 (m, 1H), 3.97-3.93 (m, 2H), 3.50-3.35 (m, 2H), 3.32 (s, 3H), 2.47-2.43(m, 2H), 2.36 (s, 3H), 1.75-1.59 (m, 2H).

LC-MS m/z (ESI) = **451.20** [M+1]

### Example 49

### 2-Fluoro-4-((9-(3-hydroxy-3-methylcyclobutyl)-7-methyl-8-oxo-8,9-dihydro-7H-purin-2-yl)amino)-5-methylbenzamide (compound 49)

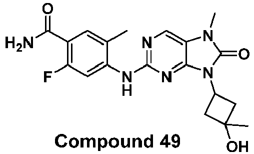

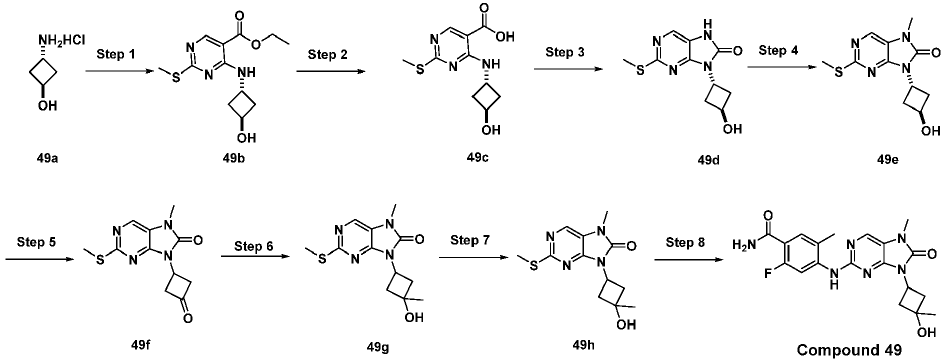

### Step 1:

### Ethyl 4-((trans-3-hydroxycyclobutyl)amino)-2-(methylthio)pyrimidine-5-carboxylate (49b)

*trans*-3-Aminocyclobutane-1-ol hydrochloride **49a** (15.00 g, 303.45 mmol) was dissolved in acetonitrile (200 mL), and potassium carbonate (41.92 g, 303.45 mmol) and ethyl 4-chloro-2-methylthio-5-pyrimidinecarboxylate (28.24 g, 121.38 mmol) were added with stirring at 0 °C. The reaction mixture was warmed to room temperature and stirred for 20 h. The reaction monitored by TLC. After completion of the reaction, a large amount of water was added to the reaction mixture, and a white solid was precipitated. The reaction mixture was filtered through celite, and the filtrate was concentrated to get the title compound ethyl 4-((*trans*-3-hydroxycyclobutyl)amino)-2-(methylthio)pyrimidine-5-carboxylate **49b** (white solid, 30 g, 87.23% yield).

¹H NMR (400 MHz, DMSO-d6) δ 8.54 (s, 1H), 8.29 (d, 1H), 5.14(s, 1H),4.60-4.55 (m, 1H), 4.32-4.25 (m, 3H), 2.47 (s, 3H), 2.24 (t, 4H), 1.30 (t, 3H).

LC-MS m/z(ESI) = 284.10 [M+1]

### Step 2:

### 4-((trans-3-Hydroxycyclobutyl)amino)-2-(methylthio)pyrimidine-5-carboxylic acid (49c)

Ethyl 4-((*trans*-3-hydroxycyclobutyl)amino)-2-(methylthio)pyrimidine-5-carboxylate **49b** (30 g, 105.88 mmol) was dissolved in methanol/water (150 mL/150 mL), and sodium hydroxide (6.4 g, 158.82 mmol) was added. The reaction mixture was stirred at room temperature for 12 h. The reaction was monitored by TLC. After completion of the reaction, the reaction mixture was concentrated to evaporate methanol and the pH was adjusted to about 4 with 2 N HCl, and a white solid was precipitated. The reaction mixture was filtered, and the filter cake was washed twice with water and petroleum ether/ethyl acetate (v/v = 10/1) to obtain the title compound 4-((*trans*-3-hydroxycyclobutyl)amino)-2-(methylthio)pyrimidine-5-carboxylic acid **49c** (white solid, 25 g, 92.49% yield).

¹H NMR (400 MHz, DMSO-d6) δ 13.28 (s, 1H), 8.51 (s, 1H), 8.47 (d, 1H), 5.11 (s, 1H), 4.55-4.61 (m,1H), 4.23-4.33 (m, 1H), 2.47 (s, 3H), 2.20-2.26 (m, 4H).

LC-MS m/z(ESI) = **256.10** [M+1]

### Step 3:

### 9-(trans-3-Hydroxycyclobutyl)-2-(methylthio)-7,9-dihydro-8H-purin-8-one (49d)

4-((trans-3-Hydroxycyclobutyl)amino)-2-(methylthio)pyrimidine-5-carboxylic acid 49c (25 g, 97.93 mmol) was dissolved in N*,N*-dimethylacetamide (250 mL), and triethylamine (11.89 g, 117.51 mmol) and diphenylphosphoryl azide (32.34 g, 117.51 mmol) were added with stirring at room temperature. The reaction mixture was stirred for 1 h, heated to 90 °C and refluxed for 5 h. The reaction was monitored by TLC. After completion of the reaction, water was added to the reaction mixture, and a large amounts of solid was precipitated. The solid was collected by filtration and dried to obtain the title compound 9-(*trans*-3-hydroxycyclobutyl)-2-(methylthio)-7,9-dihydro-8*H*-purin-8-one **49d** (white solid, 20 g, 80.95% yield).

¹H NMR (400 MHz, DMSO-d6) δ 11.30 (s, 1H), 8.10 (s, 1H), 5.18 (s, 1H), 5.07-4.99 (m, 1H), 4.56-4.53 (m, 1H), 3.07-3.00 (m, 2H), 2.51(s, 3H), 2.27-2.21 (m, 2H). LC-MS m/z(ESI) = 253.10 [M+1]

### Step 4:

### 9-(trans-3-Hydroxycyclobutyl)-7-methyl-2-(methylthio)-7,9-dihydro-8H-purin-8-one (49e)

9-(*trans*-3-Hydroxycyclobutyl)-2-(methylthio)-7,9-dihydro-8H-purin-8-one 49d (18 g, 71.35 mmol) was dissolved in *N*,*N*-dimethylformamide (300 mL), and dimethyl sulfate (9 g, 71.35 mmol) and cesium carbonate (23.25 g, 71.35 mmol) were added with stirring at 0 °C. The reaction mixture was stirred for 1 h. The reaction was monitored by TLC. After completion of the reaction,the reaction mixture was slowly added dropwise into ice water with stirring and extracted with ethyl acetate. The organic layer was concentrated and recrystallized from methanol to obtain the title compound 9-(*trans*-3-hydroxycyclobutyl)-7-methyl-2-(methylthio)-7,9-dihydro-8*H*-purin-8-one **49e** (white solid, 16 g, 84.21% yield).

¹H NMR (400 MHz, DMSO-d6) δ 8.29 (s, 1H), 5.18 (d, 1H), 5.09-5.01 (m, 1H), 4.56-4.52 (m, 1H), 3.31 (s, 3H), 3.06-2.97 (m, 2H), 2.51 (s, 3H),2.27-2.21 (m, 2H). LC-MS m/z(ESI) = 267.10 [M+1]

### Step 5:

### 7-Methyl-2-(methylthio)-9-(3-oxocyclobutyl)-7,9-dihydro-8H-purin-8-one (49f)

9-(*trans*-3-Hydroxycyclobutyl)-7-methyl-2-(methylthio)-7,9-dihydro-8*H*-purin-8-one 49e (2.0 g, 7.51 mmol) was dissolved in dichloromethane (10 mL), Dess-Martin periodinane (4.78 g, 11.26 mmol) was added with stirring at room temperature. The reaction mixture was stirred for 4 h. The reaction was monitored by TLC. After completion of the reaction, the reaction mixture was added with saturated sodium bicarbonate and extracted with dichloromethane. The organic layer was concentrated and a solid was precipitated. The reaction mixture was filtered and crystallized to obtain the title compound 7-methyl-2-(methylthio)-9-(3-oxocyclobutyl)-7,9-dihydro-8*H-*purin-8-one **49f** (white solid, 1.58 g, 79.6% yield).

LC-MS m/z (ESI) = 253.10 [M+1]

### Step 6:

### 9-(3-Hydroxy-3-methylcyclobutyl)-7-methyl-2-(methylthio)-7,9-dihydro-8H-purin-8-one (49g)

7-Methyl-2-(methylthio)-9-(3-oxocyclobutyl)-7,9-dihydro-8*H*-purin-8-one 49f (1.07 g, 4.05 mmol) was dissolved in tetrahydrofuran (10 mL), and 3 M methylmagnesium bromide (4.7 mL, 14.17 mmol) was slowly added with stirring at 0 °C. The reaction mixture was stirredfor 2.5 h. The reaction was monitored by TLC. After completion of the reaction,the reaction mixture was quenched with saturated aqueous ammonium chloride solution, and extracted with dichloromethane. The organic layer was concentrated, and the residue was purified by silica gel column chromatography (dichloromethane/methanol (v/v) = 120:1) to obtain the title compound 9-(3-hydroxy-3-methylcyclobutyl)-7-methyl-2-(methylthio)-7,9-dihydro-8*H*-purin-8-one **49g** (white solid, 820 mg, 72.25% yield).

1H NMR (600 MHz, DMSO-d6) δ 8.29 (s, 1H), 5.14 (s, 1H), 4.40-4.32 (m, 1H), 3.31 (s, 3H), 3.02-2.96 (m, 2H), 2.53 (s, 3H), 2.35-2.30 (m, 2H), 1.33 (s, 3H).

LC-MS m/z(ESI) = 281.10 [M+1]

### Step 7:

### 9-(3-Hydroxy-3-methylcyclobutyl)-7-methyl-2-(methylsulfinyl)-7,9-dihydro-8H-purin-8-one (49h)

9-(3-Hydroxy-3-methylcyclobutyl)-7-methyl-2-(methylthio)-7,9-dihydro-8*H*-purin-8-one **49g** (710 mg, 2.53 mmol) was dissolved in dichloromethane (5 mL), and *m-*chloroperoxybenzoic acid (568 mg, 3.29 mmol) was added with stirring at room temperature. The reaction mixture was stirred for 3 h. The reaction was monitored by TLC. After completion of the reaction, saturated sodium bicarbonate was added and extracted with dichloromethane. The organic layer was concentrated, and the residue was purified by silica gel column chromatography (dichloromethane/methanol (v/v) = 20:1) to obtain the title compound 9-(3-hydroxy-3-methylcyclobutyl)-7-methyl-2-(methylsulfinyl)-7,9-dihydro-8*H*-purin-8-one **49h** (white solid, 200 mg, 26.65% yield). LC-MS m/z(ESI) = 297.10 [M+1]

### Step 8:

### 2-Fluoro-4-((9-(3-hydroxy-3-methylcyclobutyl)-7-methyl-8-oxo-8,9-dihydro-7H-purin-2-yl)amino)-5-methylbenzamide (compound 49)

9-(3-Hydroxy-3-methylcyclobutyl)-7-methyl-2-(methylsulfinyl)-7,9-dihydro-8*H-*purin-8-one **49h** (100 mg, 0.34 mmol) and 4-amino-3-methyl-2-fluorobenzamide intermediate 2 (284 mg, 1.69 mmol) were dissolved in *N*,*N*-dimethylformamide (3 mL), and cesium carbonate (330 mg, 1.01 mmol) was added. The reaction mixture was gradually heated to 80 °C and stirred for 3 h. The reaction was monitored by TLC. After completion of the reaction, the reaction mixture was poured into 30 mL of purified water, and extracted with ethyl acetate. After filtrataion, the resultant purified by silica gel column chromatography purification (dichloromethane/methanol (v/v) = 35/1) to obtain the title compound 2-fluoro-4-((9-(3-hydroxy-3-methylcyclobutyl)-7-methyl-8-oxo-8,9-dihydro-7*H*-purin-2-yl)amino)-5-methylbenzamide **compound 49-1** (white solid, 12 mg, 8.88% yield) and **compound 49-2** (white solid, 20 mg, 14.80% yield).

### Compound 49-1:

¹H NMR (600 MHz, DMSO-d6) δ 8.60 (s, 1H), 8.21 (s, 1H), 7.87 (d, 1H), 7.55 (d, 1H), 7.46 (s, 1H), 7.42 (s, 1H), 5.13 (s, 1H), 4.41-4.33 (m, 1H), 3.35 (s, 3H), 3.07-2.98 (m, 2H), 2.53 (s, 3H), 2.35-2.30 (m, 2H), 1.36 (s, 3H).

LC-MS m/z(ESI) = 401.20 [M+1]

### Compound 49-2:

¹H NMR (600 MHz, DMSO-d6) δ 8.59 (s, 1H), 8.20 (s, 1H), 7.87 (d, 1H), 7.55 (d, 1H), 7.46 (s, 1H), 7.42 (s, 1H), 5.14 (s, 1H), 4.39-4.31 (m, 1H), 3.32 (s, 3H), 3.05-2.97 (m, 2H), 2.54 (s, 3H), 2.33-2.29 (m, 2H), 1.35 (s, 3H).

LC-MS m/z(ESI) = 401.20 [M+1]

### Example 50

### 2-Hydroxy-5-methyl-4-((7-methyl-8-oxo-9-(tetrahydro-2H-pyran-4-yl)-8,9-dihydro-7H-purin-2-yl)amino)benzamide (compound 50)

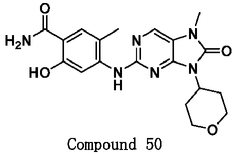

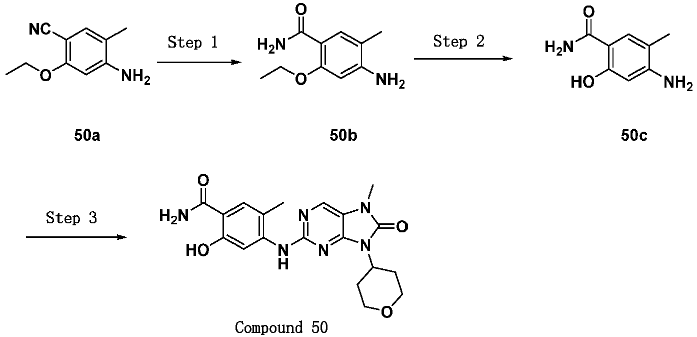

### Step 1:

### 4-Amino-2-ethoxy-5-methylbenzamide (50b)

4-Amino-2-ethoxy-5-methylbenzonitrile **50a** (1.0 g, 5.67 mmol) was dissolved in a solvent mixture of ethanol (15 mL) and dimethyl sulfoxide (1 mL), and a 1 N sodium hydroxide solution (3 mL) was added, followed by the slow dropwise addition of hydrogen peroxide (3 mL) at room temperature. The reaction mixture was stirred for 2 h. The reaction was monitored by TLC. After completion of the reaction, the reaction mixture was poured into a saturated sodium sulfite solution (20 mL) and stirred at room temperature for 2 h, and a large amounts of solid was precipitated. The solid was collected by filtration and dried by rotary evaporation under reduced pressure to obtain the title compound 4-amino-2-ethoxy-5-methylbenzamide **50b** (yellow solid, 510 mg, 48.6% yield).

¹H NMR (400 MHz, DMSO-d6) δ 7.53 (s, 1H), 7.34 (d, 1H), 7.07 (d, 1H), 6.28 (s, 1H), 5.47 (s, 2H), 4.04 (q, 2H), 1.99 (s, 3H), 1.37 (t, 3H).

LC-MS m/z(ESI) = **195.20** [M+1]

### Step 2:

### 4-Amino-2-hydroxy-5-methylbenzamide (50c)

4-Amino-2-ethoxy-5-methylbenzamide **50b** (510 mg, 2.63 mmol) was dissolved in dichloromethane (2 mL). The reaction mixture was cooled to 0 °C, and boron tribromide solution (13.1 mL, 1 M in THF) was slowly added dropwise. The reaction mixture was warmed to room temperature and stirred for 16 h. The reaction was monitored by TLC. After completion of the reaction,ethyl acetate (20 mL) and saturated ammonium chloride solution (20 mL) were added, and the resulting mixture was stirred for 0.5 h. The organic phase was extracted, concentrated, dried over anhydrous sodium sulfate and filtered, and the filtrate was dried under reduced pressure to obtain the title compound 4-amino-2-hydroxy-5-methylbenzamide **50c** (yellow solid, 350 mg, 80.1% yield).

¹H NMR (400 MHz, DMSO-d6) δ 12.26 (s, 1H), 7.79 (s, 1H), 7.38 (s, 1H), 7.23 (s, 1H), 5.98 (s, 1H), 5.50 (s, 2H), 1.96 (s, 3H).

LC-MS m/z(ESI) = **167.20** [M+1]

### Step 3:

### 2-Hydroxy-5-methyl-4-((7-methyl-8-oxo-9-(tetrahydro-2H-pyran-4-yl)-8,9-dihydro-7H-purin-2-yl)amino)benzamide (compound 50)

The title compound 2-hydroxy-5-methyl-4-((7-methyl-8-oxo-9-(tetrahydro-2*H*-pyran-4-yl)-8,9-dihydro-7*H*-purin-2-yl)amino)benzamide **compound 50** (white solid, 35 mg, 46.9% yield) was prepared according to the synthesis method of **compound 1.**

¹H NMR (400 MHz, DMSO-*d*₆) δ 12.87 (s, 1H), 8.27 (s, 1H), 8.16-8.13 (m, 2H), 7.64 (s, 1H), 7.61 (s, 1H), 7.54 (s, 1H), 4.47-4.39 (m, 1H), 4.00-3.96 (m, 2H), 3.48-3.37 (m, 2H), 3.33 (s, 3H), 2.57-2.48 (m, 2H), 2.22 (s, 3H), 1.70-1.64 (m, 2H).

LC-MS m/z(ESI) = **399.20** [M+1]

### Example 51

### 2-Fluoro-5-methyl-4-((7-methyl-9-(tetrahydro-2H-pyran-4-yl)-8,9-dihydro-7H-purin-2-yl)amino)methylbenzoate (compound 51)

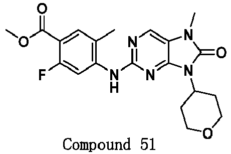

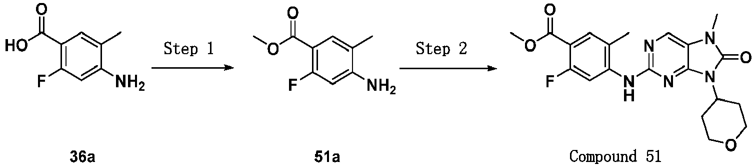

### Step 1:

### Methyl 4-amino-2-fluoro-5-methylbenzoate (51a)

4-Amino-2-fluoro-5-methylbenzoic acid **36a** (1.0 g, 5.91 mmol) was dissolved in methanol (6 mL), and 4 drops of concentrated sulfuric acid were added. The reaction mixture was heated to 60 °C and refluxed for 2 h. The reaction was monitored by TLC. After completion of the reaction, saturated sodium bicarbonate solution (20 mL) were added and extracted with ethyl acetate, and the organic phases were combined, washed once with saturated brine, dried over anhydrous sodium sulfate and concentrated, and the residue was purified by column chromatography (PE:EA (v/v) = 5:1) to obtain the title compound methyl 4-amino-2-fluoro-5-methylbenzoate **51a** (pale yellow solid, 0.8 g, 43.7% yield).

LC-MS m/z(ESI) = **184.20** [M+1]

### Step 2:

### 2-Fluoro-5-methyl-4-((7-methyl-9-(tetrahydro-2H-pyran-4-yl)-8,9-dihydro-7H-purin-2-yl)amino)methylbenzoate (compound 51)

The title compound 2-fluoro-5-methyl-4-((7-methyl-9-(tetrahydro-2*H*-pyran-4-yl)-8,9-dihydro-7*H*-purin-2-yl)amino)methylbenzoate **compound 51** (white solid, 239 mg, 79.8% yield) was prepared according to the synthesis method of **compound 1.**

¹H NMR (400 MHz, DMSO-*d*₆) δ 8.62 (s, 1H), 8.24 (s, 1H), 8.07 (d, 1H), 7.69 (d, 1H), 4.49-4.41 (m, 1H), 4.00-3.96 (m, 2H), 3.81 (s, 3H), 3.49-3.36 (m, 2H), 3.34 (s, 3H), 3.17 (s, 3H), 2.65-2.50 (m, 2H), 1.74-1.65 (m, 2H).

LC-MS m/z(ESI) = **402.20** [M+1]

### Example 52

### 2-Fluoro-5-methyl-4-((7-methyl-9-(tetrahydro-2H-pyran-4-yl)-8,9-dihydro-7H-purin-2-yl)amino)benzoic acid (compound 52)

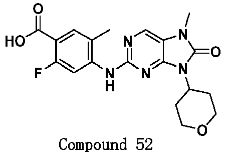

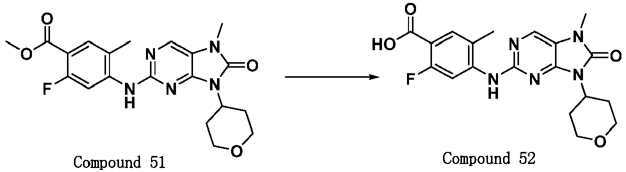

2-Fluoro-5-methyl-4-((7-methyl-9-(tetrahydro-2*H*-pyran-4-yl)-8,9-dihydro-7*H*-purin-2-yl)amino)methylbenzoate **compound 51** (300 mg, 0.75 mmol) was dissolved in tetrahydrofuran (3 mL), and a sodium hydroxide solution (80 mg in 3 mL of water) was added. The reaction mixture was heated to 50 °C and stirred for 2 h. The reaction was monitored by TLC. After completion of the reaction, the pH of reaction mixture was adjusted to about 5 with 2 N hydrochloric acid and concentrated under reduced pressure to remove tetrahydrofuran, and a large amounts of solid was precipitated. The solid was collected by filtration and purified by silica gel column chromatography (dichloromethane/methanol (v/v) = 100/1) to obtain the title compound 2-fluoro-5-methyl-4-((7-methyl-9-(tetrahydro-2*H*-pyran-4-yl)-8,9-dihydro-7*H*-purin-2-yl)amino)benzoic acid **compound 52** (white solid, 162 mg, 56.0% yield).

¹H NMR (400 MHz, DMSO-d6) δ 9.14 (s, 1H), 8.29 (s, 1H), 7.94 (d, 1H), 7.71 (d, 1H), 4.49-4.41 (m, 1H), 4.00-3.96 (m, 2H), 3.46-3.39 (m, 2H), 3.34 (s, 3H), 2.67-2.43 (m, 2H), 2.33 (s, 3H), 1.76-1.67 (m, 2H).

LC-MS m/z(ESI) = **388.20** [M+1]

### Example 53

### 2-Ethoxy-5-methyl-4-((7-methyl-8-oxo-9-(tetrahydro-2H-pyran-4-yl)-8,9-dihydro-7H-purin-2-yl)amino)benzamide (compound 53)

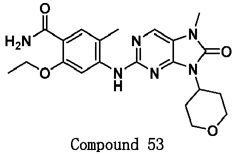

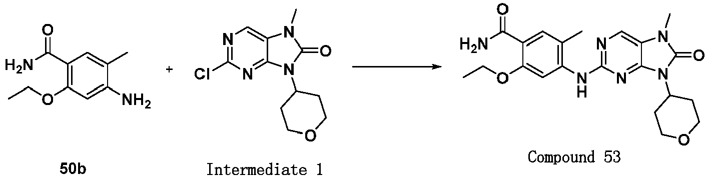

The title compound 2-ethoxy-5-methyl-4-((7-methyl-8-oxo-9-(tetrahydro-2*H*-pyran-4-yl)-8,9-dihydro-7*H*-purin-2-yl)amino)benzamide **compound 53** (pale yellow solid, 35 mg, 12.1% yield) was prepared according to the synthesis method of **compound 1.**

¹H NMR (400 MHz, DMSO-*d*₆) δ 8.38 (s, 1H), 8.18 (s, 1H), 7.76 (s, 1H), 7.71 (s, 1H), 7.51 (d, 1H), 7.41 (d, 1H), 4.48-4.40 (m, 1H), 4.17 (q, 2H), 3.98-3.95 (m, 2H), 3.47-3.36 (m, 2H), 3.34 (s, 3H), 2.57-2.53 (m, 2H), 2.24 (s, 3H), 1.72-1.63 (m, 2H), 1.41 (t, 3H).

LC-MS m/z(ESI) = **413.20** [M+1]

### Example 54

### N,4-Dimethyl-5-((7-methyl-8-oxo-9-(tetrahydro-2H-pyran-4-yl)-8,9-dihydro-7H-purin-2-yl)amino)picolinamide (compound 54)

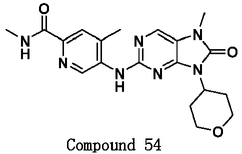

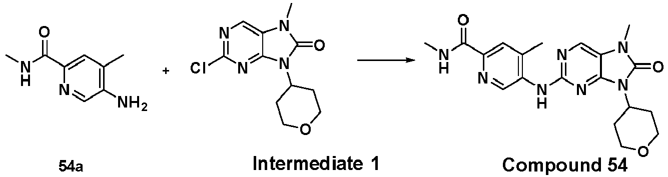

The title compound *N*,4-dimethyl-5-((7-methyl-8-oxo-9-(tetrahydro-2*H*-pyran-4-yl)-8,9-dihydro-7*H*-purin-2-yl)amino)picolinamide **compound 54** (pale yellow solid, 30 mg, 23.2% yield) was prepared according to the synthesis method of compound 1.

¹H NMR (400 MHz DMSO) δ 9.84 (s, 1H), 8.93 (d, 2H), 8.26 (d, 1H), 8.13 (s, 1H), 4.44-4.38 (m, 1H), 3.97-3.93 (m, 2H), 3.44-3.38 (m, 2H), 3.34 (s, 3H), 2.83 (d, 3H), 2.43-2.37 (m, 5H), 2.15 (s, 3H), 1.68 (dd, 2H).

LC-MS m/z(ESI) = **398.10** [M+1]

### Example 55

### 7-Methyl-2-((4-methyl-6-oxo-1,6-dihydropyridin-3-yl)amino)-9-(tetrahydro-2H-pyran-4-yl)-7,9-dihydro-8H-purin-8-one (compound 55)

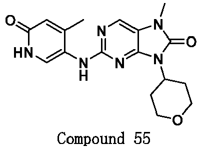

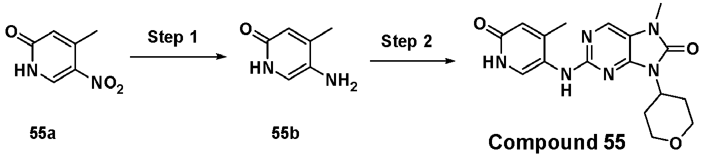

### Step 1:

### 5-Amino-4-methylpyridin-2(1H)-one (55b)

4-Methyl-5-nitropyridine-2(1*H*)-one **55a** (5.0 g, 32.44 mmol) and iron powder (9.0 g, 162.2 mmol) were added to a dry reaction flask and well mixed with ethanol/water=10/1 (110 mL), and then hydrochloric acid (7 mL, 2 mol/L) was added dropwise. The reaction mixture was stirred at 85 °C for 3 h. The reaction was monitored by TLC. After completion of the reaction, the reaction mixture was filtered, and the filter cake was washed with an appropriate amount of ethanol. The filtrate was concentrated, the pH was adjusted to about 8 with saturated sodium bicarbonate and purified by silica gel column chromatography (dichloromethane:methanol (v/v) = 10: 1) to give the title compound 5-amino-4-methylpyridin-2(1*H*)-one **55b** (dark green solid, 2.5 g, 62.08% yield).

¹H NMR (400 MHz, DMSO-d6) δ 10.63 (s, 1H), 6.75 (s, 1H), 6.11 (s, 1H), 4.06 (s, 2H), 2.02 (s, 3H).

LC-MS m/z (ESI) = **125.10** [M+1]

### Step 2:

### 7-Methyl-2-((4-methyl-6-oxo-1,6-dihydropyridin-3-yl)amino)-9-(tetrahydro-2H-pyran-4-yl)-7,9-dihydro-8H-purin-8-one (compound 55)

2-Chloro-7-methyl-9-(tetrahydro-2*H*-pyran-4-yl)-7,9-dihydro-8*H*-purin-8-one **55b** (200 mg, 0.74 mmol), 5-amino-4-methylpyridin-2(1*H*)-one (185 mg, 1.48 mmol), cesium carbonate (525 mg, 1.48 mmol) and Brettphos G3 Pd (67 mg, 0.074 mmol) were added to a dry reaction flask, followed by the addition of 1,4-dioxane (20 mL). The system was purged three times with nitrogen, and the reaction mixture was stirred at 110 °C for 2.5 h. The reaction was monitored by TLC. After completion of the reaction, the reaction mixture was concentrated, and the residue was purified by silica gel column chromatography (dichloromethane/methanol (v/v) = 20/1) to obtain the title compound 7-methyl-2-((4-methyl-6-oxo-1,6-dihydropyridin-3-yl)amino)-9-(tetrahydro-2*H-*pyran-4-yl)-7,9-dihydro-8*H*-purin-8-one **compound 55** (yellow solid, 38 mg, 10.02% yield).

¹H NMR (400 MHz, DMSO-*d*₆) δ 11.29 (s, 1H), 8.19 (s, 1H), 7.96 (s, 1H), 7.25 (s, 1H), 6.21 (s, 1H), 4.40-4.32 (m, 1H), 3.97-3.93 (m, 2H), 3.44-3.36 (m, 2H), 3.26 (s, 3H), 2.47-2.42 (m, 2H), 2.01 (s, 3H), 1.64-1.60 (m, 2H)

LC-MS m/z (ESI) = **357.20** [M+1]

### Example 56

### 2-((1,4-Dimethyl-6-oxo-1,6-dihydropyridin-3-yl)amino)-7-methyl-9-(tetrahydro-2H-pyran-4-yl)-7,9-dihydro-8H-purin-8-one (compound 56)

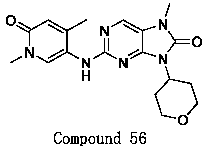

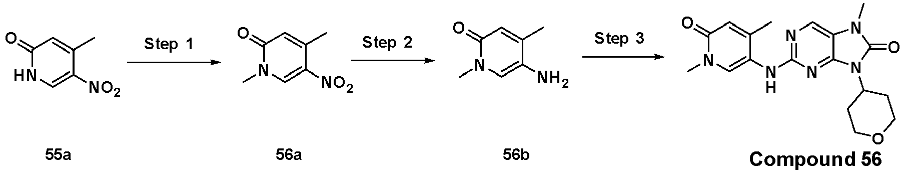

### Step 1:

### 1,4-Dimethyl-5-nitro-3,4-dihydropyridin-2-2(1H)-one (56a)

4-Methyl-5-nitropyridine-2(1*H*)-one **55a** (5.0 g, 32.44 mmol) and potassium carbonate (9.0 g, 48.66 mmol) were added to a dry reaction flask and well mixed with DMF, and iodomethane (3 mL, 64.88 mmol) was added dropwise at 0 °C. The reaction mixture was warmed to room temperature and stirred for 3 h. The reaction was monitored by TLC. After completion of the reaction, the reaction mixture was filtered, the filtrate was extracted with water and ethyl acetate, and the organic phases were combined, dried and concentrated to obtain the title compound 1,4-dimethyl-5-nitro-3,4-dihydropyridin-2-2(1*H*)-one **56a** (yellow solid, 5.2 g, 95.41% yield).

¹H NMR (400 MHz, Chloroform-d) δ 8.62 (s, 1H), 6.39 (s, 1H), 3.63 (s, 3H), 2.56-2.53 (m, 3H).

LC-MS m/z (ESI) = **169.10** [M+1]

### Step 2:

### 5-Amino-1,4-dimethylpyridin-2(1H)-one (56b)

1,4-Dimethyl-5-nitropyridine-2(1*H*)-one **56a** (5.2 g, 30.92 mmol) and iron powder (9.0 g, 154.62 mmol) were added to a dry reaction flask and well mixed with ethanol/water (v/v=10/1, 110 mL), and then hydrochloric acid (10 mL, 2 mol/L) was added. The reaction mixture was stirred at 85 °C for 3 h. The reaction was monitored by TLC. After completion of the reaction, the reaction mixture was filtered, and the filter cake was washed with an appropriate amount of ethanol. The filtrate was concentrated, the pH was adjusted to about 8 with saturated sodium bicarbonate, and purified by silica gel column chromatography (dichloromethane:methanol (v/v) = 10:1) to give the title compound 5-amino-1,4-dimethylpyridin-2(1*H*)-one **56b** (dark green solid, 2.5 g, 58.55% yield).

¹H NMR (400 MHz, DMSO-d6) δ 6.88 (s, 1H), 6.16 (s, 1H), 3.81 (s, 2H), 3.30 (s, 3H), 2.03 (s, 3H).

LC-MS m/z (ESI) = **141.10** [M+1]

### Step 3:

### 2-((1,4-Dimethyl-6-oxo-1,6-dihydropyridin-3-yl)amino)-7-methyl-9-(tetrahydro-2H-pyran-4-yl)-7,9-dihydro-8H-purin-8-one (compound 56)

2-Chloro-7-methyl-9-(tetrahydro-2*H*-pyran-4-yl)-7,9-dihydro-8*H*-purin-8-one **56b** (200 mg, 0.74 mmol), 5-amino-1,4-dimethyl-3,4-dihydropyridin-2(1*H*)-one (206 mg, 1.48 mmol), cesium carbonate (525 mg, 1.48 mmol) and Brettphos G3 Pd (67 mg, 0.074 mmol) were added to a dry reaction flask, followed by the addition of 1,4-dioxane (20 mL). The system was purged three times with nitrogen, and the reaction mixture was stirred at 110 °C for 2.5 h. The reaction was monitored by TLC. After completion of the reaction,the reaction mixture was concentrated, and the residue was purified by silica gel column chromatography (dichloromethane/methanol (v/v) = 20/1) to obtain the title compound 7-methyl-2-((4-methyl-6-oxo-1,6-dihydropyridin-3-yl)amino)-9-(tetrahydro-2*H*-pyran-4-yl)-7,9-dihydro-8*H*-purin-8-one **compound 56** (yellow solid, 97 mg, 35.36% yield).

¹H NMR (400 MHz, DMSO-*d*₆) δ 8.26 (s, 1H), 7.98 (s, 1H), 7.66 (s, 1H), 6.27 (s, 1H), 4.41-4.33 (m, 1H), 3.98-3.94 (m, 2H), 3.42 (d, 2H), 3.38 (s, 3H), 3.27 (s, 3H), 2.54-2.44 (m, 2H), 2.01 (s, 3H), 1.65-1.62 (m, 2H).

LC-MS m/z (ESI) = **371.20** [M+1]

### Example 57

### 2-Methoxy-5-methyl-4-((7-methyl-8-oxo-9-(tetrahydro-2H-pyran-4-yl)-8,9-dihydro-7H-purin-2-yl)amino)benzamide (compound 57)

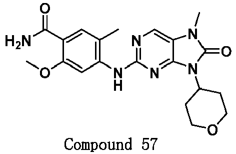

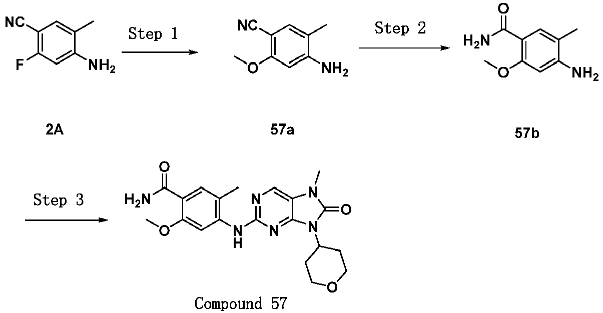

### Step 1:

### 4-Amino-2-methoxy-5-methylbenzonitrile (57a)

4-Amino-2-fluoro-5-methylbenzonitrile **2A** (1.0 g, 6.67 mmol) was dissolved in a mixture of methanol (10 mL) and purified water (2 mL), and then potassium hydroxide (1.50 g, 26.68 mmol) was added. The reaction mixture was refluxed for 4 h. The reaction was monitored by TLC. After completion of the reaction, 20 mL of purified water was added, and the resulting mixture was concentrated under reduced pressure to remove methanol and then extracted 3 times with ethyl acetate. The organic phases were combined, dried over anhydrous sodium sulfate and concentrated, and the residue was purified by column chromatography (petroleum ether:ethyl acetate (v/v) = 5:1) to obtain 4-amino-2-methoxy-5-methylbenzonitrile **57a** (white solid, 300 mg, 27.8% yield).

LC-MS m/z(ESI) = **163.10** [M+1]

### Step 2:

### 4-Amino-2-methoxy-5-methylbenzamide (57b)

4-Amino-2-methoxy-5-methylbenzonitrile **57a** (300 mg, 1.85 mmol) and potassium carbonate (51 mg, 0.37 mmol) were dissolved in DMSO (1 mL), and the mixture was added slowly dropwise with hydrogen peroxide (2 mL) at room temperature, and stirred for 2 h. The reaction was monitored by TLC. After completion of the reaction, the reaction mixture was poured into purified water (10 mL), and a large amounts of solid was precipitated. The solid was collected by filtration and dried under reduced pressure to obtain the title compound 4-amino-2-methoxy-5-methylbenzamide **57b** (yellow solid, 256 mg, 76.7% yield).

LC-MS m/z(ESI) = **181.10** [M+1]

### Step 3:

### 2-Methoxy-5-methyl-4-((7-methyl-8-oxo-9-(tetrahydro-2H-pyran-4-yl)-8,9-dihydro-7H-purin-2-yl)amino)benzamide (compound 57)

The title compound 2-methoxy-5-methyl-4-((7-methyl-8-oxo-9-(tetrahydro-2*H*-pyran-4-yl)-8,9-dihydro-7*H*-purin-2-yl)amino)benzamide **compound 57** (white solid, 35 mg, 12.1% yield) was prepared according to the synthesis method of **compound 1.**

¹H NMR (400 MHz, DMSO-d6) δ 8.39 (s, 1H), 8.19 (s, 1H), 7.78 (s, 1H), 7.70 (s, 1H), 7.55 (s, 1H), 7.37 (s, 1H), 4.47-4.41 (m, 1H), 3.98-3.94(m, 2H), 3.91(s, 3H), 3.44-3.38 (m, 2H), 3.33 (s, 3H), 2.60-2.52 (m, 2H), 2.25 (s,3H), 1.68 (d, 2H).

LC-MS m/z(ESI) = **413.20** [M+1]

### Example 58

### 5-Methyl-6-((7-methyl-8-oxo-9-(tetrahydro-2H-pyran-4-yl)-8,9-dihydro-7H-purin-2-yl)amino)nicotinamide (compound 58)

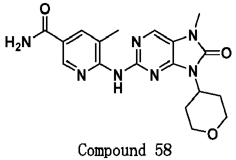

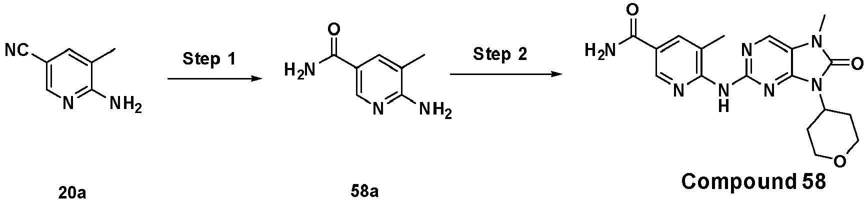

### Step 1:

### 6-Amino-5-methylnicotinamide (58a)

6-Amino-5-methylnicotinonitrile **20a** (300 mg, 2.2 mmol) and potassium carbonate (46 mg, 0.33 mmol) were dissolved in 1 mL of dimethyl sulfoxide, and 300 µL of hydrogen peroxide was added in an ice bath. Then the reaction mixture was gradually heated to 60 °C and stirred for 2 h. The reaction was monitored by TLC. After completion of the reaction, 5 mL of water was added to the reaction mixture, and a white solid was precipitated. The mixture was filtered, and the filter cake was concentrated to dryness under reduced pressure to obtain the title compound 6-amino-5-methylnicotinamide **58a** (white solid, 200 mg, 58% yield).

¹H NMR (400 MHz DMSO) δ 8.34 (s, 1H), 7.68 (s, 1H), 7.63 (s, 1H), 6.98 (s, 1H), 6.25 (s, 1H), 2.04 (s, 3H).

LC-MS m/z(ESI) = **152.10** [M+1]

### Step 2:

### 5-Methyl-6-((7-methyl-8-oxo-9-(tetrahydro-2H-pyran-4-yl)-8,9-dihydro-7H-purin-2-yl)amino)nicotinamide (compound 58)

The title compound 5-methyl-6-((7-methyl-8-oxo-9-(tetrahydro-2*H*-pyran-4-yl)-8,9-dihydro-7*H*-purin-2-yl)amino)nicotinamide **compound 58** (white solid, 17 mg, 13.1% yield) was prepared according to the synthesis method of **compound 1.**

¹H NMR (400 MHz DMSO) δ 9.19 (s, 1H), 8.59 (s, 1H), 8.12 (s, 1H), 8.00 (s, 1H), 7.96 (s, 1H), 7.38 (s, 1H), 4.45-4.35 (m, 1H), 3.94 (dd, 2H), 3.40-3.30 (m, 2H), 3.33 (s, 3H), 2.50 (s, 2H), 2.22(s,3H), 1.63(dd, 2H).

LC-MS m/z(ESI) = **384.20** [M+1]

### Example 59

### 5-Chloro-2-fluoro-4-((7-methyl-8-oxo-9-(tetrahydro-2H-pyran-4-yl)-8,9-dihydro-7H-purin-2-yl)amino)benzamide (compound 59)

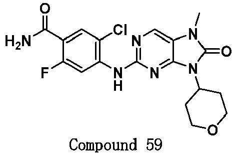

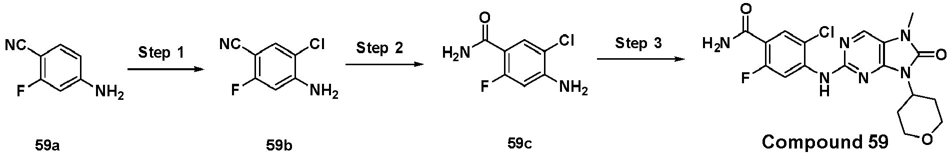

### Step 1:

### 4-Amino-5-chloro-2-fluorobenzonitrile (59b)

4-Amino-2-fluorobenzonitrile **59a** (10.0 g, 73.46 mmol) was dissolved in acetonitrile (100 mL), and N-chlorosuccinimide (10.79 g, 80.81 mmol) was added in an ice bath. The reaction mixture was gradually warmed to room temperature, heated to 80 °C and stirred for 3 h. The reaction was monitored by TLC. After completion of the reaction, the reaction mixture was poured directly into 400 mL of water and extracted with 200 mL of ethyl acetate; the organic layer was collected, dried and concentrated to obtain the title compound 4-amino-5-chloro-2-fluorobenzonitrile **59b** (white solid, 6 g, 46.7% yield).

¹H NMR (400 MHz, DMSO-d6) δ 7.79-7.77 (m, 2H), 6.78(s, 2H), 6.66 (d, 1H). LC-MS m/z(ESI) = **171.10** [M+1]

### Step 2:

### 4-Amino-5-chloro-2-fluorobenzamide (59c)

4-Amino-5-chloro-2-fluorobenzonitrile **59b** (500 mg, 2.93 mmol) was dissolved in 1 M sodium hydroxide (14.66 mL) and methanol (1.19 mL). The solution was heated to 40 °C, and then 30% hydrogen peroxide (0.3 mL) was added dropwise. The reaction mixture was reacted for 16 h with the temperature maintained. The reaction was monitored by TLC. After completion of the reaction, the reaction mixture was directly diluted with water (50 mL) and extracted with ethyl acetate, and the organic phases were combined, washed with saturated brine, dried and concentrated, and the residue was purified by silica gel column chromatography to obtain the title compound 4-amino-5-chloro-2-fluorobenzamide **59c** (280 mg, white solid, 50.7% yield).

¹H NMR (400 MHz, CDCl3-d6) δ 7.57 (d, 2H), 7.36 (s, 1H), 7.20 (s, 1H), 6.53 (d, 2H), 6.18 (s, 2H).

LC-MS m/z(ESI) = **189.10** [M+1]

### Step 3:

### 5-Chloro-2-fluoro-4-((7-methyl-8-oxo-9-(tetrahydro-2H-pyran-4-yl)-8,9-dihydro-7H-purin-2-yl)amino)benzamide (compound 59)

4-Amino-5-chloro-2-fluorobenzamide **59c** (280.74 mg, 1.49 mmol), 2-chloro-7-methyl-9-(tetrahydro-2*H*-pyran-4-yl)-7,9-dihydro-8*H*-purin-8-one **intermediate 1** (200 mg, 0.74 mmol), cesium carbonate (485 mg, 1.49 mmol), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (67.4 mg, 0.07 mmol) were dissolved in dioxane (10 mL), followed by nitrogen purging. The reaction mixture was stirred under nitrogen at 100 °C for 4 h. The reaction was monitored by TLC. After completion of the reaction, the reaction mixture was poured into ice water, and the solid was collected, and the residue was purified by silica gel column chromatography (dichloromethane/methanol (v/v) = 100/1) to yield compound 5-chloro-2-fluoro-4-((7-methyl-8-oxo-9-(tetrahydro-2*H*-pyran-4-yl)-8,9-dihydro-7*H*-purin-2-yl)amino)benzamide **compound 59** (white solid, 40 mg, 12.77 % yield).

¹H NMR (401 MHz, DMSO-d6) δ 8.44 (s, 1H), 8.29 (d, 1H), 8.25 (s, 1H), 7.77 (d, 1H), 7.64 (s, 1H), 7.60 (s, 1H), 4.49-4.43 (m, 1H), 4.00-3.97 (m, 2H), 3.48-3.44 (m, 2H), 3.34 (s, 3H), 2.57-2.53 (m, 2H), 1.72-1.68 (m, 2H).

LC-MS m/z(ESI) = **421.10** [M+1]

### Example 60

### 2-Fluoro-3,5-dimethyl-4-((7-methyl-8-oxo-9-(tetrahydro-2H-pyran-4-yl)-8,9-dihydro-7H-purin-2-yl)amino)benzamide (compound 60)

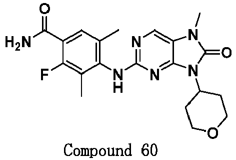

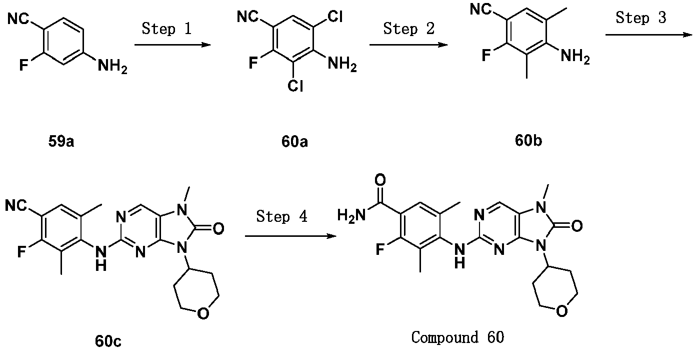

### Step 1:

### 4-Amino-3,5-dichloro-2-fluorobenzonitrile (60a)

4-Amino-2-fluorobenzonitrile **59a** (10.0 g, 73.46 mmol) was dissolved in acetonitrile (100 mL), and *N*-chlorosuccinimide (21 g, 160 mmol) was added in an ice bath. The reaction mixture was gradually warmed to room temperature, heated to 80 °C and reacted for 3 h. The reaction was monitored by TLC. After completion of the reaction, the reaction mixture was poured directly into 400 mL of water and extracted with 200 mL of ethyl acetate; the organic layer was collected, dried and concentrated to afford the title compound 4-amino-3,5-chloro-2-fluorobenzonitrile **60a** (white solid, 7 g, 46.5% yield).

¹H NMR (400 MHz, DMSO-d6) δ 7.86 (d, 1H), 7.03 (s, 2H).

LC-MS m/z(ESI) = **205.10** [M+1]

### Step 2:

### 4-Amino-2-fluoro-3,5-dimethylbenzonitrile (60b)

4-Amino-3,5-dichloro-2-fluorobenzonitrile **60a** (7 g, 34.14 mmol), methylboronic acid (20.44 g, 341.44 mmol), potassium phosphate (36.24 g, 170.72 mmol), 2-dicyclohexylphosphine-2',4',6'-triisopropylbiphenyl (4.88 g, 10.24 mmol), and palladium(□) acetate (1.15 g, 5.12 mmol) were dissolved in dioxane (100 mL), followed by nitrogen purging. The reaction mixture was stirred under nitrogen at 100 °C for 4 h. The reaction was monitored by TLC. After completion of the reaction, the reaction mixture was poured into ice water, and the solid was collected and purified by silica gel column chromatography to obtain the title compound 4-amino-2-fluoro-3,5-dimethylbenzonitrile **60b** (white solid, crude product, 6 g, 107% yield).

¹H NMR (400 MHz, CDCl3-d6) δ 7.14 (d, 1H), 5.94 (s, 2H), 1.99 (s, 3H), 1.94 (d, 2H). LC-MS m/z(ESI) = **165.10** [M+1]

### Step 3:

### 2-Fluoro-3,5-dimethyl-4-((7-methyl-8-oxo-9-(tetrahydro-2H-pyran-4-yl)-8,9-dihydro-7H-purin-2-yl)amino)benzonitrile (60c)

4-Amino-2-fluoro-3,5-dimethylbenzonitrile **60b** (1.22 g, 7.44 mmol), 2-chloro-7-methyl-9-(tetrahydro-2*H*-pyran-4-yl)-7,9-dihydro-8*H*-purin-8-one **intermediate 1** (2.0 g, 7.44 mmol), cesium carbonate (4.85 g, 14.89 mmol), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (674 mg, 0.74 mmol) were dissolved in dioxane (20 mL), followed by nitrogen purging. The reaction mixture was stirred under nitrogen at 100 °C for 4 h. The reaction was monitored by TLC. After completion of the reaction, the reaction mixture was poured into ice water, and the solid was collected and purified by silica gel column chromatography to obtain compound 2-fluoro-3,5-dimethyl-4-((7-methyl-8-oxo-9-(tetrahydro-2*H*-pyran-4-yl)-8,9-dihydro-7*H*-purin-2-yl)amino)benzonitrile **60c** (413 mg, yellow solid, 14 % yield).

¹H NMR (401 MHz, DMSO-d6) δ 9.02 (s, 1H), 8.12 (s, 1H), 7.91 (d, 1H), 4.49-4.39 (m, 1H), 4.00-3.96 (m, 2H), 3.45-3.39 (m, 2H), 2.32 (s, 3H), 2.22 (d, 3H), 1.71-1.65 (m, 2H).

LC-MS m/z(ESI) = **397.10** [M+1]

### Step 4:

### 2-Fluoro-3,5-dimethyl-4-((7-methyl-8-oxo-9-(tetrahydro-2H-pyran-4-yl)-8,9-dihydro-7H-purin-2-yl)amino)benzamide(compound 60)

2-Fluoro-3,5-dimethyl-4-((7-methyl-8-oxo-9-(tetrahydro-2*H*-pyran-4-yl)-8,9-dihydro-7*H*-purin-2-yl)amino)benzonitrile **60c** (413 mg, 1.04 mmol) was dissolved in 1 M sodium hydroxide (5 mL) and methanol (10 mL). The solution was heated to 60 °C, and then 30% hydrogen peroxide (0.16 mL) was added dropwise. The reaction mixture was stirred for 4 h with the temperature maintained. The reaction was monitored by TLC. After completion of the reaction, the reaction mixture was directly diluted with water (50 mL) and extracted with ethyl acetate, and the organic phases were combined, washed with saturated brine, dried and concentrated, and the residue was purified by silica gel column chromatography to obtain the title compound 2-fluoro-3,5-dimethyl-4-((7-methyl-8-oxo-9-(tetrahydro-2*H*-pyran-4-yl)-8,9-dihydro-7*H*-purin-2-yl)amino)benzamide **compound 60** (white solid, 40 mg, 9.3% yield).

¹H NMR (400 MHz, CDCl3-d6) δ 8.65 (s, 1H), 7.91 (s, 1H), 7.60 (s, 1H), 7.54 (s, 1H), 7.36 (d, 1H), 4.42-4.34 (m, 1H), 3.98-3.94 (m, 1H), 3.43-3.37 (m, 2H), 3.26 (s, 3H), 2.13 (s, 3H), 2.04 (d, 3H), 1.67-1.63 (m, 2H).

LC-MS m/z(ESI) = **415.10** [M+1]

### Example 61

### 4-((9-(trans-3-Cyanocyclobutyl)-7-methyl-8-oxo-8,9-dihydro-7H-purin-2-yl)amino)-2-fluoro-5-methylbenzamide (compound 61)

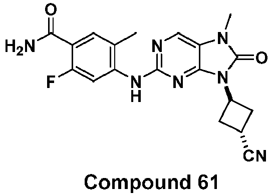

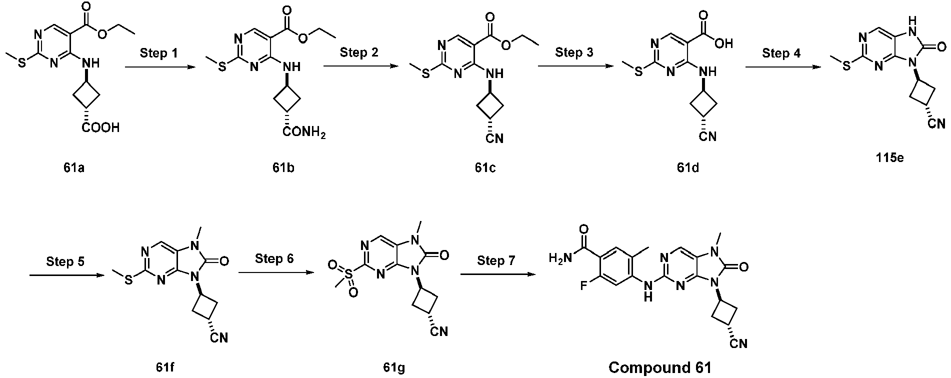

### Step 1:

### Ethyl 4-((trans-3-carbamoylcyclobutyl)amino)-2-(methylthio)pyrimidine-5-carboxylate (61b)

The starting material *trans*-3-((5-(ethoxycarbonyl)-2-(methylthio)pyrimidin-4-yl)amino)cyclobutane-1-carboxylic acid **61a** (13.0 g, 41.8 mmol) was added to dichloromethane (130 mL), and *N*,*N*,*N*,*N*-tetramethyl-*O*-(7-azabenzotriazol-1-yl)urea hexafluorophosphate (19.1 g, 50.2 mmol) and triethylamine (16.9 g, 167.2 mmol) were added. The mixture was stirred at room temperature for 10 min, and ammonium chloride (6.7 g, 125.4 mmol) was added. The reaction mixture was stirred for 2 h. The reaction was monitored by TLC. After completion of the reaction, 50 mL of saturated aqueous sodium bicarbonate and dichloromethane was added to the reaction mixture. The organic layer was concentrated over anhydrous sodium sulfate and concentrated under reduced pressure to obtain crude title compound ethyl 4-((*trans*-3-carbamoylcyclobutyl)amino)-2-(methylthio)pyrimidine-5-carboxylate **61b** (yellow viscous liquid, 12.5 g, 96.5% yield).

LCMS m/z(ESI) = 311.20 [M+1]

### Step 2:

### Ethyl 4-((trans-3-cyanocyclobutyl)amino)-2-(methylthio)pyrimidine-5-carboxylate (61c)

Ethyl 4-((*cis*-3-carbamoylcyclobutyl)amino)-2-(methylthio)pyrimidine-5-carboxylate **61b** (12.5 g, 4.03 mmol) was added to dichloromethane (130 mL), and pyridine (13.2 g, 167.2 mmol) and trifluoroacetic anhydride (26.3 g, 125.4 mmol) were added. The reaction mixture was stirred at room temperature for 1 h. The reaction was monitored by TLC. After completion of the reaction, the pH of reaction mixture was adjusted to 3 with 2 N hydrochloric acid and extracted with dichloromethane. Dried over anhydrous sodium sulfate and concentrated under reduced pressure to obtain a crude product, which was then purified by column chromatography (petroleum ether/ethyl acetate (v/v) = 5:1) to give the title compound ethyl 4-((*trans*-3-cyanocyclobutyl)amino)-2-(methylthio)pyrimidine-5-carboxylate **61c** (white solid, 1.7 g, 10.2% yield).

1HNMR (400 MHz, DMSO-d6) δ 8.65 (s, 1H), 8.46 (d, 1H), 4.70-4.61 (m, 1H), 4.37-4.31 (m, 2H), 2.92-2.81 (m, 3H), 2.52 (s, 3H), 2.46-2.35 (m, 2H), 1.38(t, 3H). LC-MS m/z(ESI) = 293.20 [M+1]

### Step 3:

### 4-((trans-3-Cyanocyclobutyl)amino)-2-(methylthio)pyrimidine-5-carboxylic acid (61d)

Ethyl 4-((*cis*-3-Cyanocyclobutyl)amino)-2-(methylthio)pyrimidine-5-carboxylate **61c** (1.7 g, 5.8 mmol) was dissolved in a mixture of tetrahydrofuran (10 mL) and water (10 mL), and lithium hydroxide (487 mg, 11.6 mmol) was added. The reaction mixture was stirred at room temperature for 1 h. The reaction was monitored by TLC. After completion of the reaction, the reaction mixture was concentrated under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound 4-((*trans*-3-cyanocyclobutyl)amino)-2-(methylthio)pyrimidine-5-carboxylic acid 61d (white solid, 1.5 g, 97.4% yield).

LC-MS m/z(ESI) = 265.10 [M+1]

### Step 4:

### trans-3-(2-(Methylthio)-8-oxo-7,8-dihydro-9H-purin-9-yl)cyclobutane-1-carbonitrile (61e)

4-((*trans*-3-Cyanocyclobutyl)amino)-2-(methylthio)pyrimidine-5-carboxylic acid **61d** (1.5 g, 5.7 mmol) was dissolved in *N*,*N*-dimethylformamide (15 mL), and triethylamine (576 mg, 5.7 mmol) and diphenylphosphoryl azide (1.57 g, 5.7 mmol) were added. The reaction mixture was gradually heated to 110 °C and stirred for 1.5 h. The reaction was monitored by TLC. After completion of the reaction,the reaction mixture was concentrated, and the residue was purified by silica gel column chromatography (petroleum ether/ethyl acetate (v/v) = 5:1 to 1:10) to obtain the title compound *trans*-3-(2-(methylthio)-8-oxo-7,8-dihydro-9*H*-purin-9-yl)cyclobutane-1-carbonitrile **61e** (white solid, 1.0 g, 67% yield).

1HNMR (400 MHz, DMSO-d6) δ 11.35 (s, 1H), 8.11 (s, 1H), 4.92-3.92 (m, 1H), 3.37-3.26 (m, 2H), 3.27-3.15 (m, 1H), 2.78-2.67 (m, 2H), 2.58 (s, 3H).

LC-MS m/z(ESI) = 262.10 [M+1]

### Step 5:

### trans-3-(7-Methyl-2-(methylthio)-8-oxo-7,8-dihydro-9H-purin-9-yl)cyclobutane-1-carbonitrile (61f)

*trans*-3-(2-(Methylthio)-8-oxo-7,8-dihydro-9*H*-purin-9-yl)cyclobutane-1-carbonitrile **61e** (500 mg, 19.1 mmol) was dissolved in dimethylformamide (5 mL), and dimethyl sulfate (2.4 g, 19.1 mmol) and cesium carbonate (12.5 g, 38.2 mmol) were added at 0 °C. The reaction mixture was stirred at 0 °C for 0.5 h. The reaction was monitored by TLC. After completion of the reaction, then 50 mL of water was added, and a solid was precipitated. The solid was collected by filtration and dried by rotary evaporation under reduced pressure to obtain the title compound the title compound *trans*-3-(7-methyl-2-(methylthio)-8-oxo-7,8-dihydro-9*H*-purin-9-yl)cyclobutane-1-carbonitrile **61f** (white solid, 462 mg, 87.7% yield).

1H NMR (400 MHz, DMSO-d6) δ 8.61 (s, 1H), 4.90-4.02 (m, 1H), 3.34(s, 3H), 3.37-3.25(m, 2H), 3.25-3.12(m, 1H), 2.89(s, 3H), 2.86-2.75(m, 2H).

LC-MS m/z (ESI) = 276.10 [M+1]

### Step 6:

### trans-3-(7-Methyl-2-(methylsulfonyl)-8-oxo-7,8-dihydro-9H-purin-9-yl)cyclobutane-1-carbonitrile (61g)

*trans*-3-(7-Methyl-2-(methylthio)-8-oxo-7,8-dihydro-9*H*-purin-9-yl)cyclobutane-1-carbonitrile **61f** (462 mg, 1.67 mmol) was dissolved in methanol (10 mL), and 2 mL of purified water was added, followed by the addition of potassium peroxymonosulfate (1.02 g, 1.67 mmol). The reaction mixture was heated to 60 °C and stirred for 2 h. The reaction was monitored by TLC. After completion of the reaction,the reaction mixture was concentrated by rotary evaporation under reduced pressure to remove methanol, and 5 mL of purified water was added. The resulting mixture was extracted with dichloromethane (20 mL × 3), and the organic phases were combined, dried over anhydrous sodium sulfate, and concentrated by rotary evaporation under reduced pressure to remove the solvent to obtain the title compound *trans-*3-(7-methyl-2-(methylsulfonyl)-8-oxo-7,8-dihydro-9*H*-purin-9-yl)cyclobutane-1-carbonitrile **61g** (pale yellow solid, 181 mg, 35.9% yield).

LC-MS m/z(ESI) = 308.10 [M+1]

### Step 7:

### 4-((9-(trans-3-Cyanocyclobutyl)-7-methyl-8-oxo-8,9-dihydro-7H-purin-2-yl)amino)-2-fluoro-5-methylbenzamide (compound 61)

*trans*-3-(7-Methyl-2-(methylsulfonyl)-8-oxo-7,8-dihydro-9*H*-purin-9-yl)cyclobutane-1-carbonitrile **61g** (181 mg, 0.59 mmol) and 4-amino-3-methyl-2-fluorobenzamide (538 mg, 3.20 mmol) were dissolved in *N,N*-dimethylformamide (3 mL), followed by nitrogen purging and the addition of potassium *tert*-butoxide (143 mg, 1.28 mmol). The reaction mixture was stirred under nitrogen at room temperature for 4 h. The reaction was monitored by TLC. After completion of the reaction,the reaction mixture was poured into 30 mL of purified water, and a large amounts of yellow solid was precipitated. The solid was collected by filtration and purified by silica gel column chromatography (dichloromethane/methanol (v/v) = 35/1) to obtain the title compound 4-((9-(*trans*-3-cyanocyclobutyl)-7-methyl-8-oxo-8,9-dihydro-7*H*-purin-2-yl)amino)-2-fluoro-5-methylbenzamide **compound 61** (yellow solid, 27 mg, 11.6 % yield).

¹H NMR (400 MHz, DMSO-d6) δ 8.62(s, 1H), 8.20(s, 1H), 7.88(d, 1H), 7.55(d, 1H), 7.49(s, 1H), 7.40(s, 1H), 5.14-5.01(m, 1H), 3.64-3.59(m, 1H), 3.31(s, 3H)3.26-3.21(m, 2H), 2.78-2.70(m, 2H), 2.32(s, 3H).

LC-MS m/z(ESI) = 396.20 [M+1]

### Example 62

### 4-((7-Ethyl-8-oxo-9-(tetrahydro-2H-pyran-4-yl)-8,9-dihydro-7H-purin-2-yl)amino)-2-fluoro-5-methylbenzamide (compound 62)

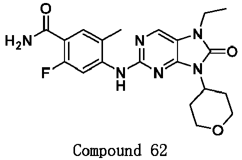

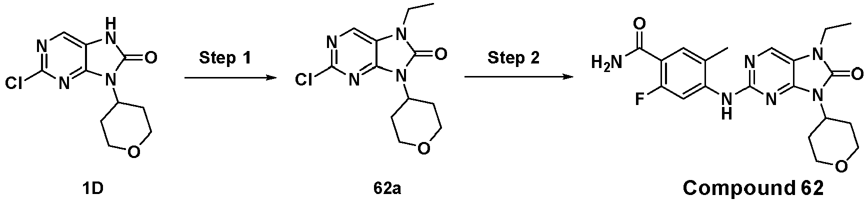

### Step 1:

### 2-Chloro-7-ethyl-9-(tetrahydro-2H-pyran-4-yl)-7,9-dihydro-8h-purin-8-one (62a)

2-Chloro-9-(tetrahydro-2*H*-pyran-4-yl)-7,9-dihydro-8*H*-purin-8-one **1D** (400 mg, 1.57 mmol) was dissolved in *N*,*N*-dimethylformamide (8 mL), and cesium carbonate (511 mg, 1.57 mmol) and iodoethane (293 mg, 1.88 mmol) were added at 0 °C. The reaction mixture was stirred for 1 h. The reaction was monitored by TLC. After completion of the reaction,then 10 mL of water was added, and the resulting mixture was extracted 3 times with ethyl acetate. The organic phase was dried over anhydrous sodium sulfate and concentrated. The reaction mixture was concentrated by rotary evaporation under reduced pressure to remove organic solvent to obtain the title compound 2-chloro-7-ethyl-9-(tetrahydro-2*H*-pyran-4-yl)-7,9-dihydro-8*H*-purin-8-one **62a** (white solid, 290 mg, 65.32% yield).

¹H NMR (400 MHz, DMSO-d6) δ 8.44 (s, 1H), 4.50-4.41 (m, 1H), 3.99-3.95 (m, 2H), 3.89 (q, 2H), 3.45 (t, 2H), 2.46-2.41 (m, 2H), 1.71-1.67 (m, 2H), 1.25 (t, 3H). LC-MS m/z(ESI) = **283.10** [M+1]

### Step 2:

### 4-((7-Ethyl-8-oxo-9-(tetrahydro-2H-pyran-4-yl)-8,9-dihydro-7H-purin-2-yl)amino)-2-fluoro-5-methylbenzamide (compound 62)

2-Chloro-7-ethyl-9-(tetrahydro-2*H*-pyran-4-yl)-7,9-dihydro-8*H*-purin-8-one **62a** (150 mg, 0.53 mmol), 4-amino-2-fluoro-5-methylbenzamide **intermediate 2** (350 mg, 2.12 mmol), cesium carbonate (690 mg, 2.12 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (72 mg, 0.08 mmol) were dissolved in 1,4-dioxane (5 mL), followed by nitrogen purging. The reaction mixture was stirred under nitrogen at 110 °C for 4 h. The reaction was monitored by TLC. After completion of the reaction,the reaction mixture was concentrated and purified by silica gel column chromatography (dichloromethane/methanol (v/v) = 20/1), and purified by Pre-HPLC to obtain the title compound 4-((7-ethyl-8-oxo-9-(tetrahydro-2*H*-pyran-4-yl)-8,9-dihydro-7*H*-purin-2-yl)amino)-2-fluoro-5-methylbenzamide **compound 62** (white solid, 38 mg, 17.28% yield).

¹H NMR (400 MHz, DMSO-d6) δ 8.53 (s, 1H), 8.25 (s, 1H), 7.89 (d, 1H), 7.55 (d, 1H), 7.42 (d, 2H), 4.48-4.40 (m, 1H), 3.98 (dd, 2H), 3.85 (q, 2H), 3.43 (t, 2H), 2.58-2.54 (m, 2H), 2.30 (s, 3H) 1.71-1.68 (m, 2H), 1.25 (t, 3H).

LC-MS m/z(ESI) = **415.20** [M+1]

### Example 63

### 2-Fluoro-4-((7-isopropyl-8-oxo-9-(tetrahydro-2H-pyran-4-yl)-8,9-dihydro-7H-purin-2-yl)amino)-5-methylbenzamide (compound 63)

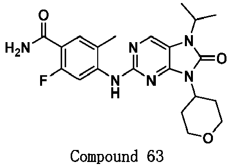

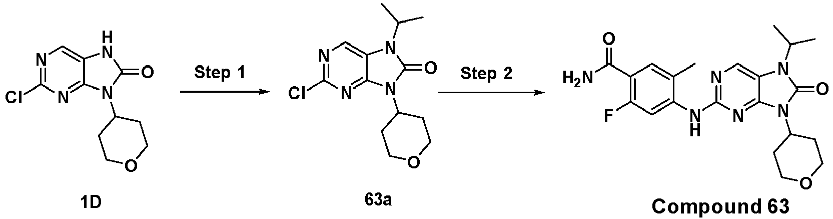

### Step 1:

### 2-Chloro-7-isopropyl-9-(tetrahydro-2H-pyran-4-yl)-7,9-dihydro-8H-purin-8-one (46a)

2-Chloro-9-(tetrahydro-2*H*-pyran-4-yl)-7,9-dihydro-8*H*-purin-8-one **1D** (520 mg, 2.04 mmol) was dissolved in *N*,*N*-dimethylformamide (10 mL), and cesium carbonate (665 mg, 2.04 mmol) and 2-iodopropane (416 mg, 2.45 mmol) were added at 0 °C. The reaction mixture was stirred for 2 h. The reaction was monitored by TLC. After completion of the reaction,then 20 mL of water was added, and the resulting mixture was extracted 3 times with ethyl acetate. The organic phase was dried over anhydrous sodium sulfate and concentrated to afford the title compound 2-chloro-7-isopropyl-9-(tetrahydro-2*H*-pyran-4-yl)-7,9-dihydro-8*H*-purin-8-one **63a** (white solid, 465 mg, 76.74% yield).

¹H NMR (400 MHz, DMSO-d6) δ 8.52 (s, 1H), 4.65-4.56 (m, 1H), 4.49-4.40 (m, 1H), 3.99-3.95 (m, 2H), 3.44 (t, 2H), 2.46-2.42 (m, 2H), 1.70-1.67 (m, 2H), 1.43 (d, 6H). LC-MS m/z(ESI) = **297.10** [M+1]

### Step 2:

### 2-Fluoro-4-((7-isopropyl-8-oxo-9-(tetrahydro-2H-pyran-4-yl)-8,9-dihydro-7H-purin-2-yl)amino)-5-methylbenzamide (compound 63)

The title compound 2-fluoro-4-((7-isopropyl-8-oxo-9-(tetrahydro-2*H*-pyran-4-yl)-8,9-dihydro-7*H*-purin-2-yl)amino)-5-methylbenzamide **compound 63** (white solid, 58 mg, 20.06% yield) was prepared according to the synthesis method of **compound 62.**

¹H NMR (400 MHz, Chloroform-d) δ 10.17 (s, 1H), 7.98 (d, 1H), 7.93 (s, 1H), 7.76 (d, 1H), 6.79 (d, 1H), 6.39 (s, 1H), 4.73 (t, 1H), 4.52 (t, 1H), 4.11 (dd, 2H), 3.49 (t, 2H), 2.63-2.55 (m, 1H), 2.40 (s, 3H), 1.75 (d, 2H), 1.51 (d, 6H).

LC-MS m/z(ESI) = **429.20** [M+1]

### Example 64

### 4-((7-Cyclopropyl-8-oxo-9-(tetrahydro-2H-pyran-4-yl)-8,9-dihydro-7H-purin-2-yl)amino)-2-fluoro-5-methylbenzamide (compound 64)

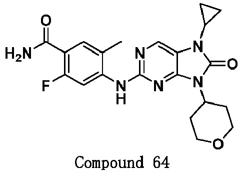

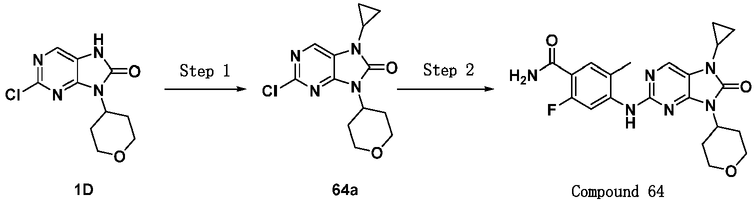

### Step 1:

### 2-Chloro-7-cyclopropyl-9-(tetrahydro-2H-pyran-4-yl)-7,9-dihydro-8H-purin-8-one (64a)

2-Chloro-9-(tetrahydro-2*H*-pyran-4-yl)-7,9-dihydro-8*H*-purine-8-one **1D** (1 g, 3.9 mmol), cyclopropylboronic acid (676 mg, 7.8 mmol), anhydrous copper(□) acetate (715 mg, 3.9 mmol), potassium carbonate (1.06 g, 7.8 mmol) and 1,10-phenanthroline (710 mg, 3.9 mmol) were dissolved in 1,2-dichloroethane (12 mL), and the reaction mixture was stirred open to air, then heated to 70 °C and stirred for 6 h. The reaction was monitored by TLC. After completion of the reaction,the reaction mixture was concentrated, and the residue was purified by silica gel column chromatography (petroleum ether/ethyl acetate (v/v) = 3:1) to obtain the title compound 2-chloro-7-cyclopropyl-9-(tetrahydro-2*H*-pyran-4-yl)-7,9-dihydro-8*H*-purin-8-one **64a** (white solid, 690 mg, 56 % yield).

¹H NMR (400 MHz CDCI3) δ 8.15 (s, 1H), 4.59-4.51 (m, 1H), 4.12 (dd, 2H), 3.55-3.49 (m, 2H), 2.96-2.91 (m, 1H), 2.77-2.67 (m, 2H), 1.77-1.68 (m, 2H), 1.26-1.13 (m, 2H), 1.04-1.00(m, 2H).

LC-MS m/z(ESI) = **295.20** [M+1]

### Step 2:

### 4-((7-Cyclopropyl-8-oxo-9-(tetrahydro-2H-pyran-4-yl)-8,9-dihydro-7H-purin-2-yl)amino)-2-fluoro-5-methylbenzamide (compound 64)

The title compound 4-((7-cyclopropyl-8-oxo-9-(tetrahydro-2*H*-pyran-4-yl)-8,9-dihydro-7*H*-purin-2-yl)amino)-2-fluoro-5-methylbenzamide **compound 64** (white solid, 20 mg, 16.2% yield) was prepared according to the synthesis method of **compound 62.**

¹H NMR (400 MHz DMSO) δ 8.56 (s, 1H), 8.11 (s, 1H), 7.88 (d, 1H), 7.54 (d, 1H), 27.43 (d, 2H), 5.98-5.87 (m, 1H), 5.24-5.19 (m, 2H), 4.49-4.42 (m, 3H), 3.99 (dd, 2H), 3.43 (t, 2H), 2.59-2.51 (m, 2H), 2.29 (s, 1H), 1.71 (dd, 2H).

LC-MS m/z(ESI) = **427.20** [M+1]

### Example 65

### 4-((9-(trans-3-Cyanocyclobutyl)-7-ethyl-8-oxo-8,9-dihydro-7H-purin-2-yl)amino)-2-fluoro-5-methylbenzamide (compound 65)

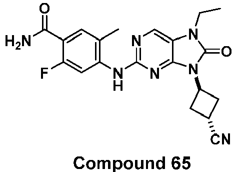

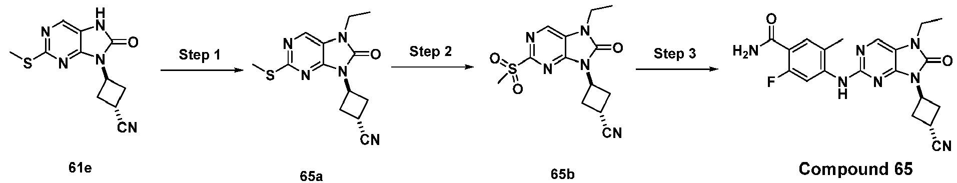

### Step 1:

### trans-3-(7-Ethyl-2-(methylthio)-8-oxo-7,8-dihydro-9H-purin-9-yl)cyclobutane-1-carbonitrile (65a)

trans-3-(2-(Methylthio)-8-oxo-7,8-dihydro-9H-purin-9-yl)cyclobutane-1-carbonitrile **61e** (500 mg, 19.1 mmol) was dissolved in dimethylformamide (5 mL), and iodoethane (3.0 g, 19.1 mmol) and sodium hydroxide (1.53 g, 38.2 mmol) were added at 0 °C. The reaction mixture was stirred at 0 °C for 0.5 h. The reaction was monitored by TLC. After completion of the reaction, 50 mL of water was added, and a solid was precipitated. The reaction mixture was filtered and concentrated by rotary evaporation under reduced pressure to obtain the title compound *trans*-3-(7-ethyl-2-(methylthio)-8-oxo-7,8-dihydro-9*H*-purin-9-yl)cyclobutane-1-carbonitrile **65a** (white solid, 457 mg, 82.5% yield).

1HNMR (400 MHz, DMSO-d6) δ 8.68 (s, 1H), 4.97-4.87 (m, 1H), 3.93(q, 2H), 3.34(s, 3H), 3.33-3.23(m, 2H), 2.91(s, 3H), 2.74-2.59(m, 2H), 1.27(t, 3H).

LC-MS m/z (ESI) = 290.10 [M+1]

### Step 2:

### trans-3-(7-Ethyl-2-(methylsulfonyl)-8-oxo-7,8-dihydro-9H-purin-9-yl)cyclobutane-1-carbonitrile (65b)

*Trans*-3-(7-Ethyl-2-(methylthio)-8-oxo-7,8-dihydro-9H-purin-9-yl)cyclobutane-1-carbonitrile **65a** (457 mg, 1.58 mmol) was dissolved in methanol (10 mL), and 2 mL of purified water was added, followed by the addition of potassium peroxymonosulfate (1.00 g, 1.58 mmol). The reaction mixture was heated to 60 °C and stirred for 2 h. The reaction was monitored by TLC. After completion of the reaction, the reaction mixture was concentrated under reduced pressure to remove methanol, and 5 mL of purified water was added. The resulting mixture was extracted 3 times with dichloromethane (20 mL × 3), and the organic phases were combined, dried over anhydrous sodium sulfate, and concentrated by rotary evaporation under reduced pressure to remove the solvent and thus to obtain *trans*-3-(7-ethyl-2-(methylsulfonyl)-8-oxo-7,8-dihydro-9H-purin-9-yl)cyclobutane-1-carbonitrile **65b** (pale yellow solid, 227 mg, 44.6% yield).

LC-MS m/z(ESI) = 322.10 [M+1]

### Step 3:

### 4-((9-(trans-3-Cyanocyclobutyl)-7-ethyl-8-oxo-8,9-dihydro-7H-purin-2-yl)amino)-2-fluoro-5-methylbenzamide (compound 65)

*trans*-3-(7-Ethyl-2-(methylsulfonyl)-8-oxo-7,8-dihydro-9*H*-purin-9-yl)cyclobutane-1-carbonitrile **65b** (200 mg, 0.62 mmol) and 4-amino-3-methyl-2-fluorobenzamide **intermediate 2** (538 mg, 3.20 mmol) were dissolved in *N*,*N*-dimethylformamide (3 mL), followed by notrogen purging and the addition of potassium *tert*-butoxide (143 mg, 1.28 mmol). The reaction mixture was stirred under nitrogen at room temperature for 4 h. The reaction was monitored by TLC. After completion of the reaction, the reaction mixture was poured into 30 mL of purified water, and a large amounts of yellow solid was precipitated. The solid was collected by filtration and purified by silica gel column chromatography (dichloromethane/methanol (v/v) = 35/1) to obtain the title compound 4-((9-(*cis*-3-cyanocyclobutyl)-7-ethyl-8-oxo-8,9-dihydro-7*H*-purin-2-yl)amino)-2-fluoro-5-methylbenzamide **compound 65** (white solid, 15 mg, 5.9% yield).

¹H NMR (400 MHz, DMSO-d6) δ8.64 (s, 1H), 8.27(s, 1H), 7.86(d 1H), 7.56-7.54(m, 1H), 7.48(s, 1H), 7.40(s, 1H), 5.14-5.09(m, 1H), 3.86-3.82(m, 2H), 3.64-3.59(m, 1H), 3.26-3.16(m, 2H), 2.75-2.68(m, 2H), 2.29(s, 3H), 1.22(t, 3H).

LC-MS m/z(ESI) = 410.20 [M+1]

### Example 66

### 2-Fluoro-4-((9-(2-isopropyl-4-methylpyridin-3-yl)-7-methyl-8-oxo-8,9-dihydro-7H-purin-2-yl)amino)-5-methylbenzamide (compound 66)

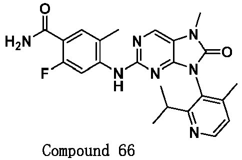

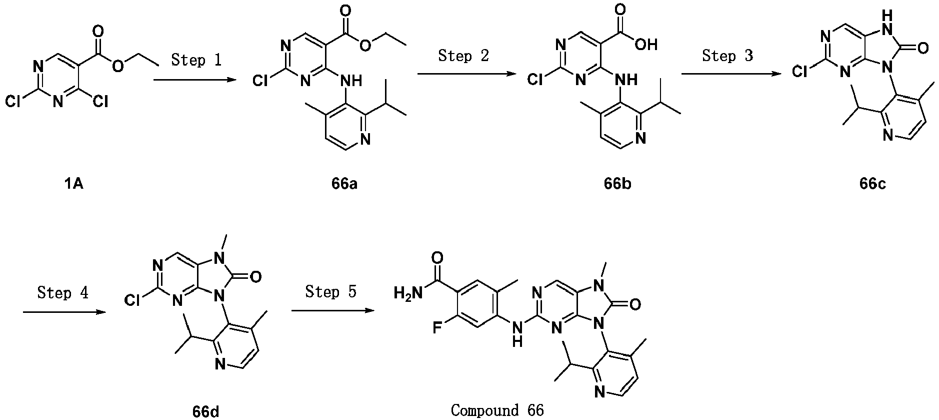

### Step 1: Ethyl 2-chloro-4-((2-isopropyl-4-methylpyridin-3-yl)amino)pyrimidine-5-carboxylate (66a)

2-Isopropyl-4-methylpyridin-3-amine (21.61 g, 143.92 mmol) and ethyl 2,4-dichloropyrimidine-5-carboxylate **1A** (26.51 g, 119.93 mmol) were dissolved in acetonitrile (400 mL), and potassium carbonate (33.15 g, 239.86 mmol) was added with stirring at 0 °C. The reaction mixture was gradually heated to 90 °C and stirred for 72 h. The reaction was monitored by TLC. After completion of the reaction, water and ethyl acetate were added to the reaction mixture for extraction, and the organic phases were combined, dried over anhydrous sodium sulfate, filtered and concentrated to obtain the title compound ethyl 2-chloro-4-((2-isopropyl-4-methylpyridin-3-yl)amino)pyrimidine-5-carboxylate **66a** (brown solid, 44.23 g, 91.95% yield).

¹H NMR (400 MHz, DMSO-d6) δ 9.83 (s, 1H), 8.77 (s, 1H), 8.39 (d, 1H), 7.20 (d, 1H), 4.39 (q, 2H), 3.10-3.03 (m, 1H), 2.13 (s, 3H), 1.36 (t, 3H), 1.12 (d, 6H).

LC-MS m/z(ESI) = **335.10** [M+1]

### Step 2:

### 2-Chloro-4-((2-isopropyl-4-methylpyridin-3-yl)amino)pyrimidine-5-carboxylic acid (66b)

Ethyl 2-chloro-4-((2-isopropyl-4-methylpyridin-3-yl)amino)pyrimidine-5-carboxylate **66a** (44.23 g, 131.42 mmol) was dissolved in tetrahydrofuran/water (200 mL/200 mL), and lithium hydroxide (11.03 g, 262.84 mmol) was added thereto. The reaction mixture was stirred at room temperature for 3 h. The reaction was monitored by TLC. After completion of the reaction, the reaction mixture was concentrated by rotary evaporation under reduced pressure to remove tetrahydrofuran and the pH was adjusted to 1-2 with 2 N hydrochloric acid, and a solid was precipitated. The reaction mixture was filtered, and the filter cake was washed with water and dried to obtain the title compound 2-chloro-4-((2-isopropyl-4-methylpyridin-3-yl)amino)pyrimidine-5-carboxylic acid **66b** (brown solid, 29.29 g, 72.66% yield).

¹H NMR (400 MHz, DMSO-d6) δ 10.23 (s, 1H), 8.72 (s, 1H), 8.38 (d, 1H), 7.20 (d, 1H), 3.11-3.04 (m, 1H), 2.13 (s, 3H), 1.26 (d, 1H), 1.12 (d, 6H).

LC-MS m/z(ESI) = **307.10** [M+1]

### Step 3:

### 2-Chloro-9-(2-isopropyl-4-methylpyridin-3-yl)-7,9-dihydro-8H-purin-8-one (66c)

2-Chloro-4-((2-isopropyl-4-methylpyridin-3-yl)amino)pyrimidine-5-carboxylic acid **66b** (25.00 g, 81.5 mmol) was dissolved in tetrahydrofuran. The solution was cooled to 0 °C, and diphenylphosphoryl azide (24.6 g, 89.5 mmol) and triethylamine (9.8 g, 97.75 mmol) were added. The reaction mixture was stirred for 1 h at room temperature, and gradually heated to 120 °C for another 2 h. The reaction was monitored by TLC. After completion of the reaction, water and ethyl acetate were added to the reaction mixture for extraction, and the organic phases were combined, dried over anhydrous sodium sulfate and filtered. The filtrate was concentrated, and the residue was purified by silica gel column chromatography (petroleum ether/ethyl acetate (v/v) = 1/1) to obtain the title compound 2-chloro-9-(2-isopropyl-4-methylpyridin-3-yl)-7,9-dihydro-8*H*-purin-8-one **66c** (white solid, 18 g, 75% yield).

¹H NMR (400 MHz, DMSO-d6) δ 12.06 (s, 1H), 8.59 (d, 1H), 8.33 (s, 1H), 7.35 (dd, 1H), 2.80-2.73(m, 1H), 2.05 (s, 3H), 1.09 (dd, 6H).

LC-MS m/z(ESI) = **304.10** [M+1]

### Step 4:

### 2-Chloro-9-(2-isopropyl-4-methylpyridin-3-yl)-7-methyl-7,9-dihydro-8H-purin-8-one (66d)

**2-Chloro-9-(2-isopropyl-4-methylpyridin-3-yl)-7,9-dihydro-8H-purin-8-one 66c (500** mg, 1.65 mmol) was dissolved in *N*,*N*-dimethylformamide (10 mL), and cesium carbonate (536 mg, 1.65 mmol) and dimethyl sulfate (269 mg, 2.14 mmol) were added at 0 °C. The reaction mixture was stirred at 0 °C for 1.5 h. The reaction was monitored by TLC. After completion of the reaction, water was added and the organics were extracted three times with ethyl acetate, and the organic phase was dried over anhydrous sodium sulfate and concentrated. The residue was purified by silica gel column chromatography (petroleum ether/ethyl acetate (v/v) = 1/1) to yield the title compound 2-chloro-9-(2-isopropyl-4-methylpyridin-3-yl)-7-methyl-7,9-dihydro-8*H*-purin-8-one **66d** (white solid, 258 mg, 49.32% yield).

¹H NMR (400 MHz, DMSO-d6) δ 8.60 (d, 1H), 8.56 (s, 1H), 7.36 (d, 1H), 3.49 (s, 3H), 2.71-2.78 (m, 1H), 2.04 (s, 3H), 1.10 (d, 3H), 1.06 (d, 3H).

LC-MS m/z(ESI) = **318.10** [M+1]

### Step 5:

### 2-Fluoro-4-((9-(2-isopropyl-4-methylpyridin-3-yl)-7-methyl-8-oxo-8,9-dihydro-7H-purin-2-yl)amino)-5-methylbenzamide (compound 66)

2-Chloro-9-(2-isopropyl-4-methylpyridin-3-yl)-7-methyl-7,9-dihydro-8*H*-purin-8-one **66d** (100 mg, 0.31 mmol), 4-amino-2-fluoro-5-methylbenzamide **intermediate 2** (210 mg, 1.26 mmol), cesium carbonate (410 mg, 1.26 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (43 mg, 0.04 mmol) were dissolved in 1,4-dioxane (4 mL), followed by nitrogen purging. The reaction mixture was stirred under nitrogen at 110 °C for 6 h. The reaction was monitored by TLC. After completion of the reaction,the reaction mixture was concentrated and purified by silica gel column chromatography (dichloromethane/methanol (v/v) = 20/1), and further purified by Pre-HPLC to obtain the title compound 2-fluoro-4-((9-(2-isopropyl-4-methylpyridin-3-yl)-7-methyl-8-oxo-8,9-dihydro-7*H*-purin-2-yl)amino)-5-methylbenzamide **compound 66** (white solid, 40 mg, 28.28% yield).

¹H NMR (400 MHz, DMSO-d6) δ 8.66 (s, 1H), 8.56 (d, 1H), 8.33 (s, 1H), 7.73 (s, 0.5H), 7.69 (s, 0.5H), 7.48 (d, 1H), 7.44 (s, 1H), 7.38 (d, 1H), 7.34 (d, 1H), 3.45 (s, 3H), 2.85-2.78 (m, 1H), 2.20 (s, 3H), 2.06 (s, 3H), 1.13 (s, 3H), 1.11 (s, 3H).

LC-MS m/z(ESI) = **450.20** [M+1]

### Example 67

### 2-Fluoro-4-((9-(3-hydroxycyclopentyl)-7-methyl-8-oxo-8,9-dihydro-7H-purin-2-yl)amino)-5-methylbenzamide (compound 67)

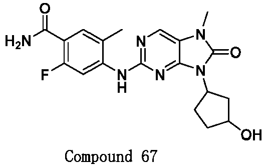

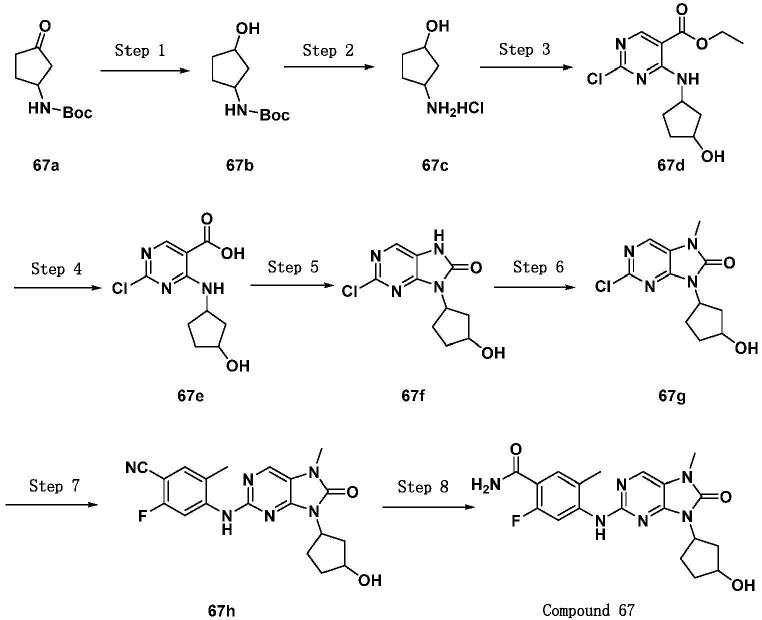

### Step 1:

### tert-Butyl (3-hydroxycyclopentyl)carbamate (67b)

*tert*-Butyl (3-oxocyclopentyl) carbamate **67a** (10 g, 50.19 mmol) was dissolved in 60 mL of tetrahydrofuran and sodium borohydride (2.85 g, 75.28 mmol) was added slowly in an ice bath. The reaction mixture was stirred for reaction for 3 h. The reaction was monitored by TLC. After completion of the reaction, saturated NaCl solution was slowly added until no air bubbles appeared, and extracted with ethyl acetate. The organic phase was concentrated to yield the title compound *tert*-butyl (3-hydroxycyclopentyl)carbamate **67b** (yellow oily liquid, 10.10 g, 99% yield).

LC-MS m/z(ESI) = **202.20** [M+1]

### Step 2:

### 3-Aminocyclopentan-1-ol hydrochloride (67c)

*tert*-Butyl (3-hydroxycyclopentyl)carbamate **67b** (10.10 g, 50.18 mmol) was added to a solution of 1,4-dioxane (20 mL), and the reaction mixture was stirred at room temperature until completely dissolved, placed in an ice bath, added slowly with 4 M hydrochloric acid-1,4 dioxane solution (8.75 g, 239.87 mmol) and stirred for 2 h. The reaction was monitored by TLC. After completion of the reaction, the reaction mixture was filtered, and the filtrate was concentrated to yield the title compound 3-aminocyclopentan-1-ol hydrochloride **67c** (black oily liquid, crude product, 6.91 g, 100% yield) which was used directly in the next step.

LC-MS m/z(ESI) = **138.10** [M+1]

### Step 3:

### Ethyl 2-chloro-4-((3-hydroxycyclopentyl)amino)pyrimidine-5-carboxylate (67d)

3-Aminocyclopentane-1-ol hydrochloride **67c** (6.91 g, 50.22 mmol) and ethyl 2,4-dichloropyrimidine-5-carboxylate (13.32 g, 60.26 mmol) were added to 75 mL of acetonitrile, and after being stirred at room temperature until dissolved, the reaction mixture was added slowly with potassium carbonate (17.35 g, 125.54 mmol) and stirred for 10 h. The reaction was monitored by TLC. After completion of the reaction, the reaction mixture was concentrated under reduced pressure to remove acetonitrile, and water was added and the organics were extracted with ethyl acetate. The organic phase was concentrated, and the residue was purified by silica gel column chromatography (petroleum ether/ethyl acetate (v/v) = 1/1) to yield the title compound ethyl 2-chloro-4-((3-hydroxycyclopentyl)amino)pyrimidine-5-carboxylate **67d** (yellow oily liquid, 7.78 g, 54% yield).

LC-MS m/z(ESI) = **286.10** [M+1]

### Step 4:

### 2-Chloro-4-((3-hydroxycyclopentyl)amino)pyrimidine-5-carboxylic acid (67e)

Ethyl 2-chloro-4-((3-hydroxycyclopentyl)amino)pyrimidine-5-carboxylate **67d** (7.78 g, 27.23 mmol) was dissolved in 50 mL of tetrahydrofuran, lithium hydroxide (1.3 g, 54.46 mmol) was dissolved in 50 mL of water, an aqueous solution of lithium hydroxide was slowly added to the THF solution in an ice bath and the reaction mixture was stirred for 2 h. The reaction was monitored by TLC. After completion of the reaction,the reaction mixture was concentrated by rotary evaporation under reduced pressure to remove tetrahydrofuran and the pH was adjusted to about 4 with hydrochloric acid solution, and a solid was precipitated. The solid was collected by filtration, and the filtrate was extracted with ethyl acetate. The organic phase and the filter residue were combined and concentrated to obtain the title compound 2-chloro-4-((3-hydroxycyclopentyl)amino)pyrimidine-5-carboxylic acid **67e** (pale yellow solid, 6.78 g, 96% yield).

LC-MS m/z(ESI) = **258.10** [M+1]

### Step 5:

### 2-Chloro-9-(3-hydroxycyclopentyl)-7,9-dihydro-8H-purin-8-one (67f)

2-Chloro-4-((3-hydroxycyclopentyl)amino)pyrimidine-5-carboxylic acid **67e** (6.0 g, 23.29 mmol) was dissolved in 60 mL of *N*,*N*-dimethylacetamide, diphenylphosphoryl azide (6.41 g, 23.29 mmol) and triethylamine (2.36 g, 23.29 mmol) were slowly added in an ice bath, and the reaction mixture was stirred at room temperature for 1 h, and then stirred at 90 °C for 5 h. The reaction was monitored by TLC. After completion of the reaction, the reaction mixture was added dropwise to 200 mL of water, and extracted with ethyl acetate. The organic phase was collected and concentrated to obtain the title compound 2-chloro-9-(3-hydroxycyclopentyl)-7,9-dihydro-8*H*-purin-8-one **67f** (yellow oily liquid, 5.47 g, 92% yield).

LC-MS m/z(ESI) = **255.10** [M+1]

### Step 6:

### 2-Chloro-9-(3-hydroxycyclopentyl)-7-methyl-7,9-dihydro-8H-purin-8-one (67h)

2-Chloro-9-(3-hydroxycyclopentyl)-7,9-dihydro-8*H*-purin-8-one **67f** (2.5 g, 9.82 mmol) and cesium carbonate (4.16 g, 12.76 mmol) were dissolved in 40 mL of DMF, and the reaction mixture was stirred in an ice bath for 10 min, then added slowly with dimethyl sulfate (1.24 g, 9.82 mmol) and reacted for 1 h. The reaction was monitored by TLC. After completion of the reaction, 100 mL of water was added and the organics were extracted with ethyl acetate, and the organic phase was collected, concentrated and purified by column chromatography to yield the title compound 2-chloro-9-(3-hydroxycyclopentyl)-7-methyl-7,9-dihydro-8*H*-purin-8-one **67g** (orange solid, 900 mg, 34% yield).

¹H NMR (400 MHz, DMSO-d6) δ 8.35 (s, 1H), 4.90 (d, 1H), 4.71-4.62 (m, 1H), 4.16-4.08 (m, 1H), 3.35 (s, 3H), 2.29-2.23 (m, 1H), 2.17-2.10(m, 2H), 1. 92-1.77 (m, 3H). LC-MS m/z(ESI) = **269.10** [M+1]

### Step 7:

### 2-Fluoro-4-((9-(3-hydroxycyclopentyl)-7-methyl-8-oxo-8,9-dihydro-7H-purin-2-yl)amino)-5-methylbenzonitrile (50h)

2-Chloro-9-(3-hydroxycyclopentyl)-7-methyl-7,9-dihydro-8*H*-purin-8-one **67g** (600 mg, 2.23 mmol), 4-amino-2-fluoro-5-methylbenzonitrile **2A** (335 mg, 2.23 mmol), cesium carbonate (2.18 g, 6.70 mmol), and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (303 mg, 0.34 mmol) were dissolved in 1,4-dioxane (10 mL), and the reaction mixture was purged with nitrogen and stirred at 110 °C for 6 h. The reaction was monitored by TLC. After completion of the reaction, the reaction mixture was concentrated, and the residue was purified by silica gel column chromatography (dichloromethane/methanol (v/v) = 20/1) to obtain the title compound 2-fluoro-4-((9-(3-hydroxycyclopentyl)-7-methyl-8-oxo-8,9-dihydro-7*H*-purin-2-yl)amino)-5-methylbenzonitrile **67h** (pale yellow solid, 460 mg, 54% yield). ¹H NMR (400 MHz, DMSO-d6) δ 8.74 (d, 1H), 8.25 (d, 1H), 8.20-8.15 (m, 1H), 7.66 (d, 1H), 5.05-4.96 (m, 0.5H), 4.83 (d, 0.5H), 4.73-4.64 (m, 0.5H), 4.66 (s, 0.5H), 4.13-4.08 (m, 1H), 3.34 (d, 3H), 2.31 (s, 3H), 2.15-2.09 (m, 3H), 1.96-1.75 (m, 3H). LC-MS m/z(ESI) = **383.20** [M+1]

### Step 8:

### 2-Fluoro-4-((9-(3-hydroxycyclopentyl)-7-methyl-8-oxo-8,9-dihydro-7H-purin-2-yl)amino)-5-methylbenzamide (compound 67)

2-Fluoro-4-((9-(3-hydroxycyclopentyl)-7-methyl-8-oxo-8,9-dihydro-7*H*-purin-2-yl)amino)-5-methylbenzonitrile **67h** (400 mg, 1.05 mmol) and potassium carbonate (43.4 mg, 313.81 mmol) were dissolved in 20 mL of dimethyl sulfoxide, and hydrogen peroxide (1.05 mL, 10.46 mmol) was slowly added with stirring at room temperature. The reaction mixture was stirred for 3 h. The reaction was monitored by TLC. After completion of the reaction, the reaction mixture was concentrated, and the residue was purified by preparative medium pressure liquid chromatography, followed by Pre-HPLC to obtain the title compound 2-fluoro-4-((9-(3-hydroxycyclopentyl)-7-methyl-8-oxo-8,9-dihydro-7*H*-purin-2-yl)amino)-5-methylbenzamide **compound 67,** which was then resolved by SFC to yield **compound 67-1** (white solid, 70 mg, 17% yield, dr = 99%) and **compound 67-2** (white solid, 58 mg, 14% yield, dr = 99%). (OZ), mobile phase: CO₂/isopropanol = 70/30; column temperature: 35 °C; column pressure: 80 bar; flow rate: 1 mL/min; detector signal channel: 220 nm@4 nm; diode array detector at a wavelength of 200-400 nm.

### Compound 67-1:

¹H NMR (400 MHz, DMSO-d6) δ 8.48 (s, 1H), 8.19 (s, 1H), 7.88 (d, 1H), 7.54 (d, 1H), 7.47 (s, 1H), 7.37 (d, 1H), 4.86 (d, 1H), 4.72-4.63 (m, 1H), 4.14-4.06 (m, 1H), 3.32(s, 3H), 2.42-2.32 (m, 1H), 2.28 (s, 3H), 2.16-2.10 (m, 2H), 1.93-1.76 (m,3H).

LC-MS m/z(ESI) = **400.20** [M+1]

### Compound 67-2:

¹H NMR (400 MHz, DMSO-d6) δ 8.51 (s, 1H), 8.18 (s, 1H), 7.86 (d, 1H), ,7.54 (d, 1H), 7.46 (s, 1H), 7.38 (d,1H), 5.03-4.94 (m, 1H), 4.64(d, 1H), 4.35 (s,1H), 3.32(s,3H), 2.38-2.31(m,1H), 2.28 (s, 3H), 2.15-2.03(m,3H)1.85-1.80(m, 1H), 1.62-1.57(m, 1H). LC-MS m/z(ESI) = **400.20** [M+1]

### Example 68

### 4-((9-Cyclohexyl-7-methyl-8-oxo-8,9-dihydro-7H-purin-2-yl)amino)-2-fluoro-5-methylbenzamide (compound 68)

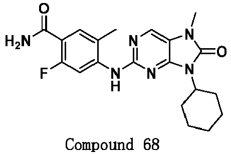

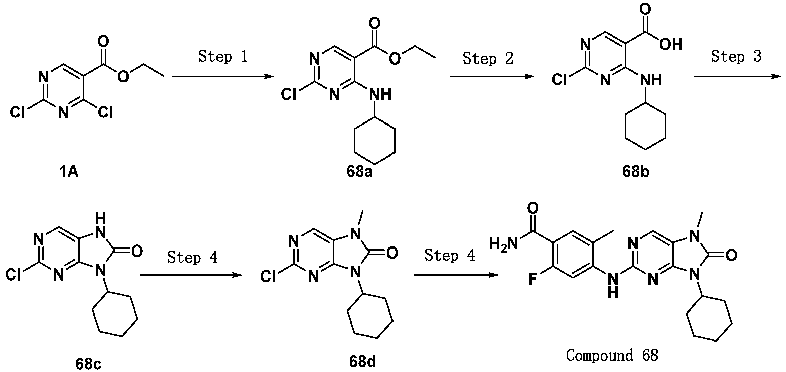

### Step 1:

### Ethyl 2-chloro-4-(cyclohexylamino)pyrimidine-5-carboxylate (68a)

Ethyl 2,4-dichloropyrimidine-5-carboxylate **1A** (5.0 g, 22.6 mmol) and potassium carbonate (6.2 g, 45.2 mmol) were dissolved in acetonitrile (50 mL), and cyclohexylamine (2.2 g, 22.6 mmol) was added at 0 °C. The reaction mixture was stirred at room temperature for 20 h. The reaction was monitored by TLC. After completion of the reaction, water was added and the organics were extracted with ethyl acetate., and was dried over anhydrous sodium sulfate and purified by silica gel column chromatography (*n*-hexane/ethyl acetate (v/v) = 10: 1) to obtain the title compound ethyl 2-chloro-4-(cyclohexylamino)pyrimidine-5-carboxylate **68a** (white solid, 4.1 g, 64% yield).

¹H NMR (400 MHz, Chloroform-d) δ 8.64 (s, 1H), 8.53-8.23 (m, 1H), 4.35 (q, 2H), 4.25-4.05 (m, 1H), 2.01-1.95 (m, 2H), 1.77-1.72(m, 2H), 1.64-1.61 (m, 1H), 1.50-1.41 (m, 3H), 1.39 (t, 3H), 1.35-1.18 (m, 2H).

LC-MS m/z(ESI) = **284.10** [M+1]

### Step 2:

### 2-Chloro-4-(cyclohexylamino)pyrimidine-5-carboxylic acid (68b)

Ethyl 2-chloro-4-(cyclohexylamino)pyrimidine-5-carboxylate **68a** (4.1 g, 14.4 mmol) was dissolved in tetrahydrofuran/water (20 mL/20 mL), and lithium hydroxide (691 mg, 28.8 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound 2-chloro-4-(cyclohexylamino)pyrimidine-5-carboxylic acid **68b** (white solid, 3.0 g, 81% yield).

¹H NMR (400 MHz, DMSO-d6) δ 13.77(s, 1H), 8.76-8.45 (m, 2H), 4.01-3.92 (m, 1H), 2.05-1.79 (m, 2H), 1.79-1.65 (m, 2H), 1.60-1.54 (m, 1H), 1.42-1.19 (m, 5H). LC-MS m/z(ESI) = **256.10** [M+1]

### Step 3:

### 2-Chloro-9-cyclohexyl-7,9-dihydro-8H-purin-8-one (68c)

2-Chloro-4-(cyclohexylamino)pyrimidine-5-carboxylic acid **68b** (3.0 g, 11.7 mmol) was dissolved in dimethylacetamide (30 mL), and triethylamine (1.18 g, 11.7 mmol) and diphenylphosphoryl azide (3.22 g, 11.7 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound 2-chloro-9-cyclohexyl-7,9-dihydro-8*H*-purin-8-one **68c** (white solid, 2.2 g, 74% yield).

¹H NMR (400 MHz, DMSO-d6) δ 11.60 (s, 1H), 8.12 (s, 1H), 4.19-4.11 (m, 1H), 1.92-1.57 (m, 6H), 1.52-0.93 (m, 4H).

LC-MS m/z(ESI) = **253.10** [M+1]

### Step 4:

### 2-Chloro-9-cyclohexyl-7-methyl-7,9-dihydro-8H-purin-8-one (68d)

2-Chloro-9-cyclohexyl-7,9-dihydro-8*H*-purin-8-one **68c** (2.2 g, 8.7 mmol) was dissolved in dimethylformamide (15 mL), and dimethyl sulfate (1.1 g, 8.7 mmol) and cesium carbonate (4.25 g, 13 mmol) were added at 0 °C. The reaction mixture was stirred for 0.5 h. The reaction was monitored by TLC. After completion of the reaction, water was added to the reaction mixture, and a solid was precipitated. The solid was collected by filtration to give the title compound 2-chloro-9-cyclohexyl-7-methyl-7,9-dihydro-8*H*-purin-8-one **68d** (yellow solid, 1.1 g, 48% yield).

¹H NMR (400 MHz, DMSO-d6) δ 8.34 (s, 1H), 4.22-4.41 (m, 1H), 3.35 (s, 3H), 2.22-2.12 (m, 2H), 1.91-1.60 (m, 5H), 1.43-1.33 (m, 2H), 1.27-1.14 (m, 1H).

LC-MS m/z(ESI) = **267.10** [M+1]

### Step 5:

### 4-((9-Cyclohexyl-7-methyl-8-oxo-8,9-dihydro-7H-purin-2-yl)amino)-2-fluoro-5-methylbenzamide (compound 68)

2-Chloro-9-cyclohexyl-7-methyl-7,9-dihydro-8*H*-purin-8-one **68d** (200 mg, 0.79 mmol), 4-amino-3-methyl-2-fluorobenzamide (292 mg, 1.74 mmol), cesium carbonate (515 mg, 1.58 mmol) and methanesulfonato(2-dicyclohexylphosphino-3,6-dimethoxy-2',4',6'-tri-i-propyl-1, 1 '-biphenyl)(2'-amino-1, 1 '-biphenyl-2-yl)palladium(II) (72 mg, 0.079 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to give the title compound 4-((9-cyclohexyl-7-methyl-8-oxo-8,9-dihydro-7*H*-purin-2-yl)amino)-2-fluoro-5-methylbenzamide **compound 68** (white solid, 80 mg, 25 % yield).

¹H NMR (400 MHz, DMSO-d6) δ 8.48 (s, 1H), 8.18 (s, 1H), 8.01 (d, 1H), 7.54 (d, 1H), 7.42 (d, 2H), 4.23-4.14 (m, 1H), 3.32 (s, 3H), 2.30 (s, 3H), 2.29-2.15 (m, 2H), 1.85-1.67 (m, 5H), 1.55-1.07 (m, 3H).

LC-MS m/z(ESI) = **399.20** [M+1]

### Example 69

### 2-Fluoro-5-methyl-4-((7-methyl-8-oxo-9-(tetrahydro-2H-pyran-3-yl)-8,9-dihydro-7H-purin-2-yl)amino)benzamide (compound 69)

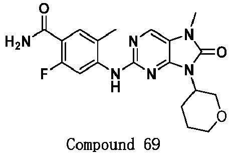

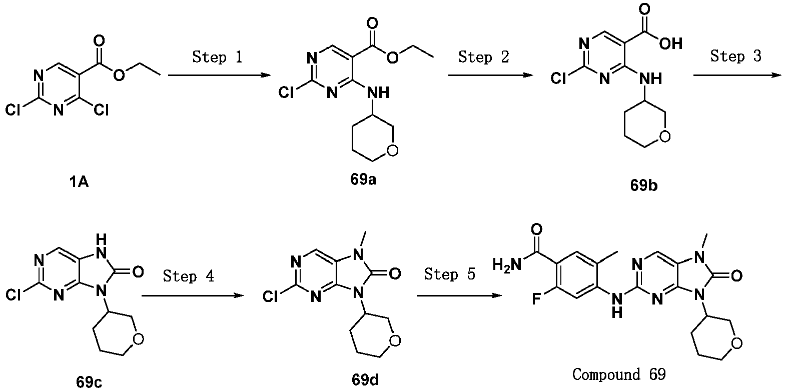

### Step 1:

### Ethyl 2-chloro-4-((tetrahydro-2H-pyran-3-yl)amino)pyrimidine-5-carboxylate (69a)

Ethyl 2,4-dichloropyrimidine-5-carboxylate **1A** (5.0 g, 22.6 mmol) and potassium carbonate (6.2 g, 45.2 mmol) were dissolved in acetonitrile (50 mL), and tetrahydro-2*H*-pyran-3-amine (2.3 g, 22.6 mmol) was added at 0 °C. The reaction mixture was stirred at room temperature for 20 h. water was added and the organics were extracted three times with ethyl acetate, and the organic phase was dried over anhydrous sodium sulfate and purified by silica gel column chromatography (*n*-hexane/ethyl acetate (v/v) = 10:1) followed by concentration to obtain the title compound ethyl 2-chloro-4-((tetrahydro-2*H*-pyran-3-yl)amino)pyrimidine-5-carboxylate **69a** (white solid, 4.1 g, 64% yield).

¹H NMR (400 MHz, Chloroform-d) δ 8.69 (s, 1H), 8.68 (s, 1H), 4.37(q, 2H),4.34-4.24 (m, 1H), 3.89-3.84 (m, 1H), 3.77-3.66 (m, 2H), 3.58-3.54 (m, 1H), 2.01-1.95 (m, 1H), 1.86-1.65 (m, 2H), 1.69-1.63 (m, 1H), 1.40 (t, 3H).

LC-MS m/z(ESI) = **286.10** [M+1]

### Step 2:

### 2-Chloro-4-((tetrahydro-2H-pyran-3-yl)amino)pyrimidine-5-carboxylic acid (69b)

Ethyl 2-chloro-4-((tetrahydro-2*H*-pyran-3-yl)amino)pyrimidine-5-carboxylate **69a** (4.1 g, 14.3 mmol) was dissolved in tetrahydrofuran/water (20 mL/20 mL), and lithium hydroxide (686 mg, 28.6 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to give the title compound 2-chloro-4-((tetrahydro-2*H*-pyran-3-yl)amino)pyrimidine-5-carboxylic acid **69b** (white solid, 3.5 g, 95% yield).

¹H NMR (400 MHz, DMSO-d6) δ 13.83 (s, 1H), 8.75 (d, 1H), 8.59 (s, 1H), 4.14-4.07 (m, 1H), 3.76-3.72 (m, 1H), 3.59 (t, 1H), 3.47-3.42 (m, 2H), 1.93-1.86 (m, 1H), 1.79-1.62 (m, 2H), 1.58-1.49(m, 1H).

LC-MS m/z(ESI) = **258.10** [M+1]

### Step 3:

### 2-Chloro-9-(tetrahydro-2H-pyran-3-yl)-7,9-dihydro-8H-purin-8-one (69c)

2-Chloro-4-((tetrahydro-2*H*-pyran-3-yl)amino)pyrimidine-5-carboxylic acid **69b** (3.5 g, 13.6 mmol) was dissolved in dimethylacetamide (30 mL), and triethylamine (1.37 g, 13.6 mmol) and diphenylphosphoryl azide (3.74 g, 13.6 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound 2-chloro-9-(tetrahydro-2*H*-pyran-3-yl)-7,9-dihydro-8*H*-purin-8-one **69c** (white solid, 2.2 g, 63% yield).

¹H NMR (400 MHz, DMSO-d6) δ 11.67 (s, 1H), 8.13 (s, 1H), 4.29-4.21 (m, 1H), 3.86-3.77 (m, 2H), 3.37-3.29 (m, 2H), 2.04-1.39 (m, 4H).

LC-MS m/z(ESI) = **255.10** [M+1]

### Step 4:

### 2-Chloro-7-methyl-9-(tetrahydro-2H-pyran-3-yl)-7,9-dihydro-8H-purin-8-one (69d)

2-Chloro-9-(tetrahydro-2*H*-pyran-3-yl)-7,9-dihydro-8*H*-purin-8-one **69c** (2.2 g, 8.7 mmol) was dissolved in dimethylformamide (15 mL), and dimethyl sulfate (1.1 g, 8.7 mmol) and cesium carbonate (4.25 g, 13 mmol) were added at 0 °C. The reaction mixture was stirred for 0.5 h. The reaction was monitored by TLC. After completion of the reaction, water was added to the reaction mixture, and a solid was precipitated. The solid was collected by filtration to give the title compound 2-chloro-9-cyclohexyl-7-methyl-7,9-dihydro-8*H*-purin-8-one **69d** (yellow solid, 1.7 g, 73% yield).

¹H NMR (400 MHz, DMSO-d6) δ 8.36 (s, 1H), 4.35-4.23 (m, 1H), 3.91-3.77 (m, 2H), 3.35 (s, 3H), 3.34-3.33(m, 2H), 2.50-2.44 (m, 1H), 1.98-1.89 (m, 1H), 1.83-1.62 (m, 2H).

LC-MS m/z(ESI) = **269.10** [M+1]

### Step 5:

### 2-Fluoro-5-methyl-4-((7-methyl-8-oxo-9-(tetrahydro-2H-pyran-3-yl)-8,9-dihydro-7H-purin-2-yl)amino)benzamide (compound 69)

2-Chloro-7-methyl-9-(tetrahydro-2*H*-pyran-3-yl)-7,9-dihydro-8H-purin-8-one **69d** (200 mg, 0.75 mmol), 4-amino-3-methyl-2-fluorobenzamide **intermediate 2** (402 mg, 1.50 mmol), cesium carbonate (489 mg, 1.50 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (68 mg, 0.075 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 2-fluoro-5-methyl-4-((7-methyl-8-oxo-9-(tetrahydro-2*H*-pyran-3-yl)-8,9-dihydro-7*H-*purin-2-yl)amino)benzamide **compound 69,** which was then resolved by SFC to yield **compound 69-1** (white solid, 32 mg, 11% yield, RT = 12.47 min) and **compound 69-**2 (white solid, 33 mg, 11% yield, RT = 14.31 min). (OZ), mobile phase: CO₂/ethanol = 65/35; column temperature: 35 °C; column pressure: 80 bar; flow rate: 1 mL/min; detector signal channel: 215 nm@4.8 nm; diode array detector at a wavelength of 200-400 nm.

¹H NMR (400 MHz, DMSO-d6) δ 8.54 (s, 1H), 8.20 (s, 1H), 7.97 (d, 1H), 7.56 (d, 1H), 7.43 (d, 2H), 4.36-4.28 (m, 1H), 3.99 (t, 1H), 3.94-3.74 (m, 2H), 3.37(d, 1H), 3.32 (s, 3H), 2.62-2.52 (m, 1H), 2.30 (s, 3H), 1.93 (d, 1H), 1.86-1.59 (m, 2H).

LC-MS m/z (ESI) = **401.20** [M+1]

### Example 70

### 2-Fluoro-4-((9-(cis-4-methoxycyclohexyl)-7-methyl-8-oxo-8,9-dihydro-7H-purin-2-yl)amino)-5-methylbenzamide (compound 70)

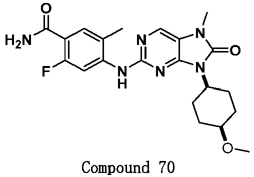

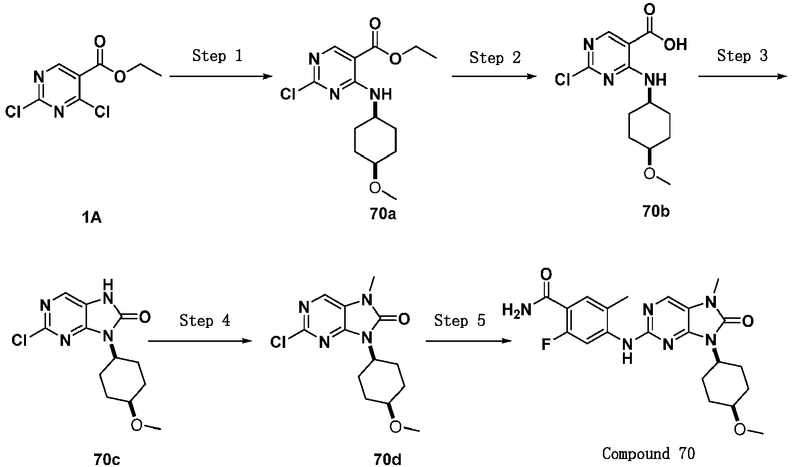

### Step 1:

### Ethyl 2-chloro-4-((cis-4-methoxycyclohexyl)amino)pyrimidine-5-carboxylate (70a)

Ethyl 2,4-dichloro-5-pyrimidinecarboxylate **1A** (5.13 g, 23.22 mmol) was dissolved in acetonitrile (20 mL), and potassium carbonate (6.42 g, 46.44 mmol) was added with stirring at 0 °C, followed by the slow dropwise addition of a solution of *cis*-4-methoxycyclohexan-1-amine (2.00 g, 15.48 mmol) in acetonitrile (10 mL). The reaction mixture was warmed to room temperature and stirred for 1 h. The reaction was monitored by TLC. After completion of the reaction, the reaction mixture was filtered through celite, and the filtrate was purified by silica gel column chromatography (petroleum ether/ethyl acetate (v/v) = 2:1) to obtain the title compound ethyl 2-chloro-4-((*cis*-4-methoxycyclohexyl)amino)pyrimidine-5-carboxylate **70a** (white solid, 2.00 g, 41.26% yield).

LC-MS m/z(ESI) = **314.10** [M+1]

### Step 2:

### 2-Chloro-4-((cis-4-methoxycyclohexyl)amino)pyrimidine-5-carboxylic acid (70b)

Ethyl 2-chloro-4-((*cis*-4-methoxycyclohexyl)amino)pyrimidine-5-carboxylate **70a** (2.00 g, 6.37 mmol) was dissolved in tetrahydrofuran/water (15 mL/15 mL), and lithium hydroxide monohydrate (0.80 g, 19.12 mmol) was added. The reaction mixture was stirred at room temperature for 2 h. The reaction was monitored by TLC. After completion of the reaction, the reaction mixture was concentrated to evaporate tetrahydrofuran and the pH was adjusted to 3-4 with 2 N HCl, and a white solid was precipitated. The mixture was filtered, and the filter cake was washed twice with water and petroleum ether/ethyl acetate (v/v = 10/1) to yield the title compound 2-chloro-4-((*cis*-4-methoxycyclohexyl)amino)pyrimidine-5-carboxylic acid **70b** (white solid, 1.67 g, 91.70% yield).

LC-MS m/z(ESI) = **286.10** [M+1]

### Step 3:

### 2-Chloro-9-(cis-4-methoxycyclohexyl)-7,9-dihydro-8H-purin-8-one (70c)

2-Chloro-4-((*cis*-4-methoxycyclohexyl)amino)pyrimidine-5-carboxylic acid **70b** (1.57 g, 5.49 mmol) was dissolved in *N,N*-dimethylacetamide (16 mL), and triethylamine (0.76 mL, 5.49 mmol) and diphenylphosphoryl azide (1.18 mL, 5.49 mmol) were added with stirring at room temperature. The reaction mixture was stirred for 2 h, then heated to 110 °C and refluxed for 2.5 h. The reaction was monitored by TLC. After completion of the reaction, water was added and the organics were extracted with dichloromethane. The organic layer was concentrated to give the title compound 2-chloro-9-(cis-4-methoxycyclohexyl)-7,9-dihydro-8*H*-purin-8-one **70c** (white solid, 1.70 g, crude product, 109.47% yield).

LC-MS m/z(ESI) = **283.00** [M+1]

### Step 4:

### 2-Chloro-9-(cis-4-methoxycyclohexyl)-7-methyl-7,9-dihydro-8H-purin-8-one (70d)

2-Chloro-9-(*cis*-4-methoxycyclohexyl)-7,9-dihydro-8*H*-purin-8-one **70c** (1.70 g, 6.01 mmol) was dissolved in *N*,*N*-dimethylformamide (20 mL), and dimethyl sulfate (0.57 mL, 6.01 mmol) and cesium carbonate (3.92 g, 12.03 mmol) were added with stirring at 0 °C. The reaction mixture was stirred for 2 h. The reaction was monitored by TLC. After completion of the reaction, the reaction mixture was slowly added dropwise to ice water. The mixture was stirred, and a white solid was precipitated. The solid was collected by filtration and washed 3 times with water and petroleum ether to give the title compound 2-chloro-9-(cis-4-methoxycyclohexyl)-7-methyl-7,9-dihydro-8*H-*purin-8-one **70d** (white solid, 1.2 g, 67.25% yield).

LC-MS m/z(ESI) = **297.10** [M+1]

### Step 5:

### 2-Fluoro-4-((9-(cis-4-methoxycyclohexyl)-7-methyl-8-oxo-8,9-dihydro-7H-purin-2-yl)amino)-5-methylbenzamide (compound 70)

2-Chloro-9-(*cis*-4-methoxycyclohexyl)-7-methyl-7,9-dihydro-8*H*-purin-8-one **70d** (0.25 g, 0.84 mmol) was dissolved in 1,4-dioxane (8 mL), and 4-amino-2-fluoro-5-methylbenzamide **intermediate 2** (0.57 g, 3.37 mmol), cesium carbonate (0.41 g, 1.26 mmol) and BrettPhos-G3-Pd (0.08 g, 0.08 mmol) were added with stirring at room temperature, followed by nitrogen purging. The reaction mixture was heated to 110 °C and refluxed under nitrogen for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(cis-4-methoxycyclohexyl)-7-methyl-8-oxo-8,9-dihydro-7*H*-purin-2-yl)amino)-5-methylbenzamide **compound 70** (white solid, 95 mg, 26.39% yield).

¹H NMR (400 MHz, DMSO-d6) δ 8.47 (s, 1H), 8.16 (s, 1H), 7.82 (d, 1H), 7.55 (d, 1H), 7.44 (s, 1H), 7.35 (d, 1H), 4.26-4.18(m, 1H), 3.42-3.40 (m, 1H), 3.31 (s, 3H), 3.21 (s, 3H), 2.49-2.39 (m, 2H), 2.28 (s, 3H), 2.00 (d, 2H), 1.65-1.37 (m, 4H).

LC-MS m/z(ESI) = **429.20** [M+1]

### Example 71

### 2-Fluoro-4-((9-(trans-4-methoxycyclohexyl)-7-methyl-8-oxo-8,9-dihydro-7H-purin-2-yl)amino)-5-methylbenzamide (compound 71)

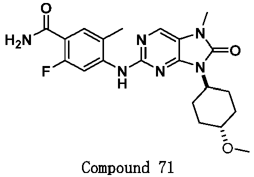

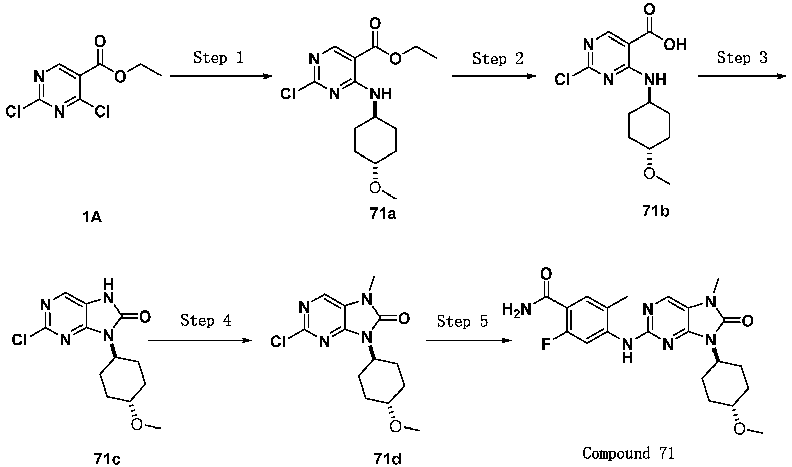

### Step 1:

### Ethyl 2-chloro-4-((trans-4-methoxycyclohexyl)amino)pyrimidine-5-carboxylate (71a)

Ethyl 2,4-dichloro-5-pyrimidinecarboxylate **1A** (5.13 g, 23.22 mmol) was dissolved in acetonitrile (20 mL), and potassium carbonate (6.42 g, 46.44 mmol) was added with stirring at 0 °C, followed by the slow dropwise addition of a solution of *trans*-4-methoxycyclohexan-1-amine (2.00 g, 15.48 mmol) in acetonitrile (10 mL). The reaction mixture was warmed to room temperature and stirred for 1 h. The reaction was monitored by TLC. After completion of the reaction, the reaction mixture was filtered through celite, and the filtrate was purified by silica gel column chromatography (petroleum ether/ethyl acetate (v/v) = 2:1) to get the title compound ethyl 2-chloro-4-((*trans*-4-methoxycyclohexyl)amino)pyrimidine-5-carboxylate **71a** (white solid, 2.00 g, 41.26% yield).

LC-MS m/z(ESI) = **314.10** [M+1]

### Step 2:

### 2-Chloro-4-((trans-4-methoxycyclohexyl)amino)pyrimidine-5-carboxylic acid (71b)

Ethyl 2-chloro-4-(((1*R*,4*R*)-4-methoxycyclohexyl)amino)pyrimidine-5-carboxylate **71a** (2.00 g, 6.37 mmol) was dissolved in tetrahydrofuran/water (15 mL/15 mL), and lithium hydroxide monohydrate (0.80 g, 19.12 mmol) was added. The reaction mixture was stirred at room temperature for 2 h. The reaction was monitored by TLC. After completion of the reaction, the reaction mixture was concentrated to evaporate tetrahydrofuran and the pH adjusted to about 3-4 with 2 N HCl, and a white solid was precipitated. The mixture was filtered, and the filter cake was washed twice with water and petroleum ether/ethyl acetate (v/v = 10/1) to get the title compound 2-chloro-4-((*trans*-4-methoxycyclohexyl)amino)pyrimidine-5-carboxylic acid **71b** (white solid, 1.67 g, 91.70% yield).

LC-MS m/z(ESI) = **286.10** [M+1]

### Step 3:

### 2-Chloro-9-(trans-4-methoxycyclohexyl)-7,9-dihydro-8H-purin-8-one (71c)

2-Chloro-4-((*trans*-4-methoxycyclohexyl)amino)pyrimidine-5-carboxylic acid **71b** (1.57 g, 5.49 mmol) was dissolved in *N*,*N*-dimethylacetamide (16 mL), and triethylamine (0.76 mL, 5.49 mmol) and diphenylphosphoryl azide (1.18 mL, 5.49 mmol) were added with stirring at room temperature. The reaction mixture was stirred for 2 h, then heated to 110 °C and refluxed for 2.5 h. The reaction was monitored by TLC. After completion of the reaction, Water was added and the organics were extracted with dichloromethane. The organic layer was concentrated to give the title compound 2-chloro-9-(*trans*-4-methoxycyclohexyl)-7,9-dihydro-8H-purin-8-one **71c** (white solid, 1.70 g, crude product, 109.47% yield).

¹H NMR (400 MHz, DMSO-d6) δ 11.61 (s, 1H), 8.11 (s, 1H), 4.20-4.10 (m, 1H), 3.25 (s, 3H), 3.22-3.14 (m, 1H), 2.30-2.18 (m, 2H), 2.15-2.06 (m, 2H), 1.81-1.73 (d, 2H), 1.31-1.18 (m, 2H).

LC-MS m/z(ESI) = **283.00** [M+1]

### Step 4:

### 2-Chloro-9-(trans-4-methoxycyclohexyl)-7-methyl-7,9-dihydro-8H-purin-8-one (71d)

2-Chloro-9-(*trans*-4-methoxycyclohexyl)-7,9-dihydro-8H-purin-8-one **71c** (1.70 g, 6.01 mmol) was dissolved in *N*,*N*-dimethylformamide (20 mL), and dimethyl sulfate (0.57 mL, 6.01 mmol) and cesium carbonate (3.92 g, 12.03 mmol) were added with stirring at 0 °C. The reaction mixture was stirred for 2 h. The reaction was monitored by TLC. After completion of the reaction, the reaction mixture was slowly added dropwise to ice water. The mixture was stirred, and a white solid was precipitated. The solid was collected by filtration and washed 3 times with water and petroleum ether to obtain the title compound 2-chloro-9-(*trans*-4-methoxycyclohexyl)-7-methyl-7,9-dihydro-8*H*-purin-8-one **71d** (white solid, 1.2 g, 67.25% yield).

¹H NMR (400 MHz, DMSO-d6) δ 8.34 (s, 1H), 4.26-4.15 (m, 1H), 3.35 (s, 3H), 3.27 (s, 3H), 3.25-3.16 (m, 1H), 2.31-2.19 (m, 2H), 2.15-2.08 (m, 2H), 1.83-1.75 (m, 2H), 1.34-1.21 (m, 2H).

LC-MS m/z(ESI) = **297.10** [M+1]

### Step 5:

### 2-Fluoro-4-((9-(trans-4-methoxycyclohexyl)-7-methyl-8-oxo-8,9-dihydro-7H-purin-2-yl)amino)-5-methylbenzamide (compound 71)

2-Chloro-9-(*trans*-4-methoxycyclohexyl)-7-methyl-7,9-dihydro-8*H*-purin-8-one **71d** (0.25 g, 0.84 mmol) was dissolved in 1,4-dioxane (8 mL), and 4-amino-2-fluoro-5-methylbenzamide (0.57 g, 3.37 mmol), cesium carbonate (0.41 g, 1.26 mmol) and BrettPhos-G3-Pd (0.08 g, 0.08 mmol) were added with stirring at room temperature, followed by nitrogen purging. The reaction mixture was heated to 110 °C and refluxed under nitrogen for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(*trans*-4-methoxycyclohexyl)-7-methyl-8-oxo-8,9-dihydro-7H-purin-2-yl)amino)-5-methylbenzamide **compound 71** (white solid, 130 mg, 36.01% yield).

¹H NMR (400 MHz, DMSO-d6) δ 8.48 (s, 1H), 8.18 (s, 1H), 7.98 (d, 1H), 7.56 (d, 1H), 7.46 (s, 1H), 7.39 (s, 1H), 4.26-4.16 (m, 1H), 3.34 (s, 3H), 3.28 (s, 3H), 3.25-3.16 (m, 1H), 2.42-2.33 (m, 2H), 2.30 (s, 3H), 2.13 (d, 2H), 1.79 (d, 2H), 1.34-1.19 (m, 2H). LC-MS m/z(ESI) = **429.20** [M+1]

### Example 72

### 2-Fluoro-4-((9-(cis-4-hydroxy-4-methylcyclohexyl)-7-methyl-8-oxo-8,9-dihydro-7H-purin-2-yl)amino)-5-methylbenzamide (compound 72)

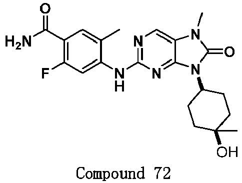

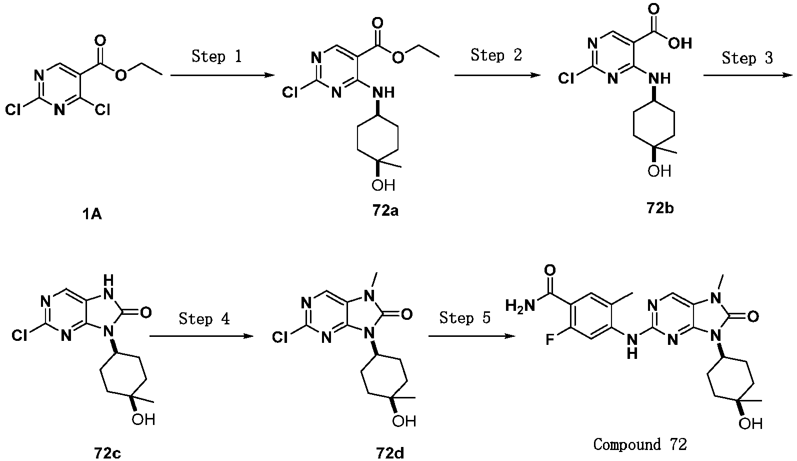

### Step 1:

### Ethyl 2-chloro-4-((cis-4-hydroxy-4-methylcyclohexyl)amino)pyrimidine-5-carboxylate (72a)

Ethyl 2,4-dichloropyrimidine-5-carboxylate **1A** (5.0 g, 22.6 mmol) and potassium carbonate (6.2 g, 45.2 mmol) were dissolved in acetonitrile (50 mL), and *cis*-4-amino-1-methylcyclohexan-1-ol (2.9 g, 22.6 mmol) was added at 0 °C. The reaction mixture was stirred at room temperature for 20 h. water was added and the organics were extracted three times with ethyl acetate., and the organic phase was dried over anhydrous sodium sulfate and purified by silica gel column chromatography (*n-*hexane/ethyl acetate (v/v) = 10:1) followed by concentration to obtain the title compound ethyl 2-chloro-4-((*cis*-4-hydroxy-4-methylcyclohexyl)amino)pyrimidine-5-carboxylate **72a** (white solid, 4.1 g, 58% yield).

¹H NMR (400 MHz, Chloroform-d) δ 8.65 (s, 1H), 8.37 (d, 1H), 4.35 (q, 2H), 4.09-4.04 (m, 1H), 1.94-1.82 (m, 2H), 1.82-1.45 (m, 6H), 1.38 (t, 3H), 1.28 (s, 3H). LC-MS m/z(ESI) = **314.10** [M+1]

### Step 2:

### 2-Chloro-4-((cis-4-hydroxy-4-methylcyclohexyl)amino)pyrimidine-5-carboxylic acid (72b)

Ethyl 2-chloro-4-((cis-4-hydroxy-4-methylcyclohexyl)amino)pyrimidine-5-carboxylate **72a** (4.1 g, 13.1 mmol) was dissolved in tetrahydrofuran/water (20 mL/20 mL), and lithium hydroxide (629 mg, 26.2 mmol) was added. The reaction mixture was stirred at room temperature for 1 h. The reaction was monitored by TLC. After completion of the reaction, the reaction mixture was concentrated under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to give the title compound 2-chloro-4-((*cis*-4-hydroxy-4-methylcyclohexyl)amino)pyrimidine-5-carboxylic acid **72b** (white solid, 3.5 g, 93% yield).

¹H NMR (400 MHz, DMSO-d6) δ 13.77(br, 1H), 8.56 (s, 1H), 8.49 (d, 1H), 3.93-3.83 (m, 2H), 1.78-1.50 (m, 6H), 1.43-1.40(m, 2H), 1.11 (s, 3H).

LC-MS m/z(ESI) = **286.10** [M+1]

### Step 3:

### 2-Chloro-9-(cis-4-hydroxy-4-methylcyclohexyl)-7,9-dihydro-8H-purin-8-one (72c)

2-Chloro-4-((*cis*-4-hydroxy-4-methylcyclohexyl)amino)pyrimidine-5-carboxylic acid **72b** (3.5 g, 12.2 mmol) was dissolved in dimethylacetamide (30 mL), and triethylamine (1.24 g, 12.2 mmol) and diphenylphosphoryl azide (3.36 g, 12.2 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound 2-chloro-9-(*cis*-4-hydroxy-4-methylcyclohexyl)-7,9-dihydro-8*H*-purin-8-one **72c** (white solid, 1.4 g, 41% yield).

¹H NMR (400 MHz, DMSO-*d*₆) δ 11.58 (s, 1H), 8.11 (d, 1H), 4.22(s, 1H), 4.22-4.02 (m, 1H), 2.65-2.56 (m, 2H), 1.83-1.60 (m, 2H), 1.47-1.43 (m, 4H), 1.18 (s, 3H). LC-MS m/z(ESI) = **283.10** [M+1]

### Step 4:

### 2-Chloro-9-(cis-4-hydroxy-4-methylcyclohexyl)-7-methyl-7,9-dihydro-8H-purin-8-one (72d)

2-Chloro-9-(cis-4-hydroxy-4-methylcyclohexyl)-7,9-dihydro-8*H*-purin-8-one **72c** (1.4 g, 4.9 mmol) was dissolved in dimethylformamide (15 mL), and dimethyl sulfate (618 mg, 4.9 mmol) and cesium carbonate (3.2 g, 9.8 mmol) were added at 0 °C. The reaction mixture was stirred for 0.5 h. The reaction was monitored by TLC. After completion of the reaction, water was added to the reaction mixture, and a solid was precipitated. The solid was collected by filtration to give the title compound 2-chloro-9-(*cis*-4-hydroxy-4-methylcyclohexyl)-7-methyl-7,9-dihydro-8*H*-purin-8-one **72d** (yellow solid, 502 mg, 34% yield).

LC-MS m/z(ESI) = **297.10** [M+1]

### Step 5:

### 2-Fluoro-4-((9-(cis-4-hydroxy-4-methylcyclohexyl)-7-methyl-8-oxo-8,9-dihydro-7H-purin-2-yl)amino)-5-methylbenzamide (compound 72)

2-Chloro-9-(cis-4-hydroxy-4-methylcyclohexyl)-7-methyl-7,9-dihydro-8-purin-8-one **72d** (250 mg, 0.84 mmol), 4-amino-3-methyl-2-fluorobenzamide **intermediate** 2 (282 mg, 1.68 mmol), cesium carbonate (446 mg, 1.68 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (76 mg, 0.084 mmol) were dissolved in dioxane (4 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 2-fluoro-4-((9-(*cis*-4-hydroxy-4-methylcyclohexyl)-7-methyl-8-oxo-8,9-dihydro-7*H-*purin-2-yl)amino)-5-methylbenzamide **compound 72** (white solid, 80 mg, 22 % yield). ¹H NMR (400 MHz, DMSO-d6) δ 8.40 (s, 1H), 8.15 (s, 1H), 7.92 (d, 1H), 7.54 (d, 1H), 7.46 (s, 1H), 7.35 (d, 1H), 4.20-4.06 (m, 1H), 4.02 (s, 1H), 3.31 (s, 3H), 2.78-2.57 (m, 2H), 2.28 (s, 3H), 1.68 (d, 2H), 1.49-1.40 (m, 4H), 1.15 (s, 3H).

LC-MS m/z (ESI) = **429.20** [M+1]

### Example 73

### 2-Fluoro-4-((9-(trans-4-hydroxy-4-methylcyclohexyl)-7-methyl-8-oxo-8,9-dihydro-7H-purin-2-yl)amino)-5-methylbenzamide (compound 73)

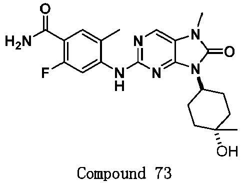

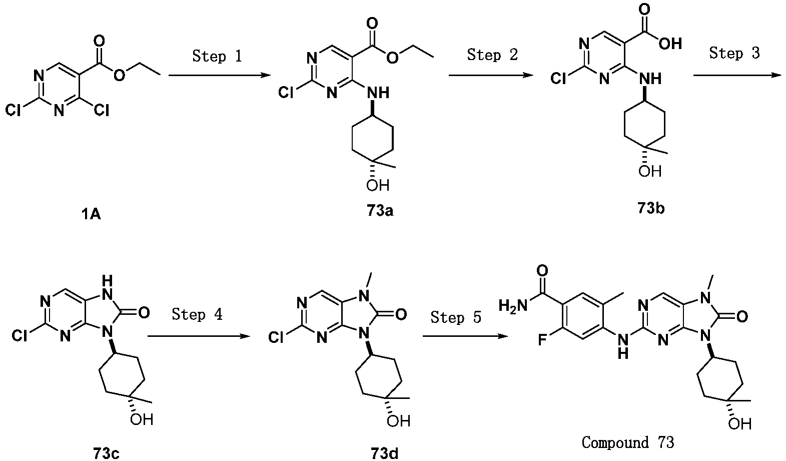

### Step 1:

### Ethyl 2-chloro-4-((trans-4-hydroxy-4-methylcyclohexyl)amino)pyrimidine-5-carboxylate (73a)

Ethyl 2,4-dichloro-5-pyrimidinecarboxylate **1A** (5.00 g, 30.18 mmol) was dissolved in acetonitrile (30 mL), and potassium carbonate (12.51 g, 90.55 mmol) was added with stirring at 0 °C, followed by the slow dropwise addition of a solution of *trans*-4-amino-1-methylcyclohexanol hydrochloride (10.01 g, 45.27 mmol) in acetonitrile (10 mL). The reaction mixture was warmed to room temperature and stirred for 1 h. The reaction was monitored by TLC. After completion of the reaction, the reaction mixture was filtered through celite, and the filtrate was purified by silica gel column chromatography (petroleum ether/ethyl acetate (v/v) = 2:1) to obtain the title compound ethyl 2-chloro-4-((*trans*-4-hydroxy-4-methylcyclohexyl)amino)pyrimidine-5-carboxylate **73a** (white solid, 4.70 g, 49.63% yield).

¹H NMR (400 MHz, Chloroform-d) δ 8.66 (s, 1H), 4.36 (q, 2H), 4.30-4.21 (m, 1H), 2.10-2.00 (m, 2H), 1.74-1.63 (m, 5H), 1.62-1.50 (m, 3H), 1.40 (t, 3H), 1.31 (s, 3H). LC-MS m/z(ESI) = **314.10** [M+1]

### Step 2:

### 2-Chloro-4-((trans-4-hydroxy-4-methyl-cyclohexyl)amino)pyrimidine-5-carboxylic acid (73b)

Ethyl 2-chloro-4-((*trans*-4-hydroxy-4-methylcyclohexyl)amino)pyrimidine-5-carboxylate **73a** (4.70 g, 14.98 mmol) was dissolved in tetrahydrofuran/water (40 mL/20 mL), and lithium hydroxide monohydrate (1.89 g, 44.94 mmol) was added. The reaction mixture was stirred at room temperature for 2 h. The reaction was monitored by TLC. After completion of the reaction, the reaction mixture was concentrated to evaporate tetrahydrofuran and the pH was adjusted to about 3-4 with 2 N HCl, and a white solid was precipitated. The mixture was filtered, and the filter cake was washed twice with water and petroleum ether/ethyl acetate (v/v = 10/1) to give the title compound 2-chloro-4-((*trans*-4-hydroxy-4-methylcyclohexyl)amino)pyrimidine-5-carboxylic acid **73b** (white solid, 4.20 g, 98.15% yield).

¹H NMR (400 MHz, DMSO-d6) δ 8.68 (d, 1H), 8.56 (s, 1H), 4.02 (s, 1H), 3.16 (s, 1H), 1.91-1.83 (m, 2H), 1.51-1.45 (m, 6H), 1.15 (s, 3H).

LC-MS m/z(ESI) = **314.10** [M+1]

### Step 3:

### 2-Chloro-9-(trans-4-hydroxy-4-methylcyclohexyl)-7,9-dihydro-8H-purin-8-one (73c)

2-Chloro-4-((*trans*-4-hydroxy-4-methylcyclohexyl)amino)pyrimidine-5-carboxylic acid **73b** (4.34 g, 15.19 mmol) was dissolved in *N*,*N*-dimethylacetamide (30 mL), and triethylamine (2.11 mL, 15.19 mmol) and diphenylphosphoryl azide (3.27 mL, 5.19 mmol) were added with stirring at room temperature. The reaction mixture was stirred for 2 h, then heated to 110 °C and refluxed for 2.5 h. The reaction was monitored by TLC. After completion of the reaction, water was added to the reaction mixture, and a white solid was precipitated. The reaction mixture was filtered, and the filter cake was washed twice with water and petroleum ether/ethyl acetate (v/v = 10/1) to obtain the title compound 2-chloro-9-(*trans*-4-hydroxy-4-methylcyclohexyl)-7,9-dihydro-8H-purin-8-one **73c** (white solid, 1.82 g, 42.36% yield).

LC-MS m/z(ESI) = **283.00** [M+1]

### Step 4:

### 2-Chloro-9-(trans-4-hydroxy-4-methylcyclohexyl)-7-methyl-7,9-dihydro-8H-purin-8-one (73d)

2-Chloro-9-(*trans*-4-hydroxy-4-methylcyclohexyl)-7,9-dihydro-8*H*-purin-8-one **73c** (1.80 g, 6.37 mmol) was dissolved in *N*,*N*-dimethylformamide (20 mL), and dimethyl sulfate (0.60 mL, 6.37 mmol) and cesium carbonate (3.11 g, 9.55 mmol) were added with stirring at 0 °C. The reaction mixture was stirred for 2 h. The reaction was monitored by TLC. After completion of the reaction, the reaction mixture was slowly added dropwise to ice water. The mixture was stirred, and a white solid was precipitated. The solid was collected by filtration and washed 3 times with water and petroleum ether to give the title compound 2-chloro-9-(*trans*-4-hydroxy-4-methylcyclohexyl)-7-methyl-7,9-dihydro-8J/-purin-8-one **73d** (white solid, 0.80 g, 42.34% yield).

¹H NMR (400 MHz, DMSO-d6) δ 8.35 (s, 1H), 4.48 (s, 1H), 4.23-4.12 (m, 1H), 3.35 (s, 3H), 2.42-2.25 (m, 2H), 1.65 (d, 4H), 1.58-1.47 (m, 2H), 1.26 (s, 3H).

LC-MS m/z(ESI) = **297.10** [M+1]

### Step 5:

### 2-Fluoro-4-((9-(trans-4-hydroxy-4-methylcyclohexyl)-7-methyl-8-oxo-8,9-dihydro-7H-purin-2-yl)amino)-5-methylbenzamide (compound 73)

2-Chloro-9-(*trans*-4-hydroxy-4-methylcyclohexyl)-7-methyl-7,9-dihydro-8*H*-purin-8-one **73d** (0.30 g, 1.01 mmol) was dissolved in 1,4-dioxane (8 mL), and 4-amino-2-fluoro-5-methylbenzamide **intermediate 2** (0.37 g, 2.22 mmol), cesium carbonate (0.49 g, 1.52 mmol) and BrettPhos-G3-Pd (0.09 g, 0.10 mmol) were added with stirring at room temperature, followed by nitrogen purging. The reaction mixture was heated to 110 °C and refluxed under nitrogen for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(*trans*-4-hydroxy-4-methylcyclohexyl)-7-methyl-8-oxo-8,9-dihydro-7*H*-purin-2-yl)amino)-5-methylbenzamide **compound 73** (pale yellow solid, 23 mg, 5.31% yield).

¹H NMR (400 MHz, DMSO-d6) δ 8.56 (s, 1H), 8.17 (s, 1H), 7.72 (d, 1H), 7.55 (d, 1H), 7.45 (s, 1H), 7.34 (d, 1H), 4.41 (s, 1H), 4.16 (tt, 1H), 2.36-2.29 (m, 2H), 2.27 (s, 3H), 1.63 (d, 4H), 1.49 (td, 2H), 1.14 (s, 3H).

LC-MS m/z(ESI) = **429.20** [M+1]

### Example 74

### 4-((9-(cis-3-Cyanocyclobutyl)-7-methyl-8-oxo-8,9-dihydro-7H-purin-2-yl)amino)-2-fluoro-5-methylbenzamide (compound 74)

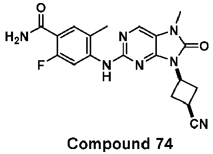

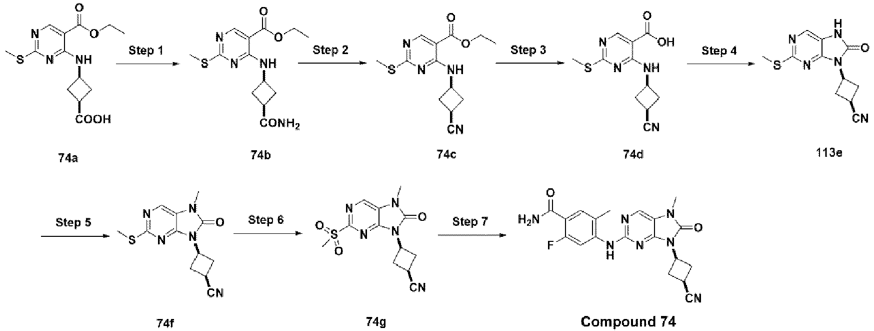

### Step 1:

### Ethyl 4-((cis-3-carbamoylcyclobutyl)amino)-2-(methylthio)pyrimidine-5-carboxylate (74b)

The starting material *cis*-3-((5-(ethoxycarbonyl)-2-(methylthio)pyrimidine-4-yl)amino)cyclobutane-1-carboxylic acid **74a** (13.0 g, 41.8 mmol) was added to dichloromethane (130 mL), and *N*,*N*,*N*,*N*-tetramethyl-O-(7-azabenzotriazol-1-yl)urea hexafluorophosphate (19.1 g, 50.2 mmol) and triethylamine (16.9 g, 167.2 mmol) were added. The mixture was stirred at room temperature for 10 min, and then ammonium chloride (6.7 g, 125.4 mmol) was added. The reaction mixture was stirred for 2 h. The reaction was monitored by TLC. After completion of the reaction, 50 mL of saturated aqueous sodium bicarbonate were added to the reaction mixture and the organics were with dichloromethane, and the organic layer was concentrated, dried over anhydrous sodium sulfate and concentrated under reduced pressure to obtain crude title compound ethyl 4-((*cis*-3-carbamoylcyclobutyl)amino)-2-(methylthio)pyrimidine-5-carboxylate **74b** (yellow viscous liquid, 17.5 g, 134.6% yield).

LC-MS m/z(ESI) = 311.20 [M+1]

### Step 2:

### Ethyl 4-((cis-3-cyanocyclobutyl)amino)-2-(methylthio)pyrimidine-5-carboxylate (74c)

Ethyl 4-((*cis*-3-carbamoylcyclobutyl)amino)-2-(methylthio)pyrimidine-5-carboxylate 74b (17.5 g, 5.64 mmol) was added to dichloromethane (130 mL), and pyridine (13.2 g, 167.2 mmol) and trifluoroacetic anhydride (26.3 g, 125.4 mmol) were added. The reaction mixture was stirred at room temperature for 1 h. The reaction was monitored by TLC. After completion of the reaction, the pH of reaction mixture was adjusted to 3 with 2 N hydrochloric acid and extracted with dichloromethane, and was dried over anhydrous sodium sulfate and concentrated under reduced pressure to get a crude product, which was then purified by column chromatography (petroleum ether/ethyl acetate (v/v) = 5:1) to obtain the title compound ethyl 4-((*cis*-3-cyanocyclobutyl)amino)-2-(methylthio)pyrimidine-5-carboxylate **74c** (white solid, 3.5 g, 21.2 % yield).

¹H NMR (400 MHz, DMSO-d6) δ 8.65 (s, 1H), 8.46 (d, 1H), 4.70-4.61 (m, 1H), 4.37-4.31 (m, 2H), 2.94-2.84 (m, 3H), 2.52 (s, 3H), 2.48-2.38 (m, 2H), 1.38(t, 3H). LC-MS m/z(ESI) = 293.20 [M+1]

### Step 3:

### 4-((cis-3-Cyanocyclobutyl)amino)-2-(methylthio)pyrimidine-5-carboxylic acid (74d)

Ethyl 4-((cis-3-cyanocyclobutyl)amino)-2-(methylthio)pyrimidine-5-carboxylate **74c** (3.5 g, 11.9 mmol) was dissolved in a mixture of tetrahydrofuran (20 mL) and water (20 mL), and lithium hydroxide (691 mg, 28.8 mmol) was added. The reaction mixture was stirred at room temperature for 1 h. The reaction was monitored by TLC. After completion of the reaction, the reaction mixture was concentrated under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to afford the title compound 4-((*cis*-3-cyanocyclobutyl)amino)-2-(methylthio)pyrimidine-5-carboxylic acid **74d** (white solid, 3.0 g, 94.6% yield).

¹H NMR (400 MHz, DMSO-d6) δ 13.30 (br, 1H) 8.58 (d, 1H), 8.53 (s, 1H), 4.66-4.56 (m, 1H), 3.18-3.09 (m, 1H), 2.74-2.65(m, 2 H), 2.47 (s, 3H), 2.48-2.40 (m, 2H). LC-MS m/z(ESI) = 265.10 [M+1]

### Step 4:

### cis-3-(2-(Methylthio)-8-oxo-7,8-dihydro-9H-purin-9-yl)cyclobutane-1-carbonitrile (74e)

4-((*cis*-3-Cyanocyclobutyl)amino)-2-(methylthio)pyrimidine-5-carboxylic acid **74d** (3.0 g, 11.3 mmol) was dissolved in *N*,*N*-dimethylformamide (30 mL), and triethylamine (1.18 g, 11.7 mmol) and diphenylphosphoryl azide (3.22 g, 11.7 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 5:1 to 1:10) to obtain the title compound *cis*-3-(2-(methylthio)-8-oxo-7,8-dihydro-9*H*-purin-9-yl)cyclobutane-1-carbonitrile **74e** (white solid, 2.7 g, 90.9% yield).

¹H NMR (400 MHz, DMSO-d6) δ 11.35 (s, 1H), 8.11 (s, 1H), 4.90-4.02 (m, 1H), 3.37-3.26 (m, 2H), 3.25-3.18 (m, 1H), 2.78-2.66 (m, 2H), 2.58 (s, 3H).

LC-MS m/z(ESI) = 262.10 [M+1]

### Step 5:

### cis-3-(7-Methyl-2-(methylthio)-8-oxo-7,8-dihydro-9H-purin-9-yl)cyclobutane-1-carbonitrile (74f)

*cis*-3-(2-(Methylthio)-8-oxo-7,8-dihydro-9*H*-purin-9-yl)cyclobutane-1-carbonitrile **74e** (500 mg, 19.1 mmol) was dissolved in dimethylformamide (5 mL), and dimethyl sulfate (2.4 g, 19.1 mmol) and cesium carbonate (12.5 g, 38.2 mmol) were added at 0 °C. The reaction mixture was stirred at 0 °C for 0.5 h. The reaction was monitored by TLC. After completion of the reaction, then 50 mL of water was added, and a solid was precipitated. The solid was collected by filtration and dried by rotary evaporation under reduced pressure to get the title compound *cis-*3-(7-methyl-2-(methylthio)-8-oxo-7,8-dihydro-9*H*-purin-9-yl)cyclobutane-1-carbonitrile **74f** (white solid, 457 mg, 86.7% yield).

¹H NMR (400 MHz, DMSO-d6) δ 8.61 (s, 1H), 4.90-4.02 (m, 1H), 3.34(s, 3H), 3.30-3.23(m, 2H), 3.25-3.18(m, 1H), 2.89(s, 3H), 2.76-2.71(m, 2H).

LC-MS m/z (ESI) = 276.10 [M+1]

### Step 6:

### cis-3-(7-Methyl-2-(methylsulfonyl)-8-oxo-7,8-dihydro-9H-purin-9-yl)cyclobutane-1-carbonitrile (74g)

*cis*-3-(7-Methyl-2-(methylthio)-8-oxo-7,8-dihydro-9*H*-purin-9-yl)cyclobutane-1-carbonitrile **74f** (457 mg, 1.66 mmol) was dissolved in methanol (10 mL), and 2 mL of purified water was added, followed by the addition of potassium peroxymonosulfate (1.02 g, 1.66 mmol). The reaction mixture was heated to 60 °C and stirred for 2 h. The reaction was monitored by TLC. After completion of the reaction, the reaction mixture was concentrated under reduced pressure to remove methanol, and 5 mL of purified water was added. The resulting mixture was extracted 3 times with dichloromethane (20 mL × 3), and the organic phases were combined, dried over anhydrous sodium sulfate, and concentrated under reduced pressure to remove the solvent and thus to provide the title compound *cis-*3-(7-methyl-2-(methylsulfonyl)-8-oxo-7,8-dihydro-9*H*-purin-9-yl)cyclobutane-1-carbonitrile **74g** (pale yellow solid, 197 mg, 38.6% yield).

LC-MS m/z(ESI) = 308.10 [M+1]

### Step 7:

### 4-((9-(cis-3-Cyanocyclobutyl)-7-methyl-8-oxo-8,9-dihydro-7H-purin-2-yl)amino)-2-fluoro-5-methylbenzamide (compound 74)

cis-3-(7-Methyl-2-(methylsulfonyl)-8-oxo-7,8-dihydro-9*H*-purin-9-yl)cyclobutane-1-carbonitrile **74g** (197 mg, 0.64 mmol) and 4-amino-3-methyl-2-fluorobenzamide (538 mg, 3.20 mmol) were dissolved in *N*,*N*-dimethylformamide (3 mL), followed by nitrogen purging and the addition of potassium *tert*-butoxide (143 mg, 1.28 mmol). The reaction mixture was stirred under nitrogen at room temperature for 4 h. The reaction was monitored by TLC. After completion of the reaction, the reaction mixture was poured into 30 mL of purified water, and a large amounts of yellow solid was precipitated. The solid was collected by filtration and purified by silica gel column chromatography (dichloromethane/methanol (v/v) = 35/1) to obtain the title compound 4-((9-(*cis*-3-cyanocyclobutyl)-7-methyl-8-oxo-8,9-dihydro-7*H*-purin-2-yl)amino)-2-fluoro-5-methylbenzamide **compound 74** (white solid, 25 mg, 10.0 % yield).

¹H NMR (400 MHz, DMSO-d6) δ8.63 (s, 1H), 8.20(s, 1H), 7.87(d 1H), 7.56-7.54(m, 1H), 7.48(s, 1H), 7.40(s, 1H), 5.14-5.09(m, 1H), 3.64-3.59(m, 1H), 3.29(s, 3H), 3.26-3.16(m, 2H), 2.75-2.68(m, 2H), 2.30(s, 3H).

LC-MS m/z(ESI) = 396.20 [M+1]

### Example 75

### (S)-2-Fluoro-5-methyl-4-((7-methyl-8-oxo-9-(tetrahydrofuran-3-yl)-8,9-dihydro-7H-purin-2-yl)amino)benzamiderile (compound 75)

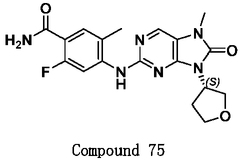

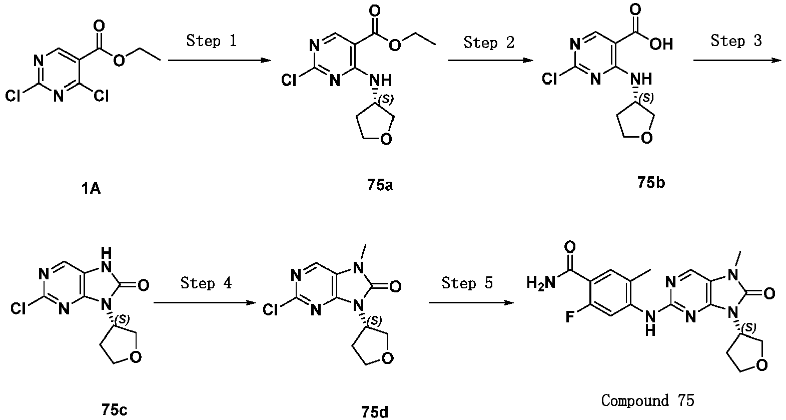

### Step 1:

### Ethyl (S)-2-chloro-4-((tetrahydrofuran-3-yl)amino)pyrimidine-5-carboxylate (75a)

Ethyl 2,4-dichloropyrimidine-5-carboxylate **1A** (5 g, 5.35 mmol) and potassium carbonate (1.4 g, 22.6 mmol) were dissolved in acetonitrile (20 mL), and (*S*)-tetrahydrofuran-3-amine (660 mg, 5.35 mmol) was added at 0 °C. The reaction mixture was stirred at room temperature for 20 h. Water was added and the organics were extracted three times with ethyl acetate, and was dried over anhydrous sodium sulfate and purified by silica gel column chromatography (n-hexane/ethyl acetate (v/v) = 10:1) followed by concentration to obtain the title compound ethyl (S)-2-chloro-4-((tetrahydrofuran-3-yl)amino)pyrimidine-5-carboxylate **75a** (white solid, 2.2 g, 36% yield).

¹H NMR (400 MHz DMSO) δ 8.68 (s, 1H), 8.58 (d, 1H), 4.79-4.77 (m, 1H), 4.39-4.33 (m, 2H), 4.04-3.98 (m, 2H), 3.90-3.87 (m, 1H), 3.76-3.73 (m, 1H), 2.43-2.33 (m, 1H), 1.95-1.88 (m, 1H), 1.39 (t, 3H).

LC-MS m/z(ESI) = **272.00** [M+1]

### Step 2:

### (S)-2-Chloro-4-((tetrahydrofuran-3-yl)amino)pyrimidine-5-carboxylic acid (75b)

Ethyl (*S*)-2-chloro-4-((tetrahydrofuran-3-yl)amino)pyrimidine-5-carboxylate **75a** (2.2 g, 8.1 mmol) was dissolved in tetrahydrofuran/water (5 mL/5 mL), and lithium hydroxide (681 mg, 16.2 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound (*S*)-2-chloro-4-((tetrahydrofuran-3-yl)amino)pyrimidine-5-carboxylic acid **75b** (white solid, 1.7 g, 89% yield).

¹H NMR (400 MHz DMSO) δ 13.83 (s, 1H), 8.65 (s, 1H), 8.63 (d, 1H), 4.61 (s, 1H), 3.89-3.80 (m, 2H), 3.76-3.70 (m, 1H), 3.65-3.63 (m, 1H), 2.50-2.25 (m, 1H), 1.87-1.86 (m, 1H).

LC-MS m/z(ESI) = **244.20** [M+1]

### Step 3:

### (S)-2-Chloro-9-(tetrahydrofuran-3-yl)-7,9-dihydro-8H-purin-8-one (75c)

(*S*)-2-Chloro-4-((tetrahydrofuran-3-yl)amino)pyrimidine-5-carboxylic acid **75b** (1.7 g, 7 mmol) was dissolved in dimethylacetamide (10 mL), and triethylamine (707 mg, 7 mmol) and diphenylphosphoryl azide (1.9 g, 7 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:6) to obtain the title compound (*S*)-2-chloro-9-(tetrahydrofuran-3-yl)-7,9-dihydro-8*H*-purin-8-one **75c** (white solid, 1.4 g, 87% yield).

¹H NMR (400 MHz DMSO) δ 8.13 (s, 1H), 5.00-4.93 (m, 1H), 4.12-4.06 (dd, 1H), 3.97 (t, 1H), 3.88-3.87 (m, 2H), 3.33 (s, 3H), 2.43-2.37 (m, 1H), 2.25-2.20 (m, 1H). LC-MS m/z(ESI) = **241.20** [M+1]

### Step 4:

### (S)-2-Chloro-7-methyl-9-(tetrahydrofuran-3-yl)-7,9-dihydro-8H-purin-8-one (75d)

(*S*)-2-Chloro-9-(tetrahydrofuran-3-yl)-7,9-dihydro-8*H*-purin-8-one **75c** (1.4 g, 5.8 mmol) was dissolved in dimethylformamide (10 mL), and dimethyl sulfate (735 mg, 5.8 mmol) and cesium carbonate (2.85 g, 8.7 mmol) were added at 0 °C. The reaction mixture was stirred for 0.5 h. The reaction was monitored by TLC. After completion of the reaction, water was added to the reaction mixture, and a solid was precipitated. The solid was collected by filtration to get the title compound (*S*)-2-chloro-7-methyl-9-(tetrahydrofuran-3-yl)-7,9-dihydro-8*H*-purin-8-one **75d** (white solid, 1.2 g, 85% yield). ¹H NMR (400 MHz DMSO) δ 8.35 (s, 1H), 5.04-4.97 (m, 1H), 4.13-4.07 (dd, 1H), 3.98 (t, 1H),3.90-3.84 (m, 2H), 3.35(s, 3H), 2.44-2.37 (m, 1H), 2.28-2.27 (m,1H).

LC-MS m/z(ESI) = **255.20** [M+1]

### Step 5:

### (S)-2-Fluoro-5-methyl-4-((7-methyl-8-oxo-9-(tetrahydrofuran-3-yl)-8,9-dihydro-7H-purin-2-yl)amino)benzamiderile (compound 75)

(*S*)-2-Chloro-7-methyl-9-(tetrahydrofuran-3-yl)-7,9-dihydro-8*H*-purin-8-one **57d** (200 mg, 0.78 mmol), 4-amino-2-fluoro-5-methylbenzamide **intermediate 2** (264 mg, 1.57 mmol), cesium carbonate (770 mg, 2.36 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (71.4 mg, 0.078 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound (*S*)-2-fluoro-5-methyl-4-((7-methyl-8-oxo-9-(tetrahydrofuran-3-yl)-8,9-dihydro-7*H-*purin-2-yl)amino)benzamiderile **compound 75** (white solid, 58 mg, 19 % yield).

¹H NMR (400 MHz DMSO) δ 8.49 (s, 1H), 8.20 (s, 1H), 7.89 (d, 1H),7.55 (d, 1H), 7.44(d, 2H), 4.95-5.02 (m, 1H), 4.07-4.13 (dd, 1H), 3.91-3.99(m,2H), 3.82-3.85(m,1H),3.32 (s, 3H), 2.44-2.50(m, 1H), 2.28(S, 3H), 2.20-2.27(m, 1 H).

LC-MS m/z(ESI) = **387.10** [M+1]

### Example 76

### (R)-2-Fluoro-5-methyl-4-((7-methyl-8-oxo-9-(tetrahydrofuran-3-yl)-8,9-dihydro-7H-purin-2-yl)amino)benzamiderile (compound 76)

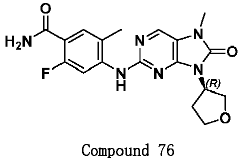

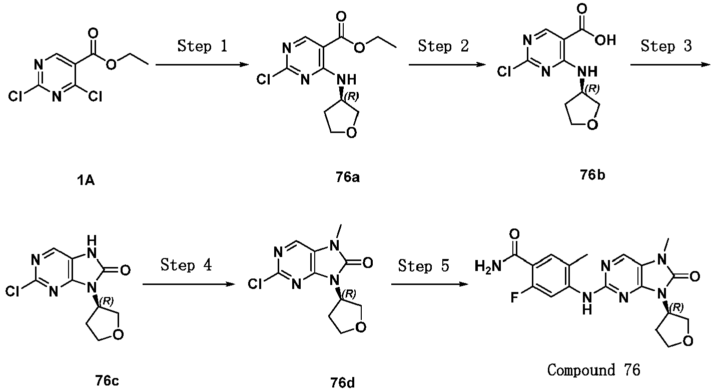

### Step 1:

### Ethyl (R)-2-Chloro-4-((tetrahydrofuran-3-yl)amino)pyrimidine-5-carboxylate (76a)

Ethyl 2,4-dichloropyrimidine-5-carboxylate **1A** (5 g, 5.35 mmol) and potassium carbonate (1.4 g, 22.6 mmol) were dissolved in acetonitrile (20 mL), and (*R*)-tetrahydrofuran-3-amine (660 mg, 5.35 mmol) was added at 0 °C. The reaction mixture was stirred at room temperature for 20 h. Water was added and the organics were extracted three times with ethyl acetate, and the organic phase was dried over anhydrous sodium sulfate and purified by silica gel column chromatography (*n*-hexane/ethyl acetate (v/v) = 10:1) followed by concentration to obtain the title compound ethyl (R)-2-chloro-4-((tetrahydrofuran-3-yl)amino)pyrimidine-5-carboxylate **76a** (white solid, 4.3 g, 65% yield).

¹H NMR (400 MHz DMSO) δ 8.68 (s, 1H), 8.58 (d, 1H), 4.83-4.77 (m, 1H), 4.39-4.33 (m, 2H), 4.02-3.98 (m, 2H), 3.90-3.86 (m, 1H), 3.76-3.73 (m, 1H), 2.43-2.34 (m,1H), 1.98-1.88 (m, 1H), 1.39 (t, 3H).

LC-MS m/z(ESI) = **272.00** [M+1]

### Step 2:

### (R)-2-Chloro-4-((tetrahydrofuran-3-yl)amino)pyrimidine-5-carboxylic acid (76b)

Ethyl (*R*)-2-chloro-4-((tetrahydrofuran-3-yl)amino)pyrimidine-5-carboxylate **76a** (4.3 g, 15.8 mmol) was dissolved in tetrahydrofuran/water (5 mL/5 mL), and lithium hydroxide (1.3 g, 31.7 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound (A)-2-chloro-4-((tetrahydrofuran-3-yl)amino)pyrimidine-5-carboxylic acid **76b** (white solid, 2.9 g, 87% yield).

¹H NMR (400 MHz DMSO) δ 13.83 (s, 1H), 8.65 (s, 1H), 8.59 (d,1H), 4.62-4.60 (m, 1H), 3.89-3.80 (m, 2H), 3.76-3.70 (m, 1H), 3.64-3.56 (m, 1H), 2.33-2.23 (m, 1H), 1.88-1.83 (m, 1H).

LC-MS m/z(ESI) = **244.20** [M+1]

### Step 3:

### (R)-2-Chloro-9-(tetrahydrofuran-3-yl)-7,9-dihydro-8H-purin-8-one (76c)

(A)-2-Chloro-4-((tetrahydrofuran-3-yl)amino)pyrimidine-5-carboxylic acid **76b** (2.9 g, 11.9 mmol) was dissolved in dimethylacetamide (20 mL), and triethylamine (1.2 g, 11.9 mmol) and diphenylphosphoryl azide (3.3 g, 11.9 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:6) to obtain the title compound (R)-2-chloro-9-(tetrahydrofuran-3-yl)-7,9-dihydro-8*H*-purin-8-one **76c** (white solid, 2.2 g, 68% yield). ¹H NMR (400 MHz DMSO) δ 8.36 (s, 1H), 5.04-4.96 (m, 1H), 4.13-4.07 (dd, 1H), 3.97 (t, 1H), 3.87-3.84 (m, 2H), 3.33 (s, 3H), 2.40-2.36 (m, 1H), 2.29-2.27 (m, 1H).

LC-MS m/z(ESI) = **241.20** [M+1]

### Step 4:

### (R)-2-Chloro-7-methyl-9-(tetrahydrofuran-3-yl)-7,9-dihydro-8H-purin-8-one (76d)

(R)-2-Chloro-9-(tetrahydrofuran-3-yl)-7,9-dihydro-8*H*-purin-8-one **76c** (2.2 g, 9.1 mmol) was dissolved in dimethylformamide (10 mL), and dimethyl sulfate (1.1 g, 9.1 mmol) and cesium carbonate (4.5 g, 13.7 mmol) were added at 0 °C. The reaction mixture was stirred for 0.5 h. The reaction was monitored by TLC. After completion of the reaction, water was added to the reaction mixture, and a solid was precipitated. The solid was collected by filtration to give the title compound (R)-2-chloro-7-methyl-9-(tetrahydrofuran-3-yl)-7,9-dihydro-8*H*-purin-8-one **76d** (white solid, 1.7 g, 82% yield). ¹H NMR (400 MHz DMSO) δ 8.41 (s, 1H), 5.05-4.97 (m, 1H), 4.13-4.08 (dd, 1H), 3.98 (t, 1H), 3.91-3.84 (m, 2H), 3.33 (s, 3H), 2.45-2.27 (m, 1H), 2.26-2.20 (m,1H).

LC-MS m/z(ESI) = **255.20** [M+1]

### Step 5:

### (R)-2-Fluoro-5-methyl-4-((7-methyl-8-oxo-9-(tetrahydrofuran-3-yl)-8,9-dihydro-7H-purin-2-yl)amino)benzamiderile (compound 76)

(*R*)-2-Chloro-7-methyl-9-(tetrahydrofuran-3-yl)-7,9-dihydro-8*H*-purin-8-one **76d** (200 mg, 0.78 mmol), 4-amino-2-fluoro-5-methylbenzamide **intermediate 2** (264 mg, 1.57 mmol), cesium carbonate (770 mg, 2.36 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (71.4 mg, 0.078 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound (*R*)-2-fluoro-5-methyl-4-((7-methyl-8-oxo-9-(tetrahydrofuran-3-yl)-8,9-dihydro-7*H-*purin-2-yl)amino)benzamiderile **compound 76** (white solid, 31 mg, 14.6 % yield). ¹H NMR (400 MHz DMSO) δ 8.51 (s, 1H), 8.20 (s, 1H), 7.89 (d, 1H),7.54 (d, 1H), 7.44(d, 2H), 4.94-5.02 (m, 1H), 4.06-4.12 (dd, 1H), 3.87-3.98(m,2H), 3.82-3.85(m,1H),3.32 (s, 3H), 2.43-2.45(m, 1H), 2.33(s, 3H), 2.19-2.22(m, 1H).

LC-MS m/z(ESI) = **387.10** [M+1]

### Example 77

### 4-((9-(cis-4-Cyanocyclohexyl)-7-methyl-8-oxo-8,9-dihydro-7H-purin-2-yl)amino)-2-fluoro-5-methylbenzamide (compound 77)

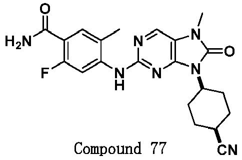

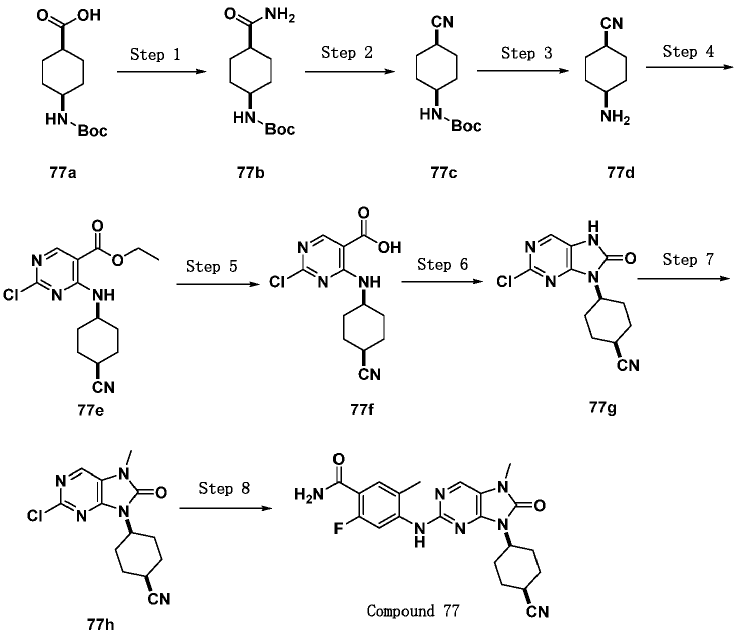

### Step 1:

### tert-Butyl(cis-4-carbamoylcyclohexyl)carbamate (77b)

*cis*-4-((*tert*-Butoxycarbonyl)amino)cyclohexane-1-carboxylic acid 77a (5.0 g, 20.5 mmol) and O-(7-azabenzotriazolyl)-*N*,*N*,*N'*,*N'*-tetramethyl-uronium-hexafluorophosphate (9.4 g, 24.7 mmol) were dissolved in dichloromethane (15 mL). The solution was stirred at 0 °C for 20 min, and *N*,*N*-diisopropylethylamine (10.5 g, 82 mmol) and ammonium chloride (3.3 g, 61.5 mmol) were added. The reaction mixture was stirred at room temperature for 4 h. The reaction was monitored by TLC. After completion of the reaction, 10 mL of water was added to the reaction mixture, and the organic phase was separated, washed with saturated brine once, dried over anhydrous sodium sulfate and mixed with silica gel to prepare a sample, which was then purified by normal phase column chromatography to give a product, and the product was concentrated to obtain the title compound *tert*-butyl(*cis*-4-carbamoylcyclohexyl)carbamate **77b** (white solid, 3.5 g, 71% yield).

LC-MS m/z(ESI) = **243.30** [M+1]

### Step 2:

### tert-Butyl(cis-4-cyanocyclohexyl)carbamate (77c)

*tert*-Butyl(*cis*-4-carbamoylcyclohexyl)carbamate **77b** (3.5 g, 14.4 mmol) was dissolved in 70 mL of pyridine. The solution was cooled in an ice bath, and then phosphorus oxychloride (7.7 mL) was added dropwise. The reaction mixture was stirred in an ice bath for 1 h. The reaction was monitored by TLC. After completion of the reaction, 20 mL of water was added in an ice bath, and the resulting mixture was extracted 4 times with ethyl acetate. The organic phase was washed 7 times with acid water, finally washed twice with saturated brine, dried over anhydrous sodium sulfate, filtered and concentrated to dryness to obtain the compound *tert*-butyl(*cis*-4-cyanocyclohexyl)carbamate **77c** (yellow solid, 1.5 g, 36% yield).

¹H NMR (400 MHz DMSO) δ 6.90 (d, 1H), 3.27-3.25 (m, 1H), 3.01-2.99 (m, 1H), 1.86-1.81 (m, 2H), 1.71-1.67 (m, 2H), 1.62-1.54 (m, 2H), 1.43-1.40 (m, 1H), 1.37(s, 3H),1.34-1.32(m, 1H).

LC-MS m/z(ESI) = **225.30** [M+1]

### Step 3:

### cis-4-Aminocyclohexane-1-carbonitrile (77d)

*tert*-Butyl(*cis*-4-cyanocyclohexyl)carbamate **77c** (1.5 g, 6.7 mmol) was dissolved in hydrogen chloride in ethyl acetate (20 mL), and the reaction mixture was heated to 45 °C and stirred for 2 h. The reaction was monitored by TLC. After completion of the reaction, a white solid was precipitated. The reaction mixture was filtered and concentrated to dryness to give the title compound *cis*-4-aminocyclohexane-1-carbonitrile **77d** (white solid, 1 g, 90% yield).

LC-MS m/z(ESI) = **125.30** [M+1]

### Step 4:

### Ethyl 2-chloro-4-(cis-4-cyanocyclohexyl)amino)pyrimidine-5-carboxylate (77e)

Ethyl 2,4-dichloropyrimidine-5-carboxylate **1A** (1.48 g, 6.7 mmol) and potassium carbonate (1.38 g, 10 mmol) were dissolved in acetonitrile (10 mL), and *cis*-4-aminocyclohexane-1-carbonitrile **77d** (1 g, 6.7 mmol) was added at 0 °C. The reaction mixture was stirred at room temperature for 20 h. The reaction was monitored by TLC. After completion of the reaction, water was added and the organics were extracted three times with ethyl acetate, and the organic phase was washed once with saturated brine, dried over anhydrous sodium sulfate, and purified by silica gel column chromatography (n-hexane/ethyl acetate (v/v) = 10:1) to obtain the title compound ethyl 2-chloro-4-(*cis-*4-cyanocyclohexyl)amino)pyrimidine-5-carboxylate **77e** (white solid, 589 mg, 60% yield).

¹H NMR (400 MHz DMSO) δ 8.63 (s, 1H), 8.38 (d, 1H), 4.35-4.29 (m, 2H), 4.07-3.99 (m, 1H), 3.11-3.09 (m, 1H), 1.92-1.73 (m, 6H), 1,63-1.54 (m, 2H), 1.47(t, 3H). LC-MS m/z(ESI) = **310.20** [M+1]

### Step 5:

### 2-Chloro-4-((cis-4-cyanocyclohexyl)amino)pyrimidine-5-carboxylic acid (77f)

Ethyl 2-chloro-4-((*cis*-4-cyanocyclohexyl)amino)pyrimidine-5-carboxylate **77e** (589 mg, 1.9 mmol) was dissolved in tetrahydrofuran/water (4 mL/4 mL), and lithium hydroxide (160 mg, 3.8 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound 2-chloro-4-((*cis*-4-cyanocyclohexyl)amino)pyrimidine-5-carboxylic acid **77f** (white solid, 490 mg, 86% yield).

¹H NMR (400 MHz DMSO) δ 13.83 (s, 1H), 8.62-8.60 (d, 2H), 4.05-3.98 (m, 1H), 3.10 (t, 1H), 1.94-1.73 (m, 6H), 1.60-1.56 (m, 2H).

LC-MS m/z(ESI) = **282.30** [M+1]

### Step 6:

### cis-4-(2-Chloro-8-oxo-7,8-dihydro-9H-purin-9-yl)cyclohexane-1-carbonitrile (77g)

2-Chloro-4-((*cis*-4-cyanocyclohexyl)amino)pyrimidine-5-carboxylic acid **77f** (490 mg, 1.75 mmol) was dissolved in dimethylacetamide (10 mL), and triethylamine (176 mg, 1.75 mmol) and diphenylphosphoryl azide (481 mg, 1.75 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound *cis*-4-(2-chloro-8-oxo-7,8-dihydro-9*H*-purin-9-yl)cyclohexane-1-carbonitrile **77g** (white solid, 436 mg, 68% yield).

¹H NMR (400 MHz DMSO) δ 11.64 (s, 1H), 8.13 (s, 1H), 4.23-4.16 (m, 1H), 3.22-3.16 (m, 1H), 2.44-2.39(m, 2H), 2.00 (d, 2H), 1.79-1.72 (m, 4H).

LC-MS m/z(ESI) = **278.30** [M+1]

### Step 7:

### cis-4-(2-Chloro-7-methyl-8-oxo-7,8-dihydro-9H-purin-9-yl)cyclohexane-1-carbonitrile (77h)

cis-4-(2-Chloro-8-oxo-7,8-dihydro-9*H*-purin-9-yl)cyclohexane-1-carbonitrile **77g** (1.6 g, 5.77 mmol) was dissolved in dimethylformamide (10 mL), and dimethyl sulfate (728 mg, 5.77 mmol) and cesium carbonate (2.8 g, 8.65 mmol) were added at 0 °C. The reaction mixture was stirred at 0 °C for 30 min. The reaction was monitored by TLC. After completion of the reaction, 10 mL of water was added to the reaction mixture, and a solid was precipitated. The solid was collected by filtration to give the title compound *cis*-4-(2-chloro-7-methyl-8-oxo-7,8-dihydro-9*H*-purin-9-yl)cyclohexane-1-carbonitrile **77h** (yellow solid, 1.25 g, 75% yield).

¹H NMR (400 MHz DMSO) δ 8.35 (s, 1H), 4.28-4.19 (m, 1H), 3.34 (s, 3H), 3.23-3.17 (m, 1H), 2.46-2.39 (m, 2H), 2.00 (d, 2H), 1.80-1.73 (m, 4H).

LC-MS m/z(ESI) = **292.30** [M+1]

### Step 8:

### 4-((9-(cis-4-Cyanocyclohexyl)-7-methyl-8-oxo-8,9-dihydro-7H-purin-2-yl)amino)-2-fluoro-5-methylbenzamide (compound 77)

*cis*-4-(2-chloro-7-methyl-8-oxo-7,8-dihydro-9*H*-purin-9-yl)cyclohexane-1-carbonitrile **77h** (100 mg, 0.34 mmol), 4-amino-2-fluoro-5-methylbenzamide **intermediate 2** (52 mg, 0.34 mmol), cesium carbonate (332 mg, 1.02 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (31 mg, 0.034 mmol) were dissolved in dioxane (2 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 4-((9-(*cis*-4-cyanocyclohexyl)-7-methyl-8-oxo-8,9-dihydro-7*H*-purin-2-yl)amino)-2-fluoro-5-methylbenzamide **compound 77** (white solid, 44 mg, 29.3 % yield).

¹H NMR (400 MHz DMSO) δ 8.36 (s, 1H), 8.17 (s, 1H), 7.89 (d, 1H), 7.52 (d, 1H), 7.44 (s, 1H), 7.26 (s, 1H), 4.20-4.26 (m, 1H), 3.32 (s, 3H), 3.19 (s, 1H), 2.44-2.47 (m, 2H), 2.26(s, 3 H), 2.02 (d, 2H), 1.67-1.81 (m, 4H).

LC-MS m/z(ESI) = **424.20** [M+1]

### Example 78

### 4-((9-(cis-4-cyanocyclohexyl)-7-methyl-8-oxo-8,9-dihydro-7H-purin-2-yl)amino)-3-methylbenzonitrile (compound 78)

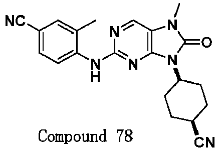

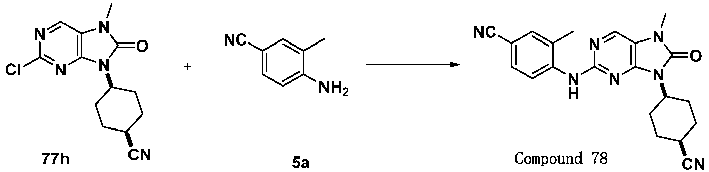

*cis*-4-(2-chloro-7-methyl-8-oxo-7,8-dihydro-9*H*-purin-9-yl)cyclohexane-1-carbonitrile **77h** (200 mg, 0.68 mmol), 4-amino-3-methylbenzonitrile **5a** (90.7 mg, 0.68 mmol), cesium carbonate (664 mg, 2 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (62 mg, 0.068 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 4-((9-(*cis*-4-cyanocyclohexyl)-7-methyl-8-oxo-8,9-dihydro-7*H*-purin-2-yl)amino)-3-methylbenzonitrile **compound 78** (white solid, 120 mg, 60 % yield).

¹H NMR (400 MHz DMSO) δ 8.48 (s, 1H), 8.22 (s, 1H), 8.20 (s, 1H),7.48 (d, 1H), 7.61 (s, 1H), 4.24-4.17 (m, 1H), 3.21 (s, 1H), 2.50-2.48 (m,2H), 2.32 (s,3H), 2.10-2.01 (m, 2H), 1.79-1.70 (m, 4H).

LC-MS m/z(ESI) = **406.20** [M+1]

### Example 79

### 4-((9-(trans-4-Cyanocyclohexyl)-7-methyl-8-oxo-8,9-dihydro-7H-purin-2-yl)amino)-2-fluoro-5-methylbenzamide (compound 79)

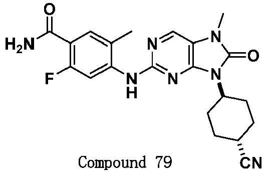

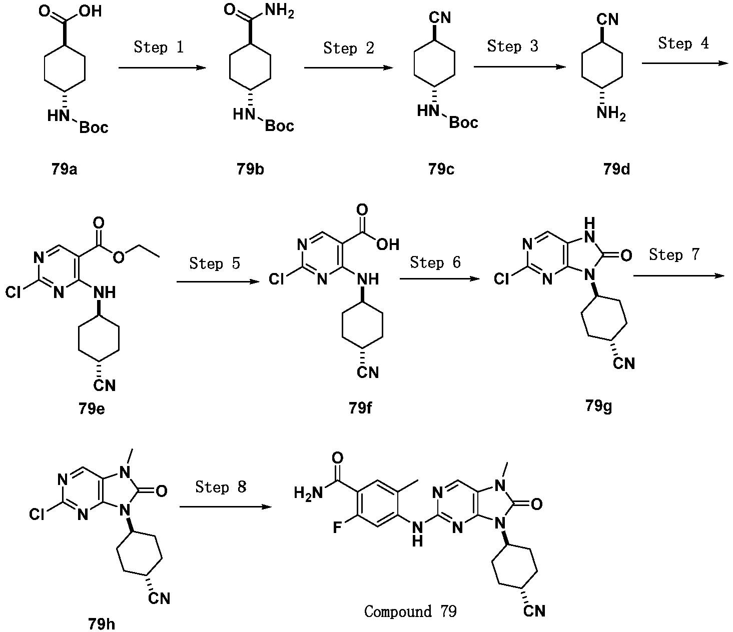

### Step 1:

### tert-Butyl(trans-4-carbamoylcyclohexyl)carbamate (79b)

*trans*-4-((*tert*-butoxycarbonyl)amino)cyclohexane-1-carboxylic acid **79a** (5.0 g, 20.5 mmol) and O-(7-azabenzotriazolyl)-*N*,*N*,*N'*,*N'*-tetramethyl-uronium-hexafluorophosphate (9.4 g, 24.7 mmol) were dissolved in dichloromethane (15 mL). The solution was stirred at 0 °C for 20 min, and *N*,*N-*diisopropylethylamine (10.5 g, 82 mmol) and ammonium chloride (3.3 g, 61.5 mmol) were added. The reaction mixture was stirred at room temperature for 4 h. The reaction was monitored by TLC. After completion of the reaction, 10 mL of water was added to the reaction mixture, and the organic phase was separated, washed with saturated brine once, dried over anhydrous sodium sulfate and concentrated. The residue was purified by preparative medium pressure liquid chromatography to give the title compound *tert-*butyl(*trans-4-*carbamoylcyclohexyl)carbamate **79b** (white solid, 4.4 g, 83% yield).

LC-MS m/z(ESI) = **243.30** [M+1]

### Step 2:

### tert-Butyl(trans-4-cyanocyclohexyl)carbamate (79c)

*trans*-Butyl(*cis*-4-carbamoylcyclohexyl)carbamate **79b** (4.4 g, 18.0 mmol) was dissolved in 70 mL of pyridine. The solution was cooled in an ice bath, and then phosphorus oxychloride (7.7 mL) was added to the reaction mixture dropwise. The reaction mixture was stirred in an ice bath for 1 h. The reaction was monitored by TLC. After completion of the reaction, 20 mL of water was added in an ice bath, and the resulting mixture was extracted 4 times with ethyl acetate. The organic phase was washed 7 times with acid water, finally washed twice with saturated brine, dried over anhydrous sodium sulfate, filtered and concentrated to obtain the compound *tert-*butyl(*trans*-4-cyanocyclohexyl)carbamate **79c** (yellow solid, 1.2 g, 30% yield).

¹H NMR (400 MHz DMSO) δ 6.80 (m, 1H), 3.24-3.22 (m, 1H), 2.63-2.55 (m, 1H), 1.99-1.95 (m, 2H), 1.77-1.73 (m, 2H), 1.56-1.46 (m, 2H), 1.36 (s, 9H), 1.21-1.11 (m, 2H).

LC-MS m/z(ESI) = **225.30** [M+1]

### Step 3:

### trans-4-Aminocyclohexane-1-carbonitrile (79d)

*tert*-Butyl(*cis*-4-cyanocyclohexyl)carbamate **79c** (1.2 g, 9.6 mmol) was dissolved in hydrogen chloride in ethyl acetate (20 mL), and the reaction mixture was heated to 45 °C and stirred for 2 h. The reaction was monitored by TLC. After completion of the reaction, a white solid was precipitated. The reaction mixture was filtered and concentrated to obtain the title compound *trans*-4-aminocyclohexane-1-carbonitrile **79d** (white solid, 660 mg, 60% yield).

LC-MS m/z(ESI) = **125.30** [M+1]

### Step 4:

### Ethyl 2-chloro-4-(trans-4-cyanocyclohexyl)amino)pyrimidine-5-carboxylate (79e)

Ethyl 2,4-dichloropyrimidine-5-carboxylate **1A** (1.4 g, 5.35 mmol) and potassium carbonate (1.4 g, 10.7 mmol) were dissolved in acetonitrile (10 mL), and *trans*-4-aminocyclohexane-1-carbonitrile **79d** (660 mg, 5.35 mmol) was added at 0 °C. The reaction mixture was stirred at room temperature for 20 h. The reaction was monitored by TLC. After completion of the reaction, water was added and the organics were extracted three times with ethyl acetate, and the organic phase was washed once with saturated brine, dried over anhydrous sodium sulfate, and purified by silica gel column chromatography (*n*-hexane/ethyl acetate (v/v) = 10:1) to obtain the title compound ethyl 2-chloro-4-(*trans*-4-cyanocyclohexyl)amino)pyrimidine-5-carboxylate **79e** (white solid, 810 mg, 62% yield).

¹H NMR (400 MHz DMSO) δ 8.62 (s, 1H), 8.27 (d, 1H), 4.30 (q, 2H), 4.04-3.96 (m, 1H), 4.04-3.67 (m, 1H), 2.76-2.70 (m, 1H), 2.04-2.00 (m, 2H), 1.96-1.92 (m, 2H), 1.72-1.62 (m, 2H), 1.47-1.37(m, 2H), 1.30(t,3H).

LC-MS m/z(ESI) = **310.20** [M+1]

### Step 5:

### 2-Chloro-4-((trans-4-cyanocyclohexyl)amino)pyrimidine-5-carboxylic acid (79f)

Ethyl 2-chloro-4-((*trans*-4-cyanocyclohexyl)amino)pyrimidine-5-carboxylate **79e** (810 mg, 2.6 mmol) was dissolved in tetrahydrofuran/water (4 mL/4 mL), and lithium hydroxide (247 mg, 5.2 mmol) was added. The reaction mixture was stirred at room temperature for 1 h. The reaction was monitored by TLC. After completion of the reaction, the reaction mixture was concentrated under reduced pressure to remove tetrahydrofuran and the pH was adjusted to 4-5, and a white solid was precipitate. The mixture was filtered, and the filter cake was washed twice with petroleum ether/ethyl acetate (v/v = 10/1) and concentrated to obtain the title compound 2-chloro-4-((*trans-*4-cyanocyclohexyl)amino)pyrimidine-5-carboxylic acid **79f** (white solid, 790 mg, 86% yield).

¹H NMR (400 MHz DMSO) δ 8.58 (s, 1H), 8.49 (d, 1H), 4.01-3.87 (m, 1H), 2.76-2.71 (m, 1H), 2.03-1.93 (m, 4H), 1.72-1.63 (m, 2H), 1.44-1.35 (m, 2H).

LC-MS m/z(ESI) = **282.30** [M+1]

### Step 6:

### trans-4-(2-Chloro-8-oxo-7,8-dihydro-9H-purin-9-yl)cyclohexane-1-carbonitrile (79g)

2-Chloro-4-((*trans*-4-cyanocyclohexyl)amino)pyrimidine-5-carboxylic acid **79f** (730 mg, 2.6 mmol) was dissolved in dimethylacetamide (10 mL), and triethylamine (263 mg, 2.6 mmol) and diphenylphosphoryl azide (715 mg, 2.6 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound *trans*-4-(2-chloro-8-oxo-7,8-dihydro-9*H*-purin-9-yl)cyclohexane-1-carbonitrile **79g** (white solid, 553 mg, 68% yield).

¹H NMR (400 MHz DMSO) δ 11.63 (s, 1H), 8.12 (s, 1H), 4.23-4.16 (m, 1H), 2.78-2.72 (m, 1H), 2.25-2.12 (m, 4H), 1.82-1.68 (m, 4H).

LC-MS m/z(ESI) = **278.30** [M+1]

### Step 7:

### trans-4-(2-Chloro-7-methyl-8-oxo-7,8-dihydro-9H-purin-9-yl)cyclohexane-1-carbonitrile (79h)

*trans*-4-(2-Chloro-8-oxo-7,8-dihydro-9*H*-purin-9-yl)cyclohexane-1-carbonitrile **79g** (553 mg, 2 mmol) was dissolved in dimethylformamide (5 mL), and dimethyl sulfate (252 mg, 2 mmol) and cesium carbonate (977 mg, 3 mmol) were added at 0 °C. The reaction mixture was stirred at 0 °C for 30 min. The reaction was monitored by TLC. After completion of the reaction, 10 mL of water was added to the reaction mixture, and a solid was precipitated. The solid was collected by filtration to give the title compound *trans*-4-(2-chloro-7-methyl-8-oxo-7,8-dihydro-9*H*-purin-9-yl)cyclohexane-1-carbonitrile **79h** (yellow solid, 420 mg, 72% yield).

¹H NMR (400 MHz DMSO) δ 8.34 (s, 1H), 4.28-4.21 (m, 1H), 2.79-2.72 (m, 1H), 2.22-2.13 (m, 4H), 1.82-1.70 (m, 4H).

LC-MS m/z(ESI) = **292.30** [M+1]

### Step 7:

### 4-((9-(trans-4-Cyanocyclohexyl)-7-methyl-8-oxo-8,9-dihydro-7H-purin-2-yl)amino)-2-fluoro-5-methylbenzamide (compound 79)

*trans*-4-(2-chloro-7-methyl-8-oxo-7,8-dihydro-9*H*-purin-9-yl)cyclohexane-1-carbonitrile **79h** (100 mg, 0.34 mmol), 4-amino-2-fluoro-5-methylbenzamide **intermediate 2** (52 mg, 0.34 mmol), cesium carbonate (332 mg, 1.02 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (31 mg, 0.034 mmol) were dissolved in dioxane (2 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 4-((9-(*trans*-4-cyanocyclohexyl)-7-methyl-8-oxo-8,9-dihydro-7*H*-purin-2-yl)amino)-2-fluoro-5-methylbenzamide **compound 79** (white solid, 57 mg, 36.2% yield).

¹H NMR (400 MHz DMSO) δ 8.48 (s, 1H), 8.18 (s, 1H), 7.95 (d, 1H), 7.54 (d, 1H), 7.45 (d, 1H), 4.20-4.29 (m, 1H), 3.31 (s, 3H), 2.60-2.67 (m, 1H), 2.25-2.34 (m, 5H), 2.15(d, 2 H), 1.83 (d, 2H), 1.72-1.76 (m, 2H).

LC-MS m/z(ESI) = **424.20** [M+1]

### Example 80

### 4-((9-(trans-4-cyanocyclohexyl)-7-methyl-8-oxo-8,9-dihydro-7H-purin-2-yl)amino)-3-methylbenzonitrile (compound 80)

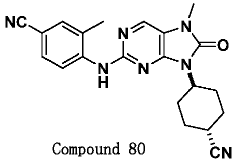

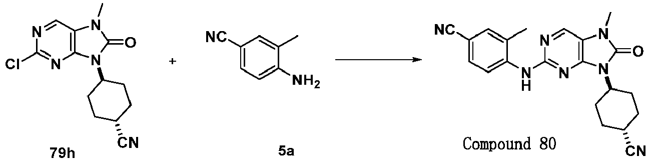

*trans*-4-(2-chloro-7-methyl-8-oxo-7,8-dihydro-9*H*-purin-9-yl)cyclohexane-1-carbonitrile **79h** (200 mg, 0.68 mmol), 4-amino-3-methylbenzonitrile **5a** (90.7 mg, 0.68 mmol), cesium carbonate (664 mg, 2 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (62 mg, 0.068 mmol) were dissolved in dioxane (3 mL), followed by nitrogen purging. The reaction mixture was stirred under nitrogen at 110 °C for 4 h. The reaction was monitored by TLC. After completion of the reaction, the reaction mixture was concentrated, and the residue was purified by silica gel column chromatography (dichloromethane/methanol (v/v) = 35/1) to obtain the title compound 4-((9-(*trans*-4-cyanocyclohexyl)-7-methyl-8-oxo-8,9-dihydro-7H-purin-2-yl)amino)-3-methylbenzonitrile **compound 80** (white solid, 120 mg, 60% yield).

¹H NMR (400 MHz DMSO) δ 8.60 (s, 1H), 8.16 (s, 1H), 8.12 (d, 1H),7.68 (d, 1H), 7.62 (s, 1H), 4.26-4.18 (m, 1H), 3.31 (s, 3H), 2.67-2.60 (m,1H), 2.32 (s,3H), 2.31-2.27(m, 2H), 2.24-2.07(m, 2H), 1.76 (dd, 2H), 1.75-1.64 (m, 2H).

LC-MS m/z(ESI) = **406.20** [M+1]

### Example 81

### 2-Fluoro-5-methyl-4-((7-methyl-8-oxo-9-(piperidin-4-yl)-8,9-dihydro-7H-purin-2-yl)amino)benzamide (compound 81)

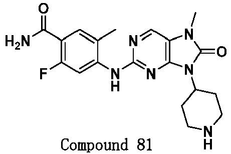

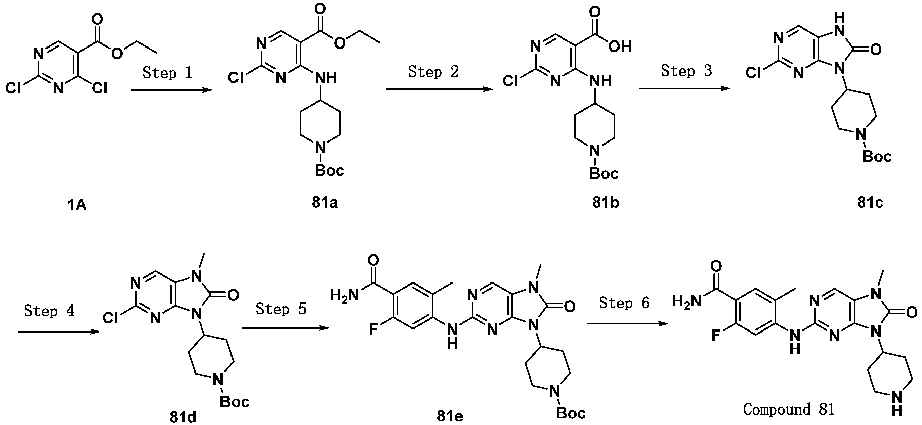

### Step 1:

### Ethyl 4-((1-(tert-butoxycarbonyl)piperidin-4-yl)amino)-2-chloropyrimidine-5-carboxylate (81a)

Ethyl 2,4-dichloropyrimidine-5-carboxylate **1A** (5 g, 22.6 mmol) and potassium carbonate (6.2 g, 45.2 mmol) were dissolved in acetonitrile (20 mL), and *tert*-butyl 4-aminopiperidine-1-carboxylate (4.5 g, 22.6 mmol) was added at 0 °C. The reaction mixture was stirred at room temperature for 20 h. The reaction was monitored by TLC. After completion of the reaction, water was added and the organics were extracted three times with ethyl acetate, and the organic phase was dried over anhydrous sodium sulfate and purified by silica gel column chromatography (*n*-hexane/ethyl acetate (v/v) = 10:1) followed by concentration to obtain the title compound ethyl 4-((1-(*tert-*butoxycarbonyl)piperidin-4-yl)amino)-2-chloropyrimidine-5-carboxylate **81a** (white solid, 8.2 g, 95% yield).

¹H NMR (400 MHz DMSO) δ 8.63 (s, 1H), 8.32 (d, 1H), 4.33-4.28 (m, 2H), 4.17-4.14 (m, 1H), 3.85 (d, 2H), 2.94 (s, 2H), 1.88-1.80 (m, 2H), 1.51-1.44 (m, 2H), 1.41 (s, 9H), 1.32-1.29 (m, 3H).

LC-MS m/z(ESI) = **385.10** [M+1]

### Step 2:

### 4-((1-(tert-Butoxycarbonyl)piperidin-4-yl)amino)-2-chloropyrimidine-5-carboxylic acid (81b)

Ethyl 4-((1-(*tert*-butoxycarbonyl)piperidin-4-yl)amino)-2-chloropyrimidine-5-carboxylate **81a** (8.2 g, 21.3 mmol) was dissolved in tetrahydrofuran/water (10 mL/5 mL), and lithium hydroxide (1.8 g, 42.7 mmol) was added. The reaction mixture was stirred at room temperature for 1 h. The reaction was monitored by TLC. After completion of the reaction, the reaction mixture was concentrated to evaporate tetrahydrofuran and the pH was adjusted to 4-5, and a white solid was precipitated. The mixture was filtered, and the filter cake was washed twice with petroleum ether/ethyl acetate (v/v = 10/1) and concentrated to obtain the title compound 4-((1-(*tert-*butoxycarbonyl)piperidin-4-yl)amino)-2-chloropyrimidine-5-carboxylic acid **81b** (white solid, 7 g, 86% yield).

¹H NMR (400 MHz DMSO) δ 8.59 (s, 1H), 8.54 (d, 1H), 4.20-4.10 (m, 1H), 3.87-3.84 (m, 2H), 2.96 (s, 3H), 1.91-1.73 (m, 3H), 1.41(s, 9H).

LC-MS m/z(ESI) = **357.10** [M+1]

### Step 3:

### tert-Butyl 4-(2-chloro-8-oxo-7,8-dihydro-9H-purin-9-yl)piperidine-1-carboxylate (81c)

4-((1-(*tert*-Butoxycarbonyl)piperidin-4-yl)amino)-2-chloropyrimidine-5-carboxylic acid **81b** (7 g, 19.6 mmol) was dissolved in dimethylacetamide (10 mL), and triethylamine (1.96 g, 19.6 mmol) and diphenylphosphoryl azide (5.4 g, 19.6 mmol) were added. The reaction mixture was gradually heated to 110 °C and stirred for 1.5 h. The reaction was monitored by TLC. After completion of the reaction, the reaction mixture was concentrated, and the residue was purified by silica gel column chromatography (petroleum ether/ethyl acetate (v/v) = 1:10) to obtain the title compound *tert*-butyl 4-(2-chloro-8-oxo-7,8-dihydro-9*H*-purin-9-yl)piperidine-1-carboxylate **81c** (white solid, 6.4 g, 87% yield).

¹H NMR (400 MHz DMSO) δ 8.14 (s, 1H), 4.41-4.33 (m, 1H), 4.06 (d, 2H), 2.88 (s, 2H), 2.30-2.20 (m, 2H), 1.84-1.71 (m, 2H), 1.43 (s, 9H).

LC-MS m/z(ESI) = **354.10** [M+1]

### Step 4:

### tert-Butyl 4-(2-chloro-7-methyl-8-oxo-7,8-dihydro-9H-purin-9-yl)piperidine-1-carboxylate (81d)

*tert*-Butyl 4-(2-chloro-8-oxo-7,8-dihydro-9*H*-purin-9-yl)piperidine-1-carboxylate **81c** (6.4 g, 18.1 mmol) was dissolved in dimethylformamide (10 mL), and dimethyl sulfate (2.28 g, 18.1 mmol) and cesium carbonate (8.5 g, 27.1 mmol) were added at 0 °C. The reaction mixture was stirred at 0 °C for 0.5 h. The reaction was monitored by TLC. After completion of the reaction, water was added to the reaction mixture, and a solid was precipitated. The mixture was filtered to give the title compound *tert*-butyl 4-(2-chloro-7-methyl-8-oxo-7,8-dihydro-9*H*-purin-9-yl)piperidine-1-carboxylate **81d** (white solid, 5.4 g, 79% yield).

¹H NMR (400 MHz DMSO) δ 8.36 (s, 1H), 4.46-4.37 (m, 1H), 4.05 (d, 2H), 3.35 (s, 3H), 2.87 (s, 2H), 2.33-2.19 (m, 2H), 1.74-1.72 (m, 2H), 1.43 (s, 9H).

LC-MS m/z(ESI) = **368.10** [M+1]

### Step 5:

### tert-Butyl 4-(2-((4-carbamoyl-5-fluoro-2-methylphenyl)amino)-7-methyl-8-oxo-7,8-dihydro-9H-purin-9-yl)piperidine-1-carboxylate (81e)

*tert*-Butyl 4-(2-chloro-7-methyl-8-oxo-7,8-dihydro-9*H*-purin-9-yl)piperidine-1-carboxylate **81d** (200 mg, 0.54 mmol), 4-amino-2-fluoro-5-methylbenzamide **intermediate 2** (181 mg, 0.54 mmol), cesium carbonate (528 mg, 1.08 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (50 mg, 0.054 mmol) were dissolved in dioxane (3 mL), followed by nitrogen purging. The reaction mixture was stirred under nitrogen atmosphere at 100 °C for 4 h. The reaction was monitored by TLC. After completion of the reaction, the reaction mixture was concentrated, and the residue was purified by silica gel column chromatography (dichloromethane/methanol (v/v) = 60/1) to obtain the title compound *tert*-butyl 4-(2-((4-carbamoyl-5-fluoro-2-methylphenyl)amino)-7-methyl-8-oxo-7,8-dihydro-9*H*-purin-9-yl)piperidine-1-carboxylate **81e** (white solid, 119 mg, 58% yield).

¹H NMR (400 MHz DMSO) δ 8.52 (s, 1H), 8.18 (s, 1H), 7.85 (d, 1H), 7.53 (d, 1H), 7.31 (s, 1H), 4.40-4.34 (m, 1H), 4.10 (dd, 2H), 3.20 (s, 3H), 2.88-2.74 (m, 2H), 2.37-2.30 (m, 2H), 2.27 (s, 3H), 1.75 (d, 2H), 1.40 (s, 9 H).

LC-MS m/z(ESI) = **500.20** [M+1]

### Step 6:

### 2-Fluoro-5-methyl-4-((7-methyl-8-oxo-9-(piperidin-4-yl)-8,9-dihydro-7H-purin-2-yl)amino)benzamide (compound 81)

*tert*-Butyl 4-(2-((4-carbamoyl-5-fluoro-2-methylphenyl)amino)-7-methyl-8-oxo-7,8-dihydro-9*H*-purin-9-yl)piperidine-1-carboxylate **81e** (119 mg, 0.23 mmol) was added to a 50 mL single-necked flask, and ethyl acetate hydrochloride 2 M (10 mL) was added thereto. The reaction mixture was stirred at room temperature for 4 h. The reaction was monitored by TLC. After completion of the reaction, the reaction mixture was concentrated, and the residue was purified by Pre-HPLC to obtain the title compound 2-fluoro-5-methyl-4-((7-methyl-8-oxo-9-(piperidin-4-yl)-8,9-dihydro-7*H-*purin-2-yl)amino)benzamide **compound 81** (white solid, 32 mg, 23% yield).

¹H NMR (400 MHz DMSO) δ 8.48 (s, 1H), 8.17 (d, 1H), 7.94 (d, 1H), 7.54 (d, 1H), 7.45-7.37 (m, 1H), 4.29-4.21 (m, 1H), 3.32 (s, 3H), 3.08-3.05 (m, 2H), 2.57-2.51 (m, 2H), 3.39-3.32 (m, 2H), 2.29 (s, 3H), 1.68-1.64 (m, 2H).

LC-MS m/z(ESI) = **400.20** [M+1]

### Example 82

### 2-Fluoro-5-methyl-4-((7-methyl-9-(1-methylpiperidin-4-yl)-8-oxo-8,9-dihydro-7H-purin-2-yl)amino)benzamide (compound 82)

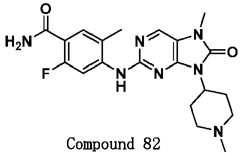

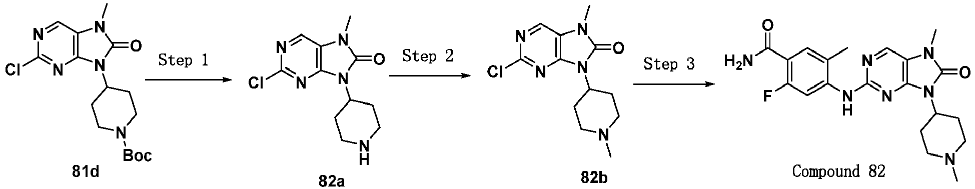

### Step 1:

### 2-Chloro-7-methyl-9-(piperidin-4-yl)-7,9-dihydro-8H-purin-8-one (82a)

*tert*-Butyl 4-(2-chloro-8-oxo-7,8-dihydro-9*H*-purin-9-yl)piperidine-1-carboxylate **81d** (2 g, 5.4 mmol) was added to a reaction flask, and 2 M hydrochloric acid-ethyl acetate solution (10 mL) was added with stirring at room temperature. The reaction mixture was stirred for 4 h. The reaction was monitored by TLC. After completion of the reaction, the reaction mixture was concentrated to give the hydrochloride salt of the title compound 2-chloro-7-methyl-9-(piperidin-4-yl)-7,9-dihydro-8*H*-purin-8-one as hydrochloride salt **82a** (white solid, 1.4 g, 91% yield).

¹H NMR (400 MHz DMSO) δ 8.39 (s, 1H), 6.42 (s, 1H), 4.59-4.53 (m, 1H), 3.39 (s, 2H), 3.36 (s, 3H), 3.16-3.04 (m, 2H), 2.62- 2.50 (m, 2H), 2.07-1.93 (m, 2H).

LC-MS m/z(ESI) = **268.10** [M+1]

### Step 2:

### 2-Chloro-7-methyl-9-(1-methylpiperidin-4-yl)-7,9-dihydro-8H-purin-8-one (82b)

2-Chloro-7-methyl-9-(piperidin-4-yl)-7,9-dihydro-8*H*-purin-8-one **82a** (1.4 g, 5.4 mmol) was dissolved in methanol (20 mL), and 4A molecular sieve (100 mg) was added, followed by the addition of polyformaldehyde (783 mg, 27 mmol). The reaction mixture was stirred at room temperature for 6 h. Sodium cyanoborohydride (1 g, 16.2 mmol) was added, and the reaction mixture was stirred for 2 h. The reaction was monitored by TLC. After completion of the reaction, the reaction mixture was filtered, and the filtrate was concentrated to give the title compound 2-chloro-7-methyl-9-(1-methylpiperidin-4-yl)-7,9-dihydro-8*H*-purin-8-one **82b** (white solid, 600 mg, 62% yield).

¹H NMR (400 MHz DMSO) δ 8.35 (s, 1H), 4.21-4.13 (m, 1H), 3.35 (s, 3H), 2.92 (d, 2H), 2.45-2.41 (m, 2H), 2.23 (s, 3H), 2.09-2.03 (m, 2H), 1.70-1.67 (m, 2H).

LC-MS m/z(ESI) = **282.10** [M+1]

### Step 3:

### 2-Fluoro-5-methyl-4-((7-methyl-9-(1-methylpiperidin-4-yl)-8-oxo-8,9-dihydro-7H-purin-2-yl)amino)benzamide (compound 82)

2-Chloro-7-methyl-9-(1-methylpiperidin-4-yl)-7,9-dihydro-8*H*-purin-8-one **82b** (150 mg, 0.53 mmol), 4-amino-2-fluoro-5-methylbenzamide **intermediate 2** (178 mg, 1.06 mmol), cesium carbonate (518 mg, 1.59 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (48 mg, 0.053 mmol) were dissolved in dioxane (3 mL), followed by nitrogen purging. The reaction mixture was stirred under nitrogen atmosphere at 100 °C for 4 h. The reaction was monitored by TLC. After completion of the reaction, the reaction mixture was concentrated, and the residue was purified by silica gel column chromatography (dichloromethane/methanol (v/v) = 20/1) followed by Pre-HPLC to obtain the title compound 2-fluoro-5-methyl-4-((7-methyl-9-(1-methylpiperidin-4-yl)-8-oxo-8,9-dihydro-7*H*-purin-2-yl)amino)benzamide **compound 82** (white solid, 20 mg, 18% yield).

¹H NMR (400 MHz DMSO) δ 8.27 (s, 1H), 8.14 (s, 1H), 7.93-7.89 (d, 1H), 7.58-7.55 (d, 1H),7.22 (s,2H), 3.34 (s, 3H), 2.95-2.92 (d, 2H), 2.67-2.55 (m, 2H), 2.30 (s, 3H), 2.25 (s, 3H), 2.10-2.04 (m, 2H), 1.91 (s, 1H), 1.72-1.68 (d, 2H).

LC-MS m/z(ESI) = **414.20** [M+1]

### Example 83

### 4-((9-(4,4-Difluorocyclohexyl)-7-methyl-8-oxo-8,9-dihydro-7H-purin-2-yl)amino)-2-fluoro-5-methylbenzamide (compound 83)

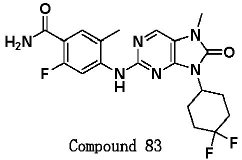

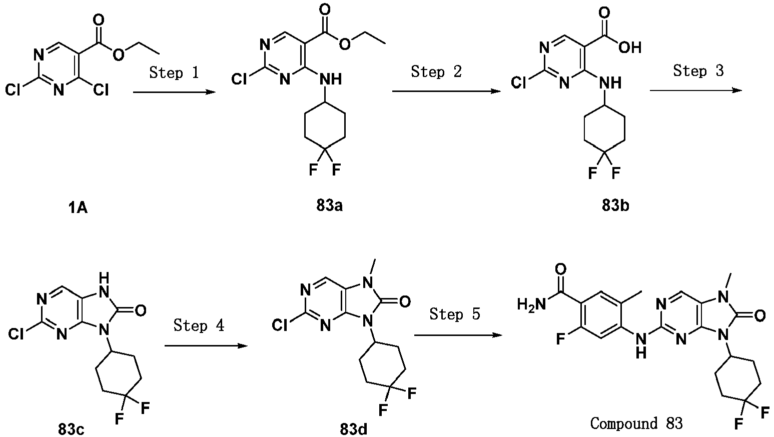

### Step 1:

### Ethyl 2-chloro-4-((4,4-difluorocyclohexyl)amino)pyrimidine-5-carboxylate (83a)

Ethyl 2,4-dichloropyrimidine-5-carboxylate **1A** (6.5 g, 29.41 mmol) and 4,4-difluorocyclohexa-1-amine hydrochloride (5.0 g, 29.41 mmol) were dissolved in acetonitrile (100 mL), and potassium carbonate (10.16 g, 73.52 mmol) was added with stirring at room temperature. The reaction mixture was stirred for 4 h. The reaction was monitored by TLC. After completion of the reaction, the reaction mixture was filtered, and the filter residue was washed with ethyl acetate. The filtrate was concentrated, and the residue was purified by silica gel column chromatography (*n*-hexane/ethyl acetate (v/v) = 1:1) to obtain the title compound ethyl 2-chloro-4-((tetrahydro-2*H*-pyran-4-yl)amino)pyrimidine-5-carboxylate **83a** (white solid, 8.0 g, 85.10% yield).

¹H NMR (400 MHz, DMSO-d6) δ 8.85 (s, 1H), 8.11 (d, 1H), 4.34-4.25 (m, 2H), 4.21-4.10 (m, 1H), 2.11-1.91 (m, 6H), 1.71-1.57 (m, 2H), 1.23 (t, 3H).

LC-MS m/z (ESI) = **320.10** [M+1]

### Step 2:

### 2-Chloro-4-((4,4-difluorocyclohexyl)amino)pyrimidine-5-carboxylic acid (83b)

Ethyl 2-chloro-4-((4,4-difluorocyclohexyl)amino)pyrimidine-5-carboxylate **83a** (4 g, 12.47 mmol) was dissolved in tetrahydrofuran/water (50 mL/50 mL), and lithium hydroxide (597 mg, 24.94 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 5 with 2 N hydrochloric acid, and a while solid was precipitated. The mixture was filtered, and the filter cake was washed twice with petroleum ether and collected to give the title compound 2-chloro-4-((4,4-difluorocyclohexyl)amino)pyrimidine-5-carboxylic acid **83b** (white solid, 3.4 g, 91.53% yield).

¹H NMR (400 MHz, DMSO-d6) δ 13.79 (s, 1H), 8.60 (s, 1H), 8.55 (d, 1H), 4.22-4.06 (m, 1H), 2.12-1.89 (m, 6H), 1.72-1.56 (m, 2H).

LC-MS m/z (ESI) = **292.00** [M+1]

### Step 3:

### 2-Chloro-9-(4,4-difluorocyclohexyl)-7,9-dihydro-8H-purin-8-one (83c)

2-Chloro-4-((tetrahydro-2*H*-pyran-4-yl)amino)pyrimidine-5-carboxylic acid **83b** (3.34 g, 11.45 mmol) was dissolved in dimethylacetamide (50 mL), and triethylamine (1.6 mL, 11.45 mmol) and diphenylphosphoryl azide (1.22 mL, 11.45 mmol) were added. The reaction mixture was gradually heated to 120 °C and stirred for 1.5 h. The reaction was monitored by TLC. The reaction mixture was poured into ice water. The solid was collected by filtration and washed 3 times with water, and concentration and drying were performed *in vacuo* to give the title compound 2-chloro-9-(4,4-difluorocyclohexyl)-7,9-dihydro-8*H*-purin-8-one **83c** (white solid, 2.2 g, 78.5% yield). ¹H NMR (400 MHz, DMSO-d6) δ 11.66 (s, 1H), 8.13 (s, 1H), 4.46-4.36 (m, 1H), 2.17-1.94 (m, 6H), 1.87-1.80 (m, 2H).

LC-MS m/z (ESI) = **289.10** [M+1]

### Step 4:

### 2-Chloro-9-(4,4-difluorocyclohexyl)-7-methyl-7,9-dihydro-8H-purin-8-one (83d)

2-Chloro-9-(4,4-difluorocyclohexyl)-7,9-dihydro-8*H*-purin-8-one **83c** (2.62 g, 9.08 mmol) was dissolved in dimethylformamide (50 mL), and dimethyl sulfate (1.14 g, 9.08 mmol) and cesium carbonate (4.44 g, 13.61 mmol) were added at 0 °C. The reaction mixture was stirred at 0 °C for 1 h. The reaction was monitored by TLC. After completion of the reaction, the reaction mixture was poured into ice water, and a solid was precipitated. The solid was collected by filtration to yield the title compound 2-chloro-7-methyl-9-(4,4-difluorocyclohexyl)-7,9-dihydro-8*H*-purin-8-one **83d** (white solid, 2.2 g, 80% yield).

¹H NMR (400 MHz, DMSO-d6) δ 8.37 (s, 1H), 4.55-4.39 (m, 1H), 3.33 (s, 3H), 2.21-2.01 (m, 6H), 1.90-1.79 (m, 2H).

LC-MS m/z (ESI) = **303.10** [M+1]

### Step 5:

### 4-((9-(4,4-Difluorocyclohexyl)-7-methyl-8-oxo-8,9-dihydro-7H-purin-2-yl)amino)-2-fluoro-5-methylbenzamide (compound 83)

2-Chloro-7-methyl-9-(4,4-difluorocyclohexyl)-7,9-dihydro-8*H*-purin-8-one **83d** (150 mg, 0.495 mmol), 4-amino-2-fluoro-5-methylbenzamide **intermediate 2** (166.6 mg, 0.991 mmol), cesium carbonate (323 mg, 0.991 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (45 mg, 0.05 mmol) were dissolved in dioxane, followed by nitrogen purging. The reaction mixture was stirred under nitrogen atmosphere at 100 °C for 4 h. The reaction was monitored by TLC. After completion of the reaction, the reaction mixture was poured into ice water, and the solid was collected and purified by silica gel column chromatography (dichloromethane/methanol (v/v) = 30/1) to obtain the title compound 4-((9-(4,4-difluorocyclohexyl)-7-methyl-8-oxo-8,9-dihydro-7*H*-purin-2-yl)amino)-2-fluoro-5-methylbenzamide **compound 83** (white solid, 30 mg, 14% yield).

¹H NMR (400 MHz, DMSO-d6) δ 8.49 (s, 1H), 8.20 (s, 1H), 7.84 (d, 1H), 7.54 (d, 1H), 7.46 (s, 1H), 7.38-7.33 (m, 1H), 4.45-4.38 (m, 1H), 3.32 (s, 3H), 2.28 (s, 3H), 2.18-1.94 (m, 6H), 1.87-1.81 (m, 2H).

LC-MS m/z (ESI) = **435.10** [M+1]

### Example 84

### 2-Fluoro-4-((9-((3aR,6aS)-5-hydroxyoctahydropentalen-2-yl)-7-methyl-8-oxo-8,9-dihydro-7H-purin-2-yl)amino)-5-methylbenzamide (compound 84)

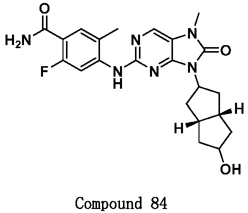

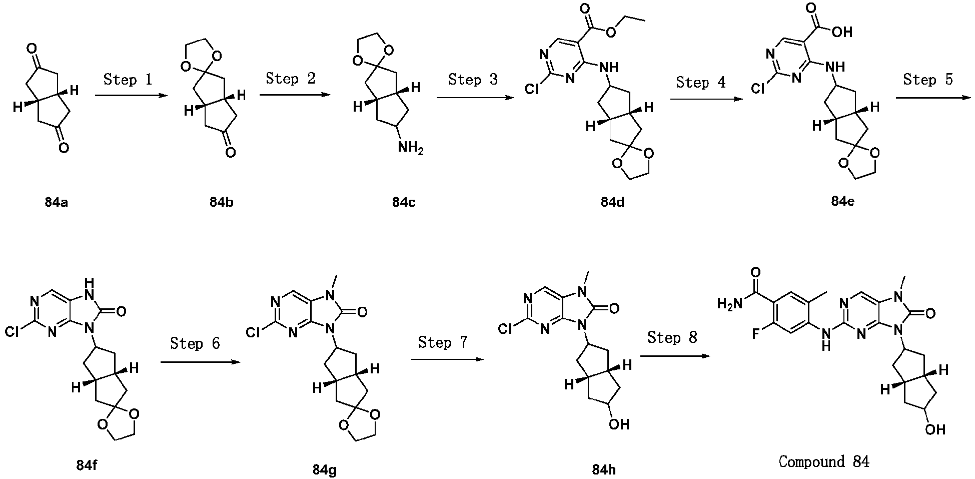

### Step 1:

### (3aR,6aS)-Tetrahydro-1H-spiro[pentalene-2,2'-[1,3]dioxolan]-5(3H)-one (84b)

(3as,6as)-Tetrahydropyrene-2,5(1*H*,3*H*)-dione **84a** (20 g, 145 mmol) and *p-*toluenesulfonic acid (2.7 g, 14.5 mmol) were added to a dry reaction flask and dissolved in toluene (250 mL), and ethylene glycol (7.3 mL, 130 mmol) was added dropwise at 120 °C. After addition, the reaction mixture was refluxed for 3 h. The reaction was monitored by TLC. After completion of the reaction, the reaction mixture was concentrated by silica gel column chromatography (petroleum ether/ethyl acetate (v/v) = 4/1) to obtain the title compound (3aR,6aS) -tetrahydro-1*H*-spiro[pentalene-2,2'-[1,3]dioxolan]-5(3*H*)-one **84b** (pale yellow liquid, 10.8 g, 40.88% yield).

LC-MS m/z (ESI) = **183.10** [M+1]

### Step 2:

### (3aR,6aS)-Hexahydro-1H-spiro[pentalene-2,2'-[1,3]dioxolan]-5-amine (84c)

(3a*R*,6a*S*)-Tetrahydro-1*H*-spiro[pentalene-2,2'-[1,3]dioxolan]-5(3*H*)-one **84b** (10.8 g, 59 mmol) was dissolved in methanol (100 mL), and ammonium acetate (45 g, 590 mmol) was added. The reaction mixture was stirred at 55 °C for 2 h. Sodium cyanoborohydride (5.5 g, 88.5 mmol) was added, and the reaction mixture was stirred for another 2 h. The reaction was monitored by TLC. After completion of the reaction, the reaction mixture was concentrated, and the residue was purified by silica gel column chromatography (dichloromethane/methanol (v/v) = 10/1) to obtain the title compound (3a*R*,6a*S*)-hexahydro-1*H*-spiro[pentalene-2,2'-[1,3]dioxolan]-5-amine **84c** (crude, yellow liquid, 5.5 g, 17.82% yield).

LC-MS m/z (ESI) = **184.10** [M+1]

### Step 3:

### Ethyl 2-chloro-4-(((3aR,6aS)-hexahydro-1H-spiro[pentalene-2,2'-[1,3]dioxolan]-5-yl)amino)pyrimidine-5-carboxylate (84d)

(3a*R*,6a*S*)-Hexahydro-1*H*-spiro[pentalene-2,2'-[1,3]dioxolane]-5-amine **84c** (5.5 g, 30.03 mmol) and potassium carbonate (12.4 g, 90.1 mmol) were well mixed with acetonitrile (100 mL), and ethyl 2,4-dichloropyrimidine-5-carboxylate **1A** (9.9 g, 45.05 mmol) was added at 0 °C. The reaction mixture was warmed to room temperature and stirred overnight. The reaction was monitored by TLC. After completion of the reaction, the reaction mixture was filtered. The filtrate was collected and concentrated, and the residue was purified by silica gel column chromatography (petroleum ether/ethyl acetate (v/v) = 8/1) to obtain the title compound ethyl 2-chloro-4-(((3a*R*,6a*S*)-hexahydro-1*H*-spiro[pentalene-2,2'-[1,3]dioxino]-5-yl)amino)pyrimidine-5-carboxylate **84d** (pale yellow solid, 0.74 g, 6.70% yield).

LC-MS m/z (ESI) = **368.10** [M+1]

### Step 4:

### 2-Chloro-4-(((3aR,6aS)-hexahydro-1H-spiro[pentalene-2,2'-[1,3]dioxolan]-5-yl)amino)pyrimidine-5-carboxylic acid (84e)

Ethyl 2-chloro-4-(((3a*R*,6a*S*)-hexahydro-1*H*-spiro[pentalene-2,2'-[1,3]dioxan]-5-yl)amino)pyrimidine-5-carboxylate **84d** (0.74 g, 2.01 mmol) was dissolved in tetrahydrofuran/water (1/1, 40 mL), and lithium hydroxide monohydrate (0.24 g, 6.03 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 3 with 2 N hydrochloric acid, and a white solid was precipitated. The mixture was filtered, and the filter cake was collected and dried to yield the title compound 2-chloro-4-(((3a*R*,6a*S*)-hexahydro-1*H-*spiro[pentalene-2,2'-[1,3]dioxido]-5-yl)amino)pyrimidine-5-carboxylic acid **84e** (white solid, 0.5 g, 73.20% yield).

LC-MS m/z (ESI) = **340.10** [M+1]

### Step 5:

### 2-Chloro-9-((3aR,6aS)-hexahydro-1H-spiro[pentalene-2,2'-[1,3]dioxolan]-5-yl)-7,9-dihydro-8H-purin-8-one (84f)

2-Chloro-4-(((3a*R*,6a*S*)-hexahydro-1*H*-spiro[pentalene-2,2'-[1,3]dioxido]-5-yl)amino)pyrimidine-5-carboxylicacid **84e** (0.5 g, 1.47 mmol) and diphenylphosphoryl azide (0.41 g, 1.47 mmol) were added to a dry reaction flask and dissolved in *N,N-*dimethylacetamide (20 mL), and triethylamine (0.2 mL, 1.47 mmol) was added dropwise at 0 °C. After addition, the reaction mixture was warmed to room temperature and stirred for 30 min, and then stirred at 120 °C for 2 h. The reaction was monitored by TLC. The reaction mixture was poured into six volumes of ice water, and a pale yellow solid was precipitated. The mixture was filtered, and the filter cake was washed with water (60 mL) and dried to give the target compound 2-chloro-9-((3a*R*,6a*S*)-hexahydro-1*H*-spiro[pentalene-2,2'-[1,3]dioxino]-5-yl)-7,9-dihydro-8*H*-purin-8-one **84f** (pale yellow solid, 0.38 g, 76.77% yield).

LC-MS m/z (ESI) = **337.10** [M+1]

### Step 6:

### 2-Chloro-9-((3aR,6aS)-hexahydro-1H-spiro[pentalene-2,2'-[1,3]dioxolan]-5-yl)-7-methyl-7,9-dihydro-8H-purin-8-one (84g)

2-Chloro-9-((3a*R*,6a*S*)-hexahydro-1*H*-spiro[pentalene-2,2'-[1,3]dioxido]-5-yl)-7,9-dihydro-8*H*-purin-8-one **84f** (0.38 g, 1.13 mmol), dimethyl sulfate (0.14 g, 1.13 mmol) and cesium carbonate (0.55 g, 1.69 mmol) were added to a dry reaction flask and dissolved in *N,N*-dimethylformamide (20 mL). The reaction mixture was stirred at room temperature for 1 h. The reaction was monitored by TLC. After completion of the reaction, water was added and the organics were extracted with ethyl acetate. The organic phases were combined, dried and concentrated to yield the title compound 2-chloro-9-((3a*R*,6a*S*)-hexahydro-1*H*-spiro[pentalene-2,2'-[1,3]dioxino]-5-yl)-7-methyl-7,9-dihydro-8*H*-purin-8-one **84g** (yellow liquid, crude, 0.68 g, 174% yield).

LC-MS m/z (ESI) = **351.10** [M+1]

### Step 7:

### 2-Chloro-9-((3aR,6aS)-5-hydroxyoctahydropentalen-2-yl)-7-methyl-7,9-dihydro-8H-purin-8-one (84h)

2-Chloro-9-((3a*R*,6a*S*)-hexahydro-1*H*-spiro[pentalene-2,2'-[1,3]dioxido]-5-yl)-7-methyl-7,9-dihydro-8*H*-purin-8-one **84g** (0.39 g, 1.13 mmol, theoretical) are dissolved in tetrahydrofuran/methanol (10/1, 11 mL), and diluted hydrochloric acid (3.8 mL, 3 mol/L) was added. The reaction mixture was stirred at room temperature for 1.5 h, concentrated to remove tetrahydrofuran and methanol, the pH was adjusted to about 8 with saturated sodium bicarbonate, and extracted with ethyl acetate. The organic phases were combined, dried and concentrated to yield a yellow solid (0.37 g), which was then dissolved in tetrahydrofuran/methanol (10/1, 11 mL), and sodium borohydride (0.05 g, 1.35 mmol) was added. The mixture was obtain at room temperature for 2 h. The reaction was monitored by TLC. After completion of the reaction, the reaction system was quenched with water (0.5 mL) and purified by silica gel column chromatography (petroleum ether/ethyl acetate (v/v) = 1/2) to obtain the title compound 2-chloro-9-((3a*R*,6a*S*)-5-hydroxyoctahydropentalen-2-yl)-7-methyl-7,9-dihydro-8*H*-purin-8-one **84h** (white solid, 0.27 g, 77.38% yield).

### Step 8:

### 2-Fluoro-4-((9-((3aR,6aS)-5-hydroxyoctahydropentalen-2-yl)-7-methyl-8-oxo-8,9-dihydro-7H-purin-2-yl)amino)-5-methylbenzamide (compound 84)

2-Chloro-9-((3a*R*,6a*S*)-5-hydroxyoctahydropenten-2-yl)-7-methyl-7,9-dihydro-8*H-*purin-8-one **84h** (0.19 g, 0.61 mmol), 4-amino-2-fluoro-5-methylbenzamide **intermediate 2** (0.41 g, 2.46 mmol), cesium carbonate (0.8 g, 2.46 mmol) and Brettphos G3 Pd (56 mg, 0.061 mmol) were dissolved in 1,4-dioxane (20 mL), and the system was purged with nitrogen three times. The reaction mixture was stirred at 110 °C for 4 h. The reaction was monitored by TLC. After completion of the reaction, the reaction mixture was purified by silica gel column chromatography (DCM/MeOH (v/v) = 30/1) to obtain the title compound 2-fluoro-4-((9-((3a*R*,6a*S*)-5-hydroxyoctahydropentan-2-yl)-7-methyl-8-oxo-8,9-dihydro-7*H*-purin-2-yl)amino)-5-methylbenzamide **compound 84** (white solid, 0.14 g, 52.10% yield).

¹H NMR (600 MHz, DMSO-*d*₆) δ 8.49 (s, 1H), 8.17 (s, 1H), 7.82 (d, 1H), 7.54 (d, 1H), 7.48 (s, 1H), 7.37 (s, 1H), 4.74 (s, 1H), 4.61-4.52 (m, 1H), 4.09-3.99 (m, 1H), 3.31 (s, 3H), 2.36-2.30 (m, 4H), 2.07-1.98 (m, 4H), 1.37-1.28 (m, 3H), 1.23 (d, 2H).

LC-MS m/z (ESI) = **441.20** [M+1]

### Example 85

### 4-((9-(8-Oxabicyclo[3.2.1]octan-3-yl)-7-methyl-8-oxo-8,9-dihydro-7H-purin-2-yl)amino)-2-fluoro-5-methylbenzamide (compound 85)

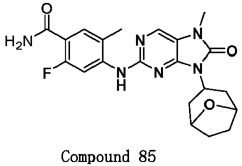

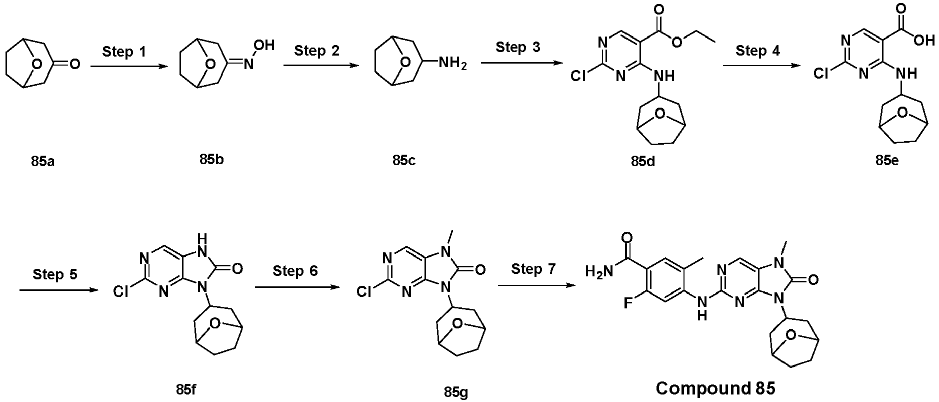

### Step 1:

### 8-Oxabicyclo[3.2.1]octan-3-one oxime (85b)

8-Oxabicyclo[3.2.1]octan-3-one **85a** (1.4 g, 11.1 mmol), hydroxylamine hydrochloride (925 mg, 13.3 mmol) and potassium carbonate (3.1 g, 22.2 mmol) were dissolved in a solvent mixture of ethanol/water (10/5 mL). The reaction mixture was stirred at 80 °C for 2 h. The reaction was monitored by TLC. After completion of the reaction, the reaction mixture was concentrated by rotary evaporation under reduced pressure to remove ethanol, and water was added and the organics were extracted with ethyl acetate. The organic layer was concentrated to give the title compound 8-oxabicyclo[3.2.1]octan-3-one oxime **85b** (pale yellow solid, crude, 1.56 g, 100% yield). LC-MS m/z(ESI) = **142.10** [M+1]

### Step 2:

### 8-Oxabicyclo[3.2.1]octan-3-amine (85c)

8-Oxabicyclo[3.2.1]octan-3-one oxime **85b** (1.5 g, 10.64 mmol) was dissolved in methanol (30 mL), and nickel chloride hexahydrate (2.53 g, 10.64 mmol) was added at room temperature. The reaction mixture was stirred at room temperature for 0.5 h and then cooled to -30 °C. Sodium borohydride (6.0 g, 159.6 mmol) was slowly added, and after addition, the reaction mixture was slowly warmed to room temperature and stirred overnight. The reaction was monitored by TLC. After completion of the reaction, water was added and the organics were extracted with ethyl acetate, and the organic layer was concentrated to yield the title compound 8-oxabicyclo[3.2.1]octan-3-amine **85c** (pale yellow oil, 639 mg, 45% yield).

LC-MS m/z(ESI) = **128.10** [M+1]

### Step 3:

### Ethyl 4-((8-oxabicyclo[3.2.1]octan-3-yl)amino)-2-chloropyrimidine-5-carboxylate (85d)

Ethyl 2,4-dichloropyrimidine-5-carboxylate **1A** (1.1 g, 5.03 mmol) and potassium carbonate (1.74 g, 12.58 mmol) were dissolved in acetonitrile (20 mL), and 8-oxabicyclo[3.2.1]octan-3-amine **85c** (639 mg, 5.03 mmol) was added at 0 °C. The reaction mixture was stirred at room temperature for 20 h. The reaction was monitored by TLC. After completion of the reaction, water (30 mL) was added to the reaction mixture, and a solid was precipitated. The mixture was filtered, and the filter cake was washed 3 times with water and dried to give the title compound ethyl 4-((8-oxabicyclo[3.2.1]octan-3-yl)amino)-2-chloropyrimidine-5-carboxylate **85d** (white solid, 1.01 g, 64.3% yield).

¹H NMR (400 MHz, DMSO) δ 8.91 (d, 1H), 8.64 (s, 1H), 4.38-4.31 (m, 4H), 4.30-4.24 (m, 1H), 2.20-2.08 (m, 2H), 2.02-1.90 (m, 4H), 1.66 (d, 2H), 1.32 (t, 3H).

LC-MS m/z(ESI) = **312.10** [M+1]

### Step 4:

### 4-((8-Oxabicyclo[3.2.1]octan-3-yl)amino)-2-chloropyrimidine-5-carboxylic acid (85e)

Ethyl 4-((8-oxabicyclo[3.2.1]octan-3-yl)amino)-2-chloropyrimidine-5-carboxylate **85d** (1.0 g, 3.21 mmol) was dissolved in 10 mL of tetrahydrofuran and 5 mL of water, and lithium hydroxide (308 mg, 12.83 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 4, and a white solid precipitated. The mixture was filtered, and the filter cake was washed twice with petroleum ether/ethyl acetate (v/v = 10/1), concentrated and dried to obtain the title compound 4-((8-oxabicyclo[3.2.1]octan-3-yl)amino)-2-chloropyrimidine-5-carboxylic acid **85e** (white solid, 808 mg, 87.9% yield).

¹H NMR (400 MHz, DMSO) δ 13.83 (s, 1H), 9.17 (d, 1H), 8.59 (s, 1H), 4.32 (s, 2H), 4.29-4.22 (m, 1H), 2.17-2.07 (m, 2H), 2.00-1.89 (m, 4H), 1.65 (d, 2H).

LC-MS m/z(ESI) = **284.20** [M+1]

### Step 5:

### 9-(8-Oxabicyclo[3.2.1]octan-3-yl)-2-chloro-7,9-dihydro-8H-purin-8-one (85f)

4-((8-Oxabicyclo[3.2.1]octan-3-yl)amino)-2-chloropyrimidine-5-carboxylic acid **85e** (808 mg, 2.85 mmol) was dissolved in dimethylacetamide (20 mL), and triethylamine (288 mg, 2.85 mmol) and diphenylphosphoryl azide (784 mg, 2.85 mmol) were added. The reaction mixture was gradually heated to 120 °C and stirred for 1.5 h. The reaction was monitored by TLC. After completion of the reaction, the reaction mixture was purified by silica gel column chromatography (petroleum ether/ethyl acetate (v/v) = 5:1 to 1:10) to obtain the title compound 9-(8-oxabicyclo[3.2.1]octan-3-yl)-2-chloro-7,9-dihydro-8*H*-purin-8-one **85f** (white solid, 653 mg, 71% yield).

¹H NMR (600 MHz, DMSO) δ 11.64 (s, 1H), 8.12 (s, 1H), 4.47-4.44 (m, 2H), 4.42-4.40 (m, 1H), 2.28-2.20 (m, 2H), 2.07-2.01 (m, 2H), 1.96-1.90 (m, 2H), 1.81-1.75 (m, 2H).

LC-MS m/z(ESI) = **281.10** [M+1]

### Step 6:

### 9-(8-Oxabicyclo[3.2.1]octan-3-yl)-2-chloro-7-methyl-7,9-dihydro-8H-purin-8-one (85g)

9-(8-Oxabicyclo[3.2.1]octan-3-yl)-2-chloro-7,9-dihydro-8*H*-purin-8-one **85f** (653 mg, 2.33 mmol) was dissolved in dimethylformamide (10 mL), and dimethyl sulfate (293 mg, 2.33 mmol) and cesium carbonate (1.52 g, 4.65 mmol) were added at 0 °C. The reaction mixture was stirred for 1 h. The reaction was monitored by TLC. After completion of the reaction, 30 mL of water and ethyl acetate was added to the reaction mixture, and the organic phase was concentrated to afford the title compound 9-(8-oxabicyclo[3.2.1]octan-3-yl)-2-chloro-7-methyl-7,9-dihydro-8*H*-purin-8-one **85g** (white solid, 533 mg, 81.6% yield).

¹H NMR (400 MHz, DMSO) δ 8.36 8.34 (m, 1H), 4.51-4.47 (m, 2H), 4.45-4.42 (m, 1H), 3.34 (s, 3H), 2.31-2.22 (m, 2H), 2.07-1.98 (m, 2H), 1.98-1.90 (m, 2H), 1.79 (t, 2H).

LC-MS m/z(ESI) = **295.20** [M+1]

### Step 7:

### 4-((9-(8-Oxabicyclo[3.2.1]octan-3-yl)-7-methyl-8-oxo-8,9-dihydro-7H-purin-2-yl)amino)-2-fluoro-5-methylbenzamide (compound 85)

9-(8-Oxabicyclo[3.2.1]octan-3-yl)-2-chloro-7-methyl-7,9-dihydro-8*H*-purin-8-one **85g** (100 mg, 0.34 mmol), 4-amino-2-fluoro-5-methylbenzamide **intermediate 2** (57 mg, 0.34 mmol), cesium carbonate (221 mg, 0.68 mmol) and Brettphos Pd G3 (31 mg, 0.034 mmol) were dissolved in dioxane, followed by nitrogen purging. The reaction mixture was stirred under nitrogen atmosphere at 100 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 4-((9-(8-oxabicyclo[3.2.1]octan-3-yl)-7-methyl-8-oxo-8,9-dihydro-7*H*-purin-2-yl)amino)-2-fluoro-5-methylbenzamide **compound 85** (white solid, 41 mg, 28.9% yield).

¹H NMR (400 MHz, DMSO) δ 8.79 (s, 1H), 8.24-8.16 (m, 1H), 7.75 (d, 1H), 7.57 (d, 1H), 7.50 (s, 1H), 7.42 (s, 1H), 4.53-4.37 (m, 3H), 2.27 (s, 3H), 2.24-2.15 (m, 2H), 2.03 (t, 2H), 1.87 (s, 2H), 1.67 (d, 2H).

LC-MS m/z(ESI) = **427.20** [M+1]

### Example 86

### 2-Fluoro-5-methyl-4-((7-methyl-8-oxo-9-(2-oxaspiro[3.5]nonan-7-yl)-8,9-dihydro-7H-purin-2-yl)amino)benzamide (compound 86)

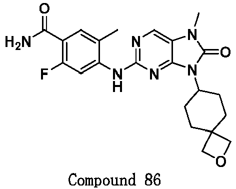

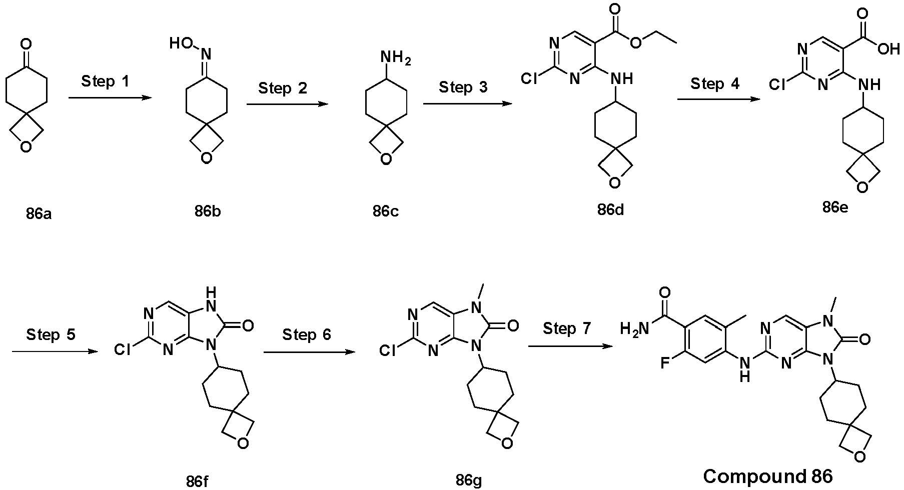

### Step 1:

### 2-Oxaspiro[3.5]nonan-7-one oxime (86b)

2-Oxaspiro [3.5]nonan-7-one **86a** (1.5 g, 10.7 mmol), hydroxylamine hydrochloride (744 mg, 10.7 mmol) and potassium carbonate (2.95 g, 21.4 mmol) were dissolved in a solvent mixture of ethanol/water (10/5 mL). The reaction mixture was stirred at 80 °C for 2 h. The reaction was monitored by TLC. After completion of the reaction, the reaction mixture was concentrated, and water was added and the organics were extracted with ethyl acetate. The organic layer was concentrated to obtain the title compound 2-oxaspiro[3.5]nonan-7-one oxime **86b** (pale yellow solid, crude, 1.45 g, 89% yield).

LC-MS m/z(ESI) = **156.10** [M+1]

### Step 2:

### 2-Oxaspiro[3.5]nonan-7-amine (86c)

2-Oxaspiro[3.5]nonan-7-one oxime **86b** (1.45 g, 9.34 mmol) was dissolved in methanol (30 mL), and nickel chloride hexahydrate (2.22 g, 9.34 mmol) was added at room temperature. The reaction mixture was stirred at room temperature for 0.5 h and then cooled to -30 °C. Sodium borohydride (5.3 g, 140.1 mmol) was slowly added, and after addition, the reaction mixture was slowly warmed to room temperature and stirred overnight. The reaction was monitored by TLC. After completion of the reaction, water was added and the organics were extracted with ethyl acetate, and the organic layer was concentrated to give the title compound 2-oxaspiro[3.5]nonan-7-amine **86c** (pale yellow oil, 481 mg, 37.6% yield).

LC-MS m/z(ESI) = **142.20** [M+1]

### Step 3:

### Ethyl 4-((2-oxaspiro[3.5]nonan-7-yl)amino)-2-chloropyrimidine-5-carboxylate (86d)

Ethyl 2,4-dichloropyrimidine-5-carboxylate **1A** (753 mg, 3.41 mmol) and potassium carbonate (940 mg, 6.81 mmol) were dissolved in acetonitrile (20 mL), and 8-oxabicyclo[3.2.1]octan-3-amine **86c** (481 mg, 3.41 mmol) was added at 0 °C. The reaction mixture was warmed to room temperature and stirred for 20 h. The reaction was monitored by TLC. After completion of the reaction, water (30 mL) was added to the reaction mixture, and a solid was precipitated. The mixture was filtered, and the filter cake was washed 3 times with water and concentrated to obtain the title compound ethyl 4-((2-oxaspiro[3.5]nonan-7-yl)amino)-2-chloropyrimidine-5-carboxylate **86d** (white solid, 640 mg, 57.6% yield).

¹H NMR (400 MHz, DMSO) δ 8.61 (s, 1H), 8.29 (d, 1H), 4.34-4.29 (m, 4H), 4.24 (s, 2H), 3.98-3.89 (m, 1H), 2.05-1.92 (m, 2H), 1.86-1.75 (m, 2H), 1.65-1.56 (m, 2H), 1.45-1.35 (m, 2H), 1.34-1.27 (m, 3H).

LC-MS m/z(ESI) = **326.20** [M+1]

### Step 4:

### 4-((2-Oxaspiro[3.5]nonan-7-yl)amino)-2-chloropyrimidine-5-carboxylic acid (86e)

Ethyl 4-((2-oxaspiro[3.5]nonan-7-yl)amino)-2-chloropyrimidine-5-carboxylate **86d** (640 mg, 1.96 mmol) was dissolved in 10 mL of tetrahydrofuran and 5 mL of water, and lithium hydroxide (189 mg, 7.86 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 4, and a white solid was precipitated. The mixture was filtered, and the filter cake was washed twice with petroleum ether/ethyl acetate (v/v = 10/1) and concentrated to obtain the title compound 4-((2-oxaspiro[3.5]nonan-7-yl)amino)-2-chloropyrimidine-5-carboxylic acid **86e** (white solid, 518 mg, 88.5% yield).

¹H NMR (400 MHz, DMSO) δ 13.74 (s, 1H), 8.57 (s, 1H), 8.48 (d, 1H), 4.32 (s, 2H), 4.24 (s, 2H), 3.97-3.84 (m, 1H), 1.99 (d, 2H), 1.86-1.76 (m, 2H), 1.66-1.54 (m, 2H), 1.42-1.26 (m, 2H).

LC-MS m/z(ESI) = **298.10** [M+1]

### Step 5:

### 2-Chloro-9-(2-oxaspiro[3.5]nonan-7-yl)-7,9-dihydro-8H-purin-8-one (86f)

4-((2-Oxaspiro[3.5]nonan-7-yl)amino)-2-chloropyrimidine-5-carboxylic acid **86e** (518 mg, 1.74 mmol) was dissolved in dimethylacetamide (20 mL), and triethylamine (175 mg, 1.74 mmol) and diphenylphosphoryl azide (479 mg, 1.74 mmol) were added. The reaction mixture was gradually heated to 120 °C and stirred for 1.5 h. The reaction was monitored by TLC. After completion of the reaction, the reaction mixture was concentrated, and the residue was purified by silica gel column chromatography (petroleum ether/ethyl acetate (v/v) = 5:1 to 1:10) to obtain the title compound 2-chloro-9-(2-oxaspiro[3.5]nonan-7-yl)-7,9-dihydro-8*H*-purin-8-one **86f** (white solid, 353 mg, 72.8% yield).

¹H NMR (600 MHz, DMSO) δ 11.61 (s, 1H), 8.11 (s, 1H), 4.41 (s, 2H), 4.24 (s, 2H), 4.15-4.08 (m, 1H), 2.24-2.08 (m, 4H), 1.69 (d, 2H), 1.64-1.51 (m, 2H).

LC-MS m/z(ESI) = **295.10** [M+1]

### Step 6:

### 2-Chloro-7-methyl-9-(2-oxaspiro[3.5]nonan-7-yl)-7,9-dihydro-8H-purin-8-one (86g)

2-Chloro-9-(2-oxaspiro[3.5]nonan-7-yl)-7,9-dihydro-8*H*-purin-8-one **86f** (353 mg, 1.18 mmol) was dissolved in dimethylformamide (10 mL), and dimethyl sulfate (150 mg, 1.18 mmol) and cesium carbonate (464 mg, 3.36 mmol) were added at 0 °C. The reaction mixture was stirred for 1 h. The reaction was monitored by TLC. After completion of the reaction, 30 mL of water was added, and the mixture was extracted with ethyl acetate. The organic phase was concentrated to afford the title compound 2-chloro-7-methyl-9-(2-oxaspiro[3.5]nonan-7-yl)-7,9-dihydro-8*H*-purin-8-one **86g** (white solid, 313 mg, 85.5% yield).

¹H NMR (400 MHz, DMSO) δ 8.34 (s, 1H), 4.41 (s, 2H), 4.25 (s, 2H), 4.22- 4.08 (m, 1H), 3.34 (s, 3H), 2.22-2.05 (m, 4H), 1.69 (d, 2H), 1.64-1.50 (m, 2H).

LC-MS m/z(ESI) = **309.20** [M+1]

### Step 7:

### 2-Fluoro-5-methyl-4-((7-methyl-8-oxo-9-(2-oxaspiro[3.5]nonan-7-yl)-8,9-dihydro-7H-purin-2-yl)amino)benzamide (compound 86)

2-Chloro-7-methyl-9-(2-oxaspiro[3.5]nonan-7-yl)-7,9-dihydro-8*H-*purin-8-one **86g** (60 mg, 0.194 mmol), 4-amino-2-fluoro-5-methylbenzamide **intermediate 2** (33 mg, 0.194 mmol), cesium carbonate (127 mg, 0.39 mmol) and Brettphos Pd G3 (17 mg, 0.019 mmol) were dissolved in dioxane, followed by nitrogen purging. The reaction mixture was stirred under nitrogen atmosphere at 100 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-5-methyl-4-((7-methyl-8-oxo-9-(2-oxaspiro[3.5]nonan-7-yl)-8,9-dihydro-7*H*-purin-2-yl)amino)benzamide **compound 86** (white solid, 21 mg, 24.7% yield).

¹H NMR (400 MHz, DMSO-*d*₆) δ 8.50 (s, 1H), 8.17 (s, 1H), 7.74 (d, 1H), 7.55 (d, 1H), 7.47 (s, 1H), 7.37 (s, 1H), 4.32 (s, 2H), 4.23 (s, 2H), 4.13 (s, 1H), 3.31 (s, 3H), 2.27 (s, 3H), 2.16 (d, 4H), 1.72-1.64 (m, 2H), 1.53 (t, 2H).

LC-MS m/z(ESI) = **441.20** [M+1]

### Example 87

### 2-Fluoro-4-((9-(6-hydroxyspiro[3.3]heptan-2-yl)-7-methyl-8-oxo-8,9-dihydro-7H-purin-2-yl)amino)-5-methylbenzamide (compound 87)

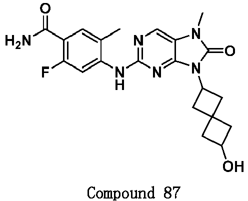

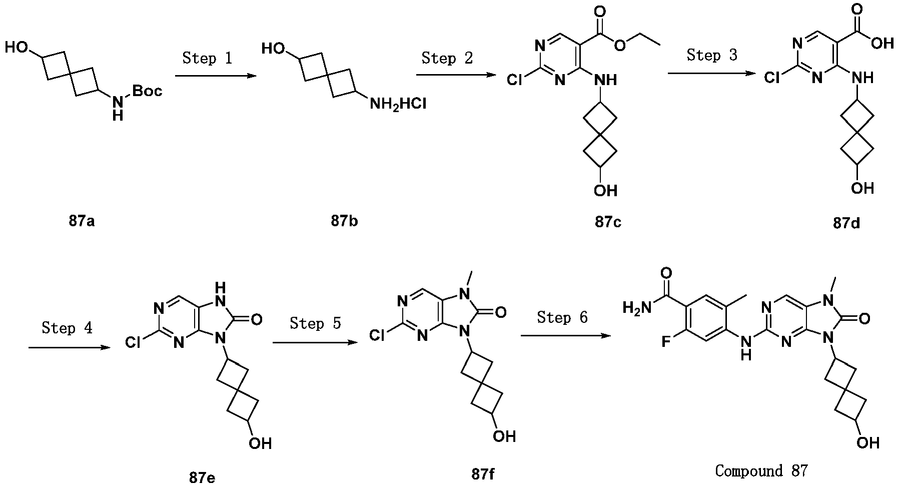

### Step 1:

### 6-Aminospiro[3.3]heptan-2-ol hydrochloride (87b)

*tert*-Butyl (6-hydroxyspiro[3.3]heptan-2-yl)carbamate **87a** (2 g, 8.80 mmol) was dissolved in 4 M hydrogen chloride-1,4-dioxane (15 mL). The reaction mixture was stirred at room temperature for 2 h. The reaction was monitored by TLC. After completion of the reaction, the reaction mixture was concentrated to evaporate 1,4-dioxane solution to give the title compound 6-aminospiro[3.3]heptan-2-ol hydrochloride **87b** (pale yellow solid, crude, 1.44 g, 100% yield).

LC-MS m/z(ESI) = **163.10** [M+1]

### Step 2:

### Ethyl 2-chloro-4-((6-hydroxyspiro[3.3]heptan-2-yl)amino)pyrimidine-5-carboxylate (87c)

Ethyl 2,4-dichloropyrimidine-5-carboxylate **1A** (2.92 g, 13.20 mmol) and 6-aminospiro[3.3]heptane-2-ol hydrochloride **87b** (1.44 g, 8.80 mmol) were dissolved in acetonitrile (20 mL), and potassium carbonate (3.65 g, 26.40 mmol) was added with stirring. The reaction mixture was stirred at room temperature for 4 h. The reaction was monitored by TLC. The reaction mixture was concentrated, and the residue was purified by column chromatography (petroleum ether: ethyl acetate (v/v) = 4:1) to obtain the title compound ethyl 2-chloro-4-((3-hydroxy-3-methylcyclohexyl)amino)pyrimidine-5-carboxylate **87c** (white solid g, 1.36 g, 49.57% yield).

¹H NMR (400 MHz, DMSO-d6) δ 8.59 (s, 1H), 8.43 (d, 1H), 4.91 (s, 1H), 4.44-4.34 (m, 1H), 4.31 (q, 2H), 4.02-3.93 (m, 1H), 2.43-2.35 (m, 2H), 2.34-2.27 (m, 1H), 2.23-2.16 (m, 1H), 2.07-1.99 (m, 2H), 1.90-1.80 (m, 2H), 1.31 (t, 3H).

LC-MS m/z(ESI) = **312.10** [M+1]

### Step 3:

### 2-Chloro-4-((6-hydroxyspiro[3.3]heptan-2-yl)amino)pyrimidine-5-carboxylic acid (87d)

Ethyl 2-chloro-4-((6-hydroxyspiro[3.3]heptan-2-yl)amino)pyrimidine-5-carboxylate **87c** (1.0 g, 3.21 mmol) was dissolved in 10 mL of tetrahydrofuran and 10 mL of water, and lithium hydroxide (269 mg, 6.42 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 2 N hydrochloric acid, and a white solid was precipitated. The reaction mixture was filtered, and the filter cake was washed twice with petroleum ether and collected to yield the title compound 2-chloro-4-((6-hydroxyspiro[3.3]heptan-2-yl)amino)pyrimidine-5-carboxylic acid **87d** (760 mg, white solid, 83.52% yield).

¹H NMR (400 MHz, DMSO-d6) δ 13.75 (s, 1H), 8.62 (d, 1H), 8.56 (s, 1H), 4.90 (s, 1H), 4.42-4.32 (m, 1H), 4.00-3.93 (m, 1H), 2.42-2.35 (m, 2H), 2.33-2.27 (m, 1H), 2.22-2.16 (m, 1H), 2.02-1.97 (m, 2H), 1.88-1.80 (m, 2H).

LC-MS m/z(ESI) = **284.10** [M+1]

### Step 4:

### 2-Chloro-9-(6-hydroxyspiro[3.3]heptan-2-yl)-7,9-dihydro-8H-purin-8-one (87e)

2-Chloro-4-((6-hydroxyspiro[3.3]heptan-2-yl)amino)pyrimidine-5-carboxylic acid **87d** (760 mg, 2.68 mmol) was dissolved in dimethylacetamide (10 mL), and triethylamine (0.37 mL, 2.68 mmol) and diphenylphosphoryl azide (0.57 mL, 2.68 mmol) were added. The reaction mixture was gradually heated to 90 °C and stirred for 2 h. The reaction was monitored by TLC. After completion of the reaction, the reaction mixture was poured into ice water. The solid was collected by filtration and washed 3 times with water, concentrated and dried to give the title compound 2-chloro-9-(6-hydroxyspiro[3.3]heptan-2-yl)-7,9-dihydro-8*H*-purin-8-one **87e** (580 mg, white solid, 77.13% yield).

¹H NMR (400 MHz, DMSO-d6) δ 11.60 (s, 1H), 8.10 (s, 1H), 4.96 (d, 1H), 4.70-4.60 (m, 1H), 2.92-2.87 (m, 2H), 2.46-2.43 (m, 2H), 2.32-2.21 (m, 3H), 1.92-1.87 (m, 2H). LC-MS m/z(ESI) = **281.10** [M+1]

### Step 5:

### 2-Chloro-9-(6-hydroxyspiro[3.3]heptan-2-yl)-7-methyl-7,9-dihydro-8H-purin-8-one (87f)

2-Chloro-9-(6-hydroxyspiro[3.3]heptan-2-yl)-7,9-dihydro-8*H*-purin-8-one **87e** (580 mg, 2.07 mmol) was dissolved in dimethylformamide (5 mL), and dimethyl sulfate (260 mg, 2.07 mmol) and cesium carbonate (1.3 g, 4.13 mmol) were added at 0 °C. The reaction mixture was stirred for 1 h. The reaction was monitored by TLC. After completion of the reaction, the reaction mixture was poured into ice water, and a solid was precipitated. The solid was collected by filtration to yield the title compound 2-chloro-9-(6-hydroxyspiro[3.3]heptan-2-yl)-7-methyl-7,9-dihydro-8*H*-purin-8-one **87f** (380 mg, white solid, 62.40% yield).

¹H NMR (400 MHz, DMSO-d6) δ 8.33 (s, 1H), 4.97 (d, 1H), 4.70-4.66 (m, 1H), 4.05-3.96 (m, 1H), 3.33 (s, 3H), 2.95-2.87 (m, 2H), 2.51-2.43 (m, 1H), 2.31-2.25 (m, 3H), 1.93-1.87 (m, 2H).

LC-MS m/z(ESI) = **295.10** [M+1]

### Step 6:

### 2-Fluoro-4-((9-(6-hydroxyspiro[3.3]heptan-2-yl)-7-methyl-8-oxo-8,9-dihydro-7H-purin-2-yl)amino)-5-methylbenzamide (compound 87)

2-Chloro-9-(6-hydroxyspiro[3.3]heptan-2-yl)-7-methyl-7,9-dihydro-8*H*-purin-8-one **87f** (130 mg, 0.44 mmol), 4-amino-2-fluoro-5-methylbenzamide **intermediate 2** (222 mg, 1.32 mmol), cesium carbonate (311 mg, 0.88 mmol) and Brettphos Pd G3 (40 mg, 0.044 mmol) were dissolved in dioxane, followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(6-hydroxyspiro[3.3]heptan-2-yl)-7-methyl-8-oxo-8,9-dihydro-7*H*-purin-2-yl)amino)-5-methylbenzamide **compound 87** (white solid, 35 mg, 18.62% yield).

1H NMR (400 MHz, DMSO-d6) δ 8.51 (s, 1H), 8.18 (s, 1H), 7.85 (d, 1H), 7.55 (d, 1H), 7.47 (s, 1H), 7.38 (s, 1H), 4.97 (d, 1H), 4.72-4.63 (m, 1H), 4.03-3.94 (m, 1H), 3.29 (s, 3H), 2.99-2.90 (m, 2H), 2.46-2.40 (m, 1H), 2.29 (s, 3H), 2.27-2.13 (m, 3H), 1.92-1.83 (m, 2H).

LC-MS m/z(ESI) = **427.20** [M+1]

### Example 88

### 2-Fluoro-4-((9-(3-hydroxybicyclo[3.2.1]octane-8-yl)-7-methyl-8-oxo-8,9-dihydro-7H-purin-2-yl)amino)-5-methylbenzamide (compound 88)

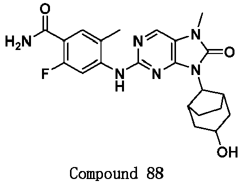

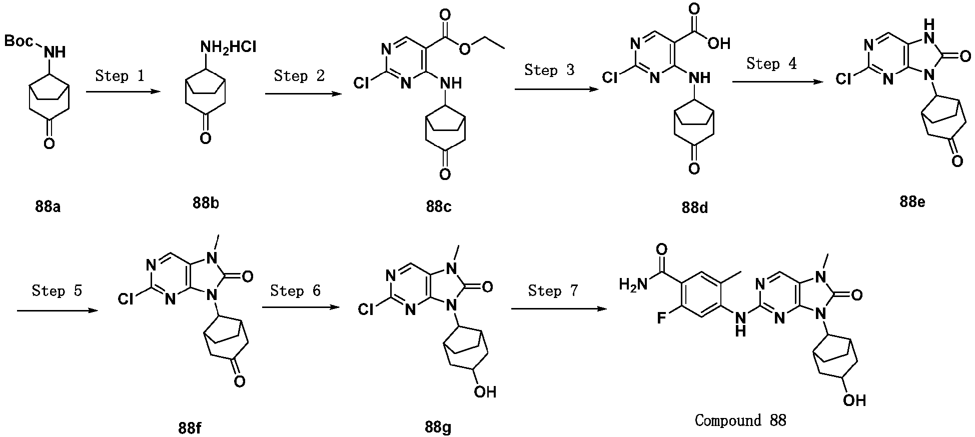

### Step 1:

### 8-Aminobicyclo[3.2.1]octan-3-one hydrochloride (88b)

*tert*-Butyl (3-oxobicyclo[3.2.1]octan-8-yl)carbamate **88a** (1.0 g, 4.18 mmol) was dissolved in 4 N dioxane hydrochloride (40 mL). The reaction mixture was stirred at room temperature for 1 h. The reaction was monitored by TLC. After completion of the reaction, the reaction mixture was directly concentrated and dried to yield the target compound 8-aminobicyclo[3.2.1]octan-3-one hydrochloride **88b** (white solid, 730 mg, 99.45% yield).

¹H NMR (401 MHz, DMSO-d6) δ 8.77 (s, 2H), 3.36 (m, 2H), 2.80-2.77 (m, 2H), 2.16-2.13 (m, 2H), 1.87-1.85 (m, 2H), 1.43-1.42 (m, 2H).

LC-MS m/z(ESI) = **176.20** [M+1]

### Step 2:

### Ethyl 2-chloro-4-((3-oxobicyclo[3.2.1]octan-8-yl)amino)pyrimidine-5-carboxylate (88c)

Ethyl 2,4-dichloropyrimidine-5-carboxylate **1A** (1.38 g, 6.26 mmol), 8-aminobicyclo[3.2.1]octan-3-one hydrochloride **88b** (730 mg, 4.17 mmol) and potassium carbonate (1.73 g, 12.52 mmol) were dissolved in acetonitrile (20 mL). The reaction mixture was stirred at room temperature for 16 h. The reaction was monitored by TLC. After completion of the reaction, the reaction mixture was filtered, and the solid was washed with a small amount of acetonitrile. The filtrates were combined and concentrated, and the crude product was purified by column chromatography (petroleum ether: ethyl acetate (v/v) = 1:1) to obtain the target compound ethyl 2-chloro-4-((3-oxobicyclo[3.2.1]octan-8-yl)amino)pyrimidine-5-carboxylate **88c** (white solid, 1.0 g, 74.01% yield).

¹H NMR (400 MHz, DMSO-d6) δ 8.86 (d, 2H), 8.68 (s, 1H), 4.36-4.30 (q, 2H), 4.20-4.16 (m, 1H), 2.66-2.65 (m, 2H), 2.56-2.55 (m, 2H), 2.21-2.17 (m, 2H), 1.97-1.93 (m, 2H), 1.53-1.33 (m, 2H), 1.31 (t, 3H).

LC-MS m/z(ESI) = **324.10** [M+1]

### Step 3:

### 2-Chloro-4-((3-oxobicyclo[3.2.1]octan-8-yl)amino)pyrimidine-5-carboxylic acid (88d)

Ethyl 2-chloro-4-((3-oxobicyclo[3.2.1]octan-8-yl)amino)pyrimidine-5-carboxylate **88c** (1.0 g, 3.09 mmol) was dissolved in 20 mL of tetrahydrofuran and 20 mL of water, and lithium hydroxide (259 mg, 6.18 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 2 N hydrochloric acid, and a white solid was precipitated. The mixture was filtered, and the filter cake was washed twice with petroleum ether and collected to give the title compound 2-chloro-4-((3-oxobicyclo[3.2.1]octan-8-yl)amino)pyrimidine-5-carboxylic acid **88d** (white solid, 800 mg, 87.59% yield).

¹H NMR (400 MHz, DMSO-d6) δ 13.93 (s, 1H), 9.20 (d, 2H), 8.64 (s, 1H), 4.20-4.16 (m, 1H), 2.66-2.64 (m, 2H), 2.54-2.53 (m, 2H), 2.21-2.16 (m, 2H), 1.96-1.93 (m, 2H), 1.53-1.47 (m, 2H).

LC-MS m/z(ESI) = **296.10** [M+1]

### Step 4:

### 2-Chloro-9-(3-oxobicyclo[3.2.1]octan-8-yl)-7,9-dihydro-8H-purin-8-one (88e)

2-Chloro-4-((3-oxobicyclo[3.2.1]octan-8-yl)amino)pyrimidine-5-carboxylic acid **88d** (800 mg, 2.71 mmol) was dissolved in dimethylacetamide (20 mL), and triethylamine (0.37 mL, 2.7 mmol) and diphenylphosphoryl azide (0.58 mL, 2.7 mmol) were added. The reaction mixture was gradually heated to 90 °C and stirred for 2 h. The reaction was monitored by TLC. After completion of the reaction, the reaction mixture was poured into ice water. The solid was collected by filtration and washed 3 times with water, and dried to obtain the title compound 2-chloro-9-(3-oxobicyclo[3.2.1]octan-8-yl)-7,9-dihydro-8*H*-purin-8-one **88e** (white solid, 630 mg, 94.71% yield).

¹H NMR (400 MHz, DMSO-d6) δ 11.66 (s, 1H), 8.13 (s, 1H), 4.01-3.99 (m, 1H), 3.65-3.64 (m, 2H), 2.66-2.61 (m, 2H), 2.12-2.08 (m, 2H), 1.89-1.86 (m, 2H), 1.54-1.49 (m, 2H).

LC-MS m/z(ESI) = **293.00** [M+1]

### Step 5:

### 2-Chloro-7-methyl-9-(3-oxobicyclo[3.2.1]octan-8-yl)-7,9-dihydro-8H-purin-8-one (88f)

2-Chloro-9-(3-oxobicyclo[3.2.1]octan-8-yl)-7,9-dihydro-8*H*-purin-8-one **88e** (630 mg, 2.15 mmol) was dissolved in dimethylformamide (10 mL), and dimethyl sulfate (0.2 mL, 2.15 mmol) and cesium carbonate (1.4 g, 4.3 mmol) were added at 0 °C. The reaction mixture was stirred for 1 h. The reaction was monitored by TLC. After completion of the reaction, the reaction mixture was poured into ice water, and a solid was precipitated. The solid was collected by filtration to yield the title compound 2-chloro-7-methyl-9-(3-oxobicyclo[3.2.1]octan-8-yl)-7,9-dihydro-8*H*-purin-8-one **88f** (white solid, 490 mg, 74.22% yield).

¹H NMR (400 MHz, DMSO-d6) δ 8.37 (s, 1H), 4.03-4.01 (m, 1H), 3.66-3.65 (m, 2H), 3.36 (s, 3H), 2.65-2.59 (m, 2H), 2.13-2.08 (m, 2H), 1.89-1.87 (m, 2H), 1.55-1.50 (m, 2H).

LC-MS m/z(ESI) = **307.10** [M+1]

### Step 6:

### 2-Chloro-9-(3-hydroxybicyclo[3.2.1]octan-8-yl)-7-methyl-7,9-dihydro-8H-purin-8-one (88g)

2-Chloro-7-methyl-9-(3-oxobicyclo[3.2.1]octan-8-yl)-7,9-dihydro-8*H*-purin-8-one **88f** (490 mg, 1.6 mmol) was dissolved in tetrahydrofuran (30 mL) and methanol (30 mL), and sodium borohydride (181 mg, 4.79 mmol) was added at 0 °C. The reaction mixture was stirred for 2 h. The reaction was monitored by TLC. After completion of the reaction, the reaction mixture was poured into ice water, and a solid was precipitated. The solid was collected by filtration to yield the title compound 2-chloro-9-(3-hydroxybicyclo[3.2.1]octan-8-yl)-7-methyl-7,9-dihydro-8*H*-purin-8-one **88g** (white solid, 380 mg, 77.04% yield).

¹H NMR (400 MHz, DMSO-d6) δ 8.37 (s, 1H), 4.28-4.20 (m, 1H), 3.74-3.66 (m, 2H), 3.45-3.43 (m, 2H), 3.37 (s, 3H), 2.14-2.07 (m, 1H), 1.74-1.50 (m, 5H), 1.28-1.22 (m, 2H).

LC-MS m/z(ESI) = **309.10** [M+1]

### Step 7:

### 2-Fluoro-4-((9-(3-hydroxybicyclo[3.2.1]octane-8-yl)-7-methyl-8-oxo-8,9-dihydro-7H-purin-2-yl)amino)-5-methylbenzamide (compound 88)

2-Chloro-9-(3-hydroxybicyclo[3.2.1]octan-8-yl)-7-methyl-7,9-dihydro-8*H*-purin-8-one **88g** (100 mg, 0.32 mmol), 4-amino-2-fluoro-5-methylbenzamide **intermediate 2** (164 mg, 0.96 mmol), cesium carbonate (228 mg, 0.64 mmol) and Brettphos Pd G3 (29 mg, 0.032 mmol) were dissolved in dioxane, followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(3-hydroxybicyclo[3.2.1]octane-8-yl)-7-methyl-8-oxo-8,9-dihydro-7H-purin-2-yl)amino)-5-methylbenzamide **compound 88** (white solid, 83 mg, 59.20% yield).

1H NMR (400 MHz, DMSO-d6) δ 8.49 (d, 1H), 8.21 (s, 1H), 7.75 (d, 1H), 7.54 (d, 1H), 7.46 (s, 1H), 7.40 (s, 1H), 4.31-4.26 (m, 1H), 3.75-3.71 (m, 1H), 3.62 (t, 1H), 3.52 (s, 2H), 3.34 (s, 3H), 2.28 (s, 3H), 1.72-1.62 (m, 4H), 1.59-1.50 (m, 2H), 1.35 (t, 2H). LC-MS m/z(ESI) = **441.20** [M+1]

### Example 89

### 2-Fluoro-5-methyl-4-((7-methyl-8-oxo-9-(2-oxaspiro[3.3]heptan-6-yl)-8,9-dihydro-7H-purin-2-yl)amino)benzamide (compound 89)

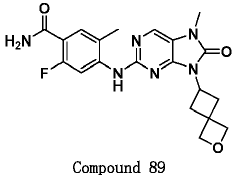

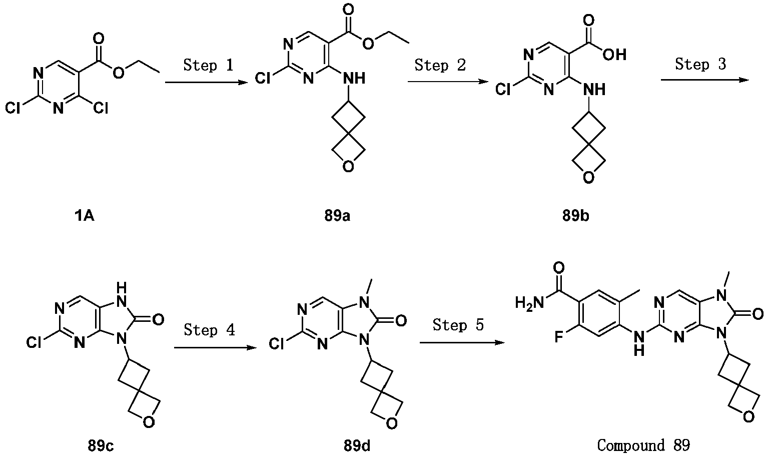

### Step 1:

### Ethyl 4-((2-oxaspiro[3.3]heptan-6-yl)amino)-2-chloropyrimidine-5-carboxylate (89a)

Ethyl 2,4-dichloropyrimidine-5-carboxylate **1A** (4.43 g, 20.05 mmol), 2-oxaspiro[3.3]heptan-6-amine hydrochloride (2.0 g, 13.37 mmol) and potassium carbonate (5.54 g, 40.10 mmol) were dissolved in acetonitrile (60 mL). The reaction mixture was stirred at room temperature for 16 h. The reaction was monitored by TLC. After completion of the reaction, the reaction mixture was filtered, and the solid was washed with a small amount of acetonitrile. The filtrates were combined and concentrated, and the crude product was purified by column chromatography (petroleum ether:ethyl acetate (v/v) = 1:1) to obtain the target compound ethyl 4-((2-oxaspiro[3.3]heptan-6-yl)amino)-2-chloropyrimidine-5-carboxylate **89a** (white solid, 3.0 g, 75.38% yield).

¹H NMR (400 MHz, DMSO-d6) δ 8.60 (s, 1H), 8.47 (d, 1H), 4.63 (s, 2H), 4.49 (s, 2H), 4.39-4.33 (m, 1H), 4.30-4.27 (q, 2H), 2.66-2.61 (m, 2H), 2.31-2.26 (m, 2H), 1.30 (t, 3H).

LC-MS m/z(ESI) = **298.10** [M+1]

### Step 2:

### 4-((2-Oxaspiro[3.3]heptan-6-yl)amino)-2-chloropyrimidine-5-carboxylic acid (89b)

Ethyl 4-((2-oxaspiro[3.3]heptan-6-yl)amino)-2-chloropyrimidine-5-carboxylate **89a** (3.0 g, 10.08 mmol) was dissolved in tetrahydrofuran/water (30 mL/30 mL), and lithium hydroxide (845 mg, 20.15 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 2 N hydrochloric acid, and a white solid was precipitated. The reaction mixture was filtered, and the filter cake was washed twice with petroleum ether and collected to obtain the title compound 4-((2-oxaspiro[3.3]heptan-6-yl)amino)-2-chloropyrimidine-5-carboxylic acid **89b** (white solid, 1.8 g, 66.24% yield). ¹H NMR (400 MHz, DMSO-d6) δ 13.76 (s, 1H), 8.64 (d, 1H), 8.57 (s, 1H), 4.64 (s, 2H), 4.49 (s, 2H), 4.40-4.30 (m, 1H), 2.67-2.61 (m, 2H), 2.28-2.23 (m, 2H).

LC-MS m/z(ESI) = **270.20** [M+1]

### Step 3:

### 2-Chloro-9-(2-oxaspiro[3.3]heptan-6-yl)-7,9-dihydro-8H-purin-8-one (89c)

4-((2-Oxaspiro[3.3]heptan-6-yl)amino)-2-chloropyrimidine-5-carboxylic acid **89b** (1.8 g, 6.67 mmol) was dissolved in dimethylacetamide (40 mL), and triethylamine (0.92 mL, 6.67 mmol) and diphenylphosphoryl azide (1.4 mL, 6.67 mmol) were added. The reaction mixture was gradually heated to 90 °C and stirred for 2 h. The reaction was monitored by TLC. After completion of the reaction, the reaction mixture was poured into ice water. The solid was collected by filtration and washed 3 times with water, and dried to give 2-chloro-9-(2-oxaspiro[3.3]heptan-6-yl)-7,9-dihydro-8J/-purin-8-one **89c** (white solid, 1.3 g, 73.03% yield).

¹H NMR (400 MHz, DMSO-d6) δ 11.61 (s, 1H), 8.11 (s, 1H), 4.71-4.63 (m, 5H), 3.02-2.94 (m, 2H), 2.69-2.66 (m, 2H).

LC-MS m/z(ESI) = **267.10** [M+1]

### Step 4:

### 2-Chloro-7-methyl-9-(2-oxaspiro[3.3]heptan-6-yl)-7,9-dihydro-8H-purin-8-one (89d)

2-Chloro-9-(2-oxaspiro[3.3]heptan-6-yl)-7,9-dihydro-8*H*-purin-8-one **89c** (1.3 g, 4.87 mmol) was dissolved in dimethylformamide (10 mL), and dimethyl sulfate (0.46 mL, 4.87 mmol) and cesium carbonate (3.18 g, 9.75 mmol) were added at 0 °C. The reaction mixture was stirred for 1 h. The reaction was monitored by TLC. After completion of the reaction, the reaction mixture was poured into ice water, and a solid was precipitated. The solid was collected by filtration to obtain the title compound 2-chloro-7-methyl-9-(2-oxaspiro[3.3]heptan-6-yl)-7,9-dihydro-8*H*-purin-8-one **89d** (white solid, 875 mg, 63.94% yield).

¹H NMR (400 MHz, DMSO-d6) δ 8.35 (s, 1H), 4.76-4.69 (m, 1H), 4.66-4.63 (m, 4H), 3.33 (s, 3H), 3.01-2.95 (m, 2H), 2.71-2.66 (m, 2H).

LC-MS m/z(ESI) = **281.10** [M+1]

### Step 5:

### 2-Fluoro-5-methyl-4-((7-methyl-8-oxo-9-(2-oxaspiro[3.3]heptan-6-yl)-8,9-dihydro-7H-purin-2-yl)amino)benzamide (compound 89)

2-Chloro-7-methyl-9-(2-oxaspiro[3.3]heptan-6-yl)-7,9-dihydro-8*H*-purin-8-one **89d** (200 mg, 0.71 mmol), 4-amino-2-fluoro-5-methylbenzamide **intermediate 2** (359 mg, 2.13 mmol), cesium carbonate (502 mg, 1.42 mmol) and Brettphos Pd G3 (65 mg, 0.071 mmol) were dissolved in dioxane, followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-5-methyl-4-((7-methyl-8-oxo-9-(2-oxaspiro[3.3]heptan-6-yl)-8,9-dihydro-7*H*-purin-2-yl)amino)benzamide **compound 89** (white solid, 58 mg, 19.82% yield).

¹H NMR (400 MHz, DMSO-d6) δ 8.49 (s, 1H), 8.19 (s, 1H), 7.80 (d, 1H), 7.56 (d, 1H), 7.47 (s, 1H), 7.41 (s, 1H), 4.67 (s, 3H), 4.52 (s, 2H), 3.30 (s, 3H), 3.08-3.02 (m, 2H), 2.65-2.57 (m, 2H), 2.27 (s, 3H).

LC-MS m/z(ESI) = **413.20** [M+1]

### Example 90

### 4-((9-(4-Cyanobicyclo[2.2.2]octan-1-yl)-7-methyl-8-oxo-8,9-dihydro-7H-purin-2-yl)amino)-2-fluoro-5-methylbenzamide (compound 90)

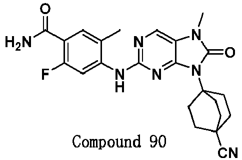

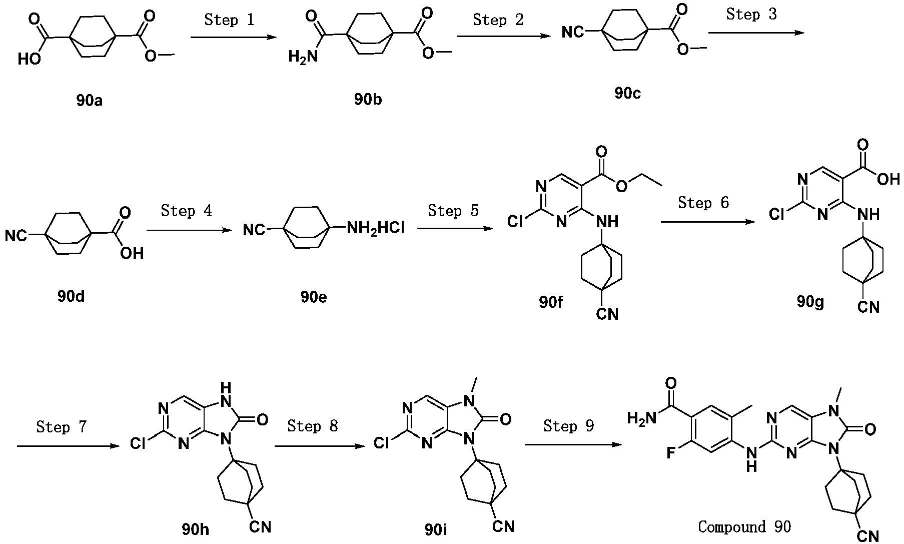

### Step 1:

### Methyl 4-carbamoylbicyclo[2.2.2]octane-1-carboxylate (90b)

4-(Methoxycarbonyl)bicyclo[2.2.2]octane-1-carboxylic acid **90a** (10.0 g, 47.12 mmol) was dissolved in anhydrous dichloromethane (200 mL), and 2-(7-azabenzotriazole)-*N*,*N*,*N*',*N*'-tetramethylurea hexafluorophosphate (18.81 g, 49.47 mmol) was added in an ice bath. The mixture was stirred for 30 min with the temperature maintained. *N,N-*Diisopropylethylamine (24.62 mL, 141.35 mmol) was added, and then solid ammonium chloride (3.78 g, 70.67 mmol) was slowly added several times. The reaction mixture was gradually warmed to room temperature and stirred overnight. The reaction was monitored by TLC. After completion of the reaction, 100 mL of 0.5 N hydrochloric acid solution was added, the organic phase was separated, successively with water and saturated brine (100 mL each), dried and concentrated to give the title compound methyl 4-carbamoylbicyclo[2.2.2]octane-1-carboxylate **90b** (white solid, 21 g, crude). ¹H NMR (400 MHz, DMSO-d6) δ 6.94 (s, 1H), 6.73 (s, 1H), 3.57 (s, 3H), 1.72-1.63 (m, 12 H).

### LC-MS m/z(ESI) = 212.10 [M+1]

### Step 2:

### Methyl 4-cyanobicyclo[2.2.2]octane-1-carboxylate (90c)

Methyl 4-carbamoylbicyclo[2.2.2]octane-1-carboxylate **90b** (21 g, 99.40 mmol) was dissolved in dichloromethane (200 mL), and pyridine (16.02 mL, 198.81 mmol) and trifluoroacetic anhydride (21.00 mL, 149.10 mmol) were added in the ice bath. The reaction mixture was stirred for 1 h with the temperature maintained. The reaction was monitored by TLC. After completion of the reaction, the reaction mixture was filtered directly, and the filter cake was washed with 100 mL of dichloromethane. The organic phases were combined, then extracted with 1 N hydrochloric acid solution, water and saturated brine (100 mL each), dried, and concentrated, and the residue was purified by column chromatography to obtain the target product methyl 4-cyanobicyclo[2.2.2]octane-1-carboxylate **90c** (white solid, 5.3 g, 58.21% yield over two steps).

¹H NMR (400 MHz, CDCl3-d6) δ 3.66 (s, 3H), 1.98-1.94 (m, 6H), 1.86-1.82 (m, 6H). LC-MS m/z(ESI) = **194.10** [M+1]

### Step 3:

### 4-Cyanobicyclo[2.2.2]octane-1-carboxylic acid (90d)

Methyl 4-cyanobicyclo[2.2.2]octane-1-carboxylate **90c** (5.3 g, 27.43 mmol) was dissolved in 50 mL of tetrahydrofuran and 50 mL of water, and lithium hydroxide (1.73 g, 41.14 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 2 N hydrochloric acid, and a white solid was precipitated. The mixture was filtered, and the filter cake was washed twice with petroleum ether and collected to obtain the title compound 4-cyanobicyclo[2.2.2]octane-1-carboxylic acid **90d** (white solid, 5.0 g, crude).

¹H NMR (400 MHz, DMSO-d6) δ 12.23 (s, 1H), 1.89-1.85 (m, 6H), 1.72 -1.68 (m, 6H).

LC-MS m/z(ESI) = **180.10** [M+1]

### Step 4:

### 4-Aminobicyclo[2.2.2]octane-1-carbonitrile hydrochloride (90e)

4-Cyanobicyclo[2.2.2]octane-1-carboxylic acid **90d** (5.0 g, 27.90 mmol) was dissolved in toluene (60 mL), and diphenylphosphoryl azide (6.01 mL, 27.90 mmol) and triethylamine (3.88 mL, 27.90 mmol) were added in an ice bath. The reaction mixture was stirred at room temperature for 1 h, then heated to 90 °C and stirred for another 3 h. The reaction was monitored by TLC. After completion of the reaction, the reaction mixture was cooled to room temperature and slowly poured into 100 mL of 1 N hydrochloric acid solution, and a large amounts of solid was precipitated. The solid was collected by filtration, slurried with ethyl acetate (150 mL), and dried *in vacuo* to yield the title compound 4-aminobicyclo[2.2.2]octane-1-carbonitrile hydrochloride **90e** (white solid, 7.3 g, crude).

¹H NMR (401 MHz, DMSO-d6) δ 8.45 (s, 2H), 2.01-1.97 (m, 6H), 1.81-1.76 (m, 6H). LC-MS m/z(ESI) = **187.10** [M+1]

### Step 5:

### Ethyl 2-chloro-4-((4-cyanobicyclo[2.2.2]octan-1-yl) amino)pyrimidine-5-carboxylate (90f)

Ethyl 2,4-dichloropyrimidine-5-carboxylate **1A** (8.74 g, 39.53 mmol), 4-aminobicyclo[2.2.2]octane-1-carbonitrile hydrochloride **90e** (7.38 g, 39.53 mmol) and potassium carbonate (21.85 g, 158.13 mmol) were dissolved in acetonitrile (200 mL). The reaction mixture was stirred at room temperature for 16 h. The reaction was monitored by TLC. After completion of the reaction, the reaction mixture was filtered, and the solid was washed with a small amount of acetonitrile. The filtrates were combined and concentrated, and the crude product was purified by silica gel column chromatography (petroleum ether/ethyl acetate (v/v) = 1:1) to obtain the title compound ethyl 2-chloro-4-((4-cyanobicyclo[2.2.2]octan-1-yl) amino)pyrimidine-5-carboxylate **90f** (white solid, 4.0 g, 30.23% yield).

¹H NMR (400 MHz, DMSO-d6) δ 8.63 (s, 1H), 8.29 (s, 1H), 4.30 (q, 2H), 2.10-2.00 (m, 12H), 1.30 (t, 3H).

LC-MS m/z(ESI) = **335.10** [M+1]

### Step 6:

### 2-Chloro-4-((4-cyanobicyclo[2.2.2]octan-1-yl)amino)pyrimidine-5-carboxylic acid (90g)

Ethyl 2-chloro-4-((4-cyanobicyclo[2.2.2]octan-1-yl) amino)pyrimidine-5-carboxylate **90f** (4 g, 11.95 mmol) was dissolved in 50 mL of tetrahydrofuran and 50 mL of water, and lithium hydroxide (1.01 g, 23.90 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 2 N hydrochloric acid, and a white solid was precipitated. The mixture was filtered, and the filter cake was washed twice with petroleum ether and collected to afford the title compound 2-chloro-4-((4-cyanobicyclo[2.2.2]octan-1-yl)amino)pyrimidine-5-carboxylic acid **90g** (white solid, 3.6 g, 98.23% yield).

¹H NMR (600 MHz, DMSO-d6) δ 13.85 (s, 1H), 8.59 (s, 1H), 8.56 (s, 1H), 2.07-1.98 (m, 12H).

LC-MS m/z (ESI) = **307.10** [M+1]

### Step 7:

### 4-(2-Chloro-8-oxo-7,8-dihydro-9H-purin-9-yl)bicyclo[2.2.2]octane-1-carbonitrile (90h)

2-Chloro-4-((4-cyanobicyclo[2.2.2]octan-1-yl)amino)pyrimidine-5-carboxylic acid **90g** (3.8 g, 12.39 mmol) was dissolved in dimethylacetamide (50 mL), and triethylamine (1.72 mL, 12.39 mmol) and diphenylphosphoryl azide (2.67 mL, 12.39 mmol) were added. The reaction mixture was gradually heated to 120 °C and stirred for 1.5 h. The reaction was monitored by TLC. After completion of the reaction, the reaction mixture was poured into ice water. The solid was collected by filtration and washed 3 times with water, and dried to give the title compound 4-(2-chloro-8-oxo-7,8-dihydro-9*H*-purin-9-yl)bicyclo[2.2.2]octane-1-carbonitrile **90h** (white solid, 3.3 g, 87.7% yield).

¹H NMR (400 MHz, DMSO-d6) δ 11.61 (s, 1H), 8.09 (s, 1H), 2.48-2.40 (m, 6H), 2.09-2.03 (m, 6H).

LC-MS m/z(ESI) = **304.20** [M+1]

### Step 8:

### 4-(2-Chloro-7-methyl-8-oxo-7,8-dihydro-9H-purin-9-yl)bicyclo[2.2.2]octane-1-carbonitrile (90i)

4-(2-Chloro-8-oxo-7,8-dihydro-9*H*-purin-9-yl)bicyclo[2.2.2]octane-1-carbonitrile **90h** (3.3 g, 11.43 mmol) was dissolved in dimethylformamide (50 mL), and dimethyl sulfate (1.44 g, 11.43 mmol) and cesium carbonate (5.59 g, 17.15 mmol) were added at 0 °C. The reaction mixture was stirred at 0 °C for 1 h. The reaction was monitored by TLC. After completion of the reaction, the reaction mixture was poured into ice water, and a solid was precipitated. The solid was collected by filtration to obtain the title compound 4-(2-chloro-7-methyl-8-oxo-7,8-dihydro-9*H*-purin-9-yl)bicyclo[2.2.2]octane-1-carbonitrile **90i** (white solid, 3.1 g, 89.59% yield).

¹H NMR (401 MHz, DMSO-d6) δ 8.33 (s, 1H), 3.29 (s, 3H), 2.48-2.41 (m, 6H), 2.10-2.04 (m, 6H).

LC-MS m/z(ESI) = 318.20 [M+1]

### Step 9:

### 4-((9-(4-Cyanobicyclo[2.2.2]octan-1-yl)-7-methyl-8-oxo-8,9-dihydro-7H-purin-2-yl)amino)-2-fluoro-5-methylbenzamide (compound 90)

4-(2-Chloro-7-methyl-8-oxo-7,8-dihydro-9*H*-purin-9-yl)bicyclo[2.2.2]octane-1-carbonitrile **90i** (200 mg, 0.63 mmol), 4-amino-2-fluoro-5-methylbenzamide **intermediate 2** (318 mg, 1.89 mmol), cesium carbonate (444 mg, 1.26 mmol) and Brettphos Pd G3 (57 mg, 0.063 mmol) were dissolved in dioxane, followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 4-((9-(4-cyanobicyclo [2.2.2] octan-1-yl)-7-methyl-8-oxo-8, 9-dihydro-7*H*-purin-2-yl)amino)-2-fluoro-5-methylbenzamide **compound 90** (white solid, 18 mg, 6.4% yield).

¹H NMR (401 MHz, DMSO-d6) δ 10.08 (d, 1H), 8.22-8.17 (s, 1H), 7.88-7.53 (d, 1H), 7.48-7.29 (d, 1H), 6.31-6.29 (s, 1H), 5.82 (s, 1H), 3.26 (d, 3H), 2.34-2.26 (m, 6H), 2.06 (d, 5H), 1.96 (dd, 4H).

LC-MS m/z(ESI) = **450.20** [M+1]

### Example 91

### 2-Fluoro-4-((9-(3-hydroxy-3-methylcyclohexyl)-7-methyl-8-oxo-8,9-dihydro-7H-purin-2-yl)amino)-5-methylbenzamide (compound 91)

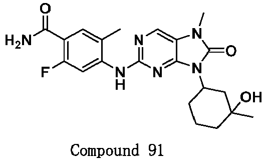

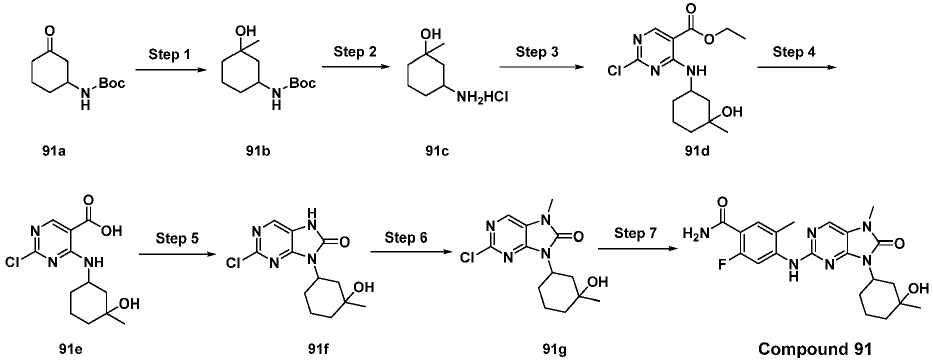

### Step 1:

### tert-Butyl (3-hydroxy-3-methylcyclohexyl)carbamate (91b)

*tert*-Butyl (3-oxocyclohexyl)carbamate **91a** (5.00 g, 23.4 mmol) was dissolved in tetrahydrofuran (80 mL). The solution was pre-cooled at -78 °C, and then 1.6 M methyllithium-diethyl ether solution (61.5 mL, 98.28 mmol) was slowly added dropwise over 45 min. The reaction mixture was stirred at -78 °C for 1 h, and then the remaining 1.6 M methyllithium-diethyl ether solution (61.5 mL, 98.28 mmol) was added. The reaction mixture was stirred at -78 °C for another hour. The reaction was monitored by TLC. After completion of the reaction, the reaction mixture was quenched with saturated ammonium chloride solution, and water was added and the organics were extracted with ethyl acetate. The organic layer was concentrated, and the residue was purified by silica gel column chromatography (petroleum ether/ethyl acetate (v/v) = 8:1) to obtain the title compound *tert*-butyl (3-hydroxy-3-methylcyclohexyl)carbamate **91b** (white solid, 2.07 g, 38.53% yield).

¹H NMR (400 MHz, Chloroform-d) δ 4.30 (s, 1H), 3.68 (s, 1H), 1.90 (d, 3H), 1.67 (q, 1H), 1.59-1.48 (m, 2H), 1.37 (s, 9H), 1.19-1.12 (m, 4H), 1.06 (t, 1H), 0.90 (qd, 1H). LC-MS m/z(ESI) = **230.20** [M+1]

### Step 2:

### 3-Amino-1-methylcyclohexan-1-ol hydrochloride (91c)

*tert-*Butyl (3-hydroxy-3-methylcyclohexyl)carbamate **91b** (2.07 g, 9.03 mmol) was dissolved in 4 M hydrogen chloride-1,4-dioxane (10 mL). The reaction mixture was stirred at room temperature. The reaction was monitored by TLC. After completion of the reaction, the reaction mixture was concentrated to evaporate 1,4-dioxane solution to give the title compound 3-amino-1-methylcyclohexan-1-ol hydrochloride **91c** (pale yellow solid, crude, 1.49 g).

LC-MS m/z(ESI) = **166.10** [M+1]

### Step 3:

### Ethyl 2-chloro-4-((3-hydroxy-3-methylcyclohexyl)amino)pyrimidine-5-carboxylate (91d)

Ethyl 2,4-dichloropyrimidine-5-carboxylate **1A** (2.99 g, 13.54 mmol) and 3-amino-1-methylcyclohexan-1-ol hydrochloride **91c** (1.49 g, 9.03 mmol) were dissolved in acetonitrile (20 mL), and potassium carbonate (3.74 g, 27.08 mmol) was added with stirring. The reaction mixture was stirred at room temperature for 4 h. The reaction was monitored by TLC. After completion of the reaction, the reaction mixture was concentrated, and the residue was purified by column chromatography (petroleum ether: ethyl acetate (v/v) = 4:1) to obtain the title compound ethyl 2-chloro-4-((3-hydroxy-3-methylcyclohexyl)amino)pyrimidine-5-carboxylate **91d** (white solid, 2.23 g, 78.74% yield).

¹H NMR (400 MHz, Chloroform-d) δ 8.57 (s, 1H), 8.19 (d, 1H), 4.43-4.32 (m, 1H), 4.27 (q, 2H), 2.07-1.95 (m, 2H), 1.83-1.69 (m, 2H), 1.64-1.56 (m, 2H), 1.31 (t, 4H), 1.26 (d, 1H), 1.21 (s, 3H), 1.10-1.05 (m, 1H).

LC-MS m/z(ESI) = **314.10** [M+1]

### Step 4:

### 2-Chloro-4-((3-hydroxy-3-methylcyclohexyl)amino)pyrimidine-5-carboxylic acid (91e)

Ethyl 2-chloro-4-((3-hydroxy-3-methylcyclohexyl)amino)pyrimidine-5-carboxylate **91d** (2.23 g, 7.11 mmol) was dissolved in tetrahydrofuran/water (15 mL/15 mL), and lithium hydroxide (895 mg, 21.32 mmol) was added. The reaction mixture was stirred at room temperature for 1 h. The reaction was monitored by TLC. After completion of the reaction, the reaction mixture was concentrated to remove tetrahydrofuran and the pH was adjusted to 3-4 with 2 N hydrochloric acid, and a white solid was precipitated. The mixture was filtered, and the filter cake was washed twice with petroleum ether and collected to give the title compound 2-chloro-4-((3-hydroxy-3-methylcyclohexyl)amino)pyrimidine-5-carboxylic acid **91e** (white solid, 1.6 g, 78.79% yield).

LC-MS m/z(ESI) = **286.10** [M+1]

### Step 5:

### 2-Chloro-9-(3-hydroxy-3-methylcyclohexyl)-7,9-dihydro-8H-purin-8-one (91f)

2-Chloro-4-((3-hydroxy-3-methylcyclohexyl)amino)pyrimidine-5-carboxylic acid **91e** (1.38 g, 4.83 mmol) was dissolved in dimethylacetamide (20 mL), and triethylamine (0.67 mL, 4.83 mmol) and diphenylphosphoryl azide (1.04 mL, 4.83 mmol) were added. The reaction mixture was gradually heated to 90 °C and stirred for 2 h. The reaction was monitored by TLC. After completion of the reaction, the reaction mixture was poured into ice water. The solid was collected by filtration and washed 3 times with water, and concentrated and dried to obtain the target compound 2-chloro-9-(3-hydroxy-3-methylcyclohexyl)-7,9-dihydro-8*H*-purin-8-one **91f** (white solid, 1.35 g, 98.86% yield).

¹H NMR (400 MHz, DMSO-d6) δ 11.60 (s, 1H), 8.10 (s, 1H), 4.62-4.54 (m, 1H), 4.35 (s, 1H), 2.19 (t, 1H), 2.10-1.99 (m, 1H), 1.70-1.53 (m, 5H), 1.30-1.22 (m, 1H), 1.16 (s, 3H).

LC-MS m/z(ESI) = **283.10** [M+1]

### Step 6:

### 2-Chloro-9-(3-hydroxy-3-methylcyclohexyl)-7-methyl-7,9-dihydro-8H-purin-8-one (91g)

2-Chloro-9-(3-hydroxy-3-methylcyclohexyl)-7,9-dihydro-8*H*-purin-8-one **91f** (1.35 g, 4.77 mmol) was dissolved in dimethylformamide (10 mL), and dimethyl sulfate (0.45 mL, 4.77 mmol) and cesium carbonate (1.56 g, 4.77 mmol) were added at 0 °C. The reaction mixture was stirred for 1 h. The reaction was monitored by TLC. After completion of the reaction, the reaction mixture was poured into ice water, and a solid was precipitated. The solid was collected by filtration to give the title compound 2-chloro-9-(3-hydroxy-3-methylcyclohexyl)-7-methyl-7,9-dihydro-8*H*-purin-8-one **91g** (white solid, 873 mg, 61.61% yield).

¹H NMR (400 MHz, DMSO-d6) δ 8.34 (s, 1H), 4.66-4.58 (m, 1H), 4.37 (s, 1H), 3.35 (s, 3H), 2.20 (t, 1H), 2.11-2.00 (m, 1H), 1.72-1.54 (m, 5H), 1.32-1.24 (m, 1H), 1.17 (s, 3H).

LC-MS m/z(ESI) = **297.10** [M+1]

### Step 7:

### 2-Fluoro-4-((9-(3-hydroxy-3-methylcyclohexyl)-7-methyl-8-oxo-8,9-dihydro-7H-purin-2-yl)amino)-5-methylbenzamide (compound 91)

2-Chloro-9-(3-hydroxy-3-methylcyclohexyl)-7-methyl-7,9-dihydro-8*H*-purin-8-one **91g** (200 mg, 0.67 mmol), 4-amino-2-fluoro-5-methylbenzamide **intermediate 2** (338 mg, 2.01 mmol), cesium carbonate (473 mg, 1.34 mmol) and Brettphos Pd G3 (61 mg, 0.067 mmol) were dissolved in dioxane, followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(3-hydroxy-3-methylcyclohexyl)-7-methyl-8-oxo-8,9-dihydro-7*H*-purin-2-yl)amino)-5-methylbenzamide **compound 91** (white solid, 140 mg, 48.80% yield).

¹H NMR (400 MHz, DMSO-*d*₆) δ 8.50 (s, 1H), 8.18 (s, 1H), 7.96 (d, 1H), 7.54 (d, 1H), 7.46 (s, 1H), 7.39 (d, 1H), 4.67-4.58 (m, 1H), 4.33 (s, 1H), 3.32 (s, 3H), 2.35 (t, 1H), 2.30 (s, 3H), 2.15-2.01 (m, 1H), 1.80-1.63 (m, 3H), 1.58 (d, 2H), 1.41-1.22 (m, 1H), 1.18 (s, 3H).

LC-MS m/z(ESI) = **429.20** [M+1]

### Example 92

### 4-((9-(4-Cyano-4-methylcyclohexyl)-7-methyl-8-oxo-8,9-dihydro-7H-purin-2-yl)amino)-2-fluoro-5-methylbenzamide (compound 92)

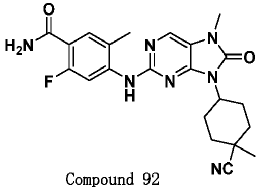

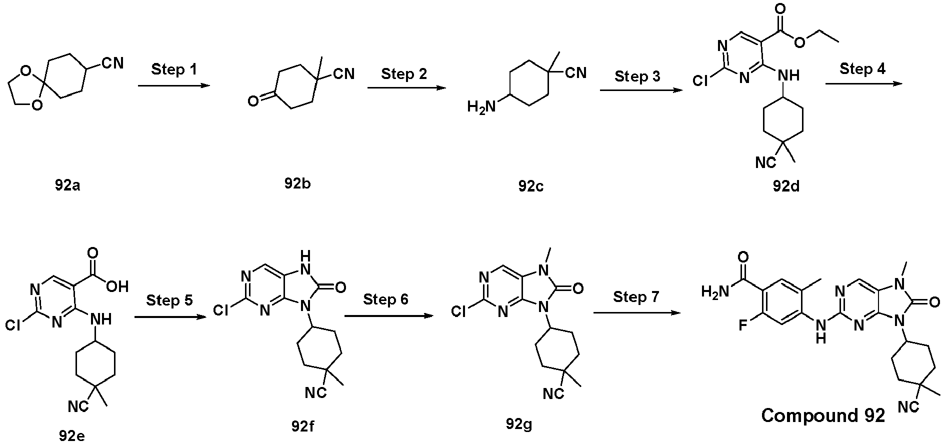

### Step 1:

### 1-Methyl-4-oxocyclohexane-1-carbonitrile (92b)

1,4-Dioxaspiro[4.5]decane-8-carbonitrile **92a** (5.00 g, 29.9 mmol) was dissolved in THF (60 mL), and lithium bistrifluoromethanesulfonimide (9.87 mL, 34.38 mmol) was slowly added dropwise at 0 °C. The reaction mixture was stirred for 1 h, and then iodomethane (4.24 g, 29.9 mmol) was slowly added. The reaction mixture was stirred for another hour. The reaction was monitored by TLC. After completion of the reaction, the reaction mixture was extracted twice with saturated ammonium chloride solution, bromine water and ethyl acetate. The organic layer was dried over anhydrous sodium sulfate, filtered, and concentrated to get an intermediate 8-methyl-1,4-dioxaspiro[4.5]decane-8-carbonitrile, which was then dissolved in THF (60 mL). 3 M HCl (60 mL) was added to the reaction mixture, which was then heated to 50 °C and stirred for 5 h. The reaction was monitored by TLC. After completion of the reaction, the reaction mixture was cooled to room temperature, the pH was adjusted to 7-8 with 3 M NaOH, and extracted 3 times with dichloromethane. The organic phase was dried over anhydrous sodium sulfate and concentrated to obtain the title compound 1-methyl-4-oxocyclohexane-1-carbonitrile **92b** (yellow liquid, 3.48 g, 84.84% yield).

LC-MS m/z(ESI) = **138.10** [M+1]

### Step 2:

### 4-Amino-1-methylcyclohexane-1-carbonitrile (92c)

1-Methyl-4-oxocyclohexane-1-carbonitrile **92b** (3.48 g, 25.37 mmol) was dissolved in absolute methanol (50 mL), and ammonium formate (16.00 g, 253.70 mmol) was added with stirring at room temperature. The reaction mixture was stirred for 4 h, and then sodium cyanoborohydride (1.88 g, 30.44 mmol) was added. The reaction mixture was stirred for 3 h. The reaction was monitored by TLC. After completion of the reaction, the pH of the reaction mixture was adjusted to 1-2 with 2 N HCl, stirred for 0.5 h, then extracted twice with ethyl acetate, the pH was adjusted to about 10 with 2 N NaOH solution, and extracted 3 times with dichloromethane. The organic phase was dried over anhydrous sodium sulfate and concentrated to obtain the title compound 4-amino-1-methylcyclohexane-1-carbonitrile **92c** (pale yellow liquid, 2.80 g, 79.85% yield).

LC-MS m/z(ESI) = **139.10** [M+1]

### Step 3:

### Ethyl 2-chloro-4-((4-cyano-4-methylcyclohexyl)amino)pyrimidine-5-carboxylate (92d)

Ethyl 2,4-dichloro-5-pyrimidinecarboxylate **1A** (6.72 g, 30.39 mmol) was dissolved in acetonitrile (30 mL), and potassium carbonate (8.40 g, 60.78 mmol) was added with stirring at 0 °C, followed by the slow dropwise addition of a solution of 4-amino-1-methylcyclohexane-1-carbonitrile **92c** (2.80 g, 20.26 mmol) in acetonitrile (10 mL). The reaction mixture was stirred for 1 h. The reaction was monitored by TLC. After completion of the reaction, the reaction mixture was filtered through celite, and the filtrate was purified by silica gel column chromatography (petroleum ether/ethyl acetate (v/v) = 20:1) to obtain the title compound ethyl 2-chloro-4-((4-cyano-4-methylcyclohexyl)amino)pyrimidine-5-carboxylate **92d** (white solid, 2.63 g, 40.22% yield).

¹H NMR (400 MHz, Chloroform-*d*) δ 8.68 (d, 1H), 4.38 (p, 2H), 2.23-1.82 (m, 4H), 1.79-1.49 (m, 6H), 1.46-1.36 (m, 6H).

LC-MS m/z(ESI) = **323.10** [M+1]

### Step 4:

### 2-Chloro-4-((4-cyano-4-methylcyclohexyl)amino)pyrimidine-5-carboxylic acid (92e)

Ethyl 2-chloro-4-((4-cyano-4-methylcyclohexyl)amino)pyrimidine-5-carboxylate **92d** (2.63 g, 8.15 mmol) was dissolved in tetrahydrofuran/water (20 mL/10 mL), and lithium hydroxide monohydrate (1.00 g, 24.45 mmol) was added. The reaction mixture was stirred at room temperature for 2 h. The reaction was monitored by TLC. After completion of the reaction, the reaction mixture was concentrated to evaporate tetrahydrofuran and the pH was adjusted to about 3-4 with 2 N HCl, and a white solid was precipitated. The mixture was filtered, and the filter cake was washed twice with water and petroleum ether/ethyl acetate (v/v = 10/1) to obtain the title compound 2-chloro-4-((4-cyano-4-methylcyclohexyl)amino)pyrimidine-5-carboxylic acid **92e** (white solid, 2.20 g, 91.59% yield).

¹H NMR (400 MHz, DMSO-d6) δ 13.75 (s, 1H), 8.59 (s, 1H), 8.52 (d, 1H), 3.97 (m, 1H), 2.06-1.92 (m, 4H), 1.63-1.47 (m, 4H), 1.35 (s, 3H).

LC-MS m/z(ESI) = **295.10** [M+1]

### Step 5:

### 4-(2-Chloro-8-oxo-7,8-dihydro-9H-purin-9-yl)-1-methylcyclohexane-1-carbonitrile (92f)

2-Chloro-4-((4-cyano-4-methylcyclohexyl)amino)pyrimidine-5-carboxylic acid **92e** (2.20 g, 7.46 mmol) was dissolved in *N*,*N*-dimethylacetamide (15 mL), and triethylamine (1.04 mL, 7.46 mmol) and diphenylphosphoryl azide (1.61 mL, 7.46 mmol) were added with stirring at room temperature. The reaction mixture was stirred for 2 h, then heated to 110 °C and refluxed for 2.5 h. The reaction was monitored by TLC. After completion of the reaction, water was added and the organics were extracted three times with ethyl acetate. The organic layer was concentrated, and the residue was purified by silica gel column chromatography (dichloromethane/methanol (v/v) = 80/1) to obtain the title compound 4-(2-chloro-8-oxo-8,9-dihydro-7h-purin-9-yl)-1-methylcyclohexane-1-carbonitrile **92f** (white solid, 0.9 g, 41.35% yield).

¹H NMR (400 MHz, DMSO-d6) δ 11.64 (s, 1H), 8.13 (s, 1H), 4.31-4.20 (m, 1H), 2.45-2.33 (m, 2H), 2.01-1.94 (m, 4H), 1.71-1.64 (m, 2H), 1.49 (s, 3H).

LC-MS m/z(ESI) = **292.10** [M+1]

### Step 6:

### 4-(2-Chloro-7-methyl-8-oxo-7,8-dihydro-9H-purin-9-yl)-1-methylcyclohexane-1-carbonitrile (92g)

4-(2-chloro-8-oxo-8,9-dihydro-7h-purin-9-yl)-1 -methylcyclohexane-1 -carbonitrile **92f** (0.90 g, 3.08 mmol) was dissolved in *N*,*N*-dimethylformamide (10 mL), and dimethyl sulfate (0.29 mL, 3.08 mmol) and cesium carbonate (1.51 g, 4.62 mmol) were added with stirring at 0 °C. The reaction mixture was stirred for 2 h. The reaction was monitored by TLC. After completion of the reaction, the reaction mixture was slowly added dropwise to ice water. The mixture was stirred, and a white solid was precipitated. The solid was washed 3 times with water and petroleum ether and collected by filtration to give the title compound 4-(2-chloro-7-methyl-8-oxo-8,9-dihydro-7h-purin-9-yl)-1-methylcyclohexane-1-carbonitrile **92g** (white solid, 0.85 g, 90.26% yield).

¹H NMR (400 MHz, DMSO-d6) δ 8.35 (s, 1H), 4.35-4.20 (m, 1H), 3.36 (s, 3H), 2.48-2.31 (m, 2H), 2.06-1.95 (m, 3H), 1.83-1.76 (m, 1H), 1.71-1.57 (m, 2H), 1.48 (s, 1H), 1.36 (s, 2H).

LC-MS m/z(ESI) = **306.10** [M+1]

### Step 7:

### 4-((9-(4-Cyano-4-methylcyclohexyl)-7-methyl-8-oxo-8,9-dihydro-7H-purin-2-yl)amino)-2-fluoro-5-methylbenzamide (compound 92)

2-Chloro-(9-(3-hydroxy-3-methylcyclohexyl)-7-methyl-7,9-dihydro-8H-purin-8-one **92g** (200 mg, 0.67 mmol), 4-amino-2-fluoro-5-methylbenzamide **intermediate 2** (338 mg, 2.01 mmol), cesium carbonate (473 mg, 1.34 mmol) and Brettphos Pd G3 (61 mg, 0.067 mmol) were dissolved in dioxane, followed by nitrogen purging. The reaction mixture was stirred under nitrogen atmosphere at 110 °C for 4 h. The reaction was s monitored by TLC. After completion of the reaction, the reaction mixture was concentrated, and the residue was purified by silica gel column chromatography (dichloromethane/methanol (v/v) = 30/1) to obtain the title compound 2-fluoro-4-((9-(3-hydroxyl-3-methylcyclohexyl)-7-methyl-8-oxo-8,9-dihydro-7*H*-purin-2-yl)amino)-5-methylbenzamide **compound 92,** which was resolved by SFC to give **compound 92-1** (white solid, 12 mg, 4.1% yield, RT = 3.58 min) and **compound 92-2** (white solid, 22 mg, 7.5% yield, RT = 4.17 min). (OZ), mobile phase: CO₂/ethanol = 70/30; column temperature: 35 °C; column pressure: 80 bar; flow rate: 1 mL/min; detector signal channel: 215 nm@4.8 nm; diode array detector at a wavelength of 200-400 nm.

### Compound 92-1:

¹H NMR (400 MHz, DMSO-*d*₆) δ 8.60 (s, 1H), 8.18 (s, 1H), 7.65 (d, 1H), 7.57 (d, 1H), 7.47 (s, 1H), 7.38 (s, 1H), 4.27-4.16 (m, 1H), 2.45-2.31 (m, 5H), 2.26 (s, 3H), 1.97-1.89 (m, 4H), 1.69-1.59 (m, 2H), 1.32 (s, 3H).

LC-MS m/z(ESI) = **438.20** [M+1]

### Compound 92-2:

¹H NMR (400 MHz, DMSO-*d*₆) δ 8.32 (s, 1H), 8.19 (s, 1H), 7.91 (d, 1H), 7.53 (d, 1H), 7.44 (s, 1H), 7.27 (s, 1H), 4.28-4.18 (m, 1H), 2.26 (s, 3H), 2.08-2.00 (m, 2H), 1.85-1.77 (m, 2H), 1.60-1.49 (m, 3H), 1.37 (s, 3H), 1.26-1.13 (m, 4H).

LC-MS m/z(ESI) = **438.20** [M+1]

### Example 93

### 2-Fluoro-4-((9-(trans-3-hydroxycyclobutyl)-7-methyl-8-oxo-8,9-dihydro-7H-purin-2-yl)amino)-5-methylbenzamide (compound 93)

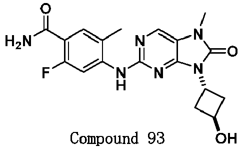

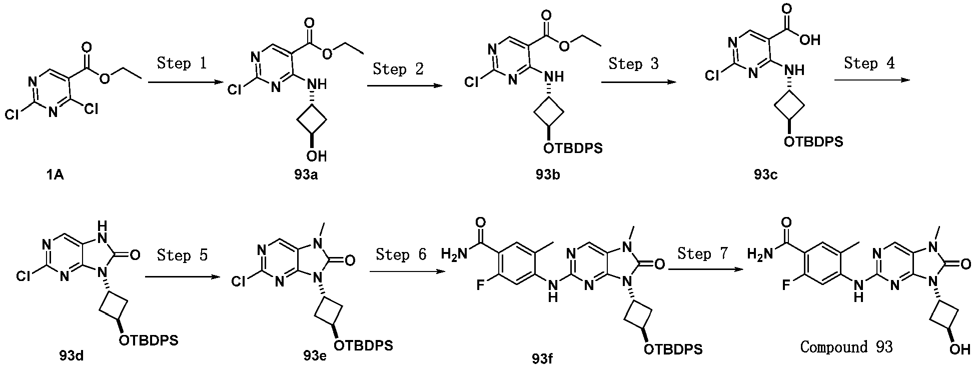

### Step 1:

### Ethyl 2-chloro-4-((trans-3-hydroxycyclobutyl)amino)pyrimidine-5-carboxylate (93a)

*trans*-3-Aminocyclobutane-1-ol hydrochloride (3.00 g, 34.48 mmol) was dissolved in acetonitrile (25 mL), and potassium carbonate (14.30 g, 103.45 mmol) and ethyl 2,4-dichloro-5-pyrimidinecarboxylate **1A** (11.43 g, 51.72 mmol) were added with stirring at 0 °C. The reaction mixture was warmed to room temperature and stirred for 1 h. The reaction was monitored by TLC. After completion of the reaction, the reaction mixture was filtered through celite, and the filtrate was purified by silica gel column chromatography (petroleum ether/ethyl acetate (v/v) = 2:1) to obtain the title compound ethyl 2-chloro-4-(((1r,3r)-3-hydroxycyclobutyl)amino)pyrimidine-5-carboxylate **93a** (white solid, 2.9 g, 30.95% yield).

¹H NMR (400 MHz, DMSO-d6) δ 8.61 (s, 1H), 8.48 (d, 1H), 5.17-5.11 (m, 1H), 4.61-4.51 (m, 1H), 4.36-4.27 (m, 3H), 2.32-2.20 (m, 4H), 1.32 (t, 3H).

LC-MS m/z(ESI) = **272.00** [M+1]

### Step 2:

### Ethyl 4-((trans-3-((tert-butyldiphenylsilyl)oxy)cyclobutyl)amino)-2-chloropyrimidine-5-carboxylate (93b)

Ethyl 2-chloro-4-((*trans*-3-hydroxycyclobutyl)amino)pyrimidine-5-carboxylate **93a** (3.10 g, 11.41 mmol) was dissolved in dichloromethane (20 mL), and *tert-*butyldiphenylchlorosilane (4.45 mL, 17.11 mmol) and imidazole (1.94 g, 28.52 mmol) were added, followed by nitrogen purging. The reaction mixture was stirred under nitrogen atmosphere at room temperature for 2 h. The reaction was monitored by TLC. After completion of the reaction, the reaction mixture was purified by silica gel column chromatography (petroleum ether/ethyl acetate (v/v) = 10:1) to obtain the title compound ethyl 4-((*trans*-3-((*tert*-butyldiphenylsilyl)oxy)cyclobutyl)amino)-2-chloropyri-midine-5-carboxylate **93b** (colorless liquid, 4.18 g, 64.26% yield).

LC-MS m/z(ESI) = **510.20** [M+1]

### Step 3:

### 4-((trans-3-((tert-Butyldiphenylsilyl)oxy)cyclobutyl)amino)-2-chloropyrimidine-5-carboxylic acid (93c)

Ethyl 4-((*trans*-3-((*tert*-butyldiphenylsilyl)oxy)cyclobutyl)amino)-2-chloropyrimidine-5-carboxylate **93b** (4.18 g, 8.19 mmol) was dissolved in tetrahydrofuran/water (30 mL/15 mL), and lithium hydroxide monohydrate (1.03 g, 24.58 mmol) was added. The reaction mixture was stirred at room temperature for 2 h. The reaction was monitored by TLC. After completion of the reaction, the reaction mixture was concentrated to evaporate tetrahydrofuran and the pH was adjusted to 3-4 with 2 N HCl, and water was added and the organics were extracted two times with ethyl acetate. The organic layer was purified by silica gel column chromatography (dichloromethane/methanol (v/v) = 20:1) to obtain the title compound 4-((*trans*-3-((*tert*-butyldiphenylsilyl)oxy)cyclobutyl)amino)-2-chloropyrimidine-5-carboxylic acid **93c** (white solid, 3.87 g, 97.97% yield).

LC-MS m/z(ESI) = **482.20** [M+1]

### Step 4:

### 9-(trans-3-((tert-Butyldiphenylsilyl)oxy)cyclobutyl)-2-chloro-7,9-dihydro-8H-purin-8-one (93d)

4-((*trans*-3-((*tert*-Butyldiphenylsilyl)oxy)cyclobutyl)amino)-2-chloropyrimidine-5-carboxylic acid **93c** (3.87 g, 8.03 mmol) was dissolved in *N*,*N*-dimethylacetamide (38 mL), and triethylamine (1.11 mL, 8.03 mmol) and diphenylphosphoryl azide (1.73 mL, 8.03 mmol) were added with stirring at room temperature. The reaction mixture was stirred for 2 h, heated to 110 °C and refluxed for 2.5 h. The reaction was monitored by TLC. After completion of the reaction, water was added and the organics were extracted with ethyl acetate. The organic layer was concentrated, and the residue was purified by silica gel column chromatography (petroleum ether/ethyl acetate (v/v) = 1:1) to obtain the title compound 9-((1r,3r)-3-((*tert*-butyldiphenylsilyl)oxy)cyclobutyl)-2-chloro-7,9-dihydro-8*H*-purin-8-one **93d** (white solid, 1.81 g, 47.06% yield).

¹H NMR (400 MHz, DMSO-d6) δ 11.59 (s, 1H), 8.09 (s, 1H), 7.65-7.62 (m, 2H), 7.62-7.60 (m, 2H), 7.47-7.39 (m, 6H), 5.05-4.97 (m, 1H), 4.96-4.89 (m, 1H), 2.98-2.84 (m, 2H), 2.54-2.46 (m, 2H), 1.02 (s, 9H).

LC-MS m/z(ESI) = **479.20** [M+1]

### Step 5:

### 9-(trans-3-((tert-Butyldiphenylsilyl)oxy)cyclobutyl)-2-chloro-7-methyl-7,9-dihydro-8H-purin-8-one (93e)

9-(*trans*-3-((*tert*-Butyldiphenylsilyl)oxy)cyclobutyl)-2-chloro-7,9-dihydro-8*H*-purin-8-one **93d** (1.81 g, 3.78 mmol) was dissolved in *N,N*-dimethylformamide (20 mL), and dimethyl sulfate (0.36 mL, 3.78 mmol) and cesium carbonate (2.47 g, 7.57 mmol) were added with stirring at 0 °C. The reaction mixture was stirred for 2 h. The reaction was monitored by TLC. After completion of the reaction, the reaction mixture was slowly added dropwise to ice water. The mixture was stirred and extracted with ethyl acetate. The organic layer was concentrated, and the residue was purified by silica gel column chromatography (petroleum ether/ethyl acetate (v/v) = 3:1) to obtain the title compound 9-(*trans*-3-((*tert*-butyldiphenylsilyl)oxy)cyclobutyl)-2-chloro-7-methyl-7,9-dihydro-8*H*-purin-8-one **93e** (white solid, 1.80 g, 96.62% yield).

LC-MS m/z(ESI) = **493.20** [M+1]

### Step 6:

### 4-((9-(trans-3-((tert-Butyldiphenylsilyl)oxy)cyclobutyl)-7-methyl-8-oxo-8,9-dihydro-7H-purin-2-yl)amino)-2-fluoro-5-methylbenzamide (93f)

9-(*trans*-3-((*tert*-Butyldiphenylsilyl)oxy)cyclobutyl)-2-chloro-7-methyl-7,9-dihydro-8H-purin-8-one **93e** (1.00 g, 2.03 mmol) was dissolved in 1,4-dioxane (20 mL), and 4-amino-2-fluoro-5-methylbenzamide **intermediate 2** (1.36 g, 8.11 mmol), cesium carbonate (0.99 g, 3.04 mmol) and BrettPhos-G3-Pd (0.18 g, 0.20 mmol) were added with stirring at room temperature, followed by nitrogen purging. The reaction mixture was heated to 110 °C and refluxed under nitrogen atmosphere for 4 h. The reaction was monitored by TLC. After completion of the reaction, the reaction mixture was concentrated, and the residue was purified by silica gel column chromatography (dichloromethane/methanol (v/v) = 80/1) to obtain the title compound 4-((9-(*trans*-3-((*tert*-butyldiphenylsilyl)oxy)cyclobutyl)-7-methyl-8-oxo-8,9-dihydro-7*H*-purin-2-yl)amino)-2-fluoro-5-methylbenzamide **93f** (pale yellow solid, 900 mg, 71.03% yield). ¹H NMR (400 MHz, DMSO-d6) δ 8.42 (s, 1H), 8.16 (s, 1H), 7.88 (d, 1H), 7.62 (dd, 4H), 7.55 (d, 1H), 7.49-7.40 (m, 7H), 7.24-7.16 (m, 1H), 5.13 (p, 1H), 4.76 (tt, 1H), 3.28 (s, 3H), 3.11-3.02 (m, 2H), 2.47-2.40 (m, 2H), 2.26 (s, 3H), 1.03 (s, 9H).

LC-MS m/z(ESI) = **625.30** [M+1]

### Step 7:

### 2-Fluoro-4-((9-(trans-3-hydroxycyclobutyl)-7-methyl-8-oxo-8,9-dihydro-7H-purin-2-yl)amino)-5-methylbenzamide (compound 93)

4-((9-(*trans*-3-((*tert*-Butyldiphenylsilyl)oxy)cyclobutyl)-7-methyl-8-oxo-8,9-dihydro-7*H*-purin-2-yl)amino)-2-fluoro-5-methylbenzamide **93f** (0.50 g, 0.80 mmol) was dissolved in 4 M hydrogen chloride-1,4-dioxane (10 mL). The reaction mixture was stirred at room temperature. The reaction was monitored by TLC. After completion of the reaction, the reaction mixture was concentrated to evaporate 1,4-dioxane solution and then the pH was adjusted to 9-10 with 2 N aqueous NaOH solution, and a white solid was precipitated. The mixture was filtered, and the filter cake was washed twice with dichloromethane/methanol (v/v = 200:1) and concentrated to obtain the title compound 2-fluoro-4-((9-(*trans*-3-hydroxycyclobutyl)-7-methyl-8-oxo-8,9-dihydro-7*H*-purin-2-yl)amino)-5-methylbenzamide **compound 93** (white solid, 300 mg, 97.02% yield).

¹H NMR (400 MHz, DMSO-d6) δ 8.51 (s, 1H), 8.19 (s, 1H), 7.93 (d, 1H), 7.55 (d, 1H), 7.46 (s, 1H), 7.39 (s, 1H), 5.15 (d, 1H), 5.09 (q, 1H), 4.53-4.44 (m, 1H), 3.31 (s, 3H), 3.14-3.05 (m, 2H), 2.30 (s, 3H), 2.27-2.18 (m, 2H).

LC-MS m/z(ESI) = **387.20** [M+1]

### Example 94

### 2-Fluoro-4-((9-(cis-3-hydroxycyclobutyl)-7-methyl-8-oxo-8,9-dihydro-7H-purin-2-yl)amino)-5-methylbenzamide (compound 94)

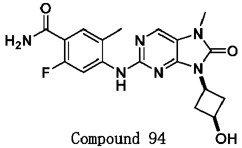

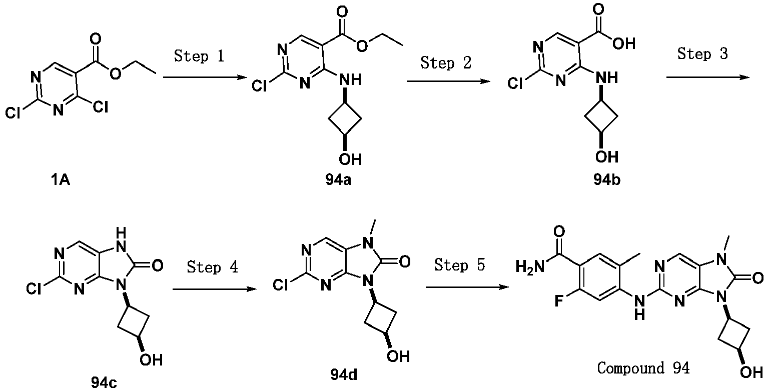

### Step 1:

### Ethyl 2-chloro-4-((cis-3-hydroxycyclobutyl)amino)pyrimidine-5-carboxylate (94a)

*cis*-3-Aminocyclobutane-1-ol hydrochloride (3.00 g, 34.48 mmol) was dissolved in acetonitrile (25 mL), and potassium carbonate (14.30 g, 103.45 mmol) and ethyl 2,4-dichloro-5-pyrimidinecarboxylate **1A** (11.43 g, 51.72 mmol) were added with stirring at 0 °C. The reaction mixture was warmed to room temperature and stirred for 1 h. The reaction was monitored by TLC. After completion of the reaction, the reaction mixture was filtered through celite, and the filtrate was purified by silica gel column chromatography (petroleum ether/ethyl acetate (v/v) = 2:1) to obtain the title compound ethyl 2-chloro-4-((*cis*-3-hydroxycyclobutyl)amino)pyrimidine-5-carboxylate **94a** (white solid, 3.3 g, 32.91% yield).

¹H NMR (400 MHz, DMSO-d6) δ 8.60 (s, 1H), 8.41 (d, 1H), 4.96 (s, 1H), 4.31 (q, 2H), 4.08-3.97 (m, 1H), 3.94-3.84 (m, 1H), 2.71-2.63 (m, 2H), 1.88-1.80 (m, 2H), 1.33-1.29 (m, 3H).

LC-MS m/z(ESI) = **272.00** [M+1]

### Step 2:

### 2-Chloro-4-((cis-3-hydroxycyclobutyl)amino)pyrimidine-5-carboxylic acid (94b)

Ethyl 2-chloro-4-((*cis*-3-hydroxycyclobutyl)amino)pyrimidine-5-carboxylate **94a** (3.30 g, 12.14 mmol) was dissolved in tetrahydrofuran/water (30 mL/15 mL), and lithium hydroxide monohydrate (1.53 g, 36.44 mmol) was added. The reaction mixture was stirred at room temperature for 2 h. The reaction was monitored by TLC. After completion of the reaction, the reaction mixture was concentrated to evaporate tetrahydrofuran and the pH was adjusted to about 3-4 with 2 N HCl, and a white solid was precipitated. The mixture was filtered, and the filter cake was washed twice with water and petroleum ether/ethyl acetate (v/v = 10/1) to obtain the title compound 2-chloro-4-((*cis*-3-hydroxycyclobutyl)amino)pyrimidine-5-carboxylic acid **94b** (white solid, 2.44 g, 82.45% yield).

¹H NMR (400 MHz, DMSO-d6) δ 8.61 (d, 1H), 8.57 (s, 1H), 4.43-4.20 (m, 1H), 4.07-3.96 (m, 1H), 3.93-3.83 (m, 1H), 2.72-2.63 (m, 2H), 1.87-1.77 (m, 2H).

LC-MS m/z(ESI) = **244.00** [M+1]

### Step 3:

### 2-Chloro-9-(cis-3-hydroxycyclobutyl)-7,9-dihydro-8H-purin-8-one (94c)

2-Chloro-4-((*cis*-3-hydroxycyclobutyl)amino)pyrimidine-5-carboxylic acid **94b** (2.44 g, 10.01 mmol) was dissolved in *N*,*N*-dimethylacetamide (20 mL), and triethylamine (1.39 mL, 10.01 mmol) and diphenylphosphoryl azide (2.16 mL, 10.01 mmol) were added with stirring at room temperature. The reaction mixture was stirred for 2 h, then heated to 110 °C and refluxed for 2.5 h. The reaction was monitored by TLC. After completion of the reaction, water was added and the organics were extracted with dichloromethane. The organic layer was concentrated to obtain the title compound 2-chloro-9-(*cis*-3-hydroxycyclobutyl)-7,9-dihydro-8*H*-purin-8-one **94c** (white solid, 0.90 g, 37.35% yield).

¹H NMR (400 MHz, DMSO-d6) δ 11.59 (s, 1H), 8.12 (s, 1H), 4.31-4.20 (m, 1H), 4.00-3.92 (m, 1H), 3.80-3.54 (m, 1H), 2.83-2.73 (m, 2H), 2.58-2.51 (m, 2H).

LC-MS m/z(ESI) = 241.00 [M+1]

### Step 4:

### 2-Chloro-9-(cis-3-hydroxycyclobutyl)-7-methyl-7,9-dihydro-8H-purin-8-one (94d)

2-Chloro-9-(*cis*-3-hydroxycyclobutyl)-7,9-dihydro-8*H*-purin-8-one **94c** (0.70 g, 1.25 mmol) was dissolved in *N*,*N*-dimethylformamide (10 mL), and dimethyl sulfate (0.28 mL, 1.25 mmol) and cesium carbonate (1.42 g, 4.36 mmol) were added with stirring at 0 °C. The reaction mixture was stirred for 2 h. The reaction was s monitored by TLC. After completion of the reaction, the reaction mixture was slowly added dropwise to ice water. The mixture was stirred and extracted with ethyl acetate. The organic layer was concentrated, and the residue was purified by silica gel column chromatography (petroleum ether/ethyl acetate (v/v) = 1:1) to obtain the title compound 2-chloro-9-(cis-3-hydroxycyclobutyl)-7-methyl-7,9-dihydro-8*H*-purin-8-one **94d** (white solid, 0.32 g, 41.20% yield).

¹H NMR (400 MHz, DMSO-d6) δ 8.33 (s, 1H), 5.31 (d, 1H), 4.34-4.23 (m, 1H), 4.02-3.91 (m, 1H), 3.34 (s, 3H), 2.81-2.72 (m, 2H), 2.58-2.51 (m, 2H).

LC-MS m/z(ESI) = **255.00** [M+1]

### Step 5:

### 2-Fluoro-4-((9-(cis-3-hydroxycyclobutyl)-7-methyl-8-oxo-8,9-dihydro-7H-purin-2-yl)amino)-5-methylbenzamide (compound 94)

2-Chloro-9-(*cis*-3-hydroxycyclobutyl)-7-methyl-7,9-dihydro-8*H*-purin-8-one **94d** (0.32 g, 1.26 mmol) was dissolved in 1,4-dioxane (8 mL), and 4-amino-2-fluoro-5-methylbenzamide **intermediate 2** (0.53 g, 3.14 mmol), cesium carbonate (0.61 g, 1.88 mmol) and BrettPhos-G3-Pd (0.11 g, 0.13 mmol) were added with stirring at room temperature, followed by nitrogen purging. The reaction mixture was heated to 110 °C and refluxed under nitrogen atmosphere for 4 h. The reaction was monitored by TLC. After completion of the reaction, the reaction mixture was concentrated, and the residue was purified by silica gel column chromatography (dichloromethane/methanol (v/v) = 40/1) to obtain the title compound 2-fluoro-4-((9-(cis-3-hydroxycyclobutyl)-7-methyl-8-oxo-8,9-dihydro-7*H*-purin-2-yl)amino)-5-methylbenzamide **compound 94** (white solid, 170 mg, 35.01% yield).

¹H NMR (400 MHz, DMSO-d6) δ 8.43 (s, 1H), 8.17 (s, 1H), 7.93 (d, 1H), 7.55 (d, 1H), 7.46 (s, 1H), 7.38 (s, 1H), 5.20 (d, 1H), 4.24 (tt, 1H), 4.02-3.90 (m, 1H), 3.31 (s, 3H), 2.79 (qd, 2H), 2.60 (qd, 2H), 2.29 (s, 3H).

LC-MS m/z(ESI) = **387.10** [M+1]

### Example 95

### 2-Fluoro-4-((9-(-3-hydroxycyclohexyl)-7-methyl-8-oxo-8,9-dihydro-7H-purin-2-yl)amino)-5-methylbenzamide (compound 95)

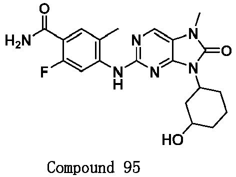

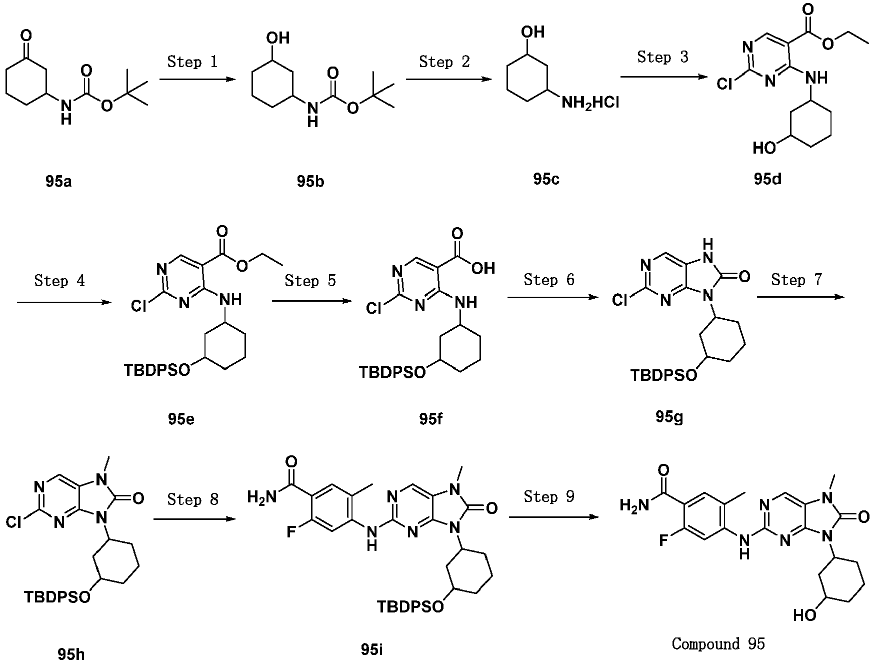

### Step 1:

### tert-Butyl (3-hydroxycyclohexyl)carbamate (95b)

*tert*-Butyl (3-oxocyclohexyl)carbamate **95a** (8.20 g, 38.45 mmol) was dissolved in tetrahydrofuran (40 mL), and sodium borohydride (4.36 g, 115.35 mmol) was added with stirring at 0 °C. The reaction mixture was stirred for 3 h. The reaction was monitored by TLC. After completion of the reaction, saturated sodium carbonate solution was slowly added to the reaction mixture, and the resulting mixture was stirred at room temperature for 3 h. Ethyl acetate was added for extraction, and the organic layer was concentrated to give the title compound *tert*-butyl (3-hydroxycyclohexyl)carbamate **95b** (yellow liquid, 8.00 g, 96.95% yield).

LC-MS m/z(ESI) = **116.10** [M+1]

### Step 2:

### 3-Aminocyclohexan-1-ol hydrochloride (95c)

*tert*-Butyl (3-hydroxycyclohexyl)carbamate **95b** (8.00 g, 37.16 mmol) was dissolved in 2 M hydrogen chloride-ethyl acetate (35 mL). The reaction mixture was stirred at room temperature for 4 h. The reaction was monitored by TLC. After completion of the reaction, the reaction mixture was concentrated to evaporate the solvent to obtain the title compound 3-aminocyclohexan-1-ol hydrochloride **95c** (yellow liquid, 4.00 g, 71.05% yield).

LC-MS m/z(ESI) = **116.10** [M+1]

### Step 3:

### Ethyl 2-chloro-4-((3-hydroxycyclohexyl)amino)pyrimidine-5-carboxylate (95d)

3-Aminocyclohexan-1-ol hydrochloride **95c** (4.00 g, 26.38 mmol) was dissolved in acetonitrile (30 mL), and ethyl 2,4-dichloro-5-pyrimidinecarboxylate **1A** (8.75 g, 39.57 mmol) and potassium carbonate (10.94 g, 79.14 mmol) were added with stirring at 0 °C. The reaction mixture was warmed to room temperature and stirred for 2 h. The reaction was monitored by TLC. After completion of the reaction, the reaction mixture was filtered through celite, and the filtrate was purified by silica gel column chromatography (petroleum ether/ethyl acetate (v/v) = 2:1) to obtain the title compound ethyl 2-chloro-4-((3-hydroxycyclohexyl)amino)pyrimidine-5-carboxylate **95d** (pale yellow solid, 1.40 g, 17.71% yield).

LC-MS m/z(ESI) = **300.10** [M+1]

### Step 4:

### Ethyl 4-((3-((tert-butyldiphenylsilyl)oxy)cyclohexyl)amino)-2-chloropyrimidine-5-carboxylate (95e)

Ethyl 2-chloro-4-((3-hydroxycyclohexyl)amino)pyrimidine-5-carboxylate **95d** (1.10 g, 3.67 mmol) was dissolved in dichloromethane (20 mL), and *tert-*butyldiphenylchlorosilane (1.43 mL, 5.50 mmol) and imidazole (0.62 g, 9.17 mmol) were added, followed by nitrogen purging. The reaction mixture was stirred under nitrogen atmosphere at room temperature for 2 h. The reaction was monitored by TLC. After completion of the reaction, the reaction mixture was purified by silica gel column chromatography (petroleum ether/ethyl acetate (v/v) = 8:1) to obtain the title compound ethyl 4-((3-((*tert*-butyldiphenylsilyl)oxy)cyclohexyl)amino)-2-chloropyrimidine-5-carboxylate **95e** (colorless liquid, 1.90 g, 96.21% yield).

LC-MS m/z(ESI) = **538.20** [M+1]

### Step 5:

### 4-((3-((tert-Butyldiphenylsilyl)oxy)cyclohexyl)amino)-2-chloropyrimidine-5-carboxylic acid (95f)

Ethyl 4-((3-((*tert*-butyldiphenylsilyl)oxy)cyclohexyl)amino)-2-chloropyrimidine-5-carboxylate **95e** (1.90 g, 3.53 mmol) was dissolved in tetrahydrofuran/water (20 mL/20 mL), and lithium hydroxide monohydrate (0.44 g, 10.59 mmol) was added. The reaction mixture was stirred at room temperature for 2 h. The reaction was monitored by TLC. After completion of the reaction, the reaction mixture was concentrated to evaporate tetrahydrofuran and the pH was adjusted to 3-4 with 2 N HCl, and water was added and the organics were extracted two times with ethyl acetate. The organic layer was concentrated to yield the title compound 4-((3-((*tert-*butyldiphenylsilyl)oxy)cyclohexyl)amino)-2-chloropyrimidine-5-carboxylic acid **95f** (white solid, 1.10 g, 61.08% yield).

LC-MS m/z(ESI) = **510.20** [M+1]

### Step 6:

### 9-(3-((tert-Butyldiphenylsilyl)oxy)cyclohexyl)-2-chloro-7,9-dihydro-8H-purin-8-one (95g)

4-((3-((*tert*-Butyldiphenylsilyl)oxy)cyclohexyl)amino)-2-chloropyrimidine-5-carboxylic acid **95f** (1.10 g, 2.16 mmol) was dissolved in *N*,*N*-dimethylacetamide (23 mL), and triethylamine (0.30 mL, 2.16 mmol) and diphenylphosphoryl azide (0.46 mL, 2.16 mmol) were added with stirring at room temperature. The reaction mixture was stirred for 2 h, heated to 110 °C and refluxed for 2.5 h. The reaction was monitored by TLC. After completion of the reaction, water was added and the organics were extracted with ethyl acetate. The organic layer was concentrated, and the residue was purified by silica gel column chromatography (petroleum ether/ethyl acetate (v/v) = 1:1) to obtain the title compound 9-(3-((*tert*-butyldiphenylsilyl)oxy)cyclohexyl)-2-chloro-7,9-dihydro-8*H*-purin-8-one **95g** (white solid, 0.31 g, 28.35% yield).

LC-MS m/z(ESI) = **507.20** [M+1]

### Step 7:

### 9-(3-((tert-Butyldiphenylsilyl)oxy)cyclohexyl)-2-chloro-7-methyl-7,9-dihydro-8H-purin-8-one (95h)

9-(3-((*tert*-Butyldiphenylsilyl)oxy)cyclohexyl)-2-chloro-7,9-dihydro-8*H*-purin-8-one **95g** (0.30 g, 0.59 mmol) was dissolved in *N,N*-dimethylformamide (10 mL), and dimethyl sulfate (0.06 mL, 0.59 mmol) and cesium carbonate (0.39 g, 1.18 mmol) were added with stirring at 0 °C. The reaction mixture was stirred for 1 h. The reaction was monitored by TLC. After completion of the reaction, the reaction mixture was slowly added dropwise to ice water. The mixture was stirred and extracted with ethyl acetate. The organic layer was concentrated to yield the title compound 9-(3-((*tert-*butyldiphenylsilyl)oxy)cyclohexyl)-2-chloro-7-methyl-7,9-dihydro-8*H*-purin-8-one **95h** (colorless liquid, 0.30 g, 97.31% yield).

LC-MS m/z(ESI) = 521.20 [M+1]

### Step 8:

### 4-((9-(3-((tert-Butyldiphenylsilyl)oxy)cyclohexyl)-7-methyl-8-oxo-8,9-dihydro-7H-purin-2-yl)amino)-2-fluoro-5-methylbenzamide (95i)

9-(3-((*tert*-Butyldiphenylsilyl)oxy)cyclohexyl)-2-chloro-7-methyl-7,9-dihydro-8*H-*purin-8-one **95h** (1.00 g, 1.92 mmol) was dissolved in 1,4-dioxane (15 mL), and 4-amino-2-fluoro-5-methylbenzamide (1.29 g, 7.68 mmol), cesium carbonate (0.94 g, 2.88 mmol) and BrettPhos-G3-Pd (0.17 g, 0.19 mmol) were added with stirring at room temperature, followed by nitrogen purging. The reaction mixture was heated to 110 °C and refluxed under nitrogen atmosphere for 4 h. The reaction was monitored by TLC. After completion of the reaction, the reaction mixture was concentrated, and the residue was purified by silica gel column chromatography (dichloromethane/methanol (v/v) = 100/1) to obtain the title compound 4-((9-(3-((*tert-*butyldiphenylsilyl)oxy)cyclohexyl)-7-methyl-8-oxo-8,9-dihydro-7*H*-purin-2-yl)amino)-2-fluoro-5-methylbenzamide **95i** (yellow solid, crude, 707 mg, 56.44% yield).

LC-MS m/z(ESI) = **653.40** [M+1]

### Step 9:

### 2-Fluoro-4-((9-(-3-hydroxycyclohexyl)-7-methyl-8-oxo-8,9-dihydro-7H-purin-2-yl)amino)-5-methylbenzamide (compound 95)

4-((9-(3-((*tert*-Butyldiphenylsilyl)oxy)cyclohexyl)-7-methyl-8-oxo-8,9-dihydro-7*H-*purin-2-yl)amino)-2-fluoro-5-methylbenzamide **95i** (0.65 g, 0.99 mmol) was dissolved in 4 M hydrogen chloride-1,4-dioxane (10 mL). The reaction mixture was stirred at room temperature. The reaction was monitored by TLC. After completion of the reaction, the reaction mixture was concentrated to evaporate 1,4-dioxane solution and then the pH was adjusted to 9-10 with 2 N aqueous NaOH solution, and ethyl acetate was added for extraction. The organic layer was concentrated, and the residue was purified by silica gel column chromatography (dichloromethane/methanol (v/v) = 30/1), followed by concentration to obtain the title compound 2-fluoro-4-((9-(-3-hydroxycyclohexyl)-7-methyl-8-oxo-8,9-dihydro-7*H*-purin-2-yl)amino)-5-methylbenzamide compound **95-1** (pale yellow solid, 100 mg, 24.35% yield) and **compound 95-2** (white solid, 47 mg, 11.44% yield).

### Compound 95-1:

¹H NMR (400 MHz, DMSO-*d*₆) δ 8.48 (s, 1H), 8.18 (s, 1H), 7.98 (d, 1H), 7.54 (d, 1H), 7.46 (s, 1H), 7.41-7.35 (m, 1H), 4.79 (d, 1H), 4.22 (tt, 1H), 3.50 (tq, 1H), 3.32 (s, 3H), 2.30 (s, 3H), 2.18 (qd, 2H), 1.95-1.83 (m, 2H), 1.79 (dd, 1H), 1.68 (d, 1H), 1.40-1.26 (m, 1H), 1.24-1.15 (m, 1H).

LC-MS m/z(ESI) = **415.20** [M+1]

### Compound 95-2:

¹H NMR (400 MHz, DMSO-*d*₆) δ 8.50 (s, 1H), 8.18 (s, 1H), 7.98 (d, 1H), 7.54 (d, 1H), 7.45 (s, 1H), 7.39 (s, 1H), 4.68 (dt, 1H), 4.63 (d, 1H), 4.15-4.10 (m, 1H), 3.32 (s, 3H), 2.53-2.47 (m, 1H), 2.30 (s, 3H), 2.21 (qd, 1H), 1.74 (dt, 4H), 1.57 (d, 1H), 1.42 (t, 1H). LC-MS m/z(ESI) = **415.10** [M+1]

### Example 96

### 2-Fluoro-4-((9-(6-(hydroxymethyl)tetrahydro-2H-pyran-3-yl)-7-methyl-8-oxo-8,9-dihydro-7H-purin-2-yl)amino)-5-methylbenzamide (compound 96)

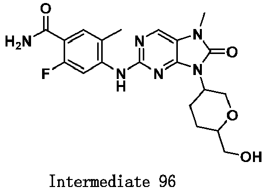

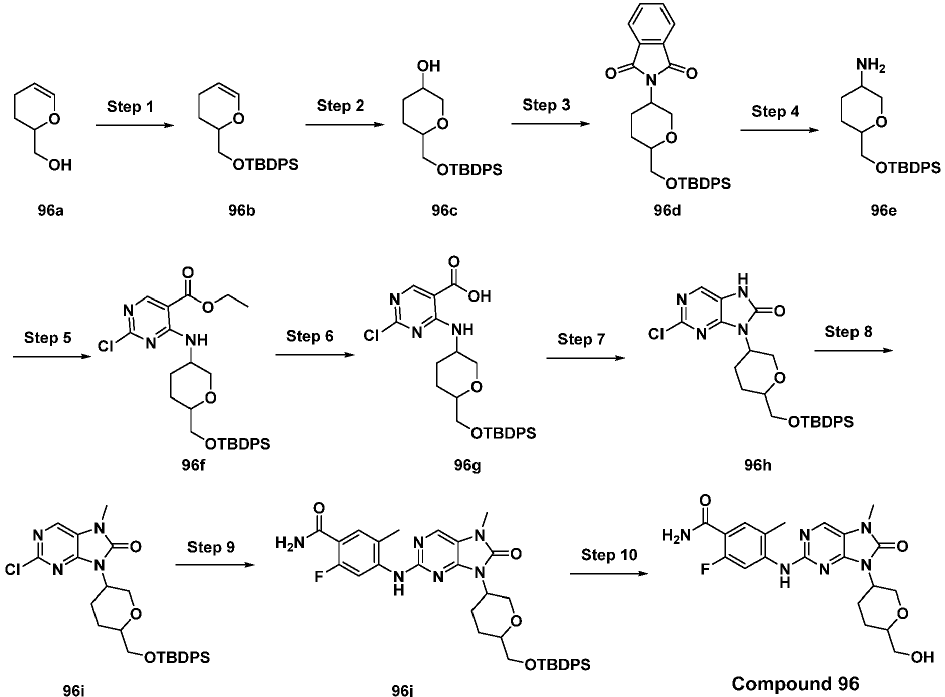

### Step 1:

### tert-Butyl((3,4-dihydro-2H-pyran-2-yl)methoxy)diphenylsilane (96b)

(3,4-Dihydro-2*H*-pyran-2-yl)methanol **96a** (20.0 g, 175.22 mmol) was dissolved in dichloromethane (400 mL), and *tert*-butyldiphenylchlorosilane (68.35 mL, 262.84 mmol) and imidazole (29.82 g, 438.06 mmol) were added, followed by nitrogen purging. The reaction mixture was stirred under nitrogen atmosphere at room temperature overnight. The reaction was monitored by TLC. After completion of the reaction, the reaction mixture was washed with water, and the organic phase was dried over anhydrous sodium sulfate and filtered. The filtrate was concentrated under reduced pressure, and the residue was purified by silica gel column chromatography (petroleum ether/ethyl acetate (v/v) = 100:1) to obtain the title compound *tert*-butyl((3,4-dihydro-2*H*-pyran-2-yl)methoxy)diphenylsilane **96b** (colorless liquid, 42.78 g, 75.00% yield). ¹H NMR (400 MHz,CDCl₃):δ7.71-7.68 (m, 4H), 7.45-7.37 (m, 6H), 6.37- 6.35 (d, 1H), 4.68-4.65 (m, 1H), 3.96-3.93 (m, 1H), 3.83-3.77 (m, 1H), 3.71-3.67 (m, 1H), 2.08-2.00 (m, 1H), 1.99-1.92 (m, 2H), 1.74-1.71 (m, 1H), 1.02 (s, 9H).

LC-MS m/z(ESI) = **353.20** [M+1]

### Step 2:

### 6-(((tert-Butyldiphenylsilyl)oxy)methyl)tetrahydro-2H-pyran-3-ol (96c)

*tert*-Butyl((3,4-dihydro-2*H*-pyran-2-yl)methoxy)diphenylsilane **96b** (23.5 g, 66.7 mmol) was added to tetrahydrofuran (200 mL). Under nitrogen atmosphere, the reaction mixture was cooled to -78 °C, and then borane-dimethyl sulfide complex (100 mL, 2 M, 200.00 mmol) was added dropwise thereto. After addition, the reaction mixture was naturally warmed to room temperature and stirred overnight. 1 N aqueous sodium hydroxide solution was slowly added dropwise to the reaction system until no borane gas was discharged, and then 30% hydrogen peroxide (90 mL) was added to the reaction mixture. The reaction mixture was stirred at 45 °C for 2 h. Water was added and the organics were extracted with ethyl acetate. The organic phase was dried over anhydrous sodium sulfate and filtered. The filtrate was concentrated under reduced pressure, and the residue was purified by reversed-phase column chromatography (acetonitrile/water (v/v) = 5:95) to obtain the title compound 6-(((*tert-*butyldiphenylsilyl)oxy)methyl)tetrahydro-2H̅-pyran-3-ol **96c** (colorless liquid, 18.03 g, 73.00% yield).

LC-MS m/z(ESI) = **371.20** [M+1]

### Step 3:

### 2-(6-(((tert-Butyldiphenylsilyl)oxy)methyl)tetrahydro-2H-pyran-3-yl)isoindoline-1,3-dione (96d)

6-(((*tert*-Butyldiphenylsilyl)oxy)methyl)tetrahydro-2*H*-pyran-3-ol **96c** (13.0 g, 35.1 mmol) was dissolved in tetrahydrofuran (150 mL), and phthalimide (5.16 g, 35.1 mmol) and triphenylphosphine (13.8 g, 52.7 mmol) were added with stirring at room temperature, followed by the slow dropwise addition of diisopropyl azodicarboxylate (10.7 g, 52.7 mmol). The reaction mixture was heated to 70 °C and refluxed for 1 h. The reaction was s monitored by TLC. After completion of the reaction, the reaction mixture was concentrated and purified by silica gel column chromatography (petroleum ether/ethyl acetate (v/v) = 100:1) to obtain the title compound 2-(6-(((*tert-*butyldiphenylsilyl)oxy)methyl)tetrahydro-2H-pyran-3-yl)isoindoline-1,3-dione **96d** (white solid, 9.0 g, 51.34% yield).

LC-MS m/z(ESI) = **500.20** [M+1]

### Step 4:

### 6-(((tert-Butyldiphenylsilyl)oxy)methyl)tetrahydro-2H-pyran-3-amine (96e)

2-(6-(((*tert*-Butyldiphenylsilyl)oxy)methyl)tetrahydro-2*H*-pyran-3-yl)isoindoline-1,3-dione **96d** (9.0 g, 18.0 mmol) was dissolved in methanol (50 mL), and hydrazine hydrate (1.8 g, 36.0 mmol) was added. The reaction mixture was refluxed for 2 h. The reaction was monitored by TLC. After completion of the reaction, dichloromethane (50 mL) was added, and a large amounts of solid was precipitated. Filtration and rotary evaporation drying were performed. The resulting viscos solid was washed twice with dichloromethane, and then the mixture was filtered. The organic phases were combined and concentrated under reduced pressure to yield the title compound 6-(((*tert-*butyldiphenylsilyl)oxy)methyl)tetrahydro-2*H*-pyran-3-amine **96e** (colorless liquid, 6.3 g, 94.10% yield).

LC-MS m/z(ESI) = **370.20** [M+1]

### Step 5:

### Ethyl 4-((6-(((tert-butyldiphenylsilyl)oxy)methyl)tetrahydro-2H-pyran-3-yl)amino)-2-chloropyrimidine-5-carboxylate (96f)

Ethyl 2,4-dichloro-5-pyrimidinecarboxylate **1A** (6.94 g, 31.40 mmol) was dissolved in acetonitrile (25 mL), and potassium carbonate (8.68 g, 62.80 mmol) was added with stirring at room temperature, followed by the slow dropwise addition of a solution of 6-((*tert*-butyldiphenylsilyl)oxy)methyltetrahydro-2*H*-pyran-3-amine **96e** (7.40 g, 20.93 mmol) in acetonitrile (15 mL). The reaction mixture was stirred for 2 h. The reaction was monitored by TLC. After completion of the reaction, the reaction mixture was filtered through celite, and the filtrate was purified by silica gel column chromatography (petroleum ether/ethyl acetate (v/v) = 20:1) to obtain the title compound ethyl 4-((6-(((*tert*-butyldiphenylsilyl)oxy)methyl)tetrahydro-2*H*-pyran-3-yl)amino)-2-chloropyrimidine-5-carboxylate **96f** (pale yellow solid, 5.20 g, 46.86% yield). LC-MS m/z(ESI) = **554.30** [M+1]

### Step 6:

### 4-((6-(((tert-Butyldiphenylsilyl)oxy)methyl)tetrahydro-2H-pyran-3-yl)amino)-2-chloropyrimidine-5-carboxylic acid (96g)

Ethyl 4-((6-(((*tert*-butyldiphenylsilyl)oxy)methyl)tetrahydro-2*H*-pyran-3-yl)amino)-2-chloropyrimidine-5-carboxylate **96f** (5.20 g, 9.383 mmol) was dissolved in tetrahydrofuran/water (30 mL/15 mL), and lithium hydroxide monohydrate (1.18 g, 28.15 mmol) was added. The reaction mixture was stirred at room temperature for 3 h, then heated to 50 °C and stirred for 2 h. The reaction was monitored by TLC. After completion of the reaction, the reaction mixture was concentrated to evaporate tetrahydrofuran and the pH was adjusted to 3-4 with 2 N HCl, and water and ethyl acetate were added for two extractions. The organic layer was purified by silica gel column chromatography (petroleum ether/ethyl acetate (v/v) = 4:1) to obtain the title compound 4-((6-(((*tert*-butyldiphenylsilyl)oxy)methyl)tetrahydro-2*H*-pyran-3-yl)amino)-2-chloropyrimidine-5-carboxylic acid **96g** (white solid, 2.57 g, 52.06% yield).

LC-MS m/z(ESI) = **526.20** [M+1]

### Step 7:

### 9-(6-(((tert-Butyldiphenylsilyl)oxy)methyl)tetrahydro-2H-pyran-3-yl)-2-chloro-7,9-dihydro-8H-purin-8-one (96h)

4-((6-(((*tert*-Butyldiphenylsilyl)oxy)methyl)tetrahydro-2*H*-pyran-3-yl)amino)-2-chloropyrimidine-5-carboxylic acid **96g** (2.57 g, 4.88 mmol) was dissolved in *N,N-*dimethylacetamide (24 mL), and triethylamine (0.68 mL, 4.88 mmol) and diphenylphosphoryl azide (1.05 mL, 4.88 mmol) were added with stirring at room temperature. The reaction mixture was stirred for 2 h, then heated to 110 °C and refluxed for 2.5 h. The reaction was monitored by TLC. Water was added and the organics were extracted with ethyl acetate. The organic layer was concentrated, and the residue was purified by silica gel column chromatography (petroleum ether/ethyl acetate (v/v) = 1:1) to obtain the title compound 9-(6-(((*tert-*butyldiphenylsilyl)oxy)methyl)tetrahydro-2*H*-pyran-3-yl)-2-chloro-7,9-dihydro-8*H-*purin-8-one **96h** (pale pink solid, 1.60 g, 62.66% yield).

¹H NMR (400 MHz, DMSO-d6) δ 11.65 (s, 1H), 8.12 (s, 1H), 7.67-7.62 (m, 4H), 7.46-7.39 (m, 6H), 4.32-4.24 (m, 1H), 4.13-4.06 (m, 1H), 4.00-3.93 (m, 1H), 3.91-3.81 (m, 2H), 3.56 (dd, 1H), 2.53-2.47 (m, 1H), 2.07-1.98 (m, 1H), 1.85-1.72 (m, 2H), 1.01 (s, 9H).

LC-MS m/z(ESI) = **523.20** [M+1]

### Step 8:

### 9-(6-(((tert-Butyldiphenylsilyl)oxy)methyl)tetrahydro-2H-pyran-3-yl)-2-chloro-7-methyl-7,9-dihydro-8H-purin-8-one (96i)

9-(6-(((*tert*-Butyldiphenylsilyl)oxy)methyl)tetrahydro-2*H*-pyran-3-yl)-2-chloro-7,9-dihydro-8*H*-purin-8-one **96h** (1.60 g, 3.06 mmol) was dissolved in *N,N-*dimethylformamide (15 mL), and dimethyl sulfate (0.29 mL, 3.06 mmol) and cesium carbonate (2.00 g, 6.13 mmol) were added with stirring at room temperature. The reaction mixture was stirred for 1 h. The reaction was monitored by TLC. After completion of the reaction, water was added and the organics were extracted with ethyl acetate. The organic layer was concentrated, and the residue was purified by silica gel column chromatography (petroleum ether/ethyl acetate (v/v) = 3:2) to obtain the title compound 9-(6-(((*tert*-butyldiphenylsilyl)oxy)methyl)tetrahydro-2*H*-pyran-3-yl)-2-chloro-7-methyl-7,9-dihydro-8*H*-purin-8-one **96i** (pale pink liquid, 1.6 g, 97.21% yield).

¹H NMR (400 MHz, DMSO-d6) δ 8.35 (s, 1H), 7.67-7.62 (m, 4H), 7.49-7.39 (m, 6H), 4.36-4.28 (m, 1H), 4.14-4.07 (m, 1H), 4.00-3.93 (m, 1H), 3.90-3.81 (m, 2H), 3.57 (dd, 1H), 3.35 (s, 3H), 2.55-2.44 (m, 1H), 2.09-1.98 (m, 1H), 1.87-1.73 (m, 2H), 1.01 (s, 9H).

LC-MS m/z(ESI) = **537.30** [M+1]

### Step 9:

### 4-((9-(6-(((tert-Butyldiphenylsilyl)oxy)methyl)tetrahydro-2H-pyran-3-yl)-7-methyl-8-oxo-8,9-dihydro-7H-purin-2-yl)amino)-2-fluoro-5-methylbenzamide (96j)

9-(6-(((*tert*-Butyldiphenylsilyl)oxy)methyl)tetrahydro-2*H*-pyran-3-yl)-2-chloro-7-methyl-7,9-dihydro-8H-purin-8-one **96i** (1.00 g, 1.86 mmol) was dissolved in 1,4-dioxane (20 mL), and 4-amino-2-fluoro-5-methylbenzamide **intermediate 2** (1.25 g, 7.45 mmol), cesium carbonate (0.91 g, 2.79 mmol) and BrettPhos-G3-Pd (0.17 g, 0.19 mmol) were added with stirring at room temperature, followed by nitrogen purging. The reaction mixture was heated to 110 °C and refluxed under nitrogen atmosphere for 4 h. The reaction was monitored by TLC. After completion of the reaction, the reaction mixture was concentrated, and the residue was purified by silica gel column chromatography (dichloromethane/methanol (v/v) = 80/1) to obtain the title compound 4-((9-(6-(((*tert*-butyldiphenylsilyl)oxy)methyl)tetrahydro-2*H*-pyran-3-yl)-7-methyl-8-oxo-8,9-dihydro-7*H*-purin-2-yl)amino)-2-fluoro-5-methylbenzamide **96j** (yellow solid, 750 mg, 60.23% yield).

LC-MS m/z(ESI) = **669.40** [M+1]

### Step 10:

### 2-Fluoro-4-((9-(6-(hydroxymethyl)tetrahydro-2H-pyran-3-yl)-7-methyl-8-oxo-8,9-dihydro-7H-purin-2-yl)amino)-5-methylbenzamide (compound 96)

4-((9-(6-(((*tert*-Butyldiphenylsilyl)oxy)methyl)tetrahydro-2*H*-pyran-3-yl)-7-methyl-8-oxo-8,9-dihydro-7*H*-purin-2-yl)amino)-2-fluoro-5-methylbenzamide **96j** (0.50 g, 0.75 mmol) was dissolved in 4 M hydrogen chloride-1,4-dioxane (8 mL). The reaction mixture was stirred at room temperature. The reaction was monitored by TLC. After completion of the reaction, the reaction mixture was concentrated to evaporate 1,4-dioxane solution and then the pH was adjusted to 9-10 with 2 N aqueous NaOH solution, and ethyl acetate was added for extraction. The organic layer was concentrated, and the residue was purified by silica gel column chromatography (dichloromethane/methanol (v/v) = 20/1), followed by concentration to yield the title compound 2-fluoro-4-((9-(6-(hydroxymethyl)tetrahydro-2*H*-pyran-3-yl)-7-methyl-8-oxo-8,9-dihydro-7*H*-purin-2-yl)amino)-5-methylbenzamide **compound 96** (white solid, 170 mg, 52.83% yield).

¹H NMR (400 MHz, DMSO-d6) δ 8.55 (s, 1H), 8.18 (s, 1H), 7.76 (d, 1H), 7.55 (d, 1H), 7.49 (s, 1H), 7.39 (s, 1H), 4.63-4.58 (m, 1H), 4.30 (tt, 1H), 4.15 (t, 1H), 3.71- 3.64 (m, 2H), 3.56 -3.45 (m, 2H), 3.32 (s, 3H), 2.65-2.35 (m, 1H), 2.28 (s, 3H), 1.86 (d, 1H), 1.79-1.67 (m, 2H).

LC-MS m/z(ESI) = **431.20** [M+1]

### Example 97

### 2-Fluoro-4-((9-(6-(hydroxymethyl)spiro[3.3]heptan-2-yl)-7-methyl-8-oxo-8,9-dihydro-7H-purin-2-yl)amino)-5-methylbenzamide (compound 97)

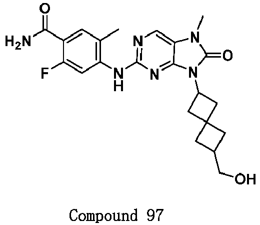

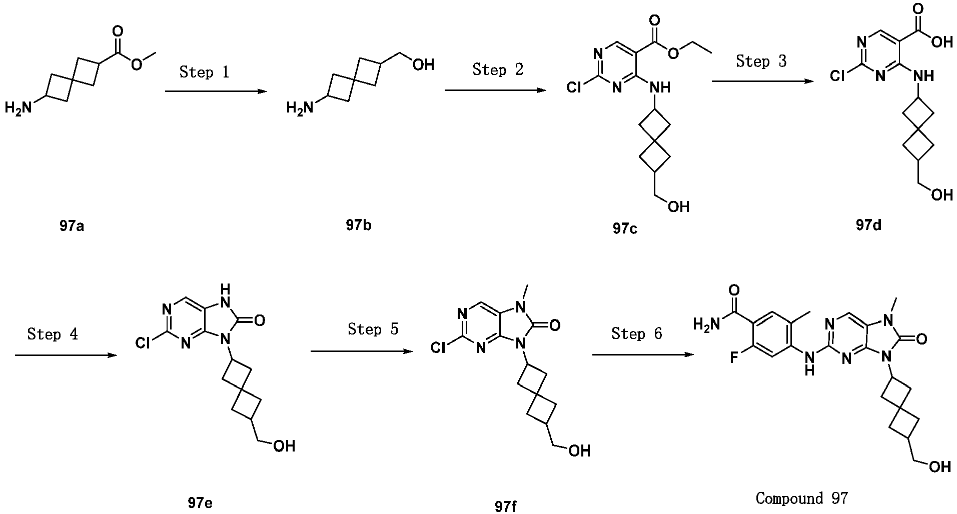

### Step 1:

### (6-Aminospiro[3.3]heptan-2-yl)methanol (97b)

Methyl 6-aminospiro[3.3]heptan-2-carboxylate **97a** (2.0 g, 11.8 mmol) was dissolved in tetrahydrofuran (15 mL). The mixture was cooled to 0 °C, and 1 N lithium aluminum hydride solution (23.6 mL, 1 mol/L in tetrahydrofuran) was slowly added dropwise. After addition, the reaction mixture was slowly warmed to room temperature and stirred for 2 h. The reaction was monitored by TLC. After completion of the reaction, the reaction mixture was quenched with methanol and concentrated under reduced pressure to evaporate the solvent. The residue was slurried with acetonitrile (50 mL), and the slurry was filtered. The filtrate was concentrated by rotary evaporation under reduced pressure to give the title compound (6-aminospiro[3.3]heptan-2-yl)methanol **97b** (white solid, 1.3 g, 77.8% yield).

LC-MS m/z(ESI) = **142.10** [M+1]

### Step 2:

### Ethyl 2-chloro-4-((6-(hydroxymethyl)spiro[3.3]heptan-2-yl)amino)pyrimidine-5-carboxylate (97c)

Ethyl 2,4-dichloropyrimidine-5-carboxylate **1A** (2.03 g, 9.19 mmol), (6-aminospiro[3.3]heptan-2-yl)methanol **97b** (1.3 g, 9.19 mmol) and potassium carbonate (1.27 g, 9.19 mmol) were dissolved in acetonitrile (25 mL). The reaction mixture was stirred at room temperature for 16 h. The reaction was monitored by TLC. After completion of the reaction, the filtrate was concentrated, and the residue was purified by silica gel column chromatography (petroleum ether:ethyl acetate (v/v) = 1:1) to obtain the title compound ethyl 2-chloro-4-((6-(hydroxymethyl)spiro[3.3]heptan-2-yl)amino)pyrimidine-5-carboxylate **97c** (white solid, 1.56 g, 52.2% yield).

¹H NMR (400 MHz, Chloroform-d) δ 8.65 (s, 1H), 8.54 (d, 1H), 4.59-4.48 (m, 1H), 4.35 (q, 2H), 3.59 (dd, 2H), 2.66-2.58 (m, 1H), 2.51-2.38 (m, 2H), 2.27-2.19 (m, 1H), 2.11-2.04 (m, 2H), 2.03-1.91 (m, 2H), 1.91-1.85 (m, 1H), 1.84-1.76 (m, 1H), 1.39 (t, 3H).

LC-MS m/z(ESI) = **326.10** [M+1]

### Step 3:

### 2-Chloro-4-((6-(hydroxymethyl)spiro[3.3]heptan-2-yl)amino)pyrimidine-5-carboxylic acid (97d)

Ethyl 2-chloro-4-((6-(hydroxymethyl)spiro[3.3]heptan-2-yl)amino)pyrimidine-5-carboxylate **97c** (1.07 g, 3.28 mmol) was dissolved in tetrahydrofuran/water (10 mL/10 mL), and lithium hydroxide monohydrate (0.41 g, 9.85 mmol) was added. The reaction mixture was stirred at room temperature for 1 h. The reaction was monitored by TLC. After completion of the reaction, the reaction mixture was concentrated to evaporate tetrahydrofuran and the pH was adjusted to about 3-4 with 2 N HCl, and a white solid was precipitated. The mixture was filtered, and the filter cake was washed twice with water and petroleum ether/ethyl acetate (v/v = 10/1) to obtain the title compound 2-chloro-4-((6-(hydroxymethyl)spiro[3.3]heptan-2-yl)amino)pyrimidine-5-carboxylic acid **97d** (white solid, 918 mg, 93.88% yield).

¹H NMR (400 MHz, DMSO-d6) δ 12.69 (s, 1H), 9.67 (d, 1H), 8.35 (s, 1H), 4.42-4.31 (m, 1H), 3.30 (s, 1H), 2.55-2.46 (m, 3H), 2.40-2.31 (m, 1H), 2.28-2.19 (m, 1H), 2.10-2.00 (m, 2H), 2.00-1.91 (m, 2H), 1.83-1.69 (m, 2H).

LC-MS m/z(ESI) = **298.10** [M+1]

### Step 4:

### 2-Chloro-9-(6-(hydroxymethyl)spiro[3.3]heptan-2-yl)-7,9-dihydro-8H-purin-8-one (97e)

2-Chloro-4-((6-(hydroxymethyl)spiro[3.3]heptan-2-yl)amino)pyrimidine-5-carboxylic acid **97d** (0.91 g, 3.06 mmol) was dissolved in *N*,*N*-dimethylacetamide (12 mL), and triethylamine (0.42 mL, 3.06 mmol) and diphenylphosphoryl azide (0.66 mL, 10.01 mmol) were added with stirring at room temperature. The reaction mixture was stirred for 2 h, then heated to 110 °C and refluxed for 2.5 h. The reaction was monitored by TLC. After completion of the reaction, water was added and the organics were extracted with ethyl acetate. The organic layer was concentrated, and the residue was purified by silica gel column chromatography (dichloromethane/methanol (v/v) = 20:1) to obtain the title compound 2-chloro-9-(6-(hydroxymethyl)spiro[3.3]heptan-2-yl)-7,9-dihydro-8*H*-purin-8-one **97e** (white solid, 516 mg, 57.28% yield).

¹H NMR (400 MHz, DMSO-d6) δ 11.60 (s, 1H), 8.11 (s, 1H), 4.68-4.57 (m, 1H), 4.46 (t, 1H), 3.37-3.32 (m, 2H), 2.95-2.81 (m, 2H), 2.42-2.35 (m, 1H), 2.32-2.19 (m, 2H), 219-212 (m, 1H), 2.06-1.98 (m, 1H), 1.89-1.77 (m, 2H).

LC-MS m/z(ESI) = **295.10** [M+1]

### Step 5:

### 2-Chloro-9-(6-(hydroxymethyl)spiro[3.3]heptan-2-yl)-7-methyl-7,9-dihydro-8H-purin-8-one (97f)

2-Chloro-9-(6-(hydroxymethyl)spiro[3.3]heptan-2-yl)-7,9-dihydro-8*H*-purin-8-one 9**7e** (516 mg, 1.75 mmol) was dissolved in *N,N*-dimethylformamide (6 mL), and dimethyl sulfate (0.17 mL, 1.75 mmol) and cesium carbonate (1.14 g, 3.50 mmol) were added with stirring at 0 °C. The reaction mixture was stirred for 1 h. The reaction was monitored by TLC. After completion of the reaction, the reaction mixture was slowly added dropwise to ice water, and the resulting mixture was stirred and extracted with ethyl acetate. The organic layer was concentrated to give the title compound 2-chloro-9-(6-(hydroxymethyl)spiro[3.3]heptan-2-yl)-7-methyl-7,9-dihydro-8*H*-purin-8-one **97f** (white solid, 470 mg, 86.95% yield).

¹H NMR (400 MHz, DMSO-d6) δ 8.33 (s, 1H), 4.71-4.60 (m, 1H), 4.10-3.96 (m, 2H), 3.35 (s, 1H), 3.33 (s, 3H), 2.93-2.81 (m, 2H), 2.44-2.36 (m, 1H), 2.32-2.21 (m, 2H), 2.20-2.13 (m, 1H), 2.07-1.99 (m, 1H), 1.89-1.78 (m, 2H).

LC-MS m/z(ESI) = **309.10** [M+1]

### Step 6:

### 2-Fluoro-4-((9-(6-(hydroxymethyl)spiro[3.3]heptan-2-yl)-7-methyl-8-oxo-8,9-dihydro-7H-purin-2-yl)amino)-5-methylbenzamide (compound 97)

2-Chloro-9-(6-(hydroxymethyl)spiro[3.3]heptan-2-yl)-7-methyl-7,9-dihydro-8*H-*purin-8-one **97f** (100 mg, 0.32 mmol) was dissolved in 1,4-dioxane (5 mL), and 4-amino-2-fluoro-5-methylbenzamide **intermediate 2** (218 mg, 1.30 mmol), cesium carbonate (158 mg, 0.49 mmol) and BrettPhos-G3-Pd (29 mg, 0.032 mmol) were added with stirring at room temperature, followed by nitrogen purging. The reaction mixture was heated to 110 °C and refluxed under nitrogen atmosphere for 4 h. The reaction was monitored by TLC. After completion of the reaction, the reaction mixture was concentrated, and the residue was purified by silica gel column chromatography (dichloromethane/methanol (v/v) = 20/1) to obtain the title compound 2-fluoro-4-((9-(6-(hydroxymethyl)spiro[3.3]heptan-2-yl)-7-methyl-8-oxo-8,9-dihydro-7*H*-purin-2-yl)amino)-5-methylbenzamide **compound 97** (white solid, 50 mg, 35.05% yield).

¹H NMR (400 MHz, DMSO-d6) δ 8.66 (s, 1H), 8.18 (s, 1H), 7.83 (d, 1H), 7.56 (d, 1H), 7.49 (s, 1H), 7.40 (s, 1H), 4.70-4.60 (m, 1H), 3.32 (s, 1H), 3.30 (s, 3H), 2.96-2.84 (m, 2H), 2.38-2.31 (m, 1H), 2.30-2.23 (m, 4H), 2.23-2.18 (m, 1H), 2.17-2.11 (m, 1H), 1.98 -1.91 (m, 1H), 1.88 -1.81 (m, 1H), 1.77-1.71 (m, 1H).

LC-MS m/z(ESI) = **441.20** [M+1]

### Example 98

### 4-[(9-(5-Hydroxy-octahydropentalen-2-yl)-7-methyl-8-oxo-8,9-dihydro-7H-purin-2-yl)amino]-3-methylbenzonitrile (compound 98)

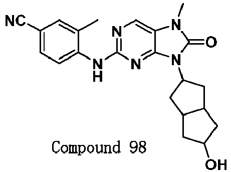

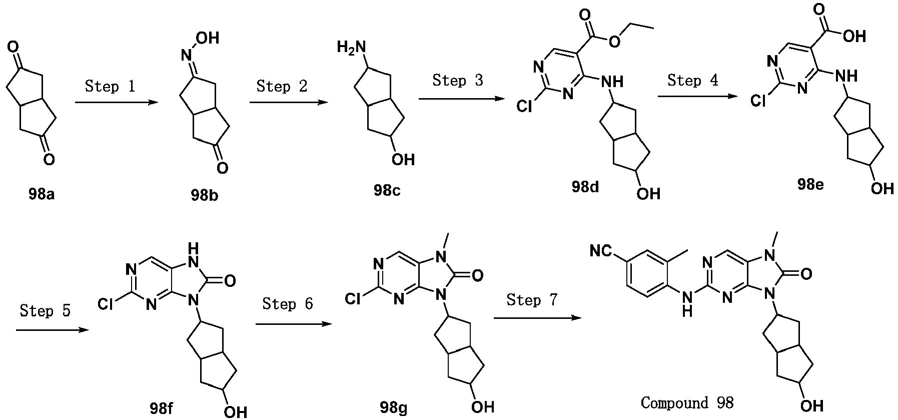

### Step 1:

### (5Z)-5-(hydroxyimino)octahydropentalen-2-one (98b)

Hydroxylamine hydrochloride (9.05 g, 130.28 mmol) and potassium carbonate (18.01, 130.28 mmol) were dissolved in 100 mL of water, and the resulting solution was added to a solution of octahydropentalene-2,5-dione **98a** (20 g, 144.76 mmol) in ethanol (200 mL). The reaction mixture was refluxed at 90 °C for 1 h. The reaction was monitored by TLC. After completion of the reaction, the reaction mixture was concentrated to remove ethanol, then diluted with 100 mL of water and extracted with ethyl acetate. The organic phases were combined, dried and filtered, and the filtrate was concentrated to get a crude product, which was then purified by column chromatography (dichloromethane:methanol = 100:1) to give the title compound 5-(hydroxyimino)hexahydropentalen-2(1H)-one **98b** (white solid, 7 g, 31.57% yield).

¹H NMR (400 MHz, DMSO-d6) δ 10.35 (s, 1H), 2.79-2.69 (m, 2H), 2.66-2.54 (m, 2H), 2.42-2.34 (m, 2H), 2.23-2.13 (m, 2H), 2.05-1.99 (m, 1H), 1.96-1.90 (m, 1H). LC-MS m/z(ESI) = **154.10** [M+1]

### Step 2:

### 5-Amino-octahydropentalen-2-ol (98c)

5-(hydroxyimino)hexahydropentalen-2(1H)-one **98b** (4 g, 26.11 mmol) was dissolved in tetrahydrofuran (100 mL), and cobalt chloride (12.42 g, 52.22 mmol) was added. The solution was cooled to -30 °C, and sodium borohydride (4.94 g, 130.55 mmol) was added in batches. The reaction mixture was stirred for 1 h with the temperature maintained at -25 °C to -30 °C throughout the reaction. The reaction was monitored by TLC. After completion of the reaction, saturated brine was added to the reaction mixture until no bubbles were formed. The reaction mixture was warmed to room temperature, stirred for another 0.5 h and filtered, and the filtrate was concentrated and dried to give the crude product 5-amino-octahydropentalen-2-ol **98c** (oily solid, 1 g, 27.12% yield).

LC-MS m/z(ESI) = **142.10** [M+1]

### Step 3:

### Ethyl 2-chloro-4-[(5-hydroxy-octahydropentalen-2-yl)amino]pyrimidine-5-carboxylate (98d)

Ethyl 2,4-dichloropyrimidine-5-carboxylate **1A** (2.0 g, 9.05 mmol) and 5-amino-octahydropentalen-2-ol **98c** (1 g, 7.08 mmol) were dissolved in acetonitrile (40 mL), and potassium carbonate (2.5 g, 18.10 mmol) was added in an ice bath. The reaction mixture was stirred at room temperature for 12 h. The reaction was monitored by TLC. After completion of the reaction, 50 mL water was added and the organics were extracted with ethyl acetate. The organic phases were combined, dried and concentrated, and the residue was purified by column chromatography (petroleum ether: acetate = 4:1) to obtain the title compound ethyl 2-chloro-4-[(5-hydroxy-octahydropentalen-2-yl)amino]pyrimidine-5-carboxylate **98d** (yellow solid, 1 g, 33.92% yield).

¹H NMR (400 MHz, Chloroform-d) δ 8.63 (s, 1H), 4.48-4.41 (m, 1H), 4.33 (q, 2H), 2.58 -2.46 (m, 2H), 2.42-2.36 (m, 2H), 2.11-2.05 (m, 2H), 1.57-1.48 (m, 4H), 1.37 (t, 3H).

LC-MS m/z(ESI) = **326.10** [M+1]

### Step 4:

### 2-Chloro-4-[(5-hydroxy-octahydropentalen-2-yl)amino]pyrimidine-5-carboxylic acid (98e)

Ethyl 2-chloro-4-[(5-hydroxy-octahydropentalen-2-yl)amino]pyrimidine-5-carboxylate **98d** (1 g, 3.07 mmol) was dissolved in 10 mL of tetrahydrofuran and 10 mL of water, and lithium hydroxide (0.15 g, 6.14 mmol) was added. The reaction mixture was stirred at room temperature for 1 h. The reaction was monitored by TLC. After completion of the reaction, the reaction mixture was concentrated to remove tetrahydrofuran and the pH was adjusted to 5 with 6 N hydrochloric acid, and a solid was precipitated. The mixture was filtered, and the filter cake was washed twice with petroleum ether and collected to obtain the title compound 2-chloro-4-[(5-hydroxy-octahydropentalen-2-yl)amino]pyrimidine-5-carboxylic acid **98e** (white solid, 0.57 g, 62.36% yield).

¹H NMR (400 MHz, DMSO-d6) δ 13.77 (s, 1H), 8.59 (d, 1H), 8.56 (s, 1H), 4.55 (s, 1H), 4.29-4.22 (m, 1H), 4.12-4.10 (m, 1H), 2.41-2.37 (m, 2H), 2.28-2.22 (m, 2H), 1.89-1.83 (m, 2H), 1.53-1.42 (m, 4H).

LC-MS m/z(ESI) = **298.10** [M+1]

### Step 5:

### 2-Chloro-9-(5-hydroxy-octahydropentalen-2-yl)-8,9-dihydro-7H-purin-8-one (98f)

2-Chloro-4-[(5-hydroxy-octahydropentalen-2-yl)amino]pyrimidine-5-carboxylic acid **98e** (0.57 g, 1.91 mmol) was dissolved in *N*,*N*-dimethylacetamide (20 mL), and diphenyl azophosphate (0.74 g, 1.91 mmol) and triethylamine (0.19 g, 1.91 mmol) were added in an ice bath. The reaction mixture was stirred at room temperature for 1 h, heated to 90 °C and stirred for another 3 h. The reaction was monitored by TLC. After completion of the reaction, the reaction mixture was naturally cooled to room temperature, diluted with 40 mL of water and extracted with ethyl acetate. The organic phases were combined, dried and concentrated to obtain the title compound 2-chloro-9-(5 -hydroxy-octahydropental en-2-yl)- 8,9-dihydro-7*H*-purin-8 -one **98f** (yellow solid, 0.5 g, 54.65% yield).

¹H NMR (400 MHz, DMSO-d6) δ 8.11 (s, 1H), 4.67 (d, 1H), 4.58-4.49 (m, 1H), 4.05 (m, 1H), 2.40-2.24 (m, 4H), 2.06-2.01 (m, 4H), 1.39-1.33 (m, 2H).

LC-MS m/z(ESI) = **295.10** [M+1]

### Step 6:

### 2-Chloro-9-(5-hydroxy-octahydropentalen-2-yl)-7-methyl-8,9-dihydro-7H-purin-8-one (98g)

2-Chloro-9-(5-hydroxy-octahydropentalen-2-yl)-8,9-dihydro-7*H*-purin-8-one **98f** (0.3 g, 1.02 mmol) was dissolved in dimethylformamide (10 mL), and cesium carbonate (0.66 g, 2.04 mmol) and dimethyl sulfate (0.13 g, 1.02 mmol) were added at 0 °C. The reaction mixture was stirred at room temperature for 1 h. The reaction was monitored by TLC. After completion of the reaction, 20 mL of water was added, and a solid was precipitated. The solid was collected by filtration and dried to give the title compound 2-chloro-9-(5-hydroxy-octahydropentalen-2-yl)-7-methyl-8,9-dihydro-7H-purin-8-one **98g** (pale yellow solid, 0.14 g, 44.45% yield).

¹H NMR (400 MHz, DMSO-d6) δ 8.34 (s, 1H), 4.63 (d, 1H), 4.60-4.52 (m, 1H), 4.10-4.01 (m, 1H), 3.34 (s, 3H), 2.40-2.24 (m, 4H), 2.07-1.99 (m, 4H), 1.40-1.33 (m, 2H). LC-MS m/z(ESI) = **309.10** [M+1]

### Step 7:

### 4-[(9-(5-Hydroxy-octahydropentalen-2-yl)-7-methyl-8-oxo-8,9-dihydro-7H-purin-2-yl)amino]-3-methylbenzonitrile (compound 98)

4-Amino-3-methylbenzonitrile **5a** (50 mg, 0.38 mmol), 2-chloro-9-(5-hydroxy-octahydropentalen-2-yl)-7-methyl-8,9-dihydro-7*H*-purin-8-one **98g** (117 mg, 0.38 mmol), cesium carbonate (248 mg, 0.76 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (34.45 mg, 0.04 mmol) were dissolved in dioxane (5 mL), followed by nitrogen purging. The reaction mixture was stirred under nitrogen atmosphere at 100 °C for 4 h. The reaction was monitored by TLC. After completion of the reaction, the reaction mixture was poured into ice water, and the solid was collected and purified by silica gel column chromatography (dichloromethane/methanol (v/v) = 100/1) to obtain the title compound 4-[(9-(5-hydroxy-octahydropentalen-2-yl)-7-methyl-8-oxo-8,9-dihydro-7*H*-purin-2-yl)amino]-3-methylbenzonitrile **compound 98** (white solid, 60 mg, 38.12% yield).

¹H NMR (400 MHz, DMSO-d6) δ 8.50 (s, 1H), 8.27 (d, 1H), 8.20 (s, 1H), 7.73 (dd, 1H),7.62 (d, 1H), 4.82 (d, 1H), 4.63-4.55 (m, 1H), 4.13-4.04 (m, 1H), 3.34 (s, 3H), 2.33-2.29 (m, 7H), 2.09-2.02 (m, 4H), 1.34-1.30 (m, 2H).

LC-MS m/z(ESI) = **405.20** [M+1]

### Example 99

### 2-Fluoro-4-((9-((3aS,6aS)-hexahydro-1H-cyclopenta[c]furan-5-yl)-7-methyl-8-oxo-8,9-dihydro-7H-purin-2-yl)amino)-5-methylbenzamide (compound 99)

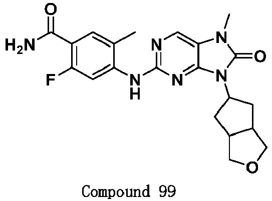

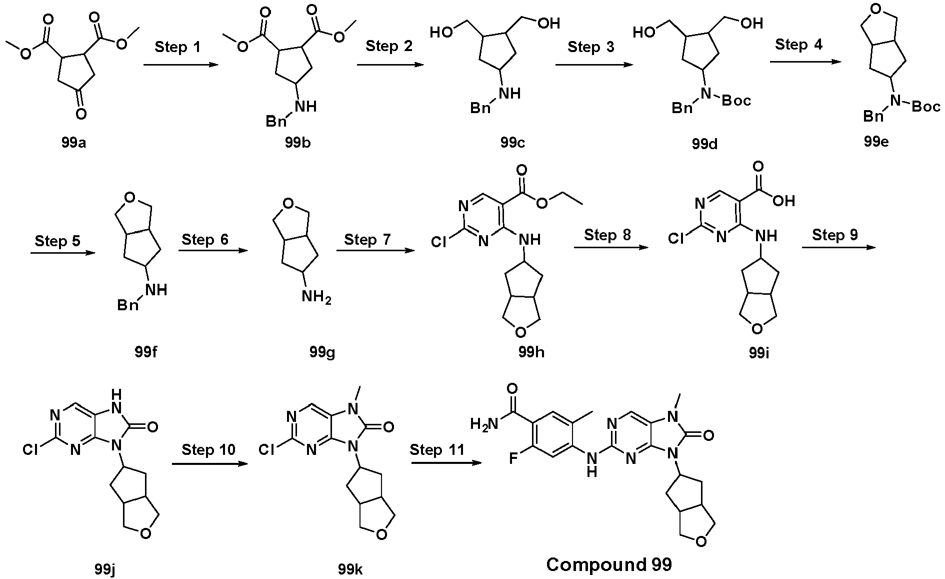

### Step 1:

### Dimethyl 4-(benzylamino)cyclopentane-1,2-dicarboxylate (99b)

Dimethyl 4-oxocyclopentane-1,2-dicarboxylate **99a** (5 g, 24.98 mmol) was dissolved in methanol (50 mL), and benzylamine (5.4 g, 49.96 mmol) was added, followed by the addition of drops of acetic acid. The reaction mixture was stirred at 40 °C for 0.5 h. After an intermediate was detected, sodium triacetyl borohydride (15.88 g, 74.94 mmol) was added at low temperature. The reaction mixture was stirred for another hour. The reaction was monitored by TLC. After completion of the reaction, the reaction mixture was filtered, and the filtrate was concentrated and extracted with water and dichloromethane. The organic phase was dried over anhydrous sodium sulfate and concentrated, and the residue was purified by column chromatography to give the title compound dimethyl 4-(benzylamino)cyclopentane-1,2-dicarboxylate **99b** (white solid, 6.0 g, 82.44% yield).

¹H NMR (400 MHz, CDCl3) δ 7.24 -7.15 (m, 5H), 3.68 (s, 2H), 3.62 (s, 6H), 3.37-3.27 (m, 2H), 3.13-3.06 (m, 1H), 2.20 (dd, 1H), 1.97 (t, 1H), 1.86 (dd, 1H), 1.77-1.70 (m, 1H).

LC-MS m/z(ESI) = **292.10** [M+1]

### Step 2:

### (4-(Benzylamino)cyclopentane-1,2-diyl)dimethanol (99c)

Dimethyl 4-(benzylamino)cyclopentane-1,2-dicarboxylate **99b** (2.0 g, 6.86 mmol) was dissolved in tetrahydrofuran (40 mL), and lithium aluminum hydride (0.63 g, 17.15 mmol) was added in batches at 0 °C. The reaction was monitored by TLC. After completion of the reaction, the lithium aluminum hydride was quenched with 10% aqueous sodium hydroxide, and the reaction mixture was filtered. The filtrate was directly concentrated to obtain the title compound (4-(benzylamino)cyclopentane-1,2-diyl)dimethanol **99c** (white solid, 1.1 g, 68.32% yield).

LC-MS m/z(ESI) = **236.20** [M+1]

### Step 3:

### tert-Butyl benzyl(3,4-bis(hydroxymethyl)cyclopentyl)carbamate (99d)

(4-(Benzylamino)cyclopentane-1,2-diyl)dimethanol **99c** (8 g, 34.0 mmol) was dissolved in methanol (50 mL), and triethylamine (3.64 g, 35.97 mmol) was added, followed by the addition of di-*tert*-butyl dicarbonate (8.16 g, 37.40 mmol). The reaction mixture was stirred for 1 h. The reaction was monitored by TLC. After completion of the reaction, the reaction mixture was concentrated, and the residue was purified by silica gel column chromatography (dichloromethane/methanol (v/v) = 50:1) to obtain the title compound *tert*-butyl benzyl *tert*-butyl benzyl(3,4-bis(hydroxymethyl)cyclopentyl)carbamate **99d** (white solid, 7.18 g, 63% yield).

¹H NMR (400 MHz, Chloroform-d) δ 7.32-7.16 (m, 5H), 4.37 (s, 2H), 3.72-3.61 (m,1H), 3.38-3.32 (m, 2H), 1.90-1.75 (m, 4H), 1.47-1.44 (m, 3H), 1.38 (s, 9H). LC-MS m/z(ESI) = **236.10** [M+1]

### Step 4:

### tert-Butyl benzyl (hexahydro-1H-cyclopenta[c]furan-5-yl)carbamate (99e)

*tert*-Butyl benzyl (3,4-bis(hydroxymethyl)cyclopentyl)carbamate **99d** (8 g, 23.85 mmol) was dissolved in tetrahydrofuran (100 mL), and *n*-butyllithium (10.7 mL, 26.75 mmol, 2.5 mol/L) was added dropwise at -20 °C under nitrogen atmosphere. The reaction mixture was stirred for 1 h, and a solution of *p*-toluenesulfonyl chloride (5 g, 26.23 mmol) in tetrahydrofuran (10 mL) was added dropwise. The reaction mixture was stirred for 0.5 h, and n-butyllithium (10.7 mL, 26.75 mmol, 2.5 mol/L) was added dropwise again. The reaction mixture was stirred for 10 min and heated at reflux for 48 h. The reaction was monitored by TLC. After completion of the reaction, the reaction mixture was quenched with ice water and extracted three times with ethyl acetate. The organic phase was dried over anhydrous sodium sulfate and concentrated, and the residue was purified by silica gel column chromatography (petroleum ether/ethyl acetate (v/v) = 5:1) to obtain the title compound *tert*-butyl benzyl(hexahydro-1*H-*cyclopenta[*c*]furan-5-yl)carbamate **99e** (white solid, 4.0 g, 52.84% yield).

¹H NMR (400 MHz, CDCl3) δ 7.32-7.17 (m, 5H), 4.37 (s, 2H), 3.73-3.63 (m, 2H), 3.40-3.34 (m, 2H), 2.06-1.25 (m, 16H).

LC-MS m/z(ESI) = **262**.**10** [M+1]

### Step 5:

### N-Benzylhexahydro-1H-cyclopenta[c]furan-5-amine (99f)

*tert*-Butyl benzyl(hexahydro-1*H*-cyclopenta[*c*]furan-5-yl)carbamate **99e** (4.0 g, 12.60 mmol) was dissolved in dichloromethane (10 mL), and a solvent mixture of dichloromethane/trifluoroacetic acid (v/v = 1:1) was added. The reaction mixture was stirred at room temperature for 1.5 h. The reaction was monitored by TLC. After completion of the reaction, the reaction mixture was quenched with water, and the aqueous phase was adjusted to be slightly basic with sodium bicarbonate. Dichloromethane was added for three extractions, and the organic phase was dried over anhydrous sodium sulfate and concentrated to give the title compound N-benzylhexahydro-1*H*-cyclopenta[*c*]furan-5-amine **99f** (white solid, 1.1 g, 40.1% yield). LC-MS m/z(ESI) = **218.10** [M+1]

### Step 6:

### Hexahydro-1H-cyclopenta[c]furan-5-amine (99g)

N benzylhexahydro-1*H*-cyclopenta[c]furan-5-amine **99f** (1.2 g, 5.52 mmol) was dissolved in ethanol (35 mL), and then 2 drops of acetic acid were added, followed by the addition of palladium hydroxide (1.2 g, 8.54 mmol). The system was purged with hydrogen, and the reaction mixture was stirred at room temperature for 5 h. The reaction was monitored by TLC. After completion of the reaction, the reaction mixture was concentrated, and the residue was purified by column chromatography to yield the title compound hexahydro-1*H*-cyclopenta[*c*]furan-5-amine **99g** (white solid, 613 mg, 87.6% yield).

¹H NMR (400 MHz, DMSO-d6) δ 4.01-4.06 (m, 1H), 3.72 (t, 2H), 3.13-3.48 (m, 4H), 2.44-2.48 (m, 1H), 1.96-2.17 (m, 2H), 1.97-1.72 (m, 1H), 1.23-1.37 (m, 1H). LC-MS m/z(ESI) = **128.10** [M+1]

### Step 7:

### Ethyl 2-chloro-4-((hexahydro-1H-cyclopenta[c]furan-5-yl)amino)pyrimidine-5-carboxylate (99h)

Ethyl 2,4-dichloropyrimidine-5-carboxylate **1A** (435 mg, 1.97 mmol) and potassium carbonate (816.8 mg, 5.91 mmol) were dissolved in acetonitrile (20 mL), and hexahydro-1*H*-cyclopenta[*c*]furan-5-amine **99g** (300 mg, 2.36 mmol) was added at 0 °C. The reaction mixture was warmed to room temperature and stirred for 20 h. The reaction was monitored by TLC. After completion of the reaction, water was added and the organics were extracted three times with ethyl acetate. The organic layer was washed once with saturated brine, dried over anhydrous sodium sulfate and concentrated, and the residue was purified by silica gel column chromatography (petroleum ether/ethyl acetate (v/v) = 10:1) to obtain the title compound ethyl 2-chloro-4-((hexahydro-1*H*-cyclopenta[*c*]furan-5-yl)amino)pyrimidine-5-carboxylate **99h** (white solid, 413 mg, 67.7% yield).

LC-MS m/z(ESI) = **312.10** [M+1]

### Step 8:

### 2-Chloro-4-((hexahydro-1H-cyclopenta[c]furan-5-yl)amino)pyrimidine-5-carboxylic acid (99i)

Ethyl 2-chloro-4-((hexahydro-1*H*-cyclopenta[*c*]furan-5-yl)amino)pyrimidine-5-carboxylate **99h** (680 mg, 2.18 mmol) was dissolved in 5 mL of tetrahydrofuran and 5 mL of water, and lithium hydroxide (104.38 mg, 4.36 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 4, and a white solid was precipitated. The mixture was filtered, and the filter cake was washed twice with petroleum ether/ethyl acetate (v/v = 10/1) and concentrated to obtain the title compound 2-chloro-4-((hexahydro-1*H*-cyclopenta[*c*]furan-5-yl)amino)pyrimidine-5-carboxylic acid **99i** (white solid, 520 mg, 67.26% yield).

LC-MS m/z(ESI) = **284.10** [M+1]

### Step 9:

### 2-Chloro-9-(hexahydro-1H-cyclopenta[c]furan-5-yl)-7,9-dihydro-8H-purin-8-one (99j)

2-Chloro-4-((hexahydro-1*H*-cyclopenta[*c*]furan-5-yl)amino)pyrimidine-5-carboxylic acid **99i** (0.5 g, 1.76 mmol) was dissolved in *N*,*N*-dimethylacetamide (20 mL), and diphenylazophosphate (0.68 g, 1.76 mmol) and triethylamine (0.18 g, 1.76 mmol) were added in an ice bath. The reaction mixture was stirred at room temperature for 1 h, then heatedto 90 °C and stirred for another 3 h. The reaction was monitored by TLC. After completion of the reaction, the reaction mixture was naturally cooled to room temperature, and 40 mL water was added and the organics were extracted with ethyl acetate. The organic phases were combined, dried and concentrated to yield the title compound 2-chloro-9-(hexahydro-1*H*-cyclopenta[*c*]furan-5-yl)-7,9-dihydro-8*H*-purin-8-one **99j** (yellow solid, 0.45 g, 54.65% yield).

LC-MS m/z(ESI) = **281.00** [M+1]

### Step 10:

### 2-Chloro-9-(hexahydro-1H-cyclopenta[c]furan-5-yl)-7-methyl-7,9-dihydro-8H-purin-8-one (99k)

2-Chloro-9-(hexahydro-1*H*-cyclopenta[*c*]furan-5-yl)-7,9-dihydro-8*H*-purin-8-one **99j** (0.45 g, 0.96 mmol) was dissolved in dimethylformamide (10 mL), and cesium carbonate (0.47 g, 1.44 mmol) and dimethyl sulfate (0.11 mL, 1.15 mmol) were added at 0 °C. The reaction mixture was stirred at room temperature for 2 h. The reaction was monitored by TLC. After completion of the reaction, then 20 mL of water was added, and a solid was precipitated. The solid was collected by filtration and dried to yield the title compound 2-chloro-9-(hexahydro-1*H*-cyclopenta[*c*]furan-5-yl)-7-methyl-7,9-dihydro-8*H*-purin-8-one **99k** (pale yellow solid, 0.14 g, 49.48% yield).

¹H NMR (400 MHz, DMSO-d6) δ 8.35 (s, 1H), 5.49-5.21 (m, 1H), 3.82-3.77 (m, 2H), 3.37 (s, 3H), 3.35-3.27 (m, 2H), 3.23-3.18 (m, 2H), 2.96-2.84 (m, 1H), 2.15-2.03 (m, 2H), 1.94-1.85 (m, 2H).

LC-MS m/z(ESI) = **295.10** [M+1]

### Step 11:

### 2-Fluoro-4-((9-((3aS,6aS)-hexahydro-1H-cyclopenta[c]furan-5-yl)-7-methyl-8-oxo-8,9-dihydro-7H-purin-2-yl)amino)-5-methylbenzamide (compound 99)

2-Chloro-9-((3a*S*,6a*S*)-hexahydro-1*H*-cyclopenta[*c*]furan-5-yl)-7-methyl-7,9-dihydro-8H-purin-8-one **99k** (100 mg, 0.34 mmol), 4-amino-2-fluoro-5-methylbenzamide **intermediate 2** (114 mg, 0.68 mmol), cesium carbonate (221 mg, 0.68 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (30 mg, 0.03 mmol) were dissolved in 1,4-dioxane (10 mL), followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 10/1) to obtain the title compound 2-fluoro-4-((9-((3a*S*,6a*S*)-hexahydro-1*H*-cyclopenta[*c*]furan-5-yl)-7-methyl-8-oxo-8,9-dihydro-7*H-*purin-2-yl)amino)-5-methylbenzamide **compound 99** (white solid, 8 mg, 6% yield). ¹H NMR (400 MHz, DMSO-d6) δ 8.60 (s, 1H), 8.18 (s, 1H), 7.64 (d, 1H), 7.54 (d, 1H), 7.48 (s, 1H), 7.36 (s, 1H), 3.74 (t, 1H), 3.68 (t, 1H), 3.32 (s, 3H), 3.22-3.13 (m, 3H), 2.81-2.71 (m, 1H), 2.25 (s, 3H), 2.03-1.99 (m, 2H), 1.92-1.84 (m, 3H).

LC-MS m/z(ESI) = 427.2 [M+1]

### Example 100

### 4-((9-(Hexahydro-1H-cyclopenta[c]furan-5-yl)-7-methyl-8-oxo-8,9-dihydro-7H-purin-2-yl)amino)-3-methylbenzonitrile (compound 100)

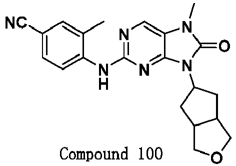

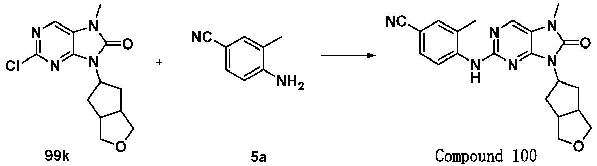

4-Amino-3-methylbenzonitrile **5a** (50 mg, 0.38 mmol), 2-chloro-9-(hexahydro-1*H-*cyclopenta[*c*]furan-5-yl)-7-methyl-7,9-dihydro-8*H*-purin-8-one **99k** (112 mg, 0.38 mmol), cesium carbonate (247.62 mg, 0.76 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (34.45 mg, 0.04 mmol) were dissolved in dioxane (10 mL), followed by nitrogen purging. The reaction mixture was stirred under nitrogen atmosphere at 100 °C for 4 h. The reaction was monitored by TLC. After completion of the reaction, the reaction mixture was poured into ice water, and the solid was collected and purified by silica gel column chromatography (dichloromethane/methanol (v/v) = 100/1) to obtain the title compound 4-((9-(hexahydro-1*H*-cyclopenta[*c*]furan-5-yl)-7-methyl-8-oxo--7-methyl-8-oxohydro-7*H-*purin-2-yl)amino)-3-methylbenzonitrile **compound 100** (pale yellow solid, 45 mg, 30.09% yield).

¹H NMR (400 MHz, Chloroform-*d*) δ 8.43 (d, 1H), 7.93 (s, 1H), 7.53 (dd, 1H), 7.48 (d, 1H), 7.05 (s, 1H), 5.47-5.40 (m, 1H), 3.96 (q, 2H), 3.48 (dd, 1H), 3.43 (s, 3H), 3.37 (dd, 1H), 5.38-5.31 (m, 1H), 2.38 (s, 3H), 2.92- 2.21 (m, 5H).

LC-MS m/z(ESI) = **391.20** [M+1]

### Example 101

### 4-((9-(1,1-Dioxidotetrahydro-2H-thiopyran-4-yl)-7-methyl-8-oxo-8,9-dihydro-7H-purin-2-yl)amino)-3-methylbenzonitrile (compound 101)

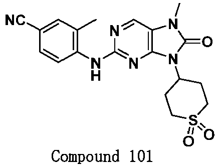

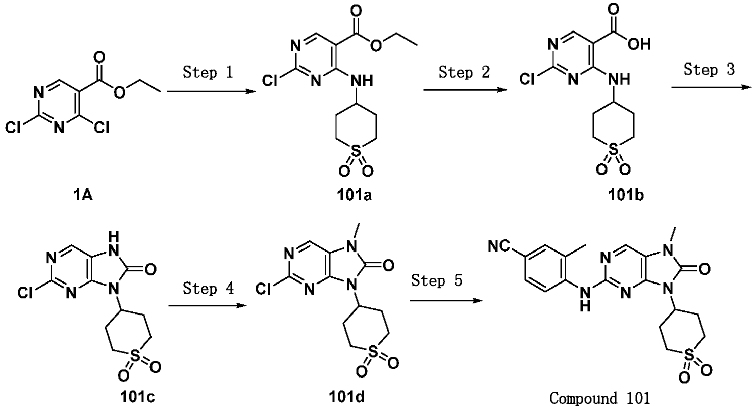

### Step 1:

### Ethyl 2-chloro-4-((1,1-dioxidotetrahydro-2H-thiopyran-4-yl)amino)pyrimidine-5-carboxylate (101a)

Ethyl 2,4-dichloropyrimidine-5-carboxylate **1A** (8 g, 36 mmol) and potassium carbonate (9.9 g, 72 mmol) were dissolved in acetonitrile (30 mL), and 4-aminotetrahydro-2*H*-thiopyran-1,1-dioxide (6.6 g, 36 mmol) was added at 0 °C. The reaction mixture was warmed to room temperature and stirred for 20 h. The reaction was monitored by TLC. After completion of the reaction, 30 mL of water was added to the reaction mixture, and a solid was precipitated. The solid was collected by filtration and dried to obtain the title compound ethyl 2-chloro-4-((1,1-dioxidotetrahydro-2*H-*thiopyran-4-yl)amino)pyrimidine-5-carboxylate **101a** (brown solid, 6.3 g, 76% yield). ¹H NMR (400 MHz DMSO) δ 8.64 (s, 1H), 8.33 (d, 1H), 4.34-4.30 (m,3H), 3.42-3.35 (m, 1H), 3.08 (d, 1H), 2.24-2.06 (m, 4H), 1.31(t, 3H).

LC-MS m/z(ESI) = **334.20** [M+1]

### Step 2:

### 2-Chloro-4-((1,1-dioxidotetrahydro-2H-thiopyran-4-yl)amino)pyrimidine-5-carboxylic acid (101b)

Ethyl 2-chloro-4-((1,1-dioxidotetrahydro-2*H*-thiopyran-4-yl)amino)pyrimidine-5-carboxylate **101a** (3 g, 9 mmol) was dissolved in 10 mL of tetrahydrofuran and 5 mL of water, and lithium hydroxide (756 mg, 18 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to about 4, and a brown solid was precipitated. The mixture was filtered, and the filter cake was washed twice with petroleum ether/ethyl acetate (v/v = 10/1), concentrated and dried to obtain the title compound 2-chloro-4-((1,1-dioxidotetrahydro-2*H*-thiopyran-4-yl)amino)pyrimidine-5-carboxylic acid **101b** (white solid, 2.3 g, 84% yield).

¹H NMR (400 MHz DMSO) δ 13.80 (s, 1H), 8.60 (s, 1H), 8.57 (d,1H) , 4.37-4.29 (m, 1H), 3.50-3.27 (m, 2H), 3.07 (d, 2H), 2.25-2.21 (m, 2H), 2.08-1.98 (m, 2H).

LC-MS m/z(ESI) = **306.20** [M+1]

### Step 3:

### 2-Chloro-9-(1,1-dioxidotetrahydro-2H-thiopyran-4-yl)-7,9-dihydro-8H-purin-8-one (101c)

2-Chloro-4-((1,1-dioxidotetrahydro-2*H*-thiopyran-4-yl)amino)pyrimidine-5-carboxylic acid **101b** (2.3 g, 7.5 mmol) was dissolved in dimethylacetamide (10 mL), and triethylamine (757 mg, 7.5 mmol) and diphenylphosphoryl azide (2 g, 7.5 mmol) were added. The reaction mixture was gradually heated to 120 °C and stirred for 1.5 h. The reaction was monitored by TLC. After completion of the reaction, the reaction mixture was poured into 30 mL of water, and a brown solid was precipitated. The solid was collected by filtration to obtain the title compound 2-chloro-9-(1,1-dioxidotetrahydro-2H-thiopyran-4-yl)-7,9-dihydro-8H-purin-8-one **101c** (white solid, 1.7 g, 58% yield).

¹H NMR (400 MHz DMSO) δ 11.67 (s, 1H), 8.14 (s, 1H), 4.69-4.61 (m,1H), 3.55-3.45 (m, 2H), 3.13 (d, 2H), 2.94-2.77 (m, 2H), 2.14-2.02 (m, 2H).

LC-MS m/z(ESI) = **303.20** [M+1]

### Step 4:

### 2-Chloro-9-(1,1-dioxidotetrahydro-2H-thiopyran-4-yl)-7-methyl-7,9-dihydro-8H-purin-8-one (101d)

2-Chloro-9-(1,1-dioxidotetrahydro-2*H*-thiopyran-4-yl)-7,9-dihydro-8*H*-purin-8-one **101c** (1.7 g, 5.6 mmol) was dissolved in dimethylformamide (10 mL), and dimethyl sulfate (709 mg, 5.6 mmol) and cesium carbonate (2.7 g, 8.4 mmol) were added at 0 °C. The reaction mixture was stirred for 1 h. The reaction was monitored by TLC. After completion of the reaction, 10 mL of water was added, and a solid was precipitated. The solid was collected by filtration to obtain the title compound 2-chloro-9-(1,1-dioxidotetrahydro-2H-thiopyran-4-yl)-7-methyl-7,9-dihydro-8H-purin-8-one **101d** (white solid, 1.3 g, 82% yield).

¹H NMR (400 MHz DMSO) δ 8.33 (s, 1H), 4.26 (s, 1H), 3.33 (s, 3H), 2.99 (s, 2H), 2.11-2.04 (m, 2H), 1.80-1.61 (m, 2H), 1.44 (d, 2H).

LC-MS m/z(ESI) = **317.20** [M+1]

### Step 5:

### 4-((9-(1,1-Dioxidotetrahydro-2H-thiopyran-4-yl)-7-methyl-8-oxo-8,9-dihydro-7H-purin-2-yl)amino)-3-methylbenzonitrile (compound 101)

2-Chloro-9-(1,1-dioxidotetrahydro-2H-thiopyran-4-yl)-7-methyl-7,9-dihydro-8H-purin-8-one **101d** (200 mg, 0.6 mmol), 4-amino-3-methylbenzonitrile (83.5 mg, 0.6 mmol), cesium carbonate (587 mg, 1.8 mmol) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (55 mg, 0.06 mmol) were dissolved in dioxane (3 mL), followed by nitrogen purging. The reaction mixture was stirred under nitrogen atmosphere at 100 °C for 4 h. The reaction was monitored by TLC. After completion of the reaction, the reaction mixture was concentrated, and the residue was purified by silica gel column chromatography (dichloromethane/methanol (v/v) = 30/1) to obtain the title compound 4-((9-(1,1-dioxidotetrahydro-2H-thiopyran-4-yl)-7-methyl-8-oxo-8,9-dihydro-7H-purin-2-yl)amino)-3-methylbenzonitrile compound 101 (white solid, 25 mg, 26% yield).

¹H NMR (400 MHz DMSO) δ 8.57 (s, 1H), 8.32 (d, 1H), 8.23 (s, 1H), 7.62-7.60 (m, 2H), 4.68-4.62 (m, 1H), 3.52-3.45 (m, 2H), 3.18-3.15 (m, 2H), 3.00-2.89 (m, 2H), 2.34 (s,3H), 2.08 (d, 2H).

LC-MS m/z(ESI) = **413.20** [M+1]

### Example 102

### 3-Methyl-4-((7-methyl-8-oxo-9-((tetrahydrofuran-3-yl)methyl)-8,9-dihydro-7H-purin-2-yl)amino)benzamide (compound 102)

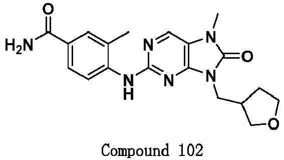

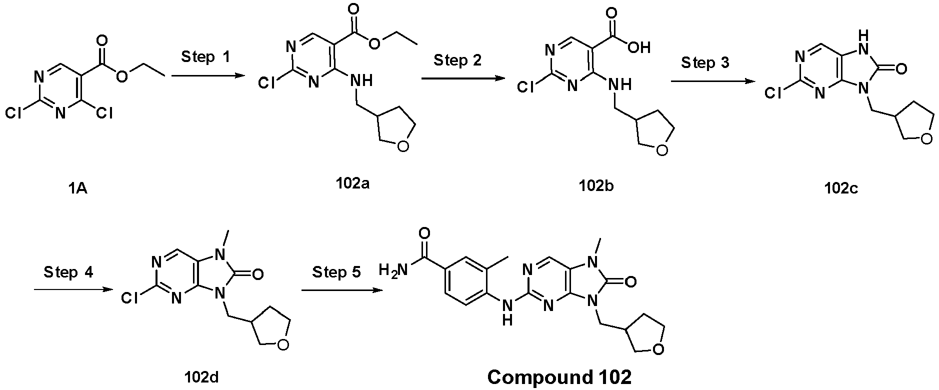

### Step 1:

### Ethyl 2-chloro-4-(((tetrahydrofuran-3-yl)methyl)amino)pyrimidine-5-carboxylate (102a)

Ethyl 2,4-dichloropyrimidine-5-carboxylate **1A** (5 g, 22.6 mmol) and potassium carbonate (6.2 g, 44.8 mmol) were dissolved in acetonitrile (20 mL), and (tetrahydrofuran-3-yl)formamide (2.3 g, 22.6 mmol) was added at 0 °C. The reaction mixture was warmed to room temperature and stirred for 20 h. The reaction was monitored by TLC. After completion of the reaction, 30 mL of water was added to the reaction mixture, and the resulting mixture was extracted with ethyl acetate. The organic phase was washed once with saturated brine, dried over anhydrous sodium sulfate, and purified by silica gel column chromatography (*n*-hexane/ethyl acetate (v/v) = 10:1) to obtain the title compound ethyl 2-chloro-4-(((tetrahydrofuran-3-yl)methyl)amino)pyrimidine-5-carboxylate **102a** (white solid, 4.4 g, 85% yield).

¹H NMR (400 MHz DMSO) δ 8.58-8.61 (m, 2H), 4.31 (q, 2H), 3.78-3.73 (m,1H), 3.69-3.58 (m, 2H), 3.48-3.44 (m, 3H), 2.57-2.53 (m, 1H), 1.98-1.91 (m, 1H), 1.62-1.58 (m, 1H), 1.31 (t, 3H).

LC-MS m/z(ESI) = **286.20** [M+1]

### Step 2:

### 2-Chloro-4-(((tetrahydrofuran-3-yl)methyl)amino)pyrimidine-5-carboxylic acid (102b)

Ethyl 2-chloro-4-(((tetrahydrofuran-3-yl)methyl)amino)pyrimidine-5-carboxylate **102a** (4.4 g, 15.3 mmol) was dissolved in 10 mL of tetrahydrofuran and 5 mL of water, and lithium hydroxide (736 mg, 30.6 mmol) was added. The reaction mixture was stirred at room temperature for 1 h. The reaction was monitored by TLC. After completion of the reaction, the reaction mixture was concentrated by rotary evaporation under reduced pressure to remove tetrahydrofuran and the pH was adjusted to pH 4-5, and a white solid was precipitated. The mixture was filtered, and the filter cake was washed twice with petroleum ether/ethyl acetate (v/v = 10/1) and concentrated to obtain the title compound 2-chloro-4-(((tetrahydrofuran-3-yl)methyl)amino)pyrimidine-5-carboxylic acid **102b** (white solid, 3.4 g, 80% yield).

¹H NMR (400 MHz DMSO) δ 13.75 (s, 1H), 8.75 (s, 1H), 8.57 (s, 1H), 3.78-3.73 (m, 3H), 3.47-3.43 (m,3H), 2.58-2.52 (m, 1H), 1.98-1.89 (m, 3H), 1.63-1.55 (m, 1H). LC-MS m/z(ESI) = **259.20** [M+1]

### Step 3:

### 2-Chloro-9-((tetrahydrofuran-3-yl)methyl)-7,9-dihydro-8H-purin-8-one (102c)

2-Chloro-4-(((tetrahydrofuran-3-yl)methyl)amino)pyrimidine-5-carboxylic acid **102b** (3.4 g, 13.0 mmol) was dissolved in dimethylacetamide (20 mL), and triethylamine (1.58 g, 13 mmol) and diphenylphosphoryl azide (3.6 g, 13 mmol) were added. The reaction mixture was gradually heated to 120 °C and stirred for 1.5 h. The reaction was monitored by TLC. After completion of the reaction, the reaction mixture was concentrated, and the residue was purified by silica gel column chromatography (petroleum ether/ethyl acetate (v/v) = 5:1 to 1:10) to obtain the title compound 2-chloro-9-((tetrahydrofuran-3-yl)methyl)-7,9-dihydro-87/-purin-8-one **102c** (white solid, 1.46 g, 50% yield).

¹H NMR (400 MHz DMSO) δ 11.65 (s, 1H), 8.14 (s, 1H), 3.80-3.74 (m, 3H), 3.66-3.57 (m, 2H), 3.52-3.49 (m, 1H), 2.73-2.69 (m, 1H), 1.98-1.89 (m, 1H), 1.67-1.60 (m, 1H). LC-MS m/z(ESI) = **255.20** [M+1]

### Step 4:

### 2-Chloro-7-methyl-9-((tetrahydrofuran-3-yl)methyl)-7,9-dihydro-8H-purin-8-one (102d)

2-Chloro-9-((tetrahydrofuran-3-yl)methyl)-7,9-dihydro-8*H*-purin-8-one **102c** (1.6 g, 5.2 mmol) was dissolved in dimethylformamide (10 mL), and dimethyl sulfate (663 mg, 5.2 mmol) and cesium carbonate (2.4 g, 7.7 mmol) were added at 0 °C. The reaction mixture was stirred for 1 h. The reaction was monitored by TLC. After completion of the reaction, then 10 mL of water was added, and a solid was precipitated. The solid was collected by filtration to obtain the title compound 2-chloro-7-methyl-9-((tetrahydrofuran-3-yl)methyl)-7,9-dihydro-8*H*-purin-8-one **102d** (white solid, 1.2 g, 80% yield).

¹H NMR (400 MHz DMSO) δ 8.35 (s, 1H), 3.81-3.75 (m, 3H), 3.65-3.57 (m, 2H), 3.52-3.49 (m, 1H), 3.37 (s, 3H), 2.73-2.70 (m, 1H), 1.96-1.91 (m, 1H), 1.67-1.61 (m, 1H). LC-MS m/z(ESI) = **268.20** [M+1]

### Step 5:

### 3-Methyl-4-((7-methyl-8-oxo-9-((tetrahydrofuran-3-yl)methyl)-8,9-dihydro-7H-purin-2-yl)amino)benzamide (compound 102)

2-Chloro-7-methyl-9-((tetrahydrofuran-3-yl)methyl)-7,9-dihydro-8H-purin-8-one **102d** (300 mg, 1.12 mmol), 4-amino-3-methylbenzamide **2a** (250 mg, 1.68 mmol), cesium carbonate (730 mg, 2.24 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (100 mg, 0.11 mmol) were dissolved in 1,4-dioxane (10 mL), followed by nitrogen purging. The reaction mixture was stirred under nitrogen atmosphere at 100 °C for 4 h. The reaction was monitored by TLC. After completion of the reaction, the reaction mixture was poured into ice water, and the solid was collected and purified by silica gel column chromatography (dichloromethane/methanol (v/v) = 100:1 to 50:1) to obtain the title compound 3-methyl-4-((7-methyl-8-oxo-9-((tetrahydrofuran-3-yl)methyl)-8,9-dihydro-7H-purin-2-yl)amino)benzamide **compound 102** (pale yellow solid, 45 mg, 10.37% yield).

¹H NMR (400 MHz, DMSO-*d*₆) δ 10.21 (s, 1H), 8.50 (s, 1H), 8.10 (s, 1H), 7.83-7.64 (m, 4H), 7.16 (s, 1H), 3.78-3.74 (m, 3H), 3.69-3.59 (m, 2H), 3.55-3.52 (m, 1H), 3.32 (s, 3H), 2.81-2.71 (m, 1H), 2.29 (s, 3H), 1.96-1.88 (m, 1H), 1.69-1.61 (m, 1H). LC-MS m/z(ESI) = **383.20** [M+1].

### Example 103

### 9-Cyclohexyl-2-((4-methoxy-2-methylphenyl)amino)-7-methyl-7,9-dihydro-8H-purin-8-one (compound 103)

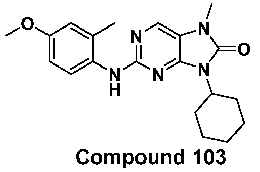

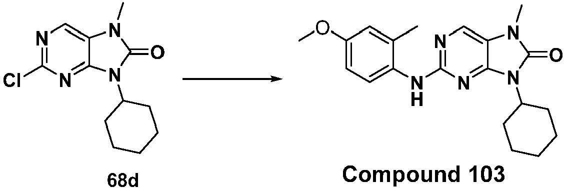

2-Chloro-9-cyclohexyl-7-methyl-7,9-dihydro-8*H*-purin-8-one **68d** (150 mg, 0.56 mmol), 4-methoxy-2-methylaniline (154 mg, 1.12 mmol), cesium carbonate (367 mg, 1.12 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (76 mg, 0.08 mmol) were dissolved in 1,4 dioxane (50 mL), followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 10/1) followed by Pre-HPLC to obtain the title compound 9-cyclohexyl-2-((4-methoxy-2-methylphenyl)amino)-7-methyl-7,9-dihydro-8*H*-purin-8-one **compound 103** (white solid, 53 mg, 26% yield).

¹H NMR (400 MHz, DMSO-d6) δ 8.26 (s, 1H), 7.94 (s, 1H), 7.30 (d, 1H), 6.78 (d, H), 6.71 (dd, 1H), 4.134.11 (m, 1H), 3.72 (s, 3H), 3.25 (s, 3H), 2.25-2.18, 2H), 2.17 (s, 3H), 1.79 (d, 2H), 1.65 (t, 3H), 1.35-1.22 (m, 2H), 1.11 (t, 1H).

LC-MS m/z(ESI) = 368.20 [M+1]

### Example 104

### 2-Fluoro-4-((9-(3-fluorotetrahydro-2H-pyran-4-yl)-7-methyl-8-oxo-8,9-dihydro-7H-purin-2-yl)amino)-5-methylbenzamide (compound 104)

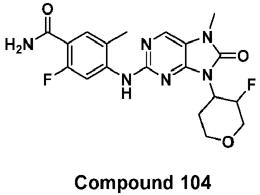

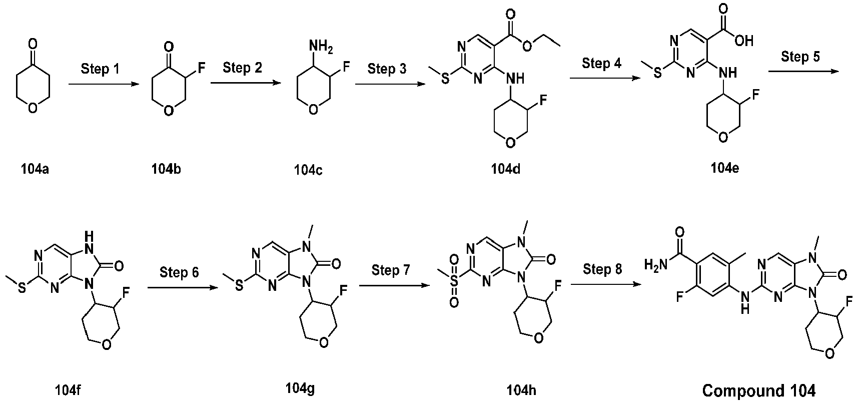

### Step 1:

### 3-Fluorotetrahydro-4H-pyran-4-one (104b)

Tetrahydro-4H-pyran-4-one **104a** (5 g, 49.94 mmol) and 1-chloromethyl-4-fluoro-1,4-diazoniabicyclo[2.2.2]octane bis(tetrafluoroborate) (21.23 g, 59.93 mmol) were dissolved in acetonitrile/water (20:1 mL). The reaction mixture was heated to 80 °C and stirred for 8 h. The reaction was monitored by TLC. After completion of the reaction, the reaction mixture was concentrated to evaporate acetonitrile, and water was added and the organics were extracted with ethyl acetate. The organic layer was concentrated, dried over anhydrous sodium sulfate, and purified by silica gel column chromatography (petroleum ether/ethyl acetate (v/v) = 1:1) to obtain the title compound 3-fluorotetrahydro-4*H*-pyran-4-one **104b** (colorless liquid, 4.4 g, 74.88% yield).

LC-MS m/z(ESI) = 119.10 [M+1]

### Step 2:

### 3-Fluorotetrahydro-2H-pyran-4-amine (104c)

3-Fluorotetrahydro-4*H*-pyran-4-one **104b** (1.4 g, 11.85 mmol) was dissolved in absolute methanol (10 mL), and ammonium formate (7.47 g, 118.53 mmol) was added with stirring at room temperature. The reaction mixture was stirred for 0.5 h, and then sodium cyanoborohydride (894 mg, 14.22 mmol) was added. The reaction mixture was stirred for 5 h. The reaction was monitored by TLC. After completion of the reaction, the reaction mixture was quenched with saturated sodium bicarbonate, concentrated to dryness by rotary evaporation under reduced pressure, filtered, and washed 3 times with methanol: dichloromethane (40:1). The filtrate was concentrated to yield the title compound 3-fluorotetrahydro-2*H*-pyran-4-amine **104c** (white solid, crude, 5.3 g).

LC-MS m/z(ESI) = 120.10 [M+1]

### Step 3:

### Ethyl 4-((3-fluorotetrahydro-2H-pyran-4-yl)amino)-2-(methylthio)pyrimidine-5-carboxylate (104d)

Ethyl 2,4-dichloropyrimidine-5-carboxylate 1A (3.3 g, 14.20 mmol) and potassium carbonate (4.9 g, 35.50 mmol) were dissolved in acetonitrile (20 mL), and 3-fluorotetrahydro-2*H*-pyran-4-amine 104c (1.41 g, 11.83 mmol) was added with stirring at room temperature. The reaction mixture was heated to 30 °C and stirred for 7 h. The reaction was monitored by TLC. After completion of the reaction, the reaction mixture was filtered, and the filter cake was washed with dichloromethane and ethyl acetate. The organic layer was concentrated, and the residue was purified by silica gel column chromatography (petroleum ether/ethyl acetate (v/v) = 10:1) to obtain the title compound ethyl 4-((3-fluorotetrahydro-2H-pyran-4-yl)amino)-2-(methylthio)pyrimidine-5-carboxylate **104d** (white solid, 1.85 g, 49.57% yield).

LC-MS m/z(ESI) = 316.10 [M+1]

### Step 4:

### 4-((3-Fluorotetrahydro-2H-pyran-4-yl)amino)-2-(methylthio)pyrimidine-5-carboxylic acid (104e)

Ethyl 4-((3-fluorotetrahydro-2*H*-pyran-4-yl)amino)-2-(methylthio)pyrimidine-5-carboxylate **104d** (1.85 g, 5.87 mmol) was dissolved in 10 mL of tetrahydrofuran and 10 mL of water, and sodium hydroxide (704 mg, 17.60 mmol) was added. The reaction mixture was stirred at room temperature for 2 h. The reaction was monitored by TLC. After completion of the reaction, the reaction mixture was concentrated under reduced pressure to remove tetrahydrofuran and the pH was adjusted to 3-4, and ethyl acetate was added for extraction. The organic phase was concentrated to yield the title compound 4-((3-fluorotetrahydro-2*H*-pyran-4-yl)amino)-2-(methylthio)pyrimidine-5-carboxylic acid **104e** (white solid, 1.73 g, 104.3% yield).

LC-MS m/z(ESI) = 288.10 [M+1]

### Step 5:

### 9-(3-Fluorotetrahydro-2H-pyran-4-yl)-2-(methylthio)-7,9-dihydro-8H-purin-8-one (104f)

4-((3-Fluorotetrahydro-2*H*-pyran-4-yl)amino)-2-(methylthio)pyrimidine-5-carboxylic acid **104e** (1.73 g, 6.02 mmol) was dissolved in dimethylacetamide (20 mL), and triethylamine (835 µL, 6.02 mmol) and diphenylphosphoryl azide (1.3 mL, 6.02 mmol) were added. The reaction mixture was stirred at room temperature for 1 h, then heated to 100 °C and stirred for 2 h. The reaction was monitored by TLC. After completion of the reaction, water was added to the reaction mixture, and a solid was precipitated. The mixture was filtered, and the filter cake was eluted with water and petroleum ether and concentrated under reduced pressure to give the title compound ethyl 4-(3-fluorotetrahydro-2*H*-pyran-4-yl)amino)-2-(methylthio)-pyrimidine-5-carboxylate **104f** (white solid, 1.27 g, 73.89% yield).

LC-MS m/z(ESI) = 285.10 [M+1]

### Step 6:

### 9-(3-Fluorotetrahydro-2H-pyran-4-yl)-7-methyl-2-(methylthio)-7,9-dihydro-8H-purin-8-one (104g)

Ethyl 4-(3-fluorotetrahydro-2*H*-pyran-4-yl)amino)-2-(methylthio)-pyrimidine-5-carboxylate **104f** (1.27 g, 4.45 mmol) was dissolved in dimethylformamide (10 mL), and dimethyl sulfate (433 µL, 4.45 mmol) and cesium carbonate (1.84 g, 13.35 mmol) were added at 0 °C. The reaction mixture was stirred for 1.5 h. The reaction was monitored by TLC. After completion of the reaction, then water was added and the organics were extracted with ethyl acetate, and the organic layer was concentrated to obtain the title compound 9-(3-fluorotetrahydro-2H-pyran-4-yl)-7-methyl-2-(methylthio)-7,9-dihydro-8H-purin-8-one **104g** (white solid, 800 mg, 60.27% yield).

LC-MS m/z(ESI) = 299.10 [M+1]

### Step 7:

### 9-(3-Fluorotetrahydro-2H-pyran-4-yl)-7-methyl-2-(methylsulfonyl)-7,9-dihydro-8H-purin-8-one (104h)

9-(3-Fluorotetrahydro-2*H*-pyran-4-yl)-7-methyl-2-(methylthio)-7,9-dihydro-8*H-*purin-8-one **104g** (800 mg, 2.68 mmol) was dissolved in dichloromethane (6 mL), and m-chloroperoxybenzoic acid (1.85 g, 10.73 mmol) was added with stirring at room temperature. The reaction mixture was stirred for 1 h. The reaction was monitored by TLC. After completion of the reaction, The reaction mixture was concentrated, and the residue was purified by silica gel column chromatography (dichloromethane/methanol (v/v) = 100:1 to 60:1) to obtain the title compound 9-(3-fluorotetrahydro-2*H*-pyran-4-yl)-7-methyl-2-(methylsulfonyl)-7,9-dihydro-8*H*-purin-8-one **104h** (white solid, 1.05 g, 119% yield).

LC-MS m/z(ESI) = 331.10 [M+1]

### Step 8:

### 2-Fluoro-4-((9-(3-fluorotetrahydro-2H-pyran-4-yl)-7-methyl-8-oxo-8,9-dihydro-7H-purin-2-yl)amino)-5-methylbenzamide (compound 104)

9-(3-Fluorotetrahydro-2*H*-pyran-4-yl)-7-methyl-2-(methylsulfonyl)-7,9-dihydro-8*H-*purin-8-one **104h** (300 mg, 0.91 mmol), 4-amino-3-methylbenzamide intermediate 2 (458 mg, 2.72 mmol) and potassium tert-butoxide (305 mg, 2.72 mmol) were dissolved in dimethylformamide (10 mL). The reaction mixture was stirred at room temperature for 1 h. The reaction was monitored by TLC. After completion of the reaction, water was added and the organics were extracted with ethyl acetate. The organic layer was concentrated, and the residue was purified by silica gel column chromatography (dichloromethane/ethyl acetate (v/v) = 5:1 to 1:1) to get the title compound 2-fluoro-4-((9-(3-fluorotetrahydro-2*H*-pyran-4-yl)-7-methyl-8-oxo-8,9-dihydro-7*H*-purin-2-yl)amino)-5-methylbenzamide compound **104-1** (pale yellow solid, 130 mg, 34.21% yield) and compound **104-2** (white solid, 85 mg, 22.37% yield).

### Compound 104-1:

¹H NMR (400 MHz, DMSO-d6) δ 8.62 (s, 1H), 8.23 (s, 1H), 7.84 (d, 1H), 7.54 (d, 1H), 7.46 (s, 1H), 7.40 (s, 1H), 5.46-5.37 (m, 0.5H), 5.34-5.25 (m, 0.5H), 4.63-4.51 (m, 1H), 4.23-4.16 (m, 1H), 3.97-3.90 (m, 1H), 3.54-3.45 (m, 1H), 3.41-3.34 (m, 4H), 2.60-2.53 (m, 1H), 2.29 (s, 3H), 1.97-1.89 (m, 1H).

LC-MS m/z(ESI) = 419.20 [M+1]

### Compound 104-2:

¹H NMR (400 MHz, DMSO-d6) δ 8.50 (s, 1H), 8.22 (s, 1H), 7.92 (d, 1H), 7.54 (d, 1H), 7.45 (s, 1H), 7.39 (s, 1H), 4.82 (s, 0.5H), 4.69 (s, 0.5H), 4.67-4.61 (m, 0.5H), 4.59-4.52 (m, 0.5H), 4.09-3.98 (m, 2H), 3.75-3.70 (m, 0.5H), 3.66-3.61 (m, 0.5H), 3.60-3.52 (m, 1H), 3.49-3.37 (m, 1H), 3.35 (s, 3H), 2.29 (s, 3H), 1.81 (dd, 1H).

LC-MS m/z(ESI) = 419.20 [M+1]

### Example 105

### 4-((7-(Ethyl-d5)-8-oxo-9-(tetrahydro-2H-pyran-4-yl)-8,9-dihydro-7H-purin-2-yl)amino)-2-fluoro-5-methylbenzamide (compound 105)

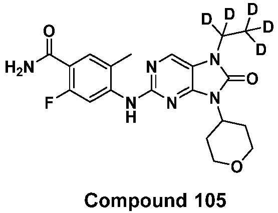

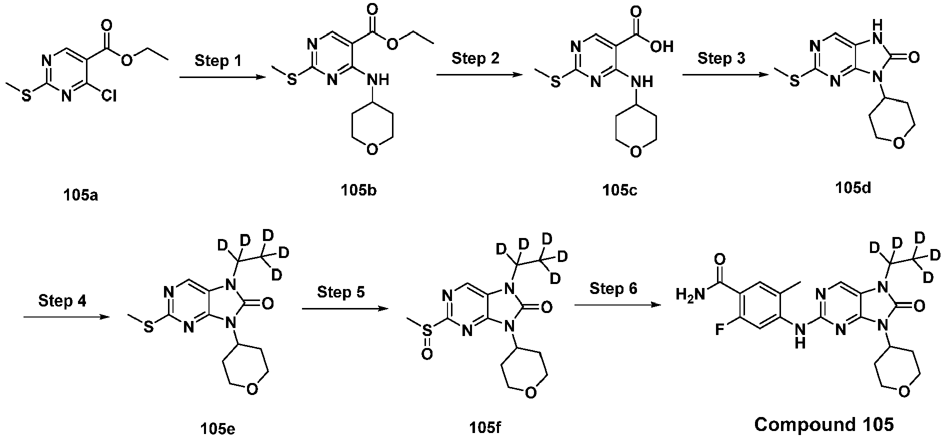

### Step 1:

### Ethyl 2-(methylthio)-4-((tetrahydro-2H-pyran-4-yl)amino)pyrimidine-5-carboxylate (105b)

Ethyl 4-chloro-2-(methylthio)pyrimidine-5-carboxylate **105a** (500 g, 2.15 mol) and tetrahydro-2*H*-pyran-4-amine hydrochloride (300 g, 2.19 mol) were dissolved in acetonitrile (3000 mL) and water (500 mL), and after the solution was stirred several times, potassium carbonate (600 g, 4.3 mol) was added. The reaction mixture was stirred at room temperature for 4 h. The reaction was monitored by TLC. After completion of the reaction, the reaction mixture was filtered, and the filter residue was washed with ethyl acetate (1000 mL). The filtrate was concentrated to get a crude product, which was then extracted with ethyl acetate and water. The organic phase was concentrated to obtain the title compound ethyl 2-(methylthio)-4-((tetrahydro-2H-pyran-4-yl)amino)pyrimidine-5-carboxylate **105b** (white solid, 642 g, 99.69% yield). ¹H NMR (400 MHz DMSO) δ 8.56 (s, 1H), 8.17 (d, 1H), 4.31-4.26 (m, 2H), 4.25-4.21 (m, 2H), 3.88-3.84 (m, 2H), 3.48-3.42 (m, 2H), 2.48 (s, 3H), 1.94-1.90 (m, 2H), 1.60-1.51 (m, 2H), 1.32-1.29 (m, 3H).

LC-MS m/z (ESI) = 298.10 [M+1]

### Step 2:

### 2-(Methylthio)-4-((tetrahydro-2H-pyran-4-yl)amino)pyrimidine-5-carboxylic acid (105c)

Ethyl 2-(methylthio)-4-((tetrahydro-2*H*-pyran-4-yl)amino)pyrimidine-5-carboxylate **105b** (640 g, 2.15 mol) was dissolved in tetrahydrofuran (1000 mL)/water (1200 mL)/methanol (200 mL), and sodium hydroxide (172.16 g, 4.30 mol) was added. The reaction mixture was stirred at room temperature for 2 h. The reaction was monitored by TLC. After completion of the reaction, the reaction mixture was concentrated to remove the organic solvent and the pH was adjusted to 3-4 with 12 N hydrochloric acid, and a white solid was precipitated. The mixture was filtered, and the filter cake was washed with petroleum ether and collected to yield the title compound 2-(methylthio)-4-((tetrahydro-2*H*-pyran-4-yl)amino)pyrimidine-5-carboxylic acid **105c** (white solid, 640 g, crude, 110.4% yield).

¹H NMR (400 MHz DMSO) δ 8.52 (s, 1H), 8.36 (d, 1H), 4.22-4.18 (m, 1H), 3.86-3.83 (m, 2H), 3.47-3.41 (m, 2H), 2.46 (s, 3H), 1.94-1.89 (m, 2H), 1.56-1.48 (m, 2H). LC-MS m/z (ESI) = 270.10 [M+1]

### Step 3:

### 2-(Methylthio)-9-(tetrahydro-2H-pyran-4-yl)-7,9-dihydro-8H-purin-8-one (105d)

2-(Methylthio)-4-((tetrahydro-2*H*-pyran-4-yl)amino)pyrimidine-5-carboxylic acid **105c** (630 g, 2.34 mol) was dissolved in dimethylacetamide (4000 mL), and diphenylphosphoryl azide (504 mL, 2.34 mol) was added with stirring at room temperature. The reaction mixture was stirred for 10 min, and then triethylamine (325 mL, 2.34 mol) was added. The reaction mixture was stirred for another 2 h, then heated to 120 °C and stirred for 1.5 h, during which a large amount of gas was produced. The reaction was monitored by TLC. After completion of the reaction, the reaction mixture was directly concentrated to remove most of the solvent. The residual liquid was poured into 6 L of ice water, and a large amounts of solid was precipitated. After the mixture was stirred for 0.5 h, the solid was collected by filtration and washed 3 times with water, and concentrated under reduced pressure to yield the title compound 2-(methylthio)-9-(tetrahydro-2*H*-pyran-4-yl)-7,9-dihydro-8*H*-purin-8-one **105d** (white solid, 880 g, crude).

¹H NMR (400 MHz DMSO) δ 11.35 (s, 1H), 8.12 (s, 1H), 4.44-4.38 (m, 1H), 4.00-43.97 (m, 2H), 3.45-3.41 (m, 2H), 2.55-2.45 (m, 5H), 1.69-1.65 (m, 2H).

LC-MS m/z (ESI) = 267.10 [M+1]

### Step 4:

### 7-(Ethyl-d5)-2-(methylthio)-9-(tetrahydro-2H-pyran-4-yl)-7,9-dihydro-8H-purin-8-one (105e)

2-(Methylthio)-9-(tetrahydro-2*H*-pyran-4-yl)-7,9-dihydro-8*H*-purin-8-one **105d** (2.5 g, 9.39 mmol) was dissolved in dimethylformamide (20 mL), and deuterated bromoethane (700 µL, 9.39 mmol) and sodium hydroxide (1.13 g, 28.16 mmol) were added with stirring at room temperature. The reaction mixture was stirred for 2.5 h. The reaction was monitored by TLC. After completion of the reaction, then water was added and the organics were extracted with ethyl acetate and the organic layer was concentrated to obtain the title compound 7-(ethyl-d5)-2-(methylthio)-9-(tetrahydro-2*H*-pyran-4-yl)-7,9-dihydro-8H-purin-8-one **105e** (pale yellow solid, 1.65 g, 58.71% yield).

LC-MS m/z(ESI) = 300.20 [M+1]

### Step 7:

### 7-(Ethyl-d5)-2-(methylsulfinyl)-9-(tetrahydro-2H-pyran-4-yl)-7,9-dihydro-8H-purin-8-one (105f)

7-(Ethyl-d5)-2-(methylthio)-9-(tetrahydro-2*H*-pyran-4-yl)-7,9-dihydro-8*H*-purin-8-one 105e (1.6 g, 5.34 mmol) was dissolved in dichloromethane (8 mL), and *m-*chloroperoxybenzoic acid (3.69 g, 21.38 mmol) was added with stirring at room temperature. The reaction mixture was reacted for 1.5 h. The reaction was monitored by TLC. After completion of the reaction, the reaction mixture was concentrated, and the residue was purified by silica gel column chromatography (dichloromethane/ethyl acetate (v/v) = 1:1) to obtain the title compound 7-(ethyl-d5)-2-(methylsulfinyl)-9-(tetrahydro-2*H*-pyran-4-yl)-7,9-dihydro-8*H*-purin-8-one **105f** (white solid, 1.4 g, 79.04% yield).

LC-MS m/z(ESI) = 332.20 [M+1]

### Step 8:

### 4-((7-(Ethyl-d5)-8-oxo-9-(tetrahydro-2H-pyran-4-yl)-8,9-dihydro-7H-purin-2-yl)amino)-2-fluoro-5-methylbenzamide (compound 105)

7-(Ethyl-d5)-2-(methylsulfinyl)-9-(tetrahydro-2*H*-pyran-4-yl)-7,9-dihydro-8*H*-purin-8-one **105f** (300 mg, 0.91 mmol), 4-amino-3-methylbenzamide intermediate 2 (457 mg, 2.72 mmol) and potassium tert-butoxide (305 mg, 2.72 mmol) were dissolved in dimethylformamide (10 mL). The reaction mixture was stirred at room temperature for 1 h. The reaction was monitored by TLC. After completion of the reaction, water was added and the organics were extracted with ethyl acetate. The organic layer was concentrated, and the residue was purified by silica gel column chromatography (dichloromethane/methanol (v/v) = 20:1) to obtain the title compound 4-((7-(ethyl-d5)-8-oxo-9-(tetrahydro-2*H*-pyran-4-yl)-8,9-dihydro-7*H*-purin-2-yl)amino)-2-fluoro-5-methylbenzamide **compound 105** (white solid, 45 mg, 11.85% yield).

¹H NMR (400 MHz, DMSO-d6) δ 8.54 (s, 1H), 8.25 (s, 1H), 7.89 (d, 1H), 7.54 (d, 1H), 7.47 (s, 1H), 7.39 (s, 1H), 4.49-4.38 (m, 1H), 3.98 (dd, 2H), 3.47-3.38 (m, 2H), 2.59-2.51 (m, 2H), 2.29 (s, 3H), 1.73-1.65 (m, 2H).

LC-MS m/z(ESI) = 420.20 [M+1]

### Example 106

### 4-((7-(2,2-Difluoroethyl)-8-oxo-9-(tetrahydro-2H-pyran-4-yl)-8,9-dihydro-7H-purin-2-yl)amino)-2-fluoro-5-methylbenzamide (compound 106)

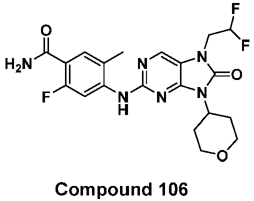

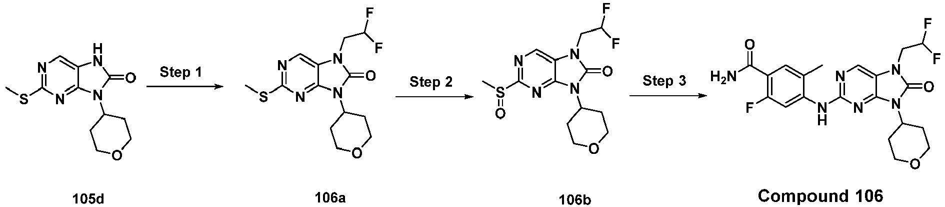

### Step 1:

### 7-(2,2-Difluoroethyl)-2-(methylthio)-9-(tetrahydro-2H-pyran-4-yl)-7,9-dihydro-8H-purin-8-one (106a)

2-(Methylthio)-9-(tetrahydro-2*H*-pyran-4-yl)-7,9-dihydro-8*H*-purin-8-one 105d (1 g, 2.53 mmol) was dissolved in dimethylformamide (5 mL), and allyl iodide (397 µL, 4.51 mmol) and potassium carbonate (1.56 g, 11.26 mmol) were added with stirring at room temperature. The reaction mixture was stirred for 1 h, then heated to 65 °C and stirred for 2.5 h. The reaction was monitored by TLC. After completion of the reaction, then water was added to the reaction mixture, and a solid was precipitated. The solid was collected by filtration and washed with water and petroleum ether, and concentrated by rotary evaporation under reduced pressure to obtain the title compound 7-(2,2-difluoroethyl)-2-(methylthio)-9-(tetrahydro-2*H*-pyran-4-yl)-7,9-dihydro-8*H*-purin-8-one **106a** (white solid, 800 mg, 64.49% yield).

1HNMR (600 MHz, DMSO-d6) δ 8.36 (s, 1H), 6.52-6.22 (m, 1H), 4.50-4.43 (m, 1H), 4.38-4.30 (m, 2H), 3.98 (dd, 2H), 3.49-3.42 (m, 2H), 2.53 (s, 3H), 2.50-2.45 (m, 2H), 1.79-1.60 (m, 2H).

LC-MS m/z(ESI) = 331.10 [M+1]

### Step 2:

### 7-(2,2-Difluoroethyl)-2-(methylsulfinyl)-9-(tetrahydro-2H-pyran-4-yl)-7,9-dihydro-8H-purin-8-one (106b)

7-(2,2-Difluoroethyl)-2-(methylthio)-9-(tetrahydro-2*H*-pyran-4-yl)-7,9-dihydro-8*H-*purin-8-one **106a** (800 mg, 2.42 mmol) was dissolved in dichloromethane (6 mL), and *m*-chloroperoxybenzoic acid (543 mg, 3.15 mmol) was added with stirring at room temperature. The reaction mixture was stirred for 3 h. The reaction was monitored by TLC. After completion of the reaction, saturated sodium bicarbonate and dichloromethane were added to the reaction mixture for extraction, and the organic layer was concentrated to obtain the title compound 7-(2,2-difluoroethyl)-2-(methylsulfinyl)-9-(tetrahydro-2*H*-pyran-4-yl)-7,9-dihydro-8*H*-purin-8-one **106b** (white solid, 740 mg, 88.23% yield).

LC-MS m/z(ESI) = 347.10 [M+1]

### Step 3:

### 4-((7-(2,2-Difluoroethyl)-8-oxo-9-(tetrahydro-2H-pyran-4-yl)-8,9-dihydro-7H-purin-2-yl)amino)-2-fluoro-5-methylbenzamide

7-(2,2-Difluoroethyl)-2-(methylsulfinyl)-9-(tetrahydro-2*H*-pyran-4-yl)-7,9-dihydro-8*H*-purin-8-one **106b** (200 mg, 0.58 mmol), 4-amino-3-methylbenzamide intermediate 2 (388 mg, 2.31 mmol) and cesium carbonate (564 mg, 1.73 mmol) were dissolved in dimethylformamide (5 mL). The reaction mixture was heated to 80 °C and stirred for 3 h. The reaction was monitored by TLC. After completion of the reaction, water was added and the organics were extracted with dichloromethane. The organic layer was concentrated, and the residue was purified by silica gel column chromatography (dichloromethane/methanol (v/v) = 30:1), followed by recrystallization to obtain the title compound 4-((7-(2,2-difluoroethyl)-8-oxo-9-(tetrahydro-2*H*-pyran-4-yl)-8,9-dihydro-7*H*-purin-2-yl)amino)-2-fluoro-5-methylbenzamide **compound 108** (white solid, 65 mg, 24.99% yield).

¹H NMR (400 MHz, DMSO-d6) δ 8.64 (s, 1H), 8.21 (s, 1H), 7.84 (d, 1H), 7.54 (d, 1H), 7.47 (s, 1H), 7.41 (s, 1H), 6.54-6.22 (m, 1H), 4.51-4.41 (m, 1H), 4.37-4.25 (m, 2H), 3.98 (dd, 2H), 3.46-3.38 (m, 2H), 2.58-2.50 (m, 2H), 2.29 (s, 3H), 1.75-1.67 (m, 2H). LC-MS m/z(ESI) = 451.20 [M+1]

### Example 107

### (S)-4-((7-Ethyl-8-oxo-9-(tetrahydrofuran-3-yl)-8,9-dihydro-7H-purin-2-yl)amino)-2-fluoro-5-methylbenzamide (compound 107)

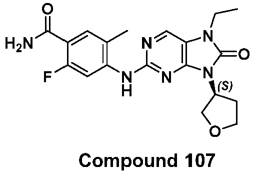

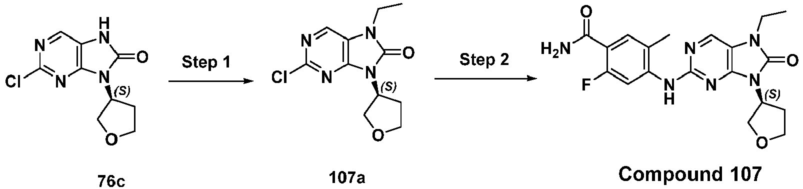

### Step 1:

### (S)-2-Chloro-7-ethyl-9-(tetrahydrofuran-3-yl)-7,9-dihydro-8H-purin-8-one (107a)

(*S*)-2-Chloro-9-(tetrahydrofuran-3-yl)-7,9-dihydro-8*H*-purin-8-one **76c** (2 g, 8.31 mmol) was dissolved in dimethylformamide (20 mL), and cesium carbonate (2.71 g, 8.31 mmol) and iodoethane (1.56 g, 9.97 mmol) were added at 0 °C. The reaction mixture was stirred for 1 h. The reaction was monitored by TLC. After completion of the reaction, then the reaction mixture was poured into 60 mL of water, and a large amounts of solid was precipitated The solid was collected by filtration and dried to give the title compound (*S*)-2-chloro-7-ethyl-9-(tetrahydrofuran-3-yl)-7,9-dihydro-8*H-*purin-8-one **107a** (white solid, 1.1 g, 49.26% yield).

¹H NMR (400 MHz, DMSO-d6) δ 8.43 (s, 1H), 5.04-4.97 (m, 1H), 4.15-4.06 (m, 1H), 3.98 (t, 1H), 3.91-3.84 (m, 4H), 2.44-2.37 (m, 1H), 2.30-2.19 (m, 1H), 1.26 (s, 3H).

LC-MS m/z(ESI) = 269.10 [M+1]

### Step 2:

### (S)-4-((7-Ethyl-8-oxo-9-(tetrahydrofuran-3-yl)-8,9-dihydro-7H-purin-2-yl)amino)-2-fluoro-5-methylbenzamide (compound 107)

(S)-2-Chl oro-7-ethyl -9-(tetrahydrofuran-3 -yl)-7,9-dihydro-8*H*-purin-8 -one **107a** (500 mg, 1.86 mmol), 4-amino-2-fluoro-5-methylbenzamide intermediate 2 (1.25 g, 7.44 mmol), cesium carbonate (2.43 g, 7.44 mmol) and methanesulfonato(2-dicyclohexylphosphino-3,6-dimethoxy-2',4',6'-tri-*i*-propyl-1,1'-biphenyl)(2'-amino-1,1'-biphenyl-2-yl)palladium(II) (253 mg, 0.28 mmol) were dissolved in 1,4-dioxane (10 mL), followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 20/1) followed by Pre-HPLC to obtain the title compound (*S*)-2-fluoro-5-methyl-4-((7-methyl-8-oxo-9-(tetrahydrofuran-3-yl)-8,9-dihydro-7H-purin-2-yl)amino)benzamide **compound 107** (white solid, 120 mg, 16.11% yield).

¹H NMR (400 MHz, DMSO-d6) δ 8.56 (s, 1H), 8.27 (s, 1H), 7.88 (d, 1H), 7.54 (d, 1H), 7.47 (s, 1H), 7.41 (s, 1H), 5.01-4.94 (m, 1H), 4.09 (q, 1H), 3.96 (t, 3H), 3.92-3.82 (m, 4H), 2.76-2.66 (m, 1H), 2.33-2.23 (m, 1H), 2.28 (s, 3H), 1.24 (t, 3H).

LC-MS m/z(ESI) = 401.20 [M+1]

### Example 108

### (R)-4-((7-Ethyl-8-oxo-9-(tetrahydrofuran-3-yl)-8,9-dihydro-7H-purin-2-yl)amino)-2-fluoro-5-methylbenzamide (compound 108)

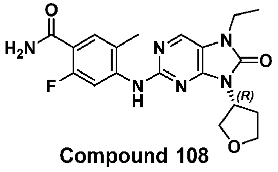

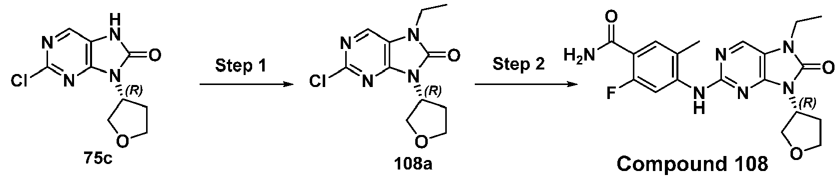

### Step 1:

### (R)-2-Chloro-7-ethyl-9-(tetrahydrofuran-3-yl)-7,9-dihydro-8H-purin-8-one (108a)

(*R*)-2-Chloro-9-(tetrahydrofuran-3-yl)-7,9-dihydro-8*H*-purin-8-one **75c** (1.4 g, 5.82 mmol) was dissolved in dimethylformamide (20 mL), and cesium carbonate (1.90 g, 5.82 mmol) and iodoethane (998 mg, 6.40 mmol) were added at 0 °C. The reaction mixture was stirred for 1 h. The reaction was monitored by TLC. After completion of the reaction, then the reaction mixture was poured into 60 mL of water, and a large amounts of solid was precipitated. The solid was collected by filtration and dried to yield the title compound (*R*)-2-chloro-7-ethyl-9-(tetrahydrofuran-3-yl)-7,9-dihydro-8*H*-purin-8-one 108a (white solid, 1.08 g, 69.09% yield).

LC-MS m/z(ESI) = 269.10 [M+1]

### Step 2:

### (R)-4-((7-Ethyl-8-oxo-9-(tetrahydrofuran-3-yl)-8,9-dihydro-7H-purin-2-yl)amino)-2-fluoro-5-methylbenzamide (compound 108)

(*R*)-2-Chloro-7-ethyl-9-(tetrahydrofuran-3-yl)-7,9-dihydro-8*H*-purin-8-one **108a** (300 mg, 1.12 mmol), 4-amino-2-fluoro-5-methylbenzamide intermediate 2 (939 mg, 5.58 mmol), cesium carbonate (1.09 g, 3.35 mmol) and methanesulfonato(2-dicyclohexylphosphino-3,6-dimethoxy-2',4',6'-tri-i-propyl- 1, I'-biphenyl)(2'-amino-1,1'-biphenyl-2-yl)palladium(II) (202 mg, 0.22 mmol) were dissolved in 1,4-dioxane (10 mL), followed by nitrogen purging. The reaction mixture was stirred under nitrogen atmosphere at 110 °C for 4 h. The reaction was monitored by TLC. After completion of the reaction, the reaction mixture was concentrated, and the residue was purified by silica gel column chromatography (dichloromethane/methanol (v/v) = 20/1) followed by Pre-HPLC to obtain the title compound (*R*)-2-fluoro-5-methyl-4-((7-methyl-8-oxo-9-(tetrahydrofuran-3-yl)-8,9-dihydro-7*H*-purin-2-yl)amino)benzamide **compound 108** (white solid, 100 mg, 22.37% yield).

¹H NMR (400 MHz, DMSO-d6) δ 8.53 (s, 1H), 8.28 (s, 1H), 7.87 (d, 1H), 7.55 (d, 1H), 7.47 (s, 1H), 7.41 (s, 1H), 5.03-4.97 (m, 1H), 4.11-4.05 (m, 1H), 3.96 (t, 3H), 3.94-3.85 (m, 4H), 2.77-2.70 (m, 1H), 2.36-2.23 (m, 1H), 2.28 (s, 3H), 1.26 (t, 3H).

LC-MS m/z(ESI) = 401.20 [M+1]

### Example 109

### 2-Fluoro-4-((7-(fluoromethyl)-8-oxo-9-(tetrahydro-2H-pyran-4-yl)-8, 9-dihydro-7H-purin-2-yl)amino)-5-methylbenzamide (compound 109)

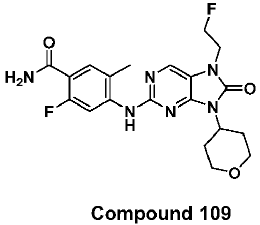

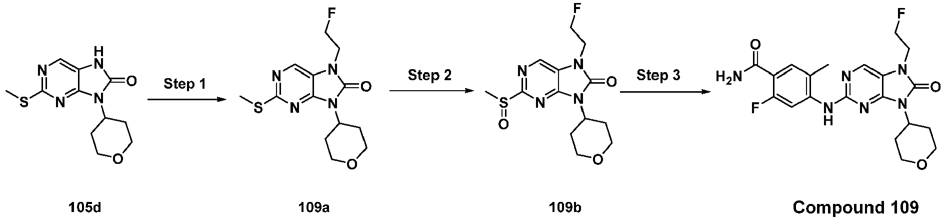

### Step 1:

### 7-(2-Fluoroethyl)-2-(methylthio)-9-(tetrahydro-2H-pyran-4-yl)-7,9-dihydro-8H-purin-8-one (109a)

2-(Methylthio)-9-(tetrahydro-2*H*-pyran-4-yl)-7,9-dihydro-8*H*-purin-8-one **105d** (1.0 g, 3.75 mmol), 1-fluoro-2-iodoethane (0.71 g, 4.1 mmol) and potassium carbonate (1.5 g, 11.2 mmol) were added to a dry reaction flask, followed by the addition of DMF (10 mL). The reaction mixture was stirred at room temperature for 2.5 h. The reaction was monitored by TLC. After completion of the reaction, water was added and the organics were extracted with dichloromethane, and the organic phases were combined, dried and concentrated to yield the title compound 7-(2-fluoroethyl)-2-(methylthio)-9-(tetrahydro-2*H*-pyran-4-yl)-7,9-dihydro-8*H*-purin-8-one **109a** (white solid, 1.0 g, 85.47% yield).

LC-MS m/z (ESI) = 313.10 [M+1]

### Step 2:

### 7-(2-Fluoroethyl)-2-(methylsulfinyl)-9-(tetrahydro-2H-pyran-4-yl)-7,9-dihydro-8H-purin-8-one (109b)

7-(2-Fluoroethyl)-2-(methylthio)-9-(tetrahydro-2*H*-pyran-4-yl)-7,9-dihydro-8*H*-purin-8-one **109a** (0.9 g, 2.88 mmol) was added to a dry reaction flask and dissolved in DCM (10 mL), and *m*-chloroperoxybenzoic acid (0.5 g, 3.17 mmol) was added in three batches at room temperature. After addition, the reaction mixture was stirred for 3 h. The reaction was monitored by TLC. After completion of the reaction, water was added and the organics were extracted with dichloromethane, and the organic phases were combined, dried and concentrated to yield the title compound 7-(2-fluoroethyl)-2-(methylsulfinyl)-9-(tetrahydro-2*H*-pyran-4-yl)-7,9-dihydro-8*H*-purin-8-one **109b** (white solid, 0.8 g, 85.11% yield).

LC-MS m/z (ESI) = 329.10 [M+1]

### Step 3:

### 2-Fluoro-4-((7-(fluoromethyl)-8-oxo-9-(tetrahydro-2H-pyran-4-yl)-8, 9-dihydro-7H-purin-2-yl)amino)-5-methylbenzamide (compound 109)

7-(2-Fluoroethyl)-2-(methylsulfinyl)-9-(tetrahydro-2*H*-pyran-4-yl)-7,9-dihydro-8*H-*purin-8-one **109b** (200 mg, 0.61 mmol), 4-amino-2-fluoro-5-methylbenzamide intermediate 2 (410 mg, 2.44 mmol) and cesium carbonate (590 mg, 1.83 mmol) were added to a dry reaction tube, followed by the addition of DMF (3 mL). The reaction mixture was stirred at 80 °C for 2.5 h. Water was added and the organics were extracted with dichloromethane, and the organic phases were combined, dried and concentrated. The residue was purified by silica gel column chromatography (DCM/MeOH (v/v) = 20:1) to obtain the title compound 2-fluoro-4-((7-(fluoromethyl)-8-oxo-9-(tetrahydro-2H-pyran-4-yl)-8,9-dihydro-7*H*-purin-2-yl)amino)-5-methylbenzamide **compound 109** (white solid, 21 mg, 7.98% yield).

¹H NMR (600 MHz, DMSO-d6) δ 8.60 (s, 1H), 8.21 (s, 1H), 7.87 (d, 1H), 7.55 (d, 1H), 7.48 (s, 1H), 7.42 (s, 1H), 4.75-4.66 (m, 2H), 4.48-4.43 (m, 1H), 4.19-4.13 (m, 2H), 4.00-3.97 (m, 2H), 3.43 (t, 2H), 2.59-2.53 (m, 2H), 2.29 (s, 3H), 1.74-1.68 (m, 2H). LC-MS m/z (ESI) = 433.20 [M+1]

### Example 110

### 2-Fluoro-5-methyl-4-((8-oxo-9-(tetrahydro-2H-pyran-4-yl)-7-(2,2,2-trifluoroethyl)-8,9-dihydro-7H-purin-2-yl)amino)benzamide (compound 110)

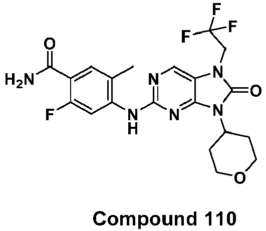

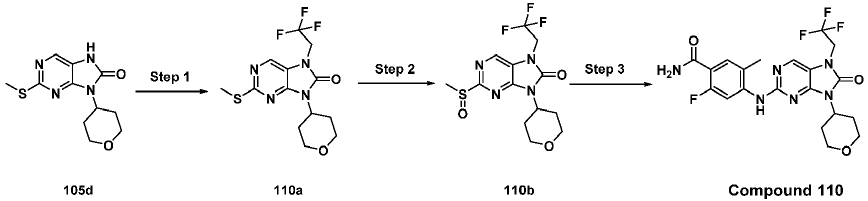

### Step 1:

### 2-(Methylthio)-9-(tetrahydro-2H-pyran-4-yl)-7-(2,2,2-trifluoroethyl)-7,9-dihydro-8H-purin-8-one (110a)

2-(Methylthio)-9-(tetrahydro-2*H*-pyran-4-yl)-7,9-dihydro-8*H*-purin-8-one **105d** (1.0 g, 3.75 mmol), 2-iodo-1,1,1-trifluoroethane (444 mg, 4.5 mmol) and cesium carbonate (3.67 g, 11.26 mmol) were added to a dry reaction flask, followed by the addition of DMF (10 mL). The reaction mixture was stirred at room temperature for 2.5 h. The reaction was monitored by TLC. After completion of the reaction, water was added and the organics were extracted with ethyl acetate, and the organic phases were combined, dried and concentrated to give the title compound 7-(2-fluoroethyl)-2-(methylthio)-9-(tetrahydro-2*H*-pyran-4-yl)-7,9-dihydro-8*H*-purin-8-one **110a** (pale yellow solid, 1.18 g, 90.22% yield).

¹H NMR (600 MHz, DMSO-d6) δ 8.43 (s, 1H), 4.83 (q, 2H), 4.52-4.43 (m, 1H), 3.98 (dd, 2H), 3.51-3.42 (m, 2H), 2.53 (s, 3H), 2.50-2.44 (m, 2H), 1.76-1.68 (m, 2H). LC-MS m/z (ESI) = 349.10 [M+1]

### Step 2:

### 2-(Methylsulfinyl)-9-(tetrahydro-2H-pyran-4-yl)-7-(2,2,2-trifluoroethyl)-7,9-dihydro-8H-purin-8-one (110b)

7-(2-Fluoroethyl)-2-(methylthio)-9-(tetrahydro-2*H*-pyran-4-yl)-7,9-dihydro-8*H*-purin-8-one **110a** (1.17 g, 3.36 mmol) was added to a dry reaction flask and dissolved in DCM (10 mL), and *m*-chloroperoxybenzoic acid (754 mg, 4.37 mmol) was added at room temperature. The reaction mixture was stirred for 1.5 h. The reaction was monitored by TLC. After completion of the reaction, saturated sodium bicarbonate and dichloromethane were added to the reaction mixture for extraction, and the organic phases were combined, dried and concentrated to obtain the title compound 2-(methylsulfinyl)-9-(tetrahydro-2*H*-pyran-4-yl)-7-(2,2,2-trifluoroethyl)-7,9-dihydro-8*H*-purin-8-one **110b** (white solid, 1 g, 81.72% yield).

LC-MS m/z (ESI) = 365.10 [M+1]

### Step 3:

### 2-Fluoro-5-methyl-4-((8-oxo-9-(tetrahydro-2H-pyran-4-yl)-7-(2,2,2-trifluoroethyl)-8,9-dihydro-7H-purin-2-yl)amino)benzamide (compound 110)

2-(Methylsulfinyl)-9-(tetrahydro-2*H*-pyran-4-yl)-7-(2,2,2-trifluoroethyl)-7,9-dihydro-8H-purin-8-one **110b** (200 mg, 0.55 mmol), 4-amino-2-fluoro-5-methylbenzamide intermediate 2 (369 mg, 2.20 mmol) and cesium carbonate (537 mg, 1.65 mmol) were added to a dry reaction tube, followed by the addition of DMF (3 mL). The reaction mixture was stirred at 40 °C for 5 h. Water was added and the organics were extracted with dichloromethane, and the organic phases were combined, dried and concentrated. The residue was purified by silica gel column chromatography (DCM/MeOH (v/v) = 20:1) to obtain the title compound 2-fluoro-5-methyl-4-((8-oxo-9-(tetrahydro-2*H-*pyran-4-yl)-7-(2,2,2-trifluoroethyl)-8,9-dihydro-7*H*-purin-2-yl)amino)benzamide **compound 110** (white solid, 45 mg, 17.5% yield).

¹H NMR (600 MHz, DMSO-d6) δ 8.54 (s, 1H), 8.26 (s, 1H), 7.87 (d, 1H), 7.55 (d, 1H), 7.47 (s, 1H), 7.42 (s, 1H), 4.85-4.69 (m, 2H), 4.53-4.42 (m, 1H), 3.98 (dd, 2H), 3.46-3.40 (m, 2H), 2.52-2.45 (m, 2H), 2.29 (s, 3H), 1.76-1.68 (m, 2H).

LC-MS m/z (ESI) = 469.20 [M+1]

### Example 111

### 4-((9-(cis-3-Cyanocyclobutyl)-7-ethyl-8-oxo-8,9-dihydro-7H-purin-2-yl)amino)-2-fluoro-5-methylbenzamide (compound 111)

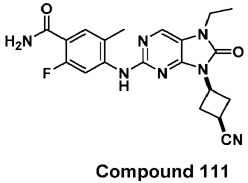

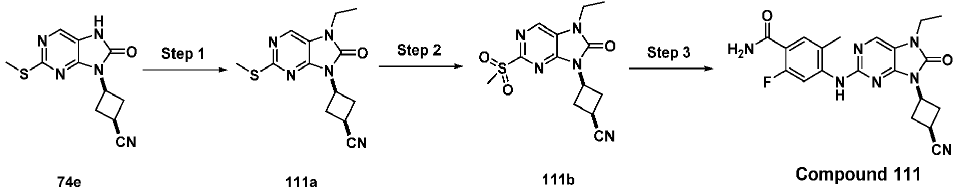

### Step 1:

### cis-3-(7-Ethyl-2-(methylthio)-8-oxo-7,8-dihydro-9H-purin-9-yl)cyclobutane-1-carbonitrile (111a)

cis-3-(2-(Methylthio)-8-oxo-7,8-dihydro-9*H*-purin-9-yl)cyclobutane-1-carbonitrile 74e (500 mg, 19.1 mmol) was dissolved in dimethylformamide (5 mL), and iodoethane (3.0 g, 19.1 mmol) and sodium hydroxide (1.53 g, 38.2 mmol) were added at 0 °C. The reaction mixture was stirred at 0 °C for 0.5 h. The reaction was monitored by TLC. After completion of the reaction, Then 50 mL of water was added, and a solid was precipitated. The solid was collected by filtration and dried by rotary evaporation under reduced pressure to obtain the title compound *cis*-3-(7-ethyl-2-(methylthio)-8-oxo-7,8-dihydro-9*H*-purin-9-yl)cyclobutane-1-carbonitrile **111a** (white solid, 487 mg, 87.9% yield).

1H NMR (400 MHz, DMSO-d6) δ 8.68 (s, 1H), 4.97-4.87 (m, 1H), 3.93 (q, 2H), 3.34 (s, 3H), 3.33-3.23 (m, 2H), 2.91 (s, 3H), 2.77-2.69 (m, 2H), 1.27 (t, 3H).

LC-MS m/z (ESI) = 290.10 [M+1]

### Step 2:

### cis-3-(7-Ethyl-2-(methylsulfonyl)-8-oxo-7,8-dihydro-9H-purin-9-yl)cyclobutane-1-carbonitrile (111b)

*cis*-3-(7-Ethyl-2-(methylthio)-8-oxo-7,8-dihydro-9*H*-purin-9-yl)cyclobutane-1-carbonitrile **111a** (487 mg, 1.66 mmol) was dissolved in methanol (10 mL), and 2 mL of purified water was added, followed by the addition of potassium peroxymonosulfate (1.02 g, 1.66 mmol). The reaction mixture was heated to 60 °C and stirred for 2 h. The reaction was monitored by TLC. After completion of the reaction, the reaction mixture was concentrated by rotary evaporation under reduced pressure to remove the methanol, and then 5 mL of purified water was added. The mixture was extracted 3 times with dichloromethane (20 mL × 3), and the organic phases were combined, dried over anhydrous sodium sulfate and concentrated by rotary evaporation under reduced pressure to remove the solvent and thus to obtain *cis*-3-(7-ethyl-2-(methylsulfonyl)-8-oxo-7,8-dihydro-9*H*-purin-9-yl)cyclobutane-1-carbonitrile **111b** (pale yellow solid, 397 mg, 74.3% yield).

LC-MS m/z(ESI) = 322.10 [M+1]

### Step 3:

### 4-((9-(cis-3-Cyanocyclobutyl)-7-ethyl-8-oxo-8,9-dihydro-7H-purin-2-yl)amino)-2-fluoro-5-methylbenzamide (compound 111)

cis-3-(7-Ethyl-2-(methylsulfonyl)-8-oxo-7,8-dihydro-9*H*-purin-9-yl)cyclobutane-1-carbonitrile **111b** (200 mg, 0.62 mmol) and 4-amino-3-methyl-2-fluorobenzamide **intermediate** 2 (538 mg, 3.20 mmol) were dissolved in 3 mL of *N,N-*dimethylformamide, followed by nitrogen purging and the addition of potassium *tert-*butoxide (143 mg, 1.28 mmol). The reaction mixture was stirred under nitrogen atmosphere at room temperature for 4 h. The reaction was monitored by TLC. After completion of the reaction, the reaction mixture was poured into 30 mL of purified water, and a large amounts of yellow solid was precipitated. The solid was collected by filtration and purified by silica gel column chromatography (dichloromethane/methanol (v/v) = 35/1) to obtain the title compound 4-((9-(*cis*-3-cyanocyclobutyl)-7-ethyl-8-oxo-8,9-dihydro-7*H*-purin-2-yl)amino)-2-fluoro-5-methylbenzamide **compound 111** (white solid, 25 mg, 10.0% yield).

¹H NMR (400 MHz, DMSO-d6) δ 8.63 (s, 1H), 8.28 (s, 1H), 7.88 (d 1H), 7.58-7.56 (m, 1H), 7.50 (s, 1H), 7.41 (s, 1H), 5.14-5.09 (m, 1H), 3.87-3.82 (m, 2H), 3.64-3.59 (m, 1H), 3.26-3.16 (m, 2H), 2.75-2.68 (m, 2H), 2.32 (s, 3H), 1.22 (t, 3H).

LC-MS m/z(ESI) = 410.20 [M+1]

### Biological experiments

### 1. Inhibition assay of DNA-PK kinase

The inhibitory activity of the compounds on DNA-PK kinase was measured by using a DNA-PK kinase assay kit (purchased from Promega, Cat.#: V4107, Lot: 0000366495). The results were quantified by chemiluminescence, and the detailed experimental protocol was as follows:
i. An ADP-fluorescence standard curve over a range of concentrations was plotted according to the kit instructions;
ii. 5 µL reaction systems were prepared in a 384-well white plate, and to each well 1 µL of a compound (each under a concentration gradient over 1 µM, 200 nM, 40 nM, 8 nM, 1.6 nM, 0.32 nM, 0.064 nM, 0.013 nM), 20 units of DNA-PK kinase, 0.2 µg/µL substrate, 10 µg/µL DNA, 50 µM ATP, and 1% DMSO were added;
iii. The mixtures were mixed well, centrifuged (1000 rpm, 30 s), and incubated at 37□ for 60 min;
iv. 5 µL of ADP-Glo^{™} Reagent was added to stop the reaction, which was then mixed well, centrifuged (1000 rpm, 30 s), and incubated at room temperature for 40 min;
v. 10 µL Kinase Detection Reagent was added, and the mixture was mixed well by shaking, centrifuged (1000 rpm, 30 s), and incubated at room temperature for 30 min;
vi. The fluorescence value was measured by using a microplate reader (Thermo fisher, Varioskan LUX). IC₅₀ was calculated using GraphPad Prism 8, and the results are shown in Table 1.

**Table 1. Inhibitory activity of compounds of the present disclosure on DNA-PK kinase**

| Compound No. | IC₅₀ (nM) |
|---|---|
| 1 | 10.10 |
| 2 | 0.08 |
| 3 | 18.58 |
| 4 | 8.30 |
| 5 | 0.78 |
| 6 | 0.78 |
| 7 | 5.47 |
| 8 | 11.70 |
| 9 | 0.47 |
| 11 | 25.03 |
| 15 | 5.28 |
| 17 | 28.66 |
| 18 | 2.10 |
| 21 | 53.00 |
| 22 | 1.36 |
| 24 | 0.56 |
| 25 | 3.44 |
| 26 | 7.60 |
| 27 | 4.36 |
| 29 | 3.90 |
| 30 | 66.60 |
| 31 | 31.00 |
| 36 | 17.95 |
| 37 | 11.88 |
| 38 | 0.47 |
| 39 | 5.70 |
| 40 | 39.2 |
| 41 | 13.42 |
| 42 | 2.05 |
| 43 | 9.80 |
| 44 | 1.10 |
| 45 | 13.53 |
| 46 | 0.99 |
| 47 | 3.69 |
| 48 | 1.96 |
| 50 | 12.72 |
| 51 | 34.09 |
| 53 | 0.32 |
| 54 | 8.07 |
| 55 | 14.76 |
| 56 | 14.33 |
| 57 | 3.98 |
| 59 | 12.12 |
| 60 | 29.74 |
| 62 | 4.17 |
| 63 | 50.47 |
| 64 | 3.34 |
| 67 | 2.96 |
| 68 | 0.01 |
| 69 | 1.25 |
| 70 | 4.14 |
| 71 | 2.17 |
| 72 | 0.05 |
| 73 | 1.34 |
| 75 | 5.69 |
| 76 | 1.96 |
| 77 | 4.67 |
| 78 | 0.038 |
| 79 | 1.86 |
| 80 | 11.15 |
| 81 | 47.12 |
| 82 | 24.5 |
| 83 | 0.43 |
| 84 | 0.24 |
| 85 | 1.10 |
| 86 | 0.44 |
| 88 | 2.85 |
| 89 | 3.83 |
| 90 | 10.82 |
| 91 | 2.44 |
| 92 | 0.02 |
| 93 | 0.75 |
| 94 | 7.20 |
| 95 | 0.60 |
| 96 | 3.89 |
| 97 | 0.68 |
| 98 | 4.01 |
| 99 | 2.13 |
| 100 | 2.20 |
| 101 | 9.60 |
| 105 | 5.54 |
| 106 | 6.75 |
| 107 | 10.11 |
| 109 | 5.54 |
| 103 (Comparative Example) | 100.40 |

The results show that the compounds of the present disclosure have a good inhibitory effect on DNA-PK kinase.

### 2. Metabolic stability in liver microsomes

### 1.1 Experimental materials

Liver microsomes (purchased from Reed Liver Disease Research (Shanghai) Co., Ltd., product: human liver microsomes (NJT)); NADPH (purchased from Roche, Lot: 40939); UDPGA; the compounds of the present invention (test substances).

### 1.2 Experimental procedure

(1) A mixed working solution of testosterone (0.5 µM) and 7-hydroxycoumarin (0.5 µM) was prepared.
(2) A mixed solution of NADPH (1 mM) and UDPGA (1 mM) was prepared, and prewarmed at 37°C in a water bath shaker before use.
(3) Liver microsomes were thawed and diluted with a buffer.
(4) A preparation of a test substance (0.5 µM) and a solution of liver microsomes (0.5 mg/mL) were added to each well of an incubation plate.
(5) Three experimental groups were set and samples were added. Test group: the test substance was incubated with liver microsomes for 0 min, 30 min, 60 min, and 120 min in the presence of the coenzyme NADPH and UDPGA; Positive control group: testosterone (0.5 µM) and 7-hydroxycoumarin (0.5 µM) were incubated with microsomes for 0 min and 120 min in the presence of the coenzyme NADPH and UDPGA; Negative control group: the test substance was incubated with microsomes for 0 and 120 min without any coenzyme.
(6) All samples were incubated at 37°C, and pre-cooled methanol was added at each termination time (0, 15, 30, 45, 60 min, 120 min) to terminate the reaction, and the time was recorded.
(7) All samples were mixed well and centrifuged at 4000 rpm for 10 min. The supernatant was taken and measured by LC-MS/MS for the amount of the test substance remaining in the samples. Data analysis was carried out as follows: ${T}_{1 / 2} = 0.693 / k , Clint = \left(0.693/{T}_{1 / 2}\right) \times \left(1/\left(microsomal protein\right)\times Scaling Factors\right) ,$ Note: k is the slope of the logarithm of the remaining amounts of compound versus time. The results are shown in Table 2.

**Table 2. Stability of compounds of the present disclosure in human liver microsomes**

| Compounds | T_{1/2} (min) |
|---|---|
| Compound 2 | >120 |
| Compound 5 | >120 |
| Compound 8 | >120 |
| Compound 9 | >120 |
| Compound 18 | >120 |
| Compound 24 | >120 |
| Compound 25 | >120 |
| Compound 26 | >120 |
| Compound 27 | >120 |
| Compound 29 | >120 |
| Compound 38 | >120 |
| Compound 39 | >120 |
| Compound 42 | >120 |
| Compound 44 | >120 |
| Compound 48 | >120 |
| Compound 53 | >120 |
| Compound 54 | >120 |
| Compound 57 | >120 |
| Compound 69 | >120 |
| Compound 72 | >120 |
| Compound 73 | >120 |
| Compound 75 | >120 |
| Compound 76 | >120 |
| Compound 77 | >120 |
| Compound 79 | >120 |
| Compound 80 | >120 |
| Compound 81 | >120 |
| Compound 82 | >120 |
| Compound 83 | >120 |
| Compound 85 | >120 |
| Compound 92-1 | >120 |
| Compound 92-2 | >120 |
| Compound 93 | >120 |

The results show that the compounds of the present application have good stability in human liver microsomes.

### 3. Pharmacokinetic assay

### 3.1 Experimental materials

ICR mice were purchased from Beijing Vital River Laboratory Animal Technology Co., Ltd.

### 3.2 Experimental procedure

(1) Healthy male ICR mice (18-22 g) were prepared, with 18 mice for each compound. The mice were divided into 2 groups (an i.v. group and a p.o. group), with 9 mice per group. 3 mice were picked for blood collection at each time point.
(2) After the animals were fasted overnight (with free access to water), the compound of the present invention dissolved (or suspended) in a solvent of 5% DMSO and 95% 30% HP-β-CD (v:v) was administered via the tail vein (i.v., 1 mg/kg) or intragastrically (p.o., 10 mg/kg), respectively.
(3) For the i.v. group, 0.1 mL blood was collected from the submandibular vein 5 min, 15 min, 0.5 h, 1 h, 2 h, 4 h, 8 h, 12 h, and 24 h after administration, respectively, anticoagulated with EDTA-K2, and centrifuged at 4°C for 5 min to obtain plasma, which was stored at -20°C until testing.
(4) For the p.o. group, 0.1 mL blood was collected from the submandibular vein before administration and 15 min, 0.5 h, 1 h, 2 h, 4 h, 6 h, 8 h, 12 h, and 24 h after administration, and treated in the same manner as that for the intravenous group.
(5) The concentration of the compound of the present invention in plasma was measured by LC/MS/MS.
(6) Calculation and fitting were performed with Analyst 1.6 from AB Company, and the results are shown in Table 3.

**Table 3. Pharmacokinetic results of the compounds of the present disclosure**

| Compounds | Administration Route | AUC (h^{∗}ng/mL) | Cmax (ng/mL) | T_{1/2} (h) | F% |
|---|---|---|---|---|---|
| Compound 2 | iv | 265 | - | 0.87 | 38.2 |
| | po | 1044 | 449 | 1.84 | |
| Compound 5 | iv | 234 | - | 0.333 | 99.4 |
| | po | 2266 | 835 | 3.21 | |
| Compound 6 | iv | 82.4 | - | 0.299 | 179 |
| | po | 1482 | 1220 | 0.964 | |
| Compound 9 | iv | 323 | - | 0.41 | 51.4 |
| | po | 1659 | 1487 | 1.34 | |
| Compound 18 | iv | 171 | - | 0.771 | 78.4 |
| | po | 1362 | 312 | 3.06 | |
| Compound 24 | iv | 466 | - | 0.95 | 135 |
| | po | 6276 | 2500 | 3.73 | |
| Compound 26 | iv | 639 | - | 1.52 | 110 |
| | po | 7104 | 2733 | 2.99 | |
| Compound 27 | iv | 679 | - | 1.48 | 213 |
| | po | 14582 | 5450 | 2.17 | |
| Compound 29 | iv | 609 | - | 0.46 | 42.1 |
| | po | 2562 | 1820 | 1.22 | |
| Compound 38 | iv | 269 | - | 0.341 | 47.7 |
| | po | 1308 | 2203 | 1.02 | |
| Compound 41 | iv | 357 | - | 0.55 | 42.3 |
| | po | 1505 | 2713 | 4.88 | |
| Compound 42 | iv | 230 | - | 1.41 | 70 |
| | po | 1499 | 858 | 1.73 | |
| Compound 54 | iv | 840 | - | 5.22 | 63.9 |
| | po | 5369 | 2057 | 3.21 | |
| Compound 62 | iv | 1191 | - | 1.69 | 44.8 |
| | po | 4717 | 1853 | 3.01 | |
| Compound 72 | iv | 1698 | - | 2.92 | 166 |
| | po | 28250 | 4680 | 2.58 | |
| Compound 73 | iv | 239 | - | 1.24 | 56 |
| | po | 1274 | 484 | 2.50 | |
| Compound 75 | iv | 109 | - | 0.31 | 69.2 |
| | po | 756 | 984 | 2.10 | |
| Compound 76 | iv | 120 | | 0.30 | 104 |
| | po | 1365 | 1571 | 0.93 | |
| Compound 77 | iv | 545 | - | 1.55 | 108 |
| | po | 5971 | 1747 | 3.24 | |
| Compound 78 | iv | 216 | - | 1.17 | 65.2 |
| | po | 1204 | 446 | 3.5 | |
| Compound 79 | iv | 482 | - | 0.63 | 100 |
| | po | 4443 | 2487 | 2.15 | |
| Compound 80 | iv | 6299 | - | 3.12 | 33.6 |
| | po | 21207 | 3570 | 2.16 | |

The results show that the compounds of the present disclosure have good pharmacokinetic properties.

### 4. TTK kinase activity assay

### 4.1 Experimental materials

TTK kinase was purchased from Invitrogen, Cat.#: PV3792.

### 4.2 Experimental procedure

(1) 1 ×buffer was prepared by diluting 5 ×buffer into 1 ×buffer with ddHzO, and adding 0.05 mM DTT.
(2) The compounds of the present invention were prepared at an initial concentration of 1000 nM, and diluted by 1:3 to obtain 10 serial dilutions.
(3) The diluted compounds of the present invention were added to a 384-well plate (purchased from Greiner, product number 784075) by an Echo 550 pipetting workstation, and centrifuged at 1000 g for 1 min.
(3) A 2×TTK enzyme solution was prepared with the 1 ×buffer. Then, 2.5 µL of the 2× enzyme solution was added to each well, centrifuged at 1000 g for 30s, and left at room temperature for 10 min.
(4) A 2×NMP substrate/ATP mixture was prepared with the 1×buffer. Then, 2.5 µL of the 2×MBP substrate/ATP mixture was added to each well, centrifuged at 1000 g for 30s, and left at room temperature for 1h.
(5) 5 µL ADP-Glo was added to each well and left at room temperature for 40 min.
(6) 10 µL of the detection reagent was added to each well and left at room temperature for 40 min.
(7) The results were read under an Envision 2104 microplate reader, and curve fitting and IC₅₀ calculation were performed with GraphPad Prism 8. The results are shown in Table 4.

**Table 4. TTK kinase inhibiting activity of compounds of the present disclosure**

| Compound No. | IC₅₀ (nM) |
|---|---|
| Compound 2 | 275.2 |
| Compound 5 | 1722 |
| Compound 9 | 10000 |
| Compound 18 | 386.6 |
| Compound 24 | 4219 |
| Compound 27 | 427.9 |

The results show that the compounds of the present disclosure have no significant inhibitory effect on TTK kinase, but have high selectivity for DNA-PK kinase.

Some specific embodiments have been described in detail in the specification of the present invention. Those skilled in the art should realize that these embodiments are exemplary and not to be construed as limiting the present invention. For those skilled in the art, various improvements and modifications may be made to the present invention without departing from the principles of the present invention, and the technical solutions obtained by such improvements and modifications also fall within the scope of the claims of the present invention.

## Claims

1. A compound of general formula (I), or a stereoisomer, solvate, prodrug, metabolite, pharmaceutically acceptable salt, or co-crystal thereof, 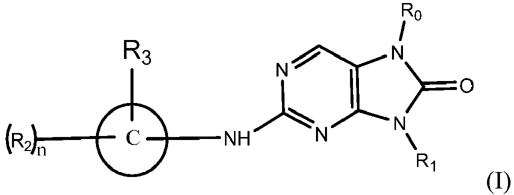 wherein 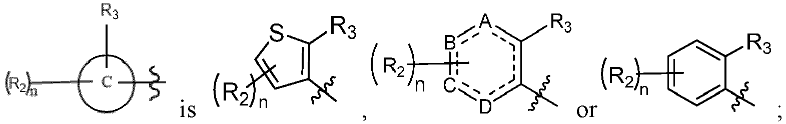
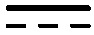 is a single bond or a double bond;
A, B, C, and D in the 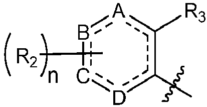 are each independently C or N, and at least one of A, B, C, and D is N;
R₀ is H, C₁₋₆ alkyl, or cyclopropyl, wherein the C₁₋₆ alkyl is optionally further substituted with one or more substituents selected from halogen and deuterium (D);
R₁ is 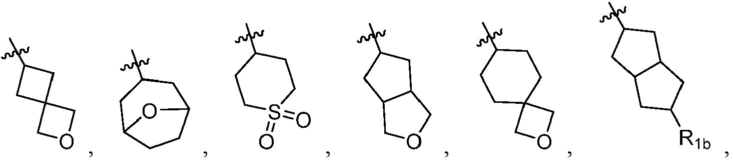 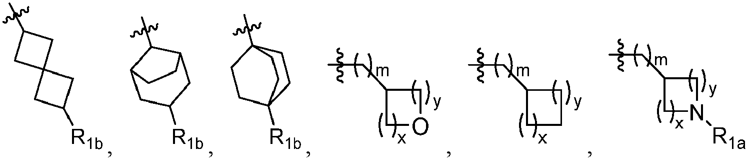 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
R₂ is H, cyano, =O, carboxyl, -C(=O)NR₂ₐR_{2b}, C₁₋₆ alkoxy, C₁₋₆ alkyl, halogen, - S(=O)₂R₂ₐ or a -C(=O)OC₁₋₆ alkyl; wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl or 3- to 5 -membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atom to which they are attached form a 5- to 6-membered heterocyclyl group containing 1, 2 or 3 heteroatoms selected from N, O and S, wherein the 5- to 6-membered heterocyclyl group is optionally further substituted with one or more substituents selected from C₁₋₆ alkyl, OH, and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2; and
x and y are each independently 1, 2, or 3;
provided that
R₁ is not 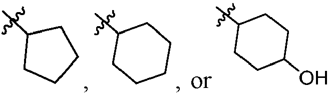 when 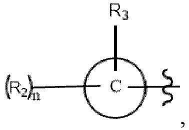 R₀, R₂, and R₃ all satisfy the following condition: 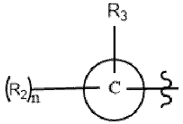 is 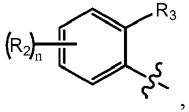 n is 1, R₀ is H or methyl, R₂ is methoxy or - S(=O)₂Me, and R₃ is methyl.

2. The compound according to claim 1, or a stereoisomer, solvate, metabolite, prodrug, pharmaceutically acceptable salt, or co-crystal thereof, wherein
R₁ is 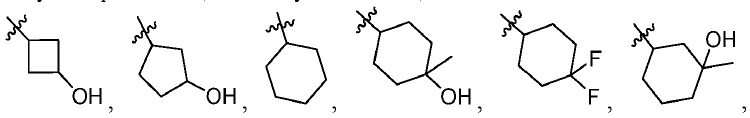 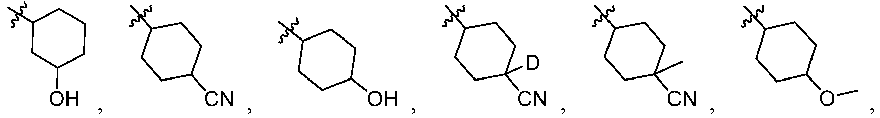 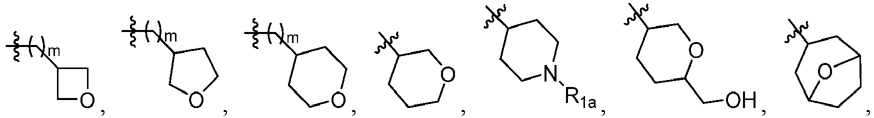 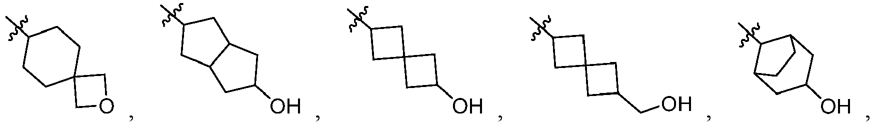 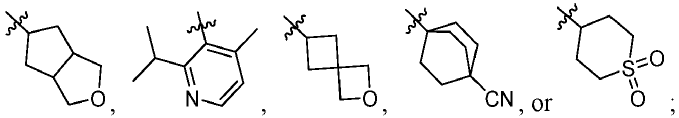
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl; wherein the -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, and halogen;
alternatively, R₂ₐ and R_{2b} together with the atom to which they are attached form a 5- to 6-membered heterocyclyl group containing 1 to 3 heteroatoms selected from N, O and S, wherein the heterocyclyl group is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1, or 2;
provided that
R₁ is not 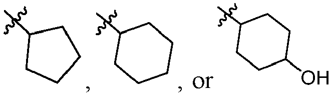 when 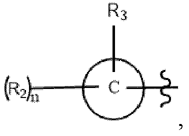 R₀, R₂, and R₃ all satisfy the following condition: 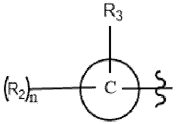 is 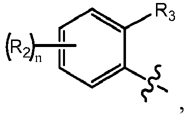 n is 1, R₀ is H or methyl, R₂ is methoxy or - S(=O)₂Me, and R₃ is methyl.

3. The compound according to claim 1, or a stereoisomer, solvate, metabolite, prodrug, pharmaceutically acceptable salt, or co-crystal thereof, wherein 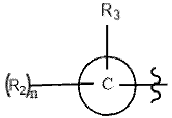 is 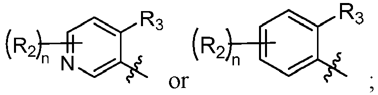
R₀ is H, C₁₋₄ alkyl, or cyclopropyl, wherein the C₁₋₄ alkyl is optionally further substituted with one or more substituents selected from halogen and D;
R₁is 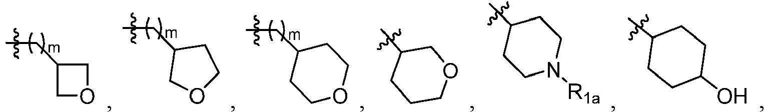 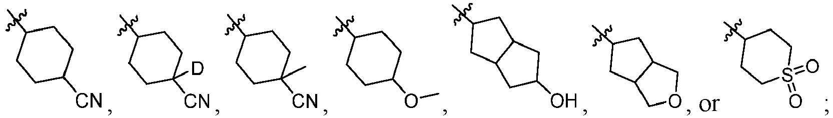
R₁ₐ is H, a C₁₋₆ alkyl or a -C(=O)C₁₋₆ alkyl;
R₂ is H, cyano, -C(=O)NR₂ₐR_{2b}, a C₁₋₆ alkoxy, halogen, -S(=O)₂R₂ₐ or a - C(=O)OC₁₋₆ alkyl; wherein the -C(=O)O alkyl or C₁₋₆ alkoxy is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, and halogen;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1, or 2;
provided that
R₁ is not 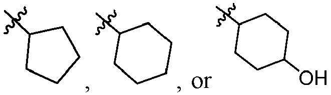 when 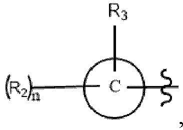 R₀, R₂, and R₃ all satisfy the following condition: 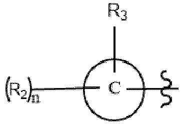 is 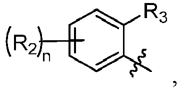 n is 1, R₀ is H or methyl, R₂ is methoxy or - S(=O)₂Me, and R₃ is methyl.

4. The compound according to claim 1, or a stereoisomer, solvate, prodrug, metabolite, pharmaceutically acceptable salt, or co-crystal thereof, wherein the compound is a compound of general formula (II): 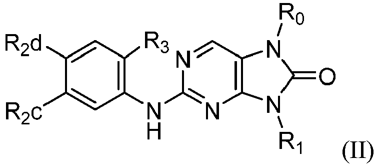 wherein
R₀ is H, C₁₋₆ alkyl, or cyclopropyl, wherein the C₁₋₆ alkyl is optionally further substituted with one or more substituents selected from halogen and deuterium;
R₁ is 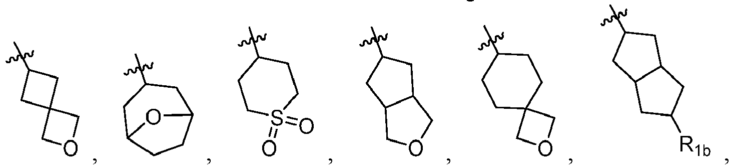 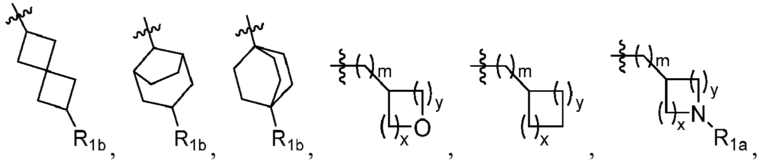 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
R_{2c} is H, cyano, halogen, or C₁₋₆ alkoxy;
R_{2d} is H, cyano, carboxyl, -C(=O)NR₂ₐR_{2b}, C₁₋₆ alkyl, halogen, -S(=O)₂R₂ₐ or - C(=O)OC₁₋₆ alkyl; wherein the C₁₋₆ alkyl or -C(=O)OC₁₋₆ alkyl is optionally substituted with one or more substituents selected from halogen and deuterium;
R₂ₐ and R_{2b} are each independently H, C₁₋₆ alkyl or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from C₁₋₆ alkyl, OH, and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2; and
x and y are each independently 1, 2, or 3;
provided that
R₁ is not 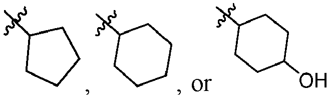 when 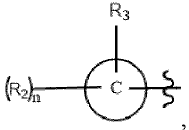 R₀, R₂, and R₃ all satisfy the following condition: 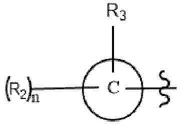 is selected from 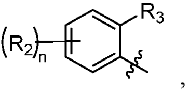 n is 1, R₀ is H or methyl, R₂ is methoxy or -S(=O)₂Me, and R₃ is methyl.

5. The compound according to claim 4, or a stereoisomer, solvate, prodrug, metabolite, pharmaceutically acceptable salt, or co-crystal thereof, wherein the compound is a compound of general formula (III): 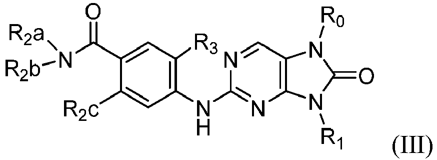 wherein
R₀ is H, C₁₋₆ alkyl, or cyclopropyl, wherein the C₁₋₆ alkyl is optionally further substituted with one or more substituents selected from halogen and deuterium;
R₁ is 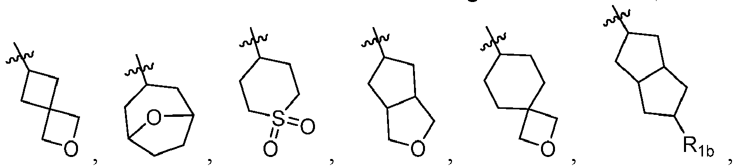 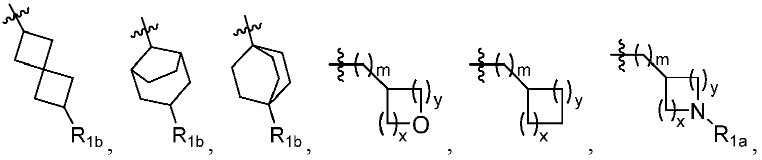 or pyridyl, and R₁ is optionally further substituted with one or two substituents selected from D, halogen, cyano, hydroxy, C₁₋₆ alkyl, and C₁₋₆ alkoxy;
R_{1b} is H, OH, cyano, or hydroxyl-substituted C₁₋₆ alkyl;
R₂ₐ and R_{2b} are each independently H, C₁₋₆ alkyl or 3- to 5-membered cycloalkyl, wherein the C₁₋₆ alkyl is optionally further substituted with one or more substituents selected from D and halogen;
alternatively, R₂ₐ and R_{2b} together with the atom to which they are attached form 5- to 6-membered heterocyclyl group containing 1 to 3 heteroatoms selected from N, O and S, wherein the 5- to 6-membered heterocyclyl group is optionally further substituted with one or more substituents selected from C₁₋₆ alkyl, OH, and halogen;
R_{2c} is H, cyano, halogen, or C₁₋₆ alkoxy, wherein the C₁₋₆ alkoxy is optionally substituted with one or more D;
R₃ is halogen or C₁₋₆ alkyl, wherein the C₁₋₆ alkyl is optionally further substituted with 1 to 3 substituents selected from D and halogen;
m is 0 or 1;
n is 0, 1, or 2; and
x and y are each independently 1, 2, or 3.

6. A compound of general formula (IV), or a stereoisomer, solvate, prodrug, metabolite, pharmaceutically acceptable salt, or co-crystal thereof, 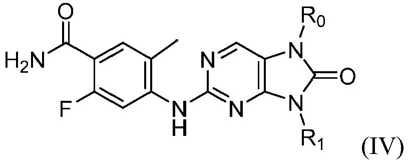 wherein
R₀ is H, C₁₋₆ alkyl, or cyclopropyl, wherein the C₁₋₆ alkyl is optionally further substituted with one or more substituents selected from halogen and deuterium; and
R₁ is -(CH)ₘ- 4 to 7-membered carbocyclyl, -(CH)ₘ- 4 to 7-membered heterocyclyl, -(CH)ₘ- 8 to 12-membered bridged ring, or -(CH)ₘ- 7 to 12 membered spiro, wherein the -(CH)ₘ- 4 to 7-membered carbocyclyl, -(CH)ₘ- 4 to 7-membered heterocyclyl, -(CH)ₘ- 8 to 12-membered bridged ring, or -(CH)ₘ- 7 to 12 membered spiro is optionally further substituted with one or more substituents selected from hydroxy, cyano, halogen, =O, C₁₋₆ alkyl, C₁₋₆ alkoxy, and hydroxyl-substituted C₁₋₆ alkyl.

7. The compound according to claim 6, or a stereoisomer, solvate, prodrug, metabolite, pharmaceutically acceptable salt, or co-crystal thereof, wherein
R₀ is H, C₁₋₆ alkyl, or cyclopropyl, wherein the C₁₋₆ alkyl is optionally further substituted with one or more substituents selected from halogen and deuterium;
R₁ is 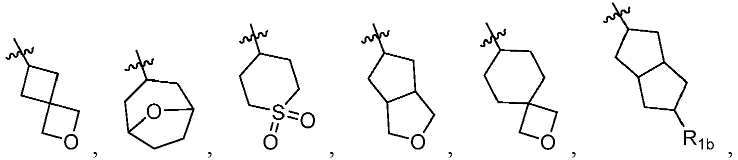 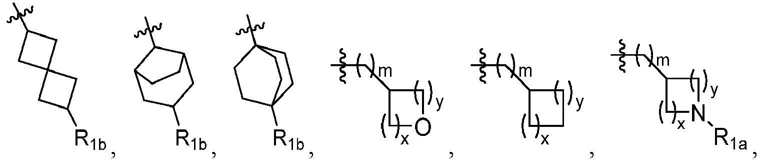 or pyridyl, and R₁ is optionally further substituted with one or two substituents selected from D, halogen, cyano, hydroxy, C₁₋₆ alkyl, and C₁₋₆ alkoxy;
R₁ₐ is H or C₁₋₆ alkyl;
R_{1b} is H, OH, cyano, or hydroxyl-substituted C₁₋₆ alkyl;
m is 0 or 1; and
x and y are each independently 1, 2, or 3.

8. The compound according to claim 7, or a stereoisomer, solvate, prodrug, metabolite, pharmaceutically acceptable salt, or co-crystal thereof, wherein
R₀ is C₁₋₄ alkyl, wherein the C₁₋₄ alkyl is optionally further substituted with one or more substituents selected from halogen and deuterium; and
R₁ is

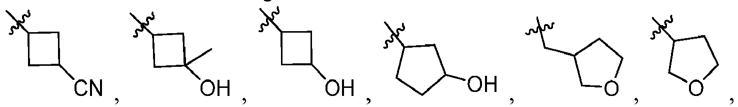

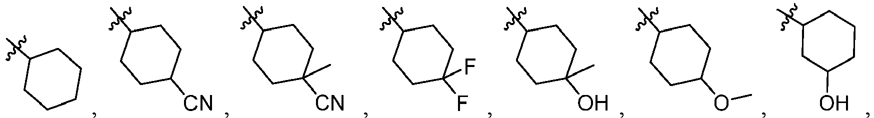

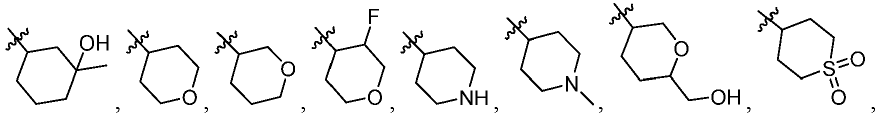

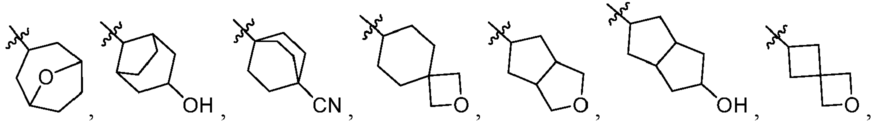

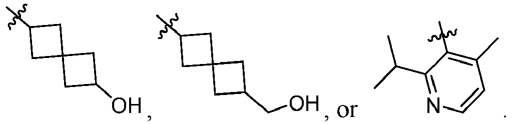

9. The compound according to any one of claims 1 to 8, or a stereoisomer, solvate, metabolite, prodrug, pharmaceutically acceptable salt, or co-crystal thereof, wherein the compound is:

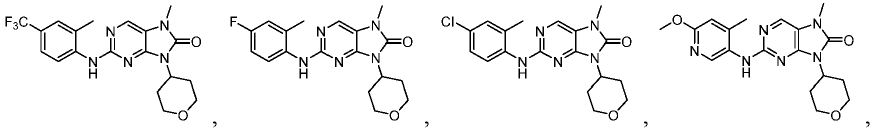

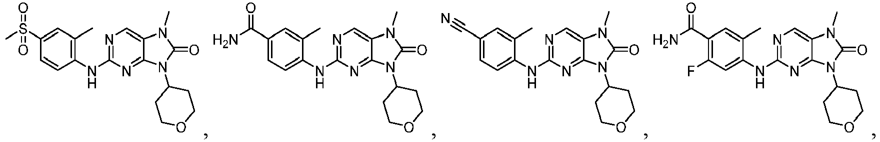

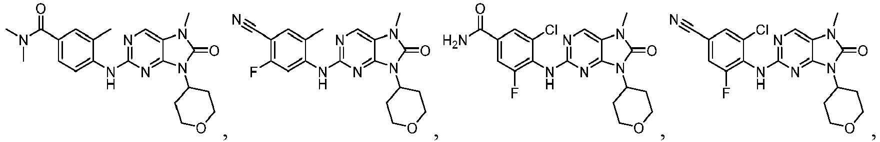

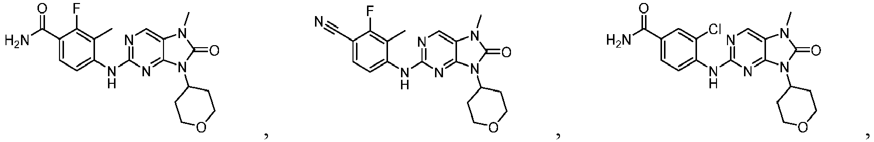

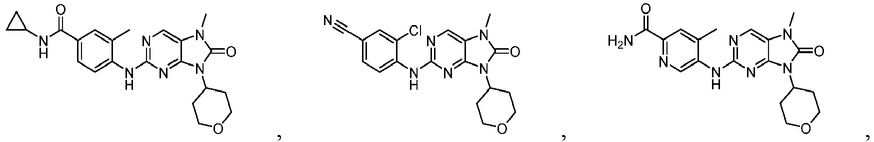

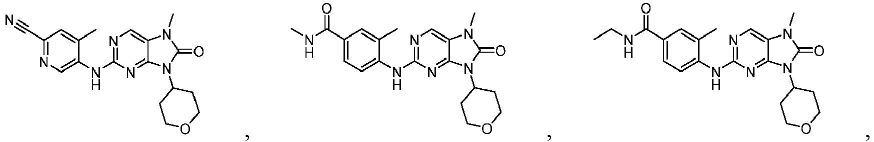

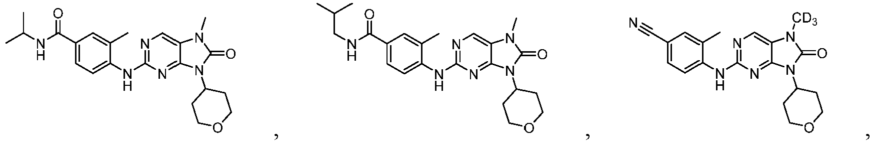

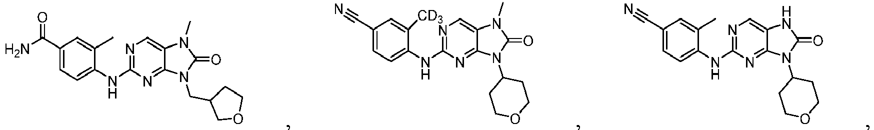

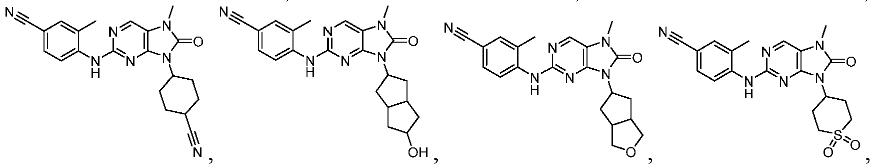

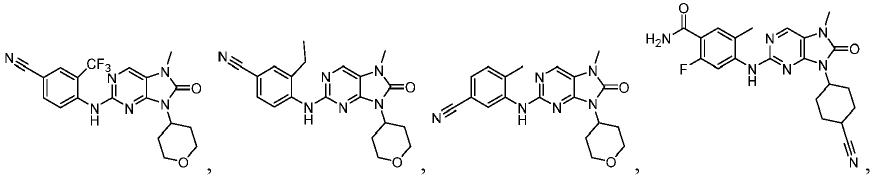

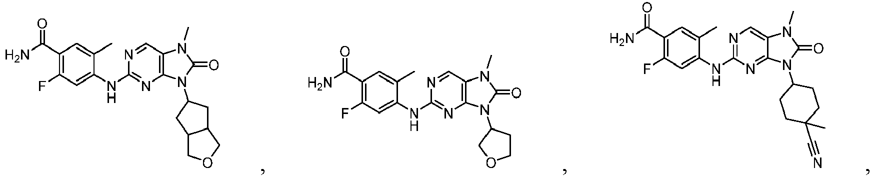

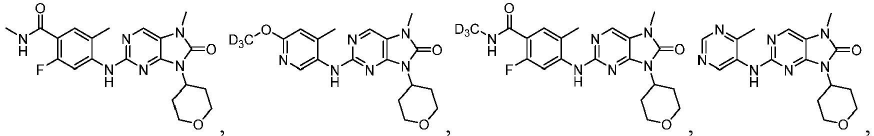

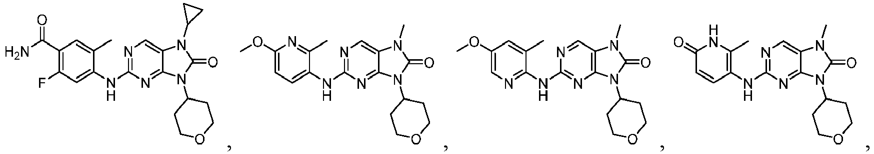

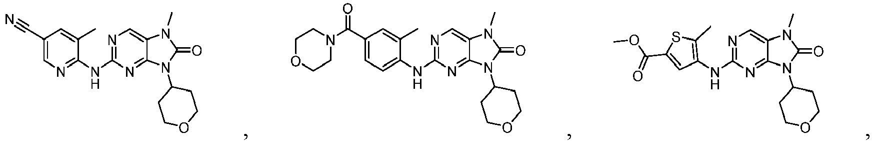

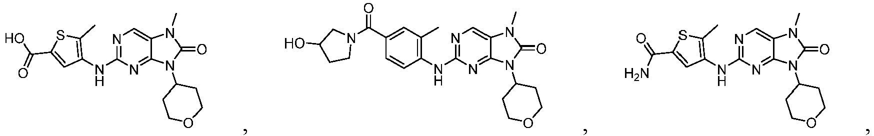

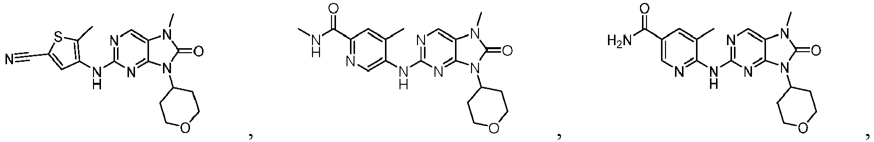

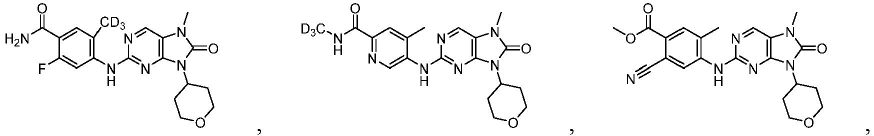

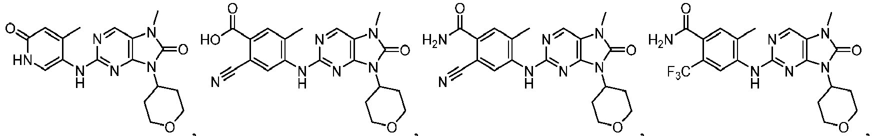

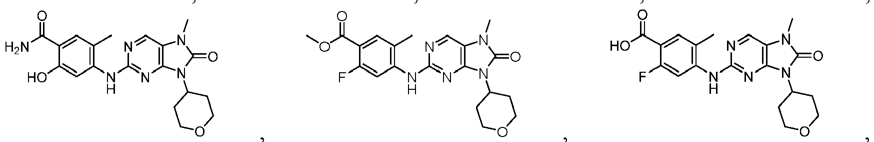

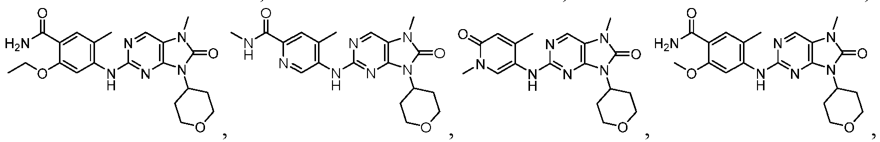

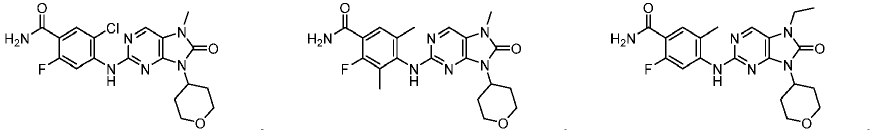

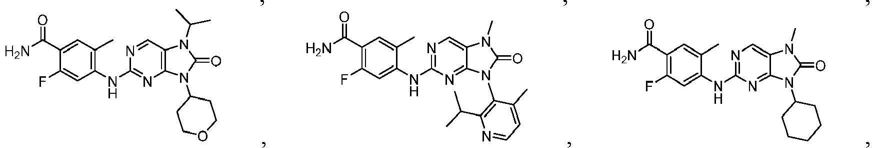

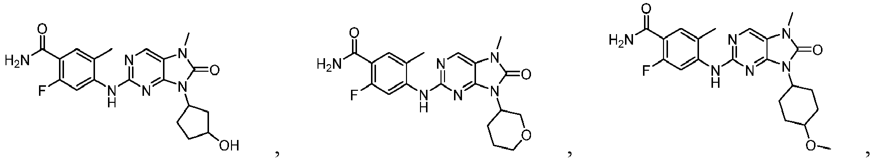

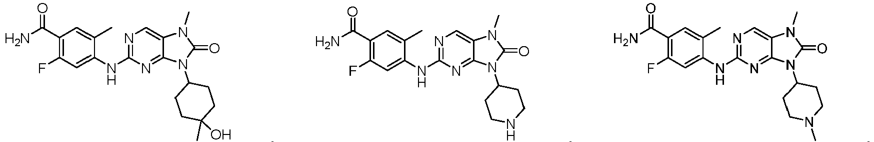

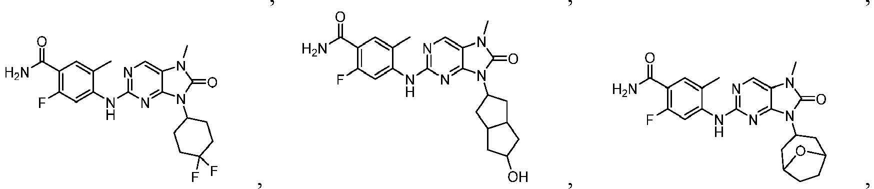

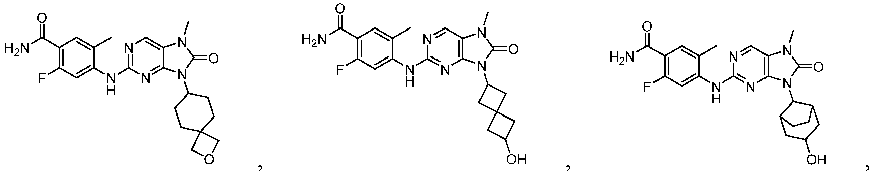

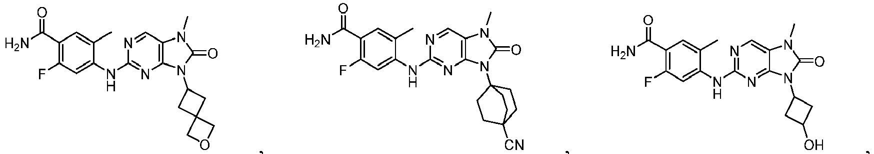

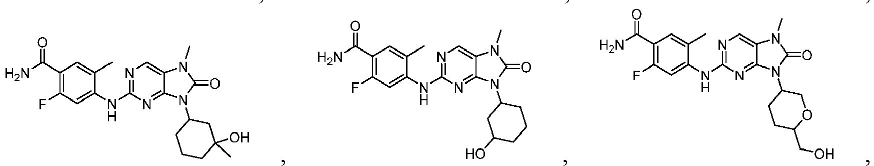

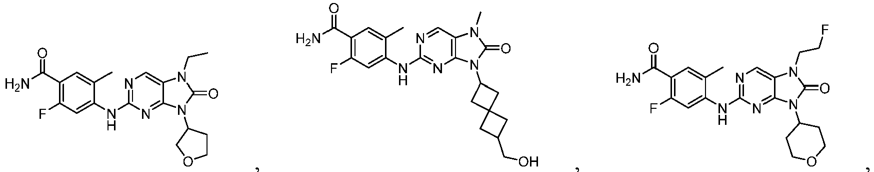

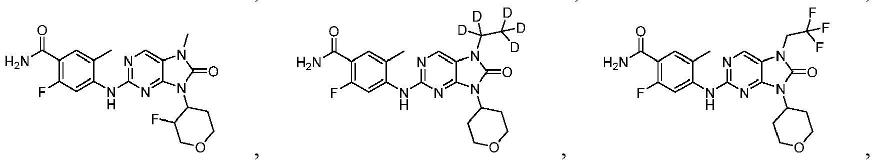

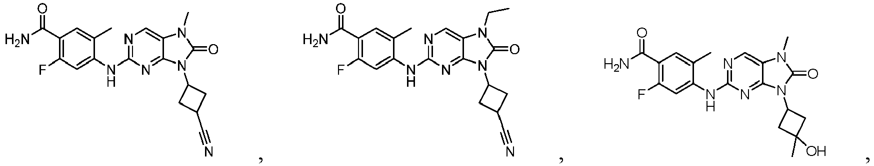

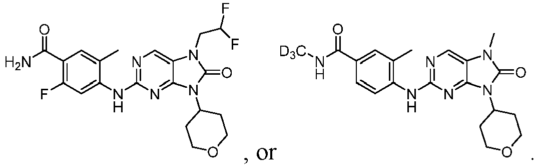

10. A pharmaceutical composition, comprising:
(1) a compound according to any one of claims 1 to 9, or a stereoisomer, solvate, metabolite, pharmaceutically acceptable salt, co-crystal, or prodrug thereof,
(2) optionally one or more additional active ingredients; and
(3) a pharmaceutically acceptable carrier and/or excipient.

11. Use of the compound according to any one of claims 1 to 9, or a stereoisomer, solvate, metabolite, pharmaceutically acceptable salt, co-crystal, or prodrug thereof, or the pharmaceutical composition according to claim 10, in the manufacture of a medicament for treatment of cancer; wherein the medicament for treatment of cancer is preferably a DNA-PK inhibitor.

12. A compound of general formula (I'), or a stereoisomer, solvate, prodrug, metabolite, pharmaceutically acceptable salt, or co-crystal thereof, 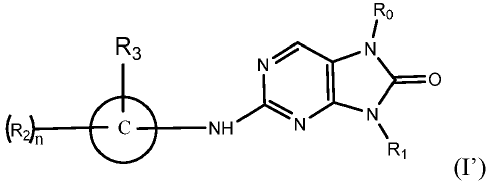 wherein
C is selected from phenyl and 6-membered monocyclic heterocyclic group, wherein the heterocyclic group may contain 1 to 3 heteroatoms selected from N;
R₀ is selected from H, C₁₋₆ alkyl, and cyclopropyl, wherein the alkyl is optionally further substituted with one or more substituents selected from halogen and deuterium;
R₁ is selected from 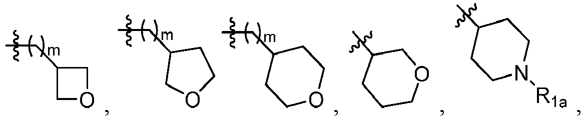 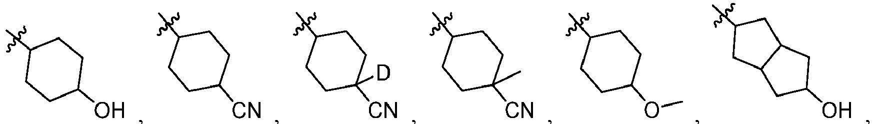 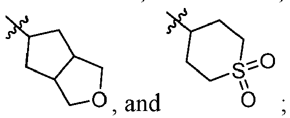
R₁ₐ is selected from H, C₁₋₆ alkyl, and -C(=O)C₁₋₆ alkyl;
R₂ is selected from cyano, carboxyl, -NR₂ₐR_{2b}, -C(=O)NR₂ₐR_{2b}, C₁₋₆ alkoxy, C₁₋₆ alkyl, halogen, -S(=O)₂R₂ₐ and -C(=O)OC₁₋₆ alkyl; wherein the alkyl or alkoxy is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH, D, halogen, C₁₋₆ alkyl and C₁₋₆ alkoxy;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from C₁₋₆ alkyl, OH, and halogen;
R₃ is selected from halogen and C₁₋₆ alkyl, wherein the alkyl is optionally further substituted with 1 to 3 substituents selected from D and halogen;
is selected from 0 and 1; and
n is selected from 0, 1, and 2;
provided that
R₁ is not 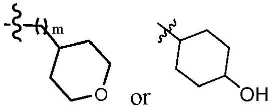 when C is selected from phenyl and 6-membered monocyclic heterocyclic group and R₀ is H.

13. The compound according to claim 12, or a stereoisomer, solvate, metabolite, prodrug, pharmaceutically acceptable salt, or co-crystal thereof, wherein 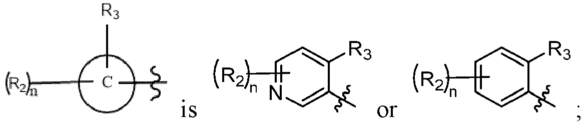
R₀ is selected from H, C₁₋₄ alkyl, and cyclopropyl, wherein the alkyl is optionally further substituted with one or more substituents selected from halogen and deuterium;
R₁ is selected from 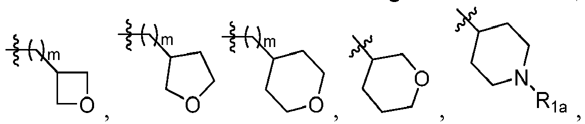 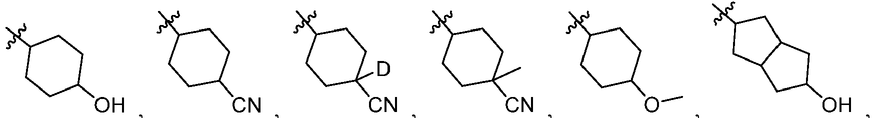 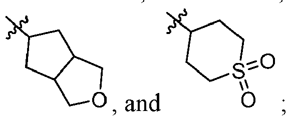
R₁ₐ is selected from H, C₁₋₆ alkyl, and -C(=O)C₁₋₆ alkyl;
R₂ is selected from cyano, -C(=O)NR₂ₐR_{2b}, C₁₋₆ alkoxy, halogen, -S(=O)₂R₂ₐ and -C(=O)OC₁₋₆ alkyl; wherein the alkyl or alkoxy is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl may be further substituted with one or more substituents selected from OH, D, and halogen;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from OH and halogen;
R₃ is selected from halogen or C₁₋₆ alkyl, wherein the alkyl is optionally further substituted with 1 to 3 substituents selected from D and halogen;
m is selected from 0 and 1; and
n is selected from 0, 1, and 2;
provided that
R₁ is not 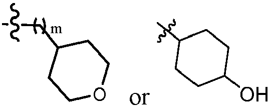 when C is selected from phenyl and 6-membered monocyclic heterocyclic group and R₀ is H.

14. A compound of general formula (I"), or a stereoisomer, solvate, prodrug, metabolite, pharmaceutically acceptable salt, or co-crystal thereof, 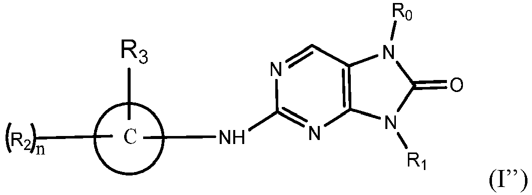 wherein
C is selected from phenyl and 6-membered monocyclic heterocyclic group, wherein the heterocyclic group may contain 1 to 3 heteroatoms selected from N;
R₀ is selected from H and C₁₋₆ alkyl, wherein the alkyl is optionally further substituted with one or more substituents selected from halogen and deuterium;
R₁ is selected from 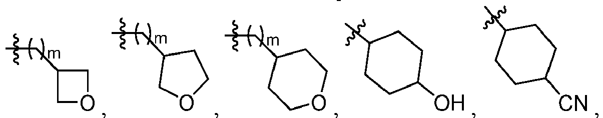 and 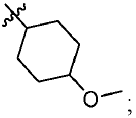
R₂ is selected from cyano, carboxyl, -NR₂ₐR_{2b}, -C(=O)NR₂ₐR_{2b}, C₁₋₆ alkoxy, C₁₋₆ alkyl, halogen, -S(=O)₂R₂ₐ and -C(=O)OC₁₋₆ alkyl; wherein the alkyl is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH, halogen, C₁₋₆ alkyl and C₁₋₆ alkoxy;
R₂ₐ and R_{2b} together with the atom to which they are attached may form 5- to 6-membered heterocyclyl group which may contain 1 to 3 heteroatoms selected from N, O and S, wherein the heterocyclyl group may be further substituted with one or more substituents selected from C₁₋₆ alkyl and halogen;
R₃ is selected from halogen and C₁₋₆ alkyl, wherein the alkyl is optionally further substituted with 1 to 3 substituents selected from D and halogen;
m is selected from 0 and 1; and
n is selected from 1 and 2.

15. The compound according to claim 14, or a stereoisomer, solvate, metabolite, prodrug, pharmaceutically acceptable salt, or co-crystal thereof, wherein 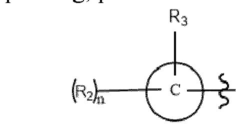 is selected from 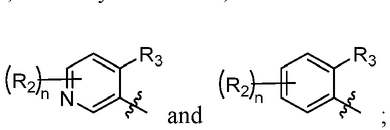
R₀ is selected from H and C₁₋₄ alkyl, wherein the alkyl is optionally further substituted with one or more substituents selected from halogen and deuterium;
R₁ is selected from R₁ is selected from 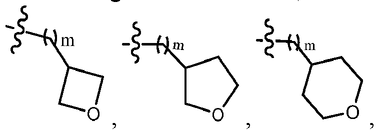 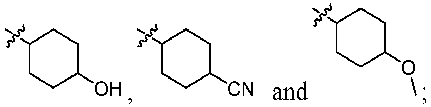
R₂ is selected from cyano, -C(=O)NR₂ₐR_{2b}, C₁₋₆ alkoxy, halogen, -S(=O)₂R₂ₐ and -C(=O)OC₁₋₆ alkyl; wherein the alkyl is optionally substituted with one or more substituents selected from halogen and deuterium;
R₂ₐ and R_{2b} are selected from H, C₁₋₆ alkyl and 3- to 5-membered cycloalkyl, wherein the C₁₋₆ alkyl is optionally further substituted with one or more substituents selected from OH and halogen;
R₂ₐ and R_{2b} together with the atom to which they are attached may form 6-membered heterocyclyl group;
R₃ is selected from halogen and C₁₋₆ alkyl, wherein the alkyl is optionally further substituted with 1 to 3 substituents selected from D and halogen;
m is selected from 0 and 1; and
n is selected from 1 and 2.

16. The compound according to claim 14 or 15, or a stereoisomer, solvate, metabolite, prodrug, pharmaceutically acceptable salt, or co-crystal thereof, wherein the compound is selected from

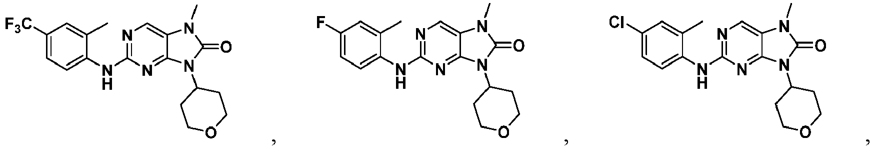

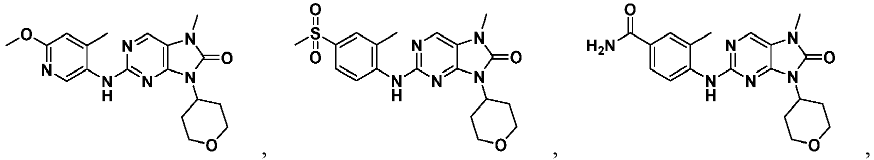

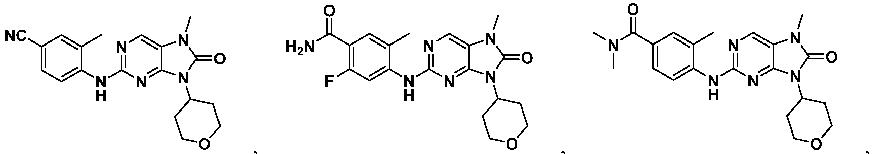

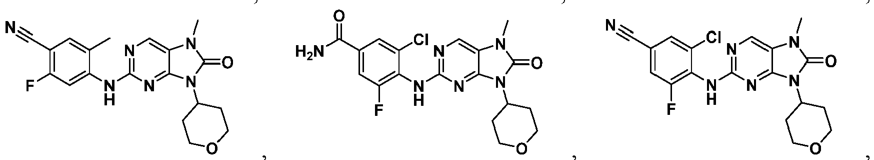

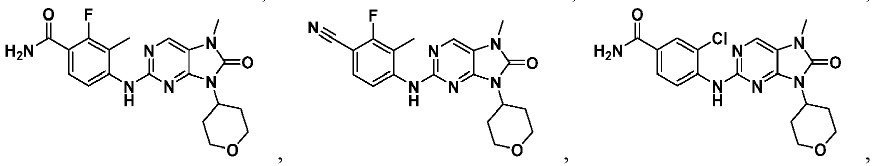

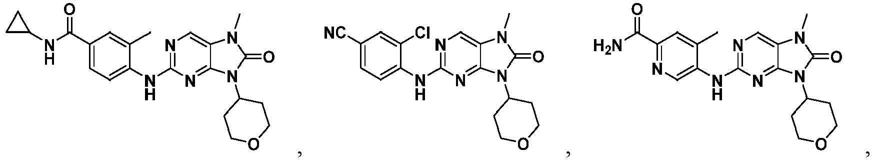

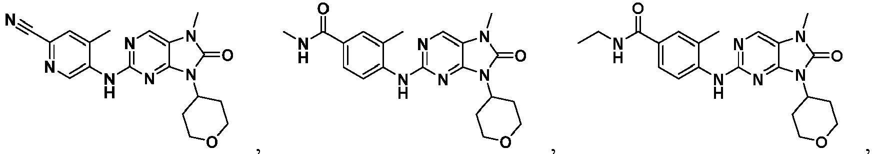

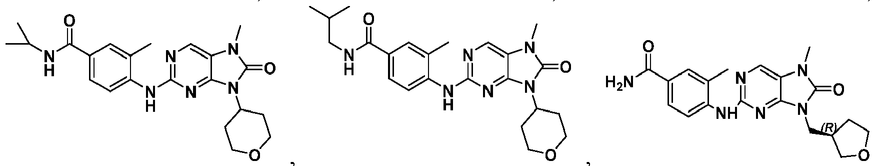

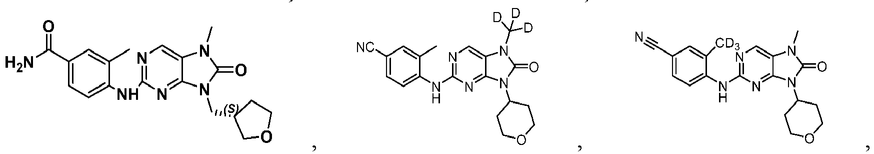

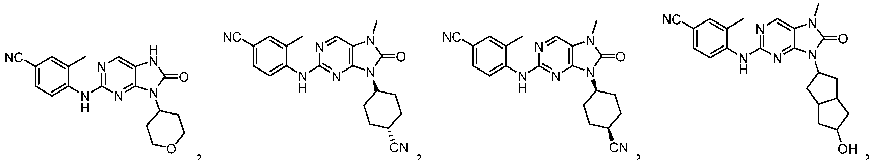

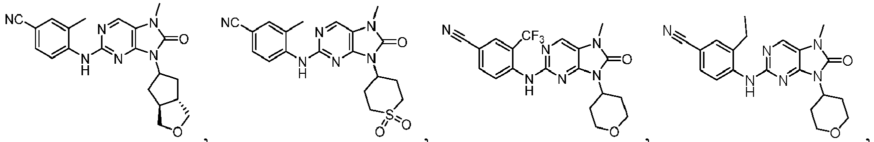

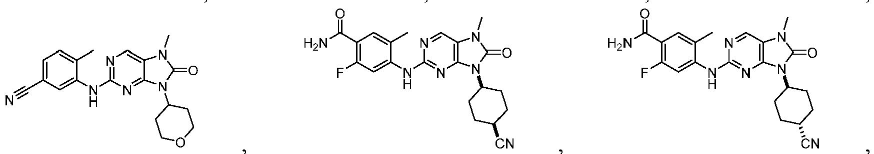

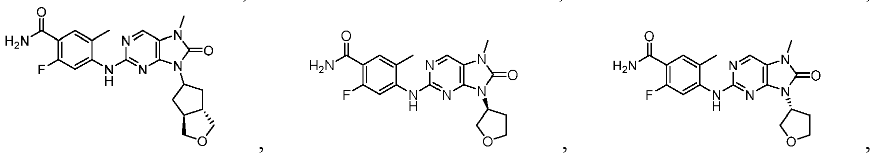

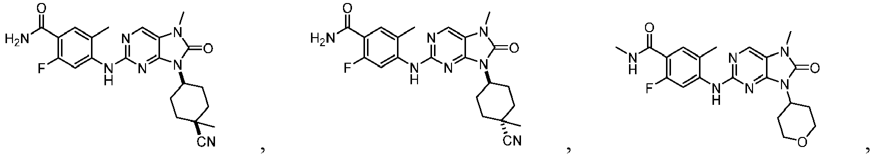

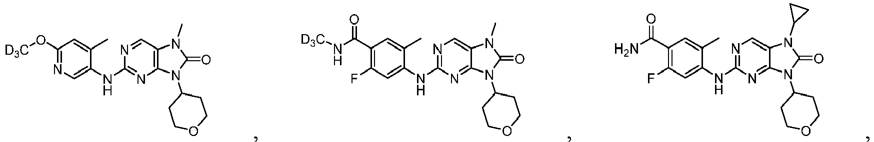

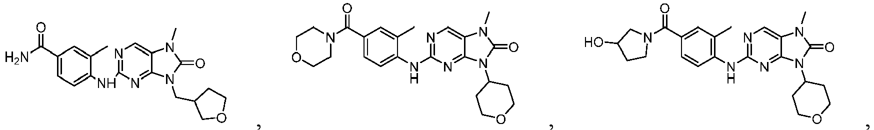

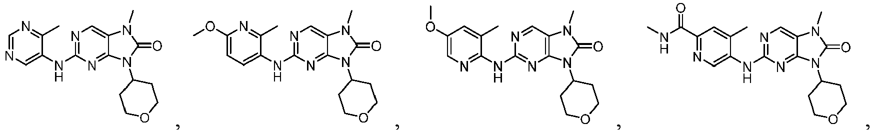

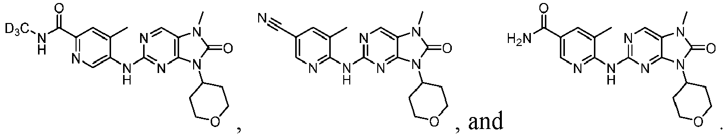

17. A pharmaceutical composition, comprising:
(1) a compound according to any one of claims 14 to 16, or a stereoisomer, solvate, metabolite, pharmaceutically acceptable salt, co-crystal, or prodrug thereof,
(2) optionally one or more additional active ingredients; and
(3) a pharmaceutically acceptable carrier and/or excipient.

18. Use of the compound according to any one of claims 14 to 16, or stereoisomer, solvate, metabolite, pharmaceutically acceptable salt, co-crystal, or prodrug thereof, or the pharmaceutical composition according to claim 17, in the manufacture of DNA-PK inhibitor.
